(19) Europäisches Patentamt / European Patent Office / Office européen des brevets

(11) EP 3 820 965 B1

(12) **EUROPÄISCHE PATENTSCHRIFT**

(45) Veröffentlichungstag und Bekanntmachung des Hinweises auf die Patenterteilung:
**25.01.2023 Patentblatt 2023/04**

(21) Anmeldenummer: **19736376.5**

(22) Anmeldetag: **05.07.2019**

(51) Internationale Patentklassifikation (IPC):
***C09K 11/06*** $^{(2006.01)}$ ***H01L 51/50*** $^{(2006.01)}$
***H01L 51/00*** $^{(2006.01)}$

(52) Gemeinsame Patentklassifikation (CPC):
**C09K 11/06; H01L 51/0072; H01L 51/0073; H01L 51/0074;** C09K 2211/1007; C09K 2211/1029; C09K 2211/1044; C09K 2211/185; H01L 51/0085; H01L 51/5016; H01L 2251/5384

(86) Internationale Anmeldenummer:
**PCT/EP2019/068066**

(87) Internationale Veröffentlichungsnummer:
**WO 2020/011658 (16.01.2020 Gazette 2020/03)**

# (54) MATERIALIEN FÜR ELEKTRONISCHE VORRICHTUNGEN

MATERIALS FOR ELECTRONIC DEVICES

MATÉRIAUX POUR DISPOSITIFS ÉLECTRONIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **09.07.2018 EP 18182505**

(43) Veröffentlichungstag der Anmeldung:
**19.05.2021 Patentblatt 2021/20**

(73) Patentinhaber: **Merck Patent GmbH**
**64293 Darmstadt (DE)**

(72) Erfinder:
- **PARHAM, Amir**
  **60486 FRANKFURT AM MAIN (DE)**
- **KROEBER, Jonas**
  **60311 FRANKFURT AM MAIN (DE)**
- **ENGELHART, Jens**
  **64285 DARMSTADT (DE)**
- **JATSCH, Anja**
  **60489 FRANKFURT AM MAIN (DE)**
- **EICKHOFF, Christian**
  **68259 MANNHEIM (DE)**
- **EHRENREICH, Christian**
  **64285 DARMSTADT (DE)**

(56) Entgegenhaltungen:
**EP-A1- 2 983 227**
**WO-A1-2006/128800**
**CN-A- 106 893 581**
**US-A1- 2013 009 543**
**US-A1- 2014 077 191**
**US-A1- 2015 060 824**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Processed by Luminess, 75001 PARIS (FR)

## Beschreibung

Die vorliegende Anmeldung betrifft heteroaromatische, polycyclisch kondensierte Verbindungen gemäß den untenstehend definierten Formeln (I-A), (I-B), (I-C), (I-D) und (II-A). Diese Verbindungen eignen sich zur Verwendung in elektronischen Vorrichtungen.

Unter elektronischen Vorrichtungen im Sinne dieser Anmeldung werden sogenannte organische elektronische Vorrichtungen verstanden (organic electronic devices), welche organische Halbleitermaterialien als Funktionsmaterialien enthalten. Insbesondere werden darunter OLEDs (organische Elektrolumineszenzvorrichtungen) verstanden. Unter der Bezeichnung OLEDs werden elektronische Vorrichtungen verstanden, welche eine oder mehrere Schichten enthaltend organische Verbindungen aufweisen und unter Anlegen von elektrischer Spannung Licht emittieren. Der Aufbau und das allgemeine Funktionsprinzip von OLEDs sind dem Fachmann bekannt.

Bei elektronischen Vorrichtungen, insbesondere OLEDs, besteht großes Interesse an einer Verbesserung der Leistungsdaten, insbesondere Lebensdauer, Effizienz und Betriebsspannung. In diesen Punkten konnte noch keine vollständig zufriedenstellende Lösung gefunden werden.

Weiterhin werden Materialien mit einer hohen Oxidationsstabilität, insbesondere in Lösung, einer hohen Temperaturstabilität, so dass sie im Hochvakuum unzersetzt verdampft werden können, und einer hohen Glasübergangstemperatur $T_G$ gesucht.

Einen großen Einfluss auf die Leistungsdaten von elektronischen Vorrichtungen haben phosphoreszierende Emissionsschichten. Diese enthalten üblicherweise mindestens zwei verschiedene Matrixmaterialien und mindestens einen phosphoreszierenden Emitter. Zur Verwendung in diesen Schichten werden weiterhin neue Matrixmaterialien gesucht, insbesondere solche, die einen hohen Energieabstand zwischen HOMO und LUMO aufweisen (wide bandgap-Materialien). Nochmals insbesondere werden neue Matrixmaterialien gesucht, welche in Kombination mit einem weiteren Matrixmaterial in emittierenden Schichten eingesetzt werden können, welche einen Triplett-Emitter enthalten. Das weitere Matrixmaterial ist dabei bevorzugt gewählt aus lochleitenden Verbindungen, aus elektronenleitenden Verbindungen und aus Verbindungen, welche sowohl lochleitende als auch elektronenleitende Eigenschaften aufweisen. Letztere Verbindungen werden als bipolare Matrixmaterialien bezeichnet.

Im Stand der Technik sind eine Vielzahl an heteroaromatischen Verbindungen zur Verwendung in OLEDs bekannt.

So werden in der US 2014/077191 A1, in der WO 2006/128800 A1, in der US 2015/060824 A1 und in der EP 2 983 227 A1 werden Verbindungen offenbart, die das folgende Strukturelement enthalten,

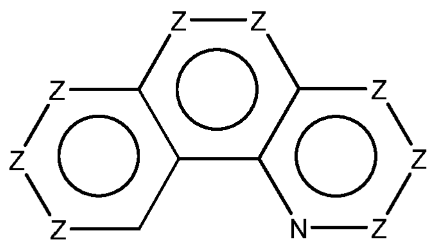

bei dem jedoch mindestens eine Gruppe Zein N-Atom ist.

In der US 2013/009543 A1 werden Verbindungen offenbart, die das obige Strukturelement mit einem ankondensierten aromatischen Ring enthalten.

In der CN 106 893 581 A werden Verbindungen offenbart, bei dem, im Gegensatz zu dem obigen Strukturelement, das N-Atom im mittleren Ring angeordnet ist und die auch am mittleren Ring substituiert sind.

Es besteht jedoch weiterhin Bedarf an alternativen Verbindungen, die hierfür geeignet sind. Es besteht auch Verbesserungsbedarf bezüglich der Leistungsdaten bei Verwendung in elektronischen Vorrichtungen, insbesondere bezüglich Lebensdauer, Betriebsspannung und Effizienz, und in Hinblick auf die oben genannten Eigenschaften Oxidationsstabilität, Temperaturstabilität, und hohe Glasübergangstemperatur $T_G$.

Nun wurde gefunden, dass sich bestimmte Verbindungen der oben genannten Strukturklasse hervorragend zur Verwendung in elektronischen Vorrichtungen eignen, insbesondere zur Verwendung in OLEDs, nochmals insbesondere darin zur Verwendung als Matrixmaterialien für phosphoreszierende Emitter, nochmals insbesondere zur Verwendung als wide-bandgap Matrixmaterialien in Kombination mit mindestens einem weiteren Matrixmaterial und mindestens einem phosphoreszierenden Emitter. Die Verbindungen führen bevorzugt zu einer Verbesserung der oben genannten Materialeigenschaften sowie zu einer Verbesserung der oben genannten Eigenschaften der OLEDs.

Diese Verbindungen sind Gegenstand der vorliegenden Anmeldung. Sie entsprechen einer Formel (I-A), (I-B), (I-C), (I-D) oder (II-A)

| | |
|---|---|
| 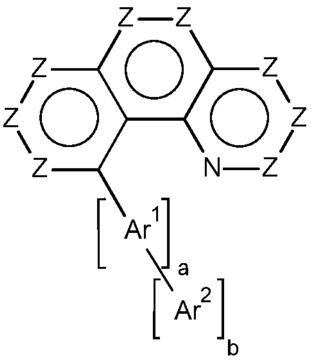 | 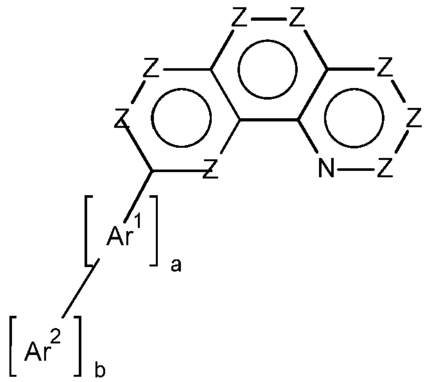 |
| Formel (I-A) | Formel (I-B) |
| 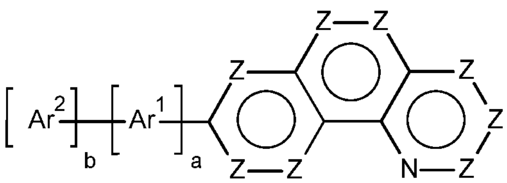 | 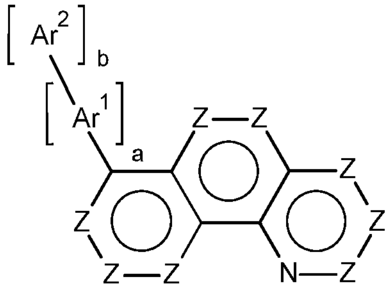 |
| Formel (I-C) | Formel (I-D) |
| 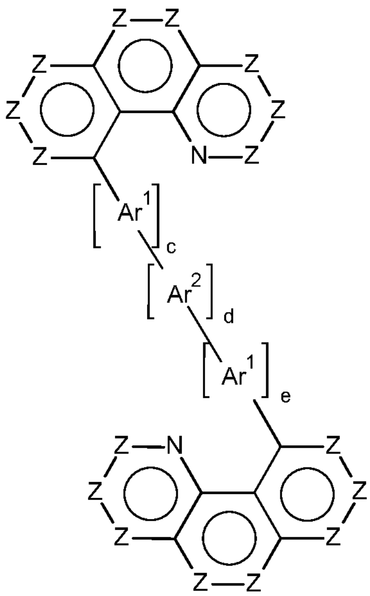 | |
| Formel (II-A) | |

wobei für die auftretenden Variablen gilt:

Z ist, wenn keine Einheit $Ar^1$ daran bindet, bei jedem Auftreten gleich oder verschieden gewählt aus $CR^1$; und Z ist, wenn eine Einheit $Ar^1$ daran bindet, gleich C;

$Ar^1$ ist bei jedem Auftreten gleich oder verschieden gewählt aus Einfachbindung, aromatischem Ringsystem mit 6 bis 40 aromatischen Ringatomen, das mit einem oder mehreren Resten $R^2$ substituiert sein kann, Dibenzothiophen, das mit einem oder mehreren Resten $R^2$ substituiert sein kann, und Dibenzofuran, das mit einem oder mehreren Resten $R^1$ substituiert sein kann;

$Ar^2$ ist bei jedem Auftreten gleich oder verschieden eine Gruppe gemäß Formel ($Ar^2$)

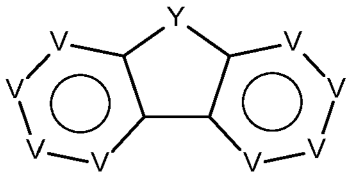

Formel ($Ar^2$),

Y ist bei jedem Auftreten gleich oder verschieden gewählt aus O, S, $C(R^3)_2$, $Si(R^3)_2$,

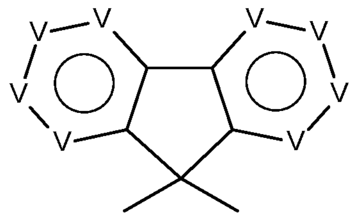

und

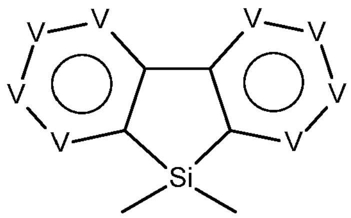

,

wobei in den Formeln

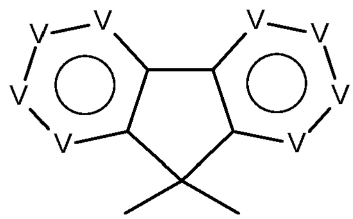

und

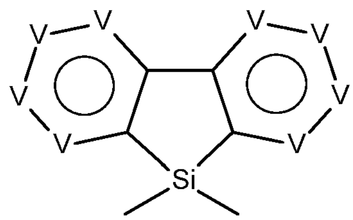

die freien Bindungen die Bindungen ausgehend von der Gruppe Y zum Rest der Gruppe der Formel ($Ar^2$) sind;
V ist bei jedem Auftreten gleich oder verschieden $CR^3$ oder N, sofern sich an der betreffenden Position nicht die Bindung an den Rest der Formel befindet, und V ist gleich C, sofern sich an der betreffenden Position die Bindung an den Rest der Formel befindet;
wobei eine oder mehrere Paare V-V jeweils durch eine Einheit gewählt aus den folgenden Einheiten

| 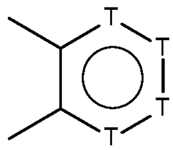 | 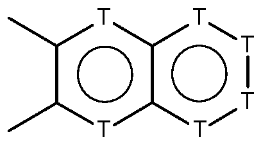 | 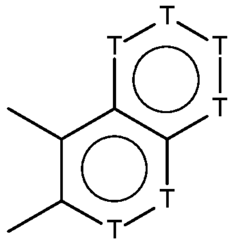 |
|---|---|---|
| (V-V-1) | (V-V-2) | (V-V-3) |

ersetzt sein können, wobei die freien Bindungen die Bindungen an den Rest der Formel kennzeichnen, und wobei T bei jedem Auftreten gleich oder verschieden $CR^3$ oder N ist, sofern sich an der betreffenden Position nicht die Bindung an den Rest der Formel befindet, und wobei T gleich C ist, sofern sich an der betreffenden Position die Bindung an den Rest der Formel befindet;

$R^1$, $R^2$ sind bei jedem Auftreten gleich oder verschieden gewählt aus H, D, F, $C(=O)R^4$, CN, $Si(R^4)_3$, $P(=O)(R^4)_2$, $OR^4$, $S(=O)R^4$, $S(=O)_2R^4$, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste $R^2$ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten $R^4$ substituiert sein können; und wobei eine oder mehrere $CH_2$-Gruppen in den genannten Alkyl-, Alkoxy-, Alkenyl- und Alkinylgruppen durch $-R^4C=CR^4-$, -C=C-, $Si(R^4)_2$, C=O, $C=NR^4$, -C(=O)O-, $-C(=O)NR^4-$, $P(=O)(R^4)$, -O-, -S-, SO oder $SO_2$ ersetzt sein können;

$R^3$ ist bei jedem Auftreten gleich oder verschieden gewählt aus H, D, F, $C(=O)R^4$, CN, $Si(R^4)_3$, $P(=O)(R^4)_2$, $OR^4$, $S(=O)R^4$, $S(=O)_2R^4$, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, heteroaromatischen N-freien Ringsystemen mit 5 bis 40 aromatischen Ringatomen; und elektronenarmen Heteroarylgruppen mit 6 bis 40 aromatischen Ringatomen, wobei zwei oder mehr Reste $R^3$ miteinander verknüpft sein können und einen Ring bilden können; wobei die genannten Alkyl-, Alkoxy, Alkenyl- und Alkinylgruppen und die genannten aromatischen Ringsysteme und heteroaromatischen N-freien Ringsysteme und elektronenarmen Heteroarylgruppen jeweils mit einem oder mehreren Resten $R^4$ substituiert sein können; und wobei eine oder mehrere $CH_2$-Gruppen in den genannten Alkyl-, Alkoxy-, Alkenyl- und Alkinylgruppen durch $-R^4C=CR^4-$, -C=C-, $Si(R^4)_2$, C=O, $C=NR^4$, -C(=O)O-, $-C(=O)NR^4-$, $P(=O)(R^4)$, -O-, -S-, SO oder $SO_2$ ersetzt sein können;

$R^4$ ist bei jedem Auftreten gleich oder verschieden gewählt aus H, D, F, $C(=O)R^5$, CN, $Si(R^5)_3$, $P(=O)(R^5)_2$, $OR^5$, $S(=O)R^5$, $S(=O)_2R^5$, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste $R^4$ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten $R^5$ substituiert sein können; und wobei eine oder mehrere $CH_2$-Gruppen in den genannten Alkyl-, Alkoxy-, Alkenyl- und Alkinylgruppen durch $-R^5C=CR^5-$, -C=C-, $Si(R^5)_2$, C=O, $C=NR^5$, -C(=O)O-, $-C(=O)NR^5-$, $P(=O)(R^5)$, -O-, -S-, SO oder $SO_2$ ersetzt sein können;

$R^5$ ist bei jedem Auftreten gleich oder verschieden gewählt aus H, D, F, CN, Alkyl- oder Alkoxygruppen mit 1 bis 20 C-Atomen, Alkenyl- oder Alkinylgruppen mit 2 bis 20 C-Atomen, aromatischen Ringsystemen mit 6 bis 40 aromatischen Ringatomen und heteroaromatischen Ringsystemen mit 5 bis 40 aromatischen Ringatomen; wobei die genannten Alkyl-, Alkoxy-, Alkenyl- und Alkinylgruppen, aromatischen Ringsysteme und heteroaromatischen Ringsysteme mit einem oder mehreren Resten gewählt aus F und CN substituiert sein können;

a, b, c, d, e sind gleich oder verschieden gewählt aus 1, 2, 3 und 4.

Die in die Sechsringe gezeichneten Kreise bedeuten, dass die betreffenden Sechsringe aromatisch bzw. heteroaromatisch sind.

Ist ein Index gewählt aus Indices a, b, c, d und e größer als 1, bedeutet dies, dass die betreffende Gruppe, die mit dem Index versehen ist, mehrfach hintereinander in einer Kette auftritt. Beispielsweise bedeutet - $[Ar^1]_a$ - für a=2, dass eine Einheit - $Ar^1$ - $Ar^1$ - vorliegt. Entsprechend bedeutet es für a=3, dass eine Einheit - $Ar^1$ - $Ar^1$ - $Ar^1$ - vorliegt.

Die Bindung, die durch die drei miteinander kondensierten Sechsringe des Grundkörpers hindurchgezogen ist, bedeutet, dass die Bindung an einer beliebigen Position des Grundkörpers vorliegen kann.

Die folgenden Definitionen gelten für die chemischen Gruppen, die in der vorliegenden Anmeldungen verwendet werden. Sie gelten, soweit keine spezielleren Definitionen angegeben sind.

Unter einer Arylgruppe im Sinne dieser Erfindung wird entweder ein einzelnder aromatischer Cyclus, also Benzol, oder ein kondensierter aromatischer Polycyclus, beispielsweise Naphthalin, Phenanthren oder Anthracen, verstanden. Ein kondensierter aromatischer Polycyclus besteht im Sinne der vorliegenden Anmeldung aus zwei oder mehr miteinander kondensierten einzelnen aromatischen Cyclen. Unter Kondensation zwischen Cyclen ist dabei zu verstehen, dass die Cyclen mindestens eine Kante miteinander teilen. Eine Arylgruppe im Sinne dieser Erfindung enthält 6 bis 40 aromatische Ringatome, von denen keines ein Heteroatom ist.

Unter einer Heteroarylgruppe im Sinne dieser Erfindung wird entweder ein einzelner heteroaromatischer Cyclus, beispielsweise Pyridin, Pyrimidin oder Thiophen, oder ein kondensierter heteroaromatischer Polycyclus, beispielsweise Chinolin oder Carbazol, verstanden. Ein kondensierter heteroaromatischer Polycyclus besteht im Sinne der vorliegenden Anmeldung aus zwei oder mehr miteinander kondensierten einzelnen aromatischen oder heteroaromatischen Cyclen, wobei wenigstens einer der aromatischen und heteroaromatischen Cyclen ein heteroaromatischer Cyclus ist. Unter Kondensation zwischen Cyclen ist dabei zu verstehen, dass die Cyclen mindestens eine Kante miteinander teilen. Eine Heteroarylgruppe im Sinne dieser Erfindung enthält 5 bis 40 aromatische Ringatome, von denen mindestens eines ein Heteroatom darstellt. Die Heteroatome der Heteroarylgruppe sind bevorzugt ausgewählt aus N, O und S.

Der Begriff "elektronenarme Heteroarylgruppe" wird verstanden wie für den Fachmann auf dem Gebiet der organischen Chemie üblich. Unter diesem Begriff wird insbesondere eine Heteroarylgruppe verstanden, die mindestens eine Einheit gewählt aus den folgenden Einheiten aufweist: i) einen heteroaromatischen Sechsring enthaltend mindestens ein N-Atom; ii) einen heteroaromatischen Fünfring enthaltend mindestens zwei N-Atome.

Unter einer Aryl- oder Heteroarylgruppe, die jeweils mit den oben genannten Resten substituiert sein kann werden insbesondere Gruppen verstanden, welche abgeleitet sind von Benzol, Naphthalin, Anthracen, Phenanthren, Pyren, Dihydropyren, Chrysen, Perylen, Triphenylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Benzimidazolo[1,2-a]benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Ein aromatisches Ringsystem im Sinne dieser Erfindung ist ein System, welches nicht notwendigerweise nur Arylgruppen enthält, sondern welches zusätzlich einen oder mehrere nicht-aromatische Ringe enthalten kann, die mit wenigstens einer Arylgruppe kondensiert sind. Diese nicht-aromatischen Ringe enthalten ausschließlich Kohlenstoffatome als Ringatome. Beispiele für Gruppen, die von dieser Definition umfasst sind, sind Tetrahydronaphthalin, Fluoren und Spirobifluoren. Weiterhin umfasst der Begriff aromatisches Ringsystem Systeme, die aus zwei oder mehr aromatischen Ringsystemen bestehen, die über Einfachbindungen miteinander verbunden sind, beispielsweise Biphenyl, Terphenyl, 7-Phenyl-2-fluorenyl, Quaterphenyl und 3,5-Diphenyl-1-phenyl. Ein aromatisches Ringsystem im Sinne dieser Erfindung enthält 6 bis 40 C-Atome und keine Heteroatome im Ringsystem. Die Definition von "aromatisches Ringsystem" umfasst nicht Heteroarylgruppen.

Ein heteroaromatisches Ringsystem entspricht der oben genannten Definition eines aromatischen Ringsystems, mit dem Unterschied dass es mindestens ein Heteroatom als Ringatom enthalten muss. Wie es beim aromatischen Ringsystem der Fall ist, muss das heteroaromatische Ringsystem nicht ausschließlich Arylgruppen und Heteroarylgruppen enthalten, sondern es kann zusätzlich einen oder mehrere nicht-aromatische Ringe enthalten, die mit wenigstens einer Aryl- oder Heteroarylgruppe kondensiert sind. Die nicht-aromatischen Ringe können ausschließlich C-Atome als Ringatome enthalten, oder sie können zusätzlich ein oder mehrere Heteroatome enthalten, wobei die Heteroatome bevorzugt gewählt sind aus N, O und S. Ein Beispiel für ein derartiges heteroaromatisches Ringsystem ist Benzopyranyl. Weiterhin werden unter dem Begriff "heteroaromatisches Ringsystem" Systeme verstanden, die aus zwei oder mehr aromatischen oder heteroaromatischen Ringsystemen bestehen, die miteinander über Einfachbindungen verbunden sind, wie beispielsweise 4,6-Diphenyl-2-triazinyl. Ein heteroaromatisches Ringsystem im Sinne dieser Erfindung enthält 5 bis 40 Ringatome, die gewählt sind aus Kohlenstoff und Heteroatomen, wobei mindestens eines der Ringatome ein Heteroatom ist. Die Heteroatome des heteroaromatischen Ringsystems sind bevorzugt ausgewählt aus N, O und S.

Die Begriffe "heteroaromatisches Ringsystem" und "aromatisches Ringsystem" gemäß der Definition der vorliegenden Anmeldung unterscheiden sich damit dadurch voneinander, dass ein aromatisches Ringsystem kein Heteroatom als Ringatom aufweisen kann, während ein heteroaromatisches Ringsystem mindestens ein Heteroatom als Ringatom aufweisen muss. Dieses Heteroatom kann als Ringatom eines nicht-aromatischen heterocyclischen Rings oder als Ringatom eines aromatischen heterocyclischen Rings vorliegen.

Entsprechend der obenstehenden Definitionen ist jede Arylgruppe vom Begriff "aromatisches Ringsystem" umfasst, und jede Heteroarylgruppe ist vom Begriff "heteroatomatisches Ringsystem" umfasst.

Unter dem Begriff "heteroaromatisches N-freies Ringsystem" wird jedes heteroaromatische Ringsystem, wie oben definiert, verstanden, dass keine N-Atome als Bestandteile des Ringsystems aufweist. Verbrückende oder verbindende nichtaromatische Gruppen werden dabei ebenfalls als Bestandteile des Ringsystems verstanden. Insbesondere schließt der Begriff "heteroaromatisches N-freies Ringsystem" Carbazol, Dihydroacridin und deren Derivate aus.

Unter einem aromatischen Ringsystem mit 6 bis 40 aromatischen Ringatomen oder einem heteroaromatischen Ringsystem mit 5 bis 40 aromatischen Ringatomen, werden insbesondere Gruppen verstanden, die abgeleitet sind von den oben unter Arylgruppen und Heteroarylgruppen genannten Gruppen sowie von Biphenyl, Terphenyl, Quaterphenyl, Fluoren, Spirobifluoren, Dihydrophenanthren, Dihydropyren, Tetrahydropyren, Indenofluoren, Truxen, Isotruxen, Spirotruxen, Spiroisotruxen, Indenocarbazol, oder von Kombinationen dieser Gruppen.

Im Rahmen der vorliegenden Erfindung werden unter einer geradkettigen Alkylgruppe mit 1 bis 20 C-Atomen bzw. einer verzweigten oder cyclischen Alkylgruppe mit 3 bis 20 C-Atomen bzw. einer Alkenyl- oder Alkinylgruppe mit 2 bis 40 C-Atomen, in der auch einzelne H-Atome oder $CH_2$-Gruppen durch die oben bei der Definition der Reste genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neoPentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden.

Unter einer Alkoxy- oder Thioalkylgruppe mit 1 bis 20 C-Atomen, in der auch einzelne H-Atome oder $CH_2$-Gruppen durch die oben bei der Definition der Reste genannten Gruppen substituiert sein können, werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy, 2,2,2-Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Unter der Formulierung, dass zwei oder mehr Reste miteinander einen Ring bilden können, soll im Rahmen der vorliegenden Anmeldung unter anderem verstanden werden, dass die beiden Reste miteinander durch eine chemische Bindung verknüpft sind. Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet.

Bevorzugt enthält die Verbindung der oben genannten Formeln keine Anthracengruppe. Besonders bevorzugt enthält die Verbindung der oben genannten Formeln keine kondensierte Arylgruppe mit mehr als 10 aromatischen Ringatomen.

Bevorzugt ist $Ar^1$ bei jedem Auftreten gleich oder verschieden gewählt aus Einfachbindung, Dibenzofuran, Dibenzothiophen, Benzol, Naphthalin, Biphenyl, Terphenyl, Fluoren, und Spirobifluoren, die jeweils mit einem oder mehreren Resten $R^2$ substituiert sein können.

Besonders bevorzugt ist $Ar^1$ bei jedem Auftreten gleich oder verschieden gewählt aus Einfachbindung und den im Folgenden gezeigten Formeln:

| | | |
|---|---|---|
| 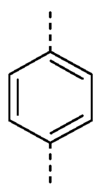 | 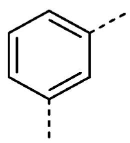 | 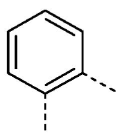 |
| $Ar^1$-1 | $Ar^1$-2 | $Ar^1$-3 |

(fortgesetzt)

| | | |
|---|---|---|
| 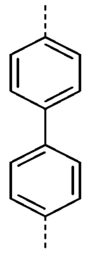 | 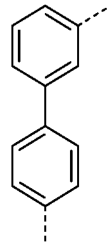 | 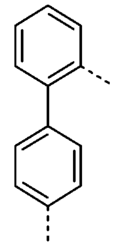 |
| $Ar^1$-4 | $Ar^1$-5 | $Ar^1$-6 |
| 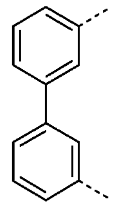 | 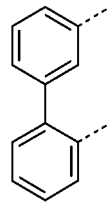 | 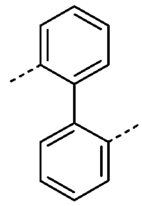 |
| $Ar^1$-7 | $Ar^1$-8 | $Ar^1$-9 |
| 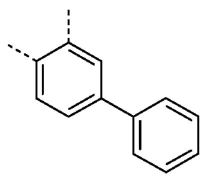 | 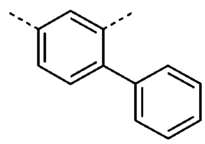 | 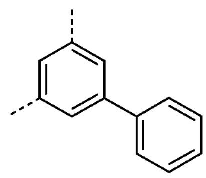 |
| $Ar^1$-10 | $Ar^1$-11 | $Ar^1$-12 |
| 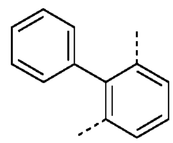 | 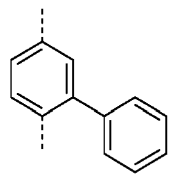 | 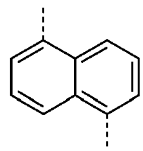 |
| $Ar^1$-13 | $Ar^1$-14 | $Ar^1$-15 |
| 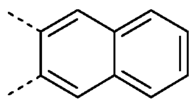 | 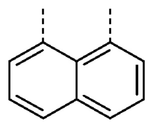 | 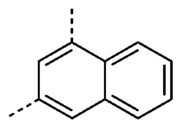 |
| $Ar^1$-16 | $Ar^1$-17 | $Ar^1$-18 |
| 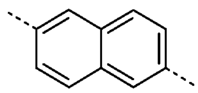 | 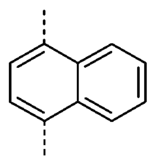 | 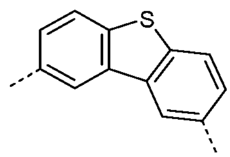 |
| $Ar^1$-19 | $Ar^1$-20 | $Ar^1$-21 |
| 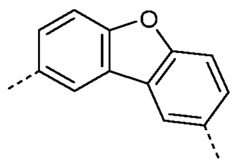 | 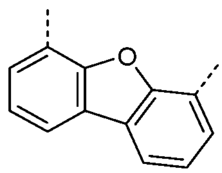 | 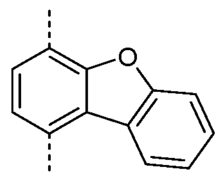 |
| $Ar^1$-22 | $Ar^1$-23 | $Ar^1$-24 |

(fortgesetzt)

| | | |
|---|---|---|
| 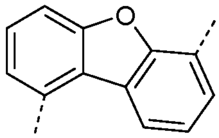 | 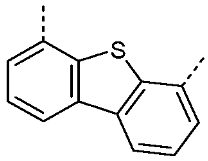 | 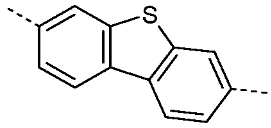 |
| Ar$^1$-25 | Ar$^1$-26 | Ar$^1$-27 |
| 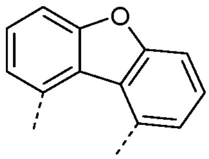 | 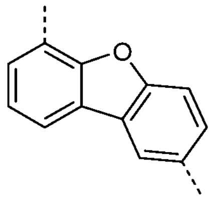 | 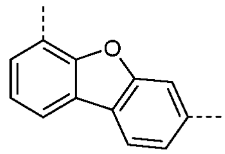 |
| Ar$^1$-28 | Ar$^1$-29 | Ar$^1$-30 |
| 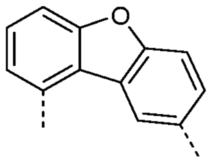 | 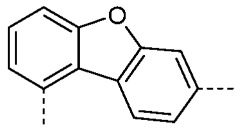 | 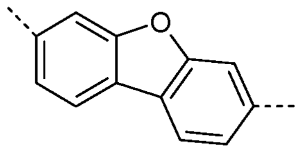 |
| Ar$^1$-31 | Ar$^1$-32 | Ar$^1$-33 |
| 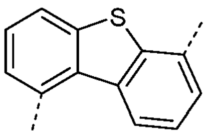 | 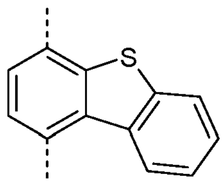 | 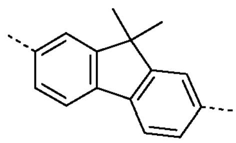 |
| Ar$^1$-34 | Ar$^1$-35 | Ar$^1$-36 |
| 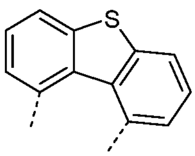 | 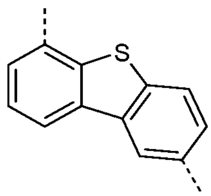 | 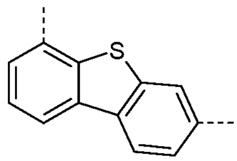 |
| Ar$^1$-37 | Ar$^1$-38 | Ar$^1$-39 |
| 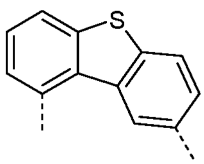 | 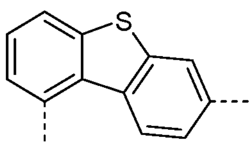 | 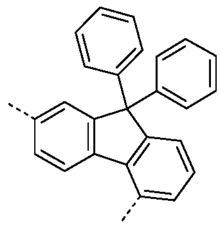 |
| Ar$^1$-40 | Ar$^1$-41 | Ar$^1$-42 |
| 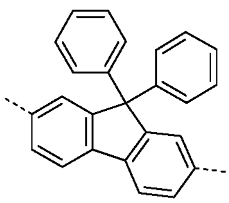 | 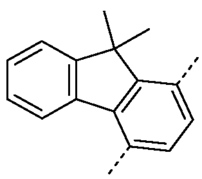 | 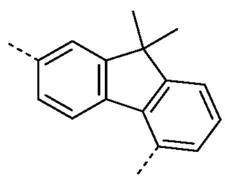 |

(fortgesetzt)

| Ar$^1$-43 | Ar$^1$-44 | Ar$^1$-45 |
|---|---|---|
| 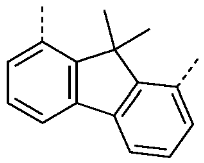 | 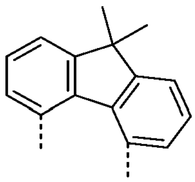 | 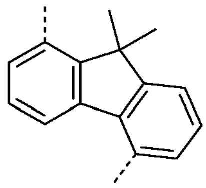 |
| Ar$^1$-46 | Ar$^1$-47 | Ar$^1$-48 |
| 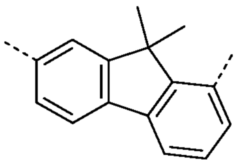 | 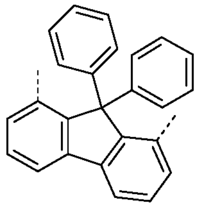 | 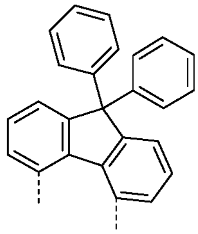 |
| Ar$^1$-49 | Ar$^1$-50 | Ar$^1$-51 |
| 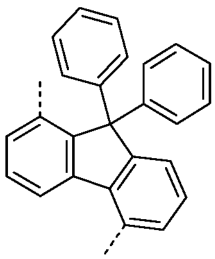 | 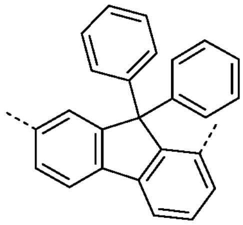 | 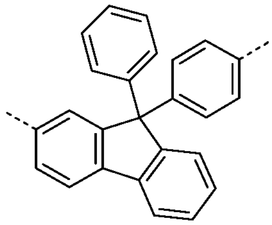 |
| Ar$^1$-52 | Ar$^1$-53 | Ar$^1$-54 |
| 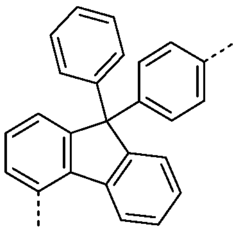 | 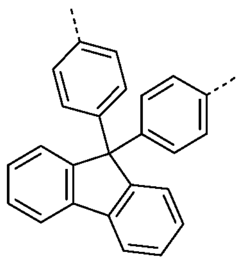 | 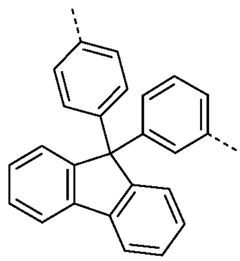 |
| Ar$^1$-55 | Ar$^1$-56 | Ar$^1$-57 |
| 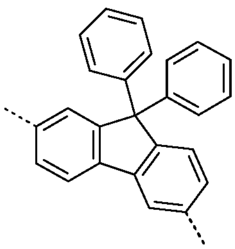 | 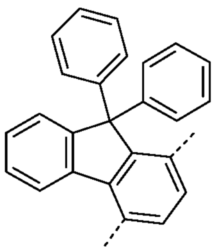 | 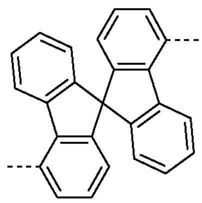 |
| Ar$^1$-58 | Ar$^1$-59 | Ar$^1$-60 |

(fortgesetzt)

| | | |
|---|---|---|
| 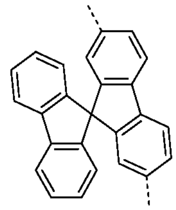 | 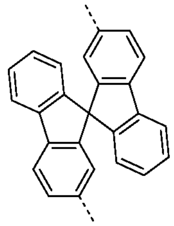 | 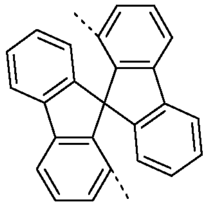 |
| $Ar^1$-61 | $Ar^1$-62 | $Ar^1$-63 |
| 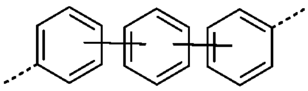 | | |
| $Ar^1$-64 | | |

wobei die Formeln jeweils mit einem oder mehreren Resten $R^2$ substituiert sein können.

Y ist bevorzugt bei jeden Auftreten gleich oder verschieden gewählt aus O und S.

Bevorzugt ist $Ar^2$ bei jedem Auftreten gleich oder verschieden gewählt aus den Formeln

| | |
|---|---|
| 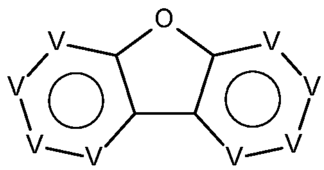 | 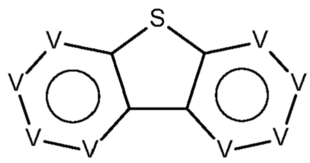 |
| Formel ($Ar^2$-1) | Formel ($Ar^2$-2) |
| 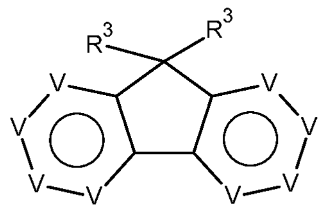 | 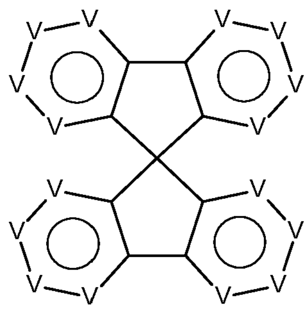 |
| Formel ($Ar^2$-3) | Formel ($Ar^2$-4) |

wobei V bei jedem Auftreten gleich oder verschieden $CR^3$ oder N ist, sofern sich an der betreffenden Position nicht die Bindung an den Rest der Formel befindet, und wobei V gleich C ist, sofern sich an der betreffenden Position die Bindung an den Rest der Formel befindet;
wobei eine oder mehrere Paare V-V jeweils durch eine Einheit gewählt aus den folgenden Einheiten

| | | |
|---|---|---|
| 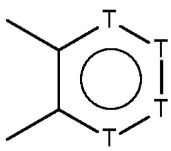 | 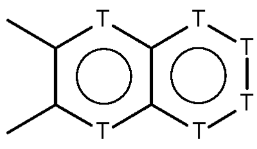 | 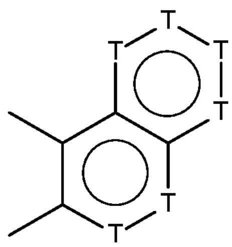 |

(fortgesetzt)

| (V-V-1) | (V-V-2) | (V-V-3) |
|---|---|---|

ersetzt sein können, wobei die freien Bindungen die Bindungen an den Rest der Formel kennzeichnen, und wobei T bei jedem Auftreten gleich oder verschieden $CR^3$ oder N ist, sofern sich an der betreffenden Position nicht die Bindung an den Rest der Formel befindet, und wobei T gleich C ist, sofern sich an der betreffenden Position die Bindung an den Rest der Formel befindet.

Bevorzugt sind höchstens 2 Gruppen V je oben genannte Formel gleich N, besonders bevorzugt ist höchstens eine Gruppe V gleich N. Ganz besonders bevorzugt ist V gleich $CR^3$, wenn sich an der betreffenden Position nicht die Bindung an den Rest der Formel befindet, und gleich C, wenn sich an der betreffenden Position die Bindung an den Rest der Formel befindet.

Bevorzugt sind höchstens 2 Gruppen T je oben genannte Formel gleich N, besonders bevorzugt ist höchstens eine Gruppe T gleich N. Ganz besonders bevorzugt ist T gleich $CR^3$, wenn sich an der betreffenden Position nicht die Bindung an den Rest der Formel befindet, und gleich C, wenn sich an der betreffenden Position die Bindung an den Rest der Formel befindet.

Besonders bevorzugt unter den oben genannten Formeln ist die Formel (V-V-1), ganz besonders bevorzugt mit allen Gruppen T gleich $CR^3$.

Bevorzugte Ausführungsformen der Formel ($Ar^2$-1) und ($Ar^2$-2) sind damit die folgenden Formeln

| | |
|---|---|
| 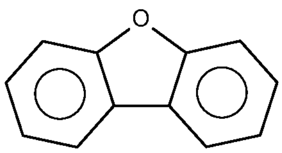 | 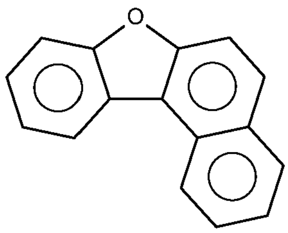 |
| Formel ($Ar^2$-1-A) | Formel ($Ar^2$-1-B) |
| 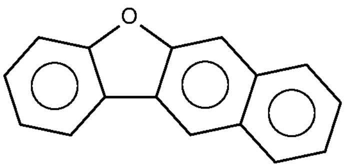 | 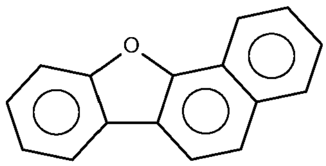 |
| Formel ($Ar^2$-1-C) | Formel ($Ar^2$-1-D) |
| 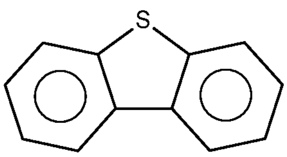 | 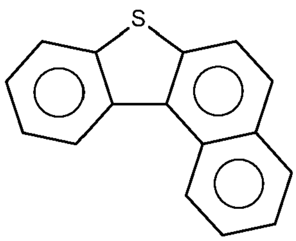 |
| Formel ($Ar^2$-2-A) | Formel ($Ar^2$-2-B) |
| 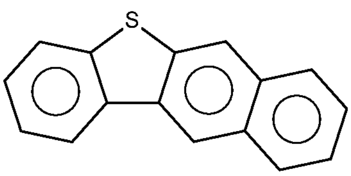 | 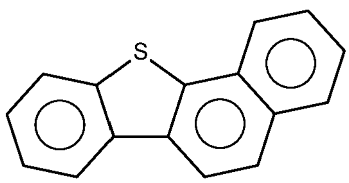 |
| Formel ($Ar^2$-2-C) | Formel ($Ar^2$-2-D) |

wobei die Gruppen jeder der freien Positionen jeweils mit einem Rest $R^3$ substituiert sein können, und wobei die Gruppen

an einer beliebigen freien Position an den Rest der Verbindung gebunden sein können.

Besonders bevorzugte Ausführungsformen der oben genannten Formeln sind die folgenden Formeln

| | |
|---|---|
| 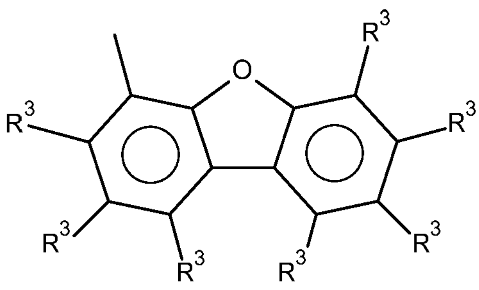 | 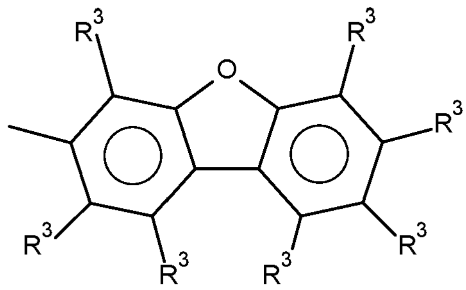 |
| Formel ($Ar^2$-1-A-1) | Formel ($Ar^2$-1-A-2) |
| 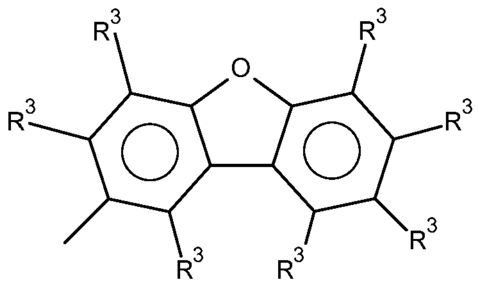 | 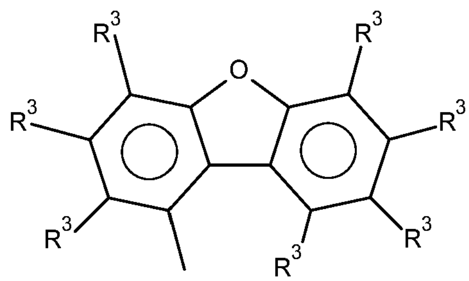 |
| Formel ($Ar^2$-1-A-3) | Formel ($Ar^2$-1-A-4) |
| 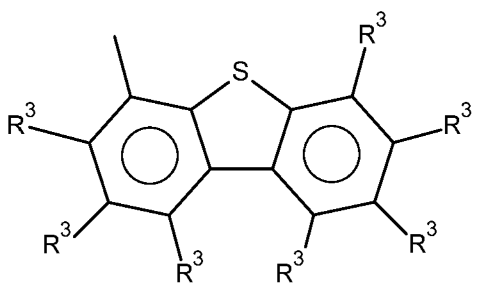 | 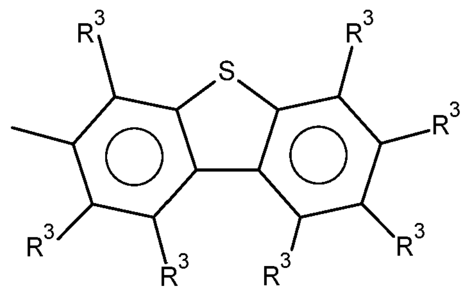 |
| Formel ($Ar^2$-2-A-1) | Formel ($Ar^2$-2-A-2) |
| 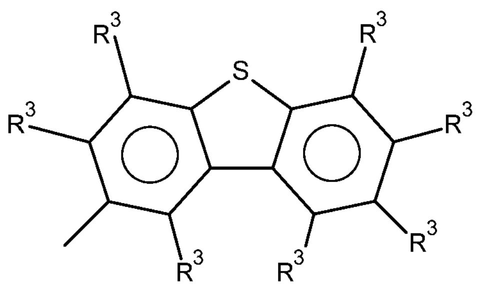 | 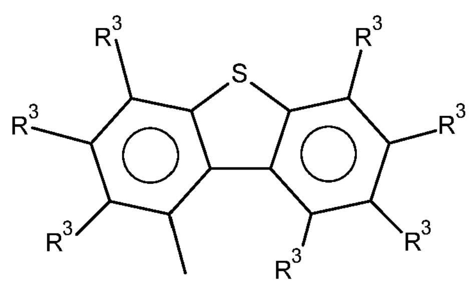 |
| Formel ($Ar^2$-2-A-3) | Formel ($Ar^2$-2-A-4) |

wobei die freie Bindung die Bindung an den Rest der Formel kennzeichnet.

Bevorzugte Einheiten $-[Ar^1]_a-[Ar^2]_b$ in den Formeln (I-A), (I-B), (I-C) und (I-D) sind gewählt aus Einheiten der folgenden Formeln $-Ar^1-Ar^2$, in denen $Ar^1$ und $Ar^2$ wie folgt gewählt sind:

| Formel | $Ar^1$ | $Ar^2$ |
|---|---|---|
| (Ar1-Ar2-1) | Einfachbindung | ($Ar^2$-1-A-1) |
| (Ar1-Ar2-2) | $Ar^1$-1 | ($Ar^2$-1-A-1) |

(fortgesetzt)

| Formel | $Ar^1$ | $Ar^2$ |
|---|---|---|
| (Ar1-Ar2-3) | $Ar^1$-2 | ($Ar^2$-1-A-1) |
| (Ar1-Ar2-4) | $Ar^1$-3 | ($Ar^2$-1-A-1) |
| (Ar1-Ar2-5) | $Ar^1$-4 | ($Ar^2$-1-A-1) |
| (Ar1 -Ar2-6) | $Ar^1$-5 | ($Ar^2$-1-A-1) |
| (Ar1-Ar2-7) | $Ar^1$-6 | ($Ar^2$-1-A-1) |
| (Ar1-Ar2-8) | $Ar^1$-7 | ($Ar^2$-1-A-1) |
| (Ar1-Ar2-9) | $Ar^1$-8 | ($Ar^2$-1-A-1) |
| (Ar1-Ar2-10) | $Ar^1$-9 | ($Ar^2$-1-A-1) |
| (Ar1-Ar2-11) | $Ar^1$-15 | ($Ar^2$-1-A-1) |
| (Ar1-Ar2-12) | $Ar^1$-16 | ($Ar^2$-1-A-1) |
| (Ar1-Ar2-13) | $Ar^1$-17 | ($Ar^2$-1-A-1) |
| (Ar1-Ar2-14) | $Ar^1$-18 | ($Ar^2$-1-A-1) |
| (Ar1-Ar2-15) | $Ar^1$-19 | ($Ar^2$-1-A-1) |
| (Ar1-Ar2-16) | $Ar^1$-20 | ($Ar^2$-1-A-1) |
| (Ar1-Ar2-17) | $Ar^1$-36 | ($Ar^2$-1-A-1) |
| (Ar1-Ar2-18) | $Ar^1$-42 | ($Ar^2$-1-A-1) |
| (Ar1-Ar2-19) | $Ar^1$-43 | ($Ar^2$-1-A-1) |
| (Ar1-Ar2-20) | $Ar^1$-44 | ($Ar^2$-1-A-1) |
| (Ar1-Ar2-21) | $Ar^1$-45 | ($Ar^2$-1-A-1) |
| (Ar1-Ar2-22) | $Ar^1$-46 | ($Ar^2$-1-A-1) |
| (Ar1-Ar2-23) | $Ar^1$-47 | ($Ar^2$-1-A-1) |
| (Ar1-Ar2-24) | $Ar^1$-48 | ($Ar^2$-1-A-1) |
| (Ar1-Ar2-25) | $Ar^1$-49 | ($Ar^2$-1-A-1) |
| (Ar1-Ar2-26) | $Ar^1$-50 | ($Ar^2$-1-A-1) |
| (Ar1-Ar2-27) | $Ar^1$-51 | ($Ar^2$-1-A-1) |
| (Ar1-Ar2-28) | $Ar^1$-52 | ($Ar^2$-1-A-1) |
| (Ar1-Ar2-29) | $Ar^1$-53 | ($Ar^2$-1-A-1) |
| (Ar1-Ar2-30) | $Ar^1$-58 | ($Ar^2$-1-A-1) |
| (Ar1-Ar2-31) | $Ar^1$-59 | ($Ar^2$-1-A-1) |
| (Ar1-Ar2-32) | $Ar^1$-60 | ($Ar^2$-1-A-1) |
| (Ar1-Ar2-33) | $Ar^1$-61 | ($Ar^2$-1-A-1) |
| (Ar1-Ar2-34) | $Ar^1$-62 | ($Ar^2$-1-A-1) |
| (Ar1-Ar2-35) | $Ar^1$-63 | ($Ar^2$-1-A-1) |
| (Ar1-Ar2-36) | $Ar^1$-64 | ($Ar^2$-1-A-1) |
| (Ar1-Ar2-37) | Einfachbindung | ($Ar^2$-1-A-2) |
| (Ar1-Ar2-38) | $Ar^1$-1 | ($Ar^2$-1-A-2) |
| (Ar1-Ar2-39) | $Ar^1$-2 | ($Ar^2$-1-A-2) |
| (Ar1-Ar2-40) | $Ar^1$-3 | ($Ar^2$-1-A-2) |

(fortgesetzt)

| Formel | $Ar^1$ | $Ar^2$ |
|---|---|---|
| (Ar1-Ar2-41) | $Ar^1$-4 | ($Ar^2$-1-A-2) |
| (Ar1-Ar2-42) | $Ar^1$-5 | ($Ar^2$-1-A-2) |
| (Ar1-Ar2-43) | $Ar^1$-6 | ($Ar^2$-1-A-2) |
| (Ar1-Ar2-44) | $Ar^1$-7 | ($Ar^2$-1-A-2) |
| (Ar1-Ar2-45) | $Ar^1$-8 | ($Ar^2$-1-A-2) |
| (Ar1-Ar2-46) | $Ar^1$-9 | ($Ar^2$-1-A-2) |
| (Ar1-Ar2-47) | $Ar^1$-15 | ($Ar^2$-1-A-2) |
| (Ar1-Ar2-48) | $Ar^1$-16 | ($Ar^2$-1-A-2) |
| (Ar1-Ar2-49) | $Ar^1$-17 | ($Ar^2$-1-A-2) |
| (Ar1-Ar2-50) | $Ar^1$-18 | ($Ar^2$-1-A-2) |
| (Ar1-Ar2-51) | $Ar^1$-19 | ($Ar^2$-1-A-2) |
| (Ar1-Ar2-52) | $Ar^1$-20 | ($Ar^2$-1-A-2) |
| (Ar1-Ar2-53) | $Ar^1$-36 | ($Ar^2$-1-A-2) |
| (Ar1-Ar2-54) | $Ar^1$-42 | ($Ar^2$-1-A-2) |
| (Ar1-Ar2-55) | $Ar^1$-43 | ($Ar^2$-1-A-2) |
| (Ar1-Ar2-56) | $Ar^1$-44 | ($Ar^2$-1-A-2) |
| (Ar1-Ar2-57) | $Ar^1$-45 | ($Ar^2$-1-A-2) |
| (Ar1-Ar2-58) | $Ar^1$-46 | ($Ar^2$-1-A-2) |
| (Ar1-Ar2-59) | $Ar^1$-47 | ($Ar^2$-1-A-2) |
| (Ar1-Ar2-60) | $Ar^1$-48 | ($Ar^2$-1-A-2) |
| (Ar1-Ar2-61) | $Ar^1$-49 | ($Ar^2$-1-A-2) |
| (Ar1-Ar2-62) | $Ar^1$-50 | ($Ar^2$-1-A-2) |
| (Ar1-Ar2-63) | $Ar^1$-51 | ($Ar^2$-1-A-2) |
| (Ar1-Ar2-64) | $Ar^1$-52 | ($Ar^2$-1-A-2) |
| (Ar1-Ar2-65) | $Ar^1$-53 | ($Ar^2$-1-A-2) |
| (Ar1-Ar2-66) | $Ar^1$-58 | ($Ar^2$-1-A-2) |
| (Ar1-Ar2-67) | $Ar^1$-59 | ($Ar^2$-1-A-2) |
| (Ar1-Ar2-68) | $Ar^1$-60 | ($Ar^2$-1-A-2) |
| (Ar1-Ar2-69) | $Ar^1$-61 | ($Ar^2$-1-A-2) |
| (Ar1-Ar2-70) | $Ar^1$-62 | ($Ar^2$-1-A-2) |
| (Ar1-Ar2-71) | $Ar^1$-63 | ($Ar^2$-1-A-2) |
| (Ar1-Ar2-72) | $Ar^1$-64 | ($Ar^2$-1-A-2) |
| (Ar1-Ar2-73) | Einfachbindung | ($Ar^2$-1-A-3) |
| (Ar1-Ar2-74) | $Ar^1$-1 | ($Ar^2$-1-A-3) |
| (Ar1-Ar2-75) | $Ar^1$-2 | ($Ar^2$-1-A-3) |
| (Ar1-Ar2-76) | $Ar^1$-3 | ($Ar^2$-1-A-3) |
| (Ar1-Ar2-77) | $Ar^1$-4 | ($Ar^2$-1-A-3) |
| (Ar1-Ar2-78) | $Ar^1$-5 | ($Ar^2$-1-A-3) |

(fortgesetzt)

| Formel | $Ar^1$ | $Ar^2$ |
|---|---|---|
| (Ar1-Ar2-79) | $Ar^1$-6 | ($Ar^2$-1-A-3) |
| (Ar1-Ar2-80) | $Ar^1$-7 | ($Ar^2$-1-A-3) |
| (Ar1-Ar2-81) | $Ar^1$-8 | ($Ar^2$-1-A-3) |
| (Ar1-Ar2-82) | $Ar^1$-9 | ($Ar^2$-1-A-3) |
| (Ar1-Ar2-83) | $Ar^1$-15 | ($Ar^2$-1-A-3) |
| (Ar1-Ar2-84) | $Ar^1$-16 | ($Ar^2$-1-A-3) |
| (Ar1-Ar2-85) | $Ar^1$-17 | ($Ar^2$-1-A-3) |
| (Ar1-Ar2-86) | $Ar^1$-18 | ($Ar^2$-1-A-3) |
| (Ar1-Ar2-87) | $Ar^1$-19 | ($Ar^2$-1-A-3) |
| (Ar1-Ar2-88) | $Ar^1$-20 | ($Ar^2$-1-A-3) |
| (Ar1-Ar2-89) | $Ar^1$-36 | ($Ar^2$-1-A-3) |
| (Ar1-Ar2-90) | $Ar^1$-42 | ($Ar^2$-1-A-3) |
| (Ar1-Ar2-91) | $Ar^1$-43 | ($Ar^2$-1-A-3) |
| (Ar1-Ar2-92) | $Ar^1$-44 | ($Ar^2$-1-A-3) |
| (Ar1-Ar2-93) | $Ar^1$-45 | ($Ar^2$-1-A-3) |
| (Ar1-Ar2-94) | $Ar^1$-46 | ($Ar^2$-1-A-3) |
| (Ar1-Ar2-95) | $Ar^1$-47 | ($Ar^2$-1-A-3) |
| (Ar1-Ar2-96) | $Ar^1$-48 | ($Ar^2$-1-A-3) |
| (Ar1-Ar2-97) | $Ar^1$-49 | ($Ar^2$-1-A-3) |
| (Ar1-Ar2-98) | $Ar^1$-50 | ($Ar^2$-1-A-3) |
| (Ar1-Ar2-99) | $Ar^1$-51 | ($Ar^2$-1-A-3) |
| (Ar1-Ar2-100) | $Ar^1$-52 | ($Ar^2$-1-A-3) |
| (Ar1-Ar2-101) | $Ar^1$-53 | ($Ar^2$-1-A-3) |
| (Ar1-Ar2-102) | $Ar^1$-58 | ($Ar^2$-1-A-3) |
| (Ar1-Ar2-103) | $Ar^1$-59 | ($Ar^2$-1-A-3) |
| (Ar1-Ar2-104) | $Ar^1$-60 | ($Ar^2$-1-A-3) |
| (Ar1-Ar2-105) | $Ar^1$-61 | ($Ar^2$-1-A-3) |
| (Ar1-Ar2-106) | $Ar^1$-62 | ($Ar^2$-1-A-3) |
| (Ar1-Ar2-107) | $Ar^1$-63 | ($Ar^2$-1-A-3) |
| (Ar1-Ar2-108) | $Ar^1$-64 | ($Ar^2$-1-A-3) |
| (Ar1-Ar2-109) | Einfachbindung | ($Ar^2$-1-A-4) |
| (Ar1-Ar2-110) | $Ar^1$-1 | ($Ar^2$-1-A-4) |
| (Ar1-Ar2-111) | $Ar^1$-2 | ($Ar^2$-1-A-4) |
| (Ar1-Ar2-112) | $Ar^1$-3 | ($Ar^2$-1-A-4) |
| (Ar1-Ar2-113) | $Ar^1$-4 | ($Ar^2$-1-A-4) |
| (Ar1-Ar2-114) | $Ar^1$-5 | ($Ar^2$-1-A-4) |
| (Ar1-Ar2-115) | $Ar^1$-6 | ($Ar^2$-1-A-4) |
| (Ar1-Ar2-116) | $Ar^1$-7 | ($Ar^2$-1-A-4) |

(fortgesetzt)

| Formel | $Ar^1$ | $Ar^2$ |
|---|---|---|
| (Ar1-Ar2-117) | $Ar^1$-8 | ($Ar^2$-1-A-4) |
| (Ar1-Ar2-118) | $Ar^1$-9 | ($Ar^2$-1-A-4) |
| (Ar1-Ar2-119) | $Ar^1$-15 | ($Ar^2$-1-A-4) |
| (Ar1-Ar2-120) | $Ar^1$-16 | ($Ar^2$-1-A-4) |
| (Ar1-Ar2-121) | $Ar^1$-17 | ($Ar^2$-1-A-4) |
| (Ar1-Ar2-122) | $Ar^1$-18 | ($Ar^2$-1-A-4) |
| (Ar1-Ar2-123) | $Ar^1$-19 | ($Ar^2$-1-A-4) |
| (Ar1-Ar2-124) | $Ar^1$-20 | ($Ar^2$-1-A-4) |
| (Ar1-Ar2-125) | $Ar^1$-36 | ($Ar^2$-1-A-4) |
| (Ar1-Ar2-126) | $Ar^1$-42 | ($Ar^2$-1-A-4) |
| (Ar1-Ar2-127) | $Ar^1$-43 | ($Ar^2$-1-A-4) |
| (Ar1-Ar2-128) | $Ar^1$-44 | ($Ar^2$-1-A-4) |
| (Ar1-Ar2-129) | $Ar^1$-45 | ($Ar^2$-1-A-4) |
| (Ar1-Ar2-130) | $Ar^1$-46 | ($Ar^2$-1-A-4) |
| (Ar1-Ar2-131) | $Ar^1$-47 | ($Ar^2$-1-A-4) |
| (Ar1-Ar2-132) | $Ar^1$-48 | ($Ar^2$-1-A-4) |
| (Ar1-Ar2-133) | $Ar^1$-49 | ($Ar^2$-1-A-4) |
| (Ar1-Ar2-134) | $Ar^1$-50 | ($Ar^2$-1-A-4) |
| (Ar1-Ar2-135) | $Ar^1$-51 | ($Ar^2$-1-A-4) |
| (Ar1-Ar2-136) | $Ar^1$-52 | ($Ar^2$-1-A-4) |
| (Ar1-Ar2-137) | $Ar^1$-53 | ($Ar^2$-1-A-4) |
| (Ar1-Ar2-138) | $Ar^1$-58 | ($Ar^2$-1-A-4) |
| (Ar1-Ar2-139) | $Ar^1$-59 | ($Ar^2$-1-A-4) |
| (Ar1-Ar2-140) | $Ar^1$-60 | ($Ar^2$-1-A-4) |
| (Ar1-Ar2-141) | $Ar^1$-61 | ($Ar^2$-1-A-4) |
| (Ar1-Ar2-142) | $Ar^1$-62 | ($Ar^2$-1-A-4) |
| (Ar1-Ar2-143) | $Ar^1$-63 | ($Ar^2$-1-A-4) |
| (Ar1-Ar2-144) | $Ar^1$-64 | ($Ar^2$-1-A-4) |
| (Ar1-Ar2-145) | Einfachbindung | ($Ar^2$-2-A-1) |
| (Ar1-Ar2-146) | $Ar^1$-1 | ($Ar^2$-2-A-1) |
| (Ar1-Ar2-147) | $Ar^1$-2 | ($Ar^2$-2-A-1) |
| (Ar1-Ar2-148) | $Ar^1$-3 | ($Ar^2$-2-A-1) |
| (Ar1-Ar2-149) | $Ar^1$-4 | ($Ar^2$-2-A-1) |
| (Ar1-Ar2-150) | $Ar^1$-5 | ($Ar^2$-2-A-1) |
| (Ar1-Ar2-151) | $Ar^1$-6 | ($Ar^2$-2-A-1) |
| (Ar1-Ar2-152) | $Ar^1$-7 | ($Ar^2$-2-A-1) |
| (Ar1-Ar2-153) | $Ar^1$-8 | ($Ar^2$-2-A-1) |
| (Ar1-Ar2-154) | $Ar^1$-9 | ($Ar^2$-2-A-1) |

(fortgesetzt)

| Formel | $Ar^1$ | $Ar^2$ |
|---|---|---|
| (Ar1-Ar2-155) | $Ar^1$-15 | ($Ar^2$-2-A-1) |
| (Ar1-Ar2-156) | $Ar^1$-16 | ($Ar^2$-2-A-1) |
| (Ar1-Ar2-157) | $Ar^1$-17 | ($Ar^2$-2-A-1) |
| (Ar1-Ar2-158) | $Ar^1$-18 | ($Ar^2$-2-A-1) |
| (Ar1-Ar2-159) | $Ar^1$-19 | ($Ar^2$-2-A-1) |
| (Ar1-Ar2-160) | $Ar^1$-20 | ($Ar^2$-2-A-1) |
| (Ar1-Ar2-161) | $Ar^1$-36 | ($Ar^2$-2-A-1) |
| (Ar1-Ar2-162) | $Ar^1$-42 | ($Ar^2$-2-A-1) |
| (Ar1-Ar2-163) | $Ar^1$-43 | ($Ar^2$-2-A-1) |
| (Ar1-Ar2-164) | $Ar^1$-44 | ($Ar^2$-2-A-1) |
| (Ar1-Ar2-165) | $Ar^1$-45 | ($Ar^2$-2-A-1) |
| (Ar1-Ar2-166) | $Ar^1$-46 | ($Ar^2$-2-A-1) |
| (Ar1-Ar2-167) | $Ar^1$-47 | ($Ar^2$-2-A-1) |
| (Ar1-Ar2-168) | $Ar^1$-48 | ($Ar^2$-2-A-1) |
| (Ar1-Ar2-169) | $Ar^1$-49 | ($Ar^2$-2-A-1) |
| (Ar1-Ar2-170) | $Ar^1$-50 | ($Ar^2$-2-A-1) |
| (Ar1-Ar2-171) | $Ar^1$-51 | ($Ar^2$-2-A-1) |
| (Ar1-Ar2-172) | $Ar^1$-52 | ($Ar^2$-2-A-1) |
| (Ar1-Ar2-173) | $Ar^1$-53 | ($Ar^2$-2-A-1) |
| (Ar1-Ar2-174) | $Ar^1$-58 | ($Ar^2$-2-A-1) |
| (Ar1-Ar2-175) | $Ar^1$-59 | ($Ar^2$-2-A-1) |
| (Ar1-Ar2-176) | $Ar^1$-60 | ($Ar^2$-2-A-1) |
| (Ar1-Ar2-177) | $Ar^1$-61 | ($Ar^2$-2-A-1) |
| (Ar1-Ar2-178) | $Ar^1$-62 | ($Ar^2$-2-A-1) |
| (Ar1-Ar2-179) | $Ar^1$-63 | ($Ar^2$-2-A-1) |
| (Ar1-Ar2-180) | $Ar^1$-64 | ($Ar^2$-2-A-1) |
| (Ar1-Ar2-181) | Einfachbindung | ($Ar^2$-2-A-2) |
| (Ar1-Ar2-182) | $Ar^1$-1 | ($Ar^2$-2-A-2) |
| (Ar1-Ar2-183) | $Ar^1$-2 | ($Ar^2$-2-A-2) |
| (Ar1-Ar2-184) | $Ar^1$-3 | ($Ar^2$-2-A-2) |
| (Ar1-Ar2-185) | $Ar^1$-4 | ($Ar^2$-2-A-2) |
| (Ar1-Ar2-186) | $Ar^1$-5 | ($Ar^2$-2-A-2) |
| (Ar1-Ar2-187) | $Ar^1$-6 | ($Ar^2$-2-A-2) |
| (Ar1-Ar2-188) | $Ar^1$-7 | ($Ar^2$-2-A-2) |
| (Ar1-Ar2-189) | $Ar^1$-8 | ($Ar^2$-2-A-2) |
| (Ar1-Ar2-190) | $Ar^1$-9 | ($Ar^2$-2-A-2) |
| (Ar1-Ar2-191) | $Ar^1$-15 | ($Ar^2$-2-A-2) |
| (Ar1-Ar2-192) | $Ar^1$-16 | ($Ar^2$-2-A-2) |

(fortgesetzt)

| Formel | $Ar^1$ | $Ar^2$ |
|---|---|---|
| (Ar1-Ar2-193) | $Ar^1$-17 | ($Ar^2$-2-A-2) |
| (Ar1-Ar2-194) | $Ar^1$-18 | ($Ar^2$-2-A-2) |
| (Ar1-Ar2-195) | $Ar^1$-19 | ($Ar^2$-2-A-2) |
| (Ar1-Ar2-196) | $Ar^1$-20 | ($Ar^2$-2-A-2) |
| (Ar1-Ar2-197) | $Ar^1$-36 | ($Ar^2$-2-A-2) |
| (Ar1-Ar2-198) | $Ar^1$-42 | ($Ar^2$-2-A-2) |
| (Ar1-Ar2-199) | $Ar^1$-43 | ($Ar^2$-2-A-2) |
| (Ar1-Ar2-200) | $Ar^1$-44 | ($Ar^2$-2-A-2) |
| (Ar1-Ar2-201) | $Ar^1$-45 | ($Ar^2$-2-A-2) |
| (Ar1-Ar2-202) | $Ar^1$-46 | ($Ar^2$-2-A-2) |
| (Ar1-Ar2-203) | $Ar^1$-47 | ($Ar^2$-2-A-2) |
| (Ar1-Ar2-204) | $Ar^1$-48 | ($Ar^2$-2-A-2) |
| (Ar1-Ar2-205) | $Ar^1$-49 | ($Ar^2$-2-A-2) |
| (Ar1-Ar2-206) | $Ar^1$-50 | ($Ar^2$-2-A-2) |
| (Ar1-Ar2-207) | $Ar^1$-51 | ($Ar^2$-2-A-2) |
| (Ar1-Ar2-208) | $Ar^1$-52 | ($Ar^2$-2-A-2) |
| (Ar1-Ar2-209) | $Ar^1$-53 | ($Ar^2$-2-A-2) |
| (Ar1-Ar2-210) | $Ar^1$-58 | ($Ar^2$-2-A-2) |
| (Ar1-Ar2-211) | $Ar^1$-59 | ($Ar^2$-2-A-2) |
| (Ar1-Ar2-212) | $Ar^1$-60 | ($Ar^2$-2-A-2) |
| (Ar1-Ar2-213) | $Ar^1$-61 | ($Ar^2$-2-A-2) |
| (Ar1-Ar2-214) | $Ar^1$-62 | ($Ar^2$-2-A-2) |
| (Ar1-Ar2-215) | $Ar^1$-63 | ($Ar^2$-2-A-2) |
| (Ar1-Ar2-216) | $Ar^1$-64 | ($Ar^2$-2-A-2) |
| (Ar1-Ar2-217) | Einfachbindung | ($Ar^2$-2-A-3) |
| (Ar1-Ar2-218) | $Ar^1$-1 | ($Ar^2$-2-A-3) |
| (Ar1-Ar2-219) | $Ar^1$-2 | ($Ar^2$-2-A-3) |
| (Ar1-Ar2-220) | $Ar^1$-3 | ($Ar^2$-2-A-3) |
| (Ar1-Ar2-221) | $Ar^1$-4 | ($Ar^2$-2-A-3) |
| (Ar1-Ar2-222) | $Ar^1$-5 | ($Ar^2$-2-A-3) |
| (Ar1-Ar2-223) | $Ar^1$-6 | ($Ar^2$-2-A-3) |
| (Ar1-Ar2-224) | $Ar^1$-7 | ($Ar^2$-2-A-3) |
| (Ar1-Ar2-225) | $Ar^1$-8 | ($Ar^2$-2-A-3) |
| (Ar1-Ar2-226) | $Ar^1$-9 | ($Ar^2$-2-A-3) |
| (Ar1-Ar2-227) | $Ar^1$-15 | ($Ar^2$-2-A-3) |
| (Ar1-Ar2-228) | $Ar^1$-16 | ($Ar^2$-2-A-3) |
| (Ar1-Ar2-229) | $Ar^1$-17 | ($Ar^2$-2-A-3) |
| (Ar1-Ar2-230) | $Ar^1$-18 | ($Ar^2$-2-A-3) |

(fortgesetzt)

| Formel | $Ar^1$ | $Ar^2$ |
|---|---|---|
| (Ar1-Ar2-231) | $Ar^1$-19 | ($Ar^2$-2-A-3) |
| (Ar1-Ar2-232) | $Ar^1$-20 | ($Ar^2$-2-A-3) |
| (Ar1-Ar2-233) | $Ar^1$-36 | ($Ar^2$-2-A-3) |
| (Ar1-Ar2-234) | $Ar^1$-42 | ($Ar^2$-2-A-3) |
| (Ar1-Ar2-235) | $Ar^1$-43 | ($Ar^2$-2-A-3) |
| (Ar1-Ar2-236) | $Ar^1$-44 | ($Ar^2$-2-A-3) |
| (Ar1-Ar2-237) | $Ar^1$-45 | ($Ar^2$-2-A-3) |
| (Ar1-Ar2-238) | $Ar^1$-46 | ($Ar^2$-2-A-3) |
| (Ar1-Ar2-239) | $Ar^1$-47 | ($Ar^2$-2-A-3) |
| (Ar1-Ar2-240) | $Ar^1$-48 | ($Ar^2$-2-A-3) |
| (Ar1-Ar2-241) | $Ar^1$-49 | ($Ar^2$-2-A-3) |
| (Ar1-Ar2-242) | $Ar^1$-50 | ($Ar^2$-2-A-3) |
| (Ar1-Ar2-243) | $Ar^1$-51 | ($Ar^2$-2-A-3) |
| (Ar1-Ar2-244) | $Ar^1$-52 | ($Ar^2$-2-A-3) |
| (Ar1-Ar2-245) | $Ar^1$-53 | ($Ar^2$-2-A-3) |
| (Ar1-Ar2-246) | $Ar^1$-58 | ($Ar^2$-2-A-3) |
| (Ar1-Ar2-247) | $Ar^1$-59 | ($Ar^2$-2-A-3) |
| (Ar1-Ar2-248) | $Ar^1$-60 | ($Ar^2$-2-A-3) |
| (Ar1-Ar2-249) | $Ar^1$-61 | ($Ar^2$-2-A-3) |
| (Ar1-Ar2-250) | $Ar^1$-62 | ($Ar^2$-2-A-3) |
| (Ar1-Ar2-251) | $Ar^1$-63 | ($Ar^2$-2-A-3) |
| (Ar1-Ar2-252) | $Ar^1$-64 | ($Ar^2$-2-A-3) |
| (Ar1-Ar2-253) | Einfachbindung | ($Ar^2$-2-A-4) |
| (Ar1-Ar2-254) | $Ar^1$-1 | ($Ar^2$-2-A-4) |
| (Ar1-Ar2-255) | $Ar^1$-2 | ($Ar^2$-2-A-4) |
| (Ar1-Ar2-256) | $Ar^1$-3 | ($Ar^2$-2-A-4) |
| (Ar1-Ar2-257) | $Ar^1$-4 | ($Ar^2$-2-A-4) |
| (Ar1-Ar2-258) | $Ar^1$-5 | ($Ar^2$-2-A-4) |
| (Ar1-Ar2-259) | $Ar^1$-6 | ($Ar^2$-2-A-4) |
| (Ar1-Ar2-260) | $Ar^1$-7 | ($Ar^2$-2-A-4) |
| (Ar1-Ar2-261) | $Ar^1$-8 | ($Ar^2$-2-A-4) |
| (Ar1-Ar2-262) | $Ar^1$-9 | ($Ar^2$-2-A-4) |
| (Ar1-Ar2-263) | $Ar^1$-15 | ($Ar^2$-2-A-4) |
| (Ar1-Ar2-264) | $Ar^1$-16 | ($Ar^2$-2-A-4) |
| (Ar1-Ar2-265) | $Ar^1$-17 | ($Ar^2$-2-A-4) |
| (Ar1-Ar2-266) | $Ar^1$-18 | ($Ar^2$-2-A-4) |
| (Ar1-Ar2-267) | $Ar^1$-19 | ($Ar^2$-2-A-4) |
| (Ar1-Ar2-268) | $Ar^1$-20 | ($Ar^2$-2-A-4) |

(fortgesetzt)

| Formel | $Ar^1$ | $Ar^2$ |
|---|---|---|
| (Ar1-Ar2-269) | $Ar^1$-36 | ($Ar^2$-2-A-4) |
| (Ar1-Ar2-270) | $Ar^1$-42 | ($Ar^2$-2-A-4) |
| (Ar1-Ar2-271) | $Ar^1$-43 | ($Ar^2$-2-A-4) |
| (Ar1-Ar2-272) | $Ar^1$-44 | ($Ar^2$-2-A-4) |
| (Ar1-Ar2-273) | $Ar^1$-45 | ($Ar^2$-2-A-4) |
| (Ar1-Ar2-274) | $Ar^1$-46 | ($Ar^2$-2-A-4) |
| (Ar1-Ar2-275) | $Ar^1$-47 | ($Ar^2$-2-A-4) |
| (Ar1-Ar2-276) | $Ar^1$-48 | ($Ar^2$-2-A-4) |
| (Ar1-Ar2-277) | $Ar^1$-49 | ($Ar^2$-2-A-4) |
| (Ar1-Ar2-278) | $Ar^1$-50 | ($Ar^2$-2-A-4) |
| (Ar1-Ar2-279) | $Ar^1$-51 | ($Ar^2$-2-A-4) |
| (Ar1-Ar2-280) | $Ar^1$-52 | ($Ar^2$-2-A-4) |
| (Ar1-Ar2-281) | $Ar^1$-53 | ($Ar^2$-2-A-4) |
| (Ar1-Ar2-282) | $Ar^1$-58 | ($Ar^2$-2-A-4) |
| (Ar1-Ar2-283) | $Ar^1$-59 | ($Ar^2$-2-A-4) |
| (Ar1-Ar2-284) | $Ar^1$-60 | ($Ar^2$-2-A-4) |
| (Ar1-Ar2-285) | $Ar^1$-61 | ($Ar^2$-2-A-4) |
| (Ar1-Ar2-286) | $Ar^1$-62 | ($Ar^2$-2-A-4) |
| (Ar1-Ar2-287) | $Ar^1$-63 | ($Ar^2$-2-A-4) |
| (Ar1-Ar2-288) | $Ar^1$-64 | ($Ar^2$-2-A-4) |

oder aus Einheiten der folgenden Formeln $-Ar^1-Ar^2-Ar^2$, wobei $Ar^1$ eine Einfachbindung ist

| Formel | $-Ar^2-$ | $-Ar^2$ |
|---|---|---|
| (Ar1-Ar2-Ar2-1) | 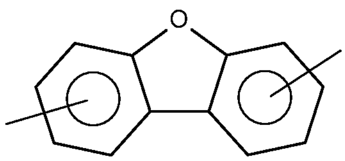 | ($Ar^2$-1-A-1) |
| (Ar1-Ar2-Ar2-2) | 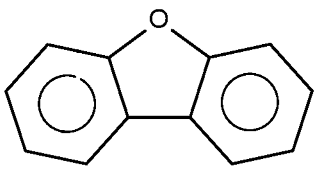 | ($Ar^2$-1-A-1) |
| (Ar1-Ar2-Ar2-3) | 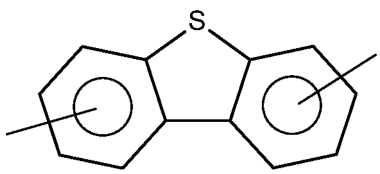 | ($Ar^2$-1-A-1) |

(fortgesetzt)

| **Formel** | **-Ar²-** | **-Ar²** |
|---|---|---|
| (Ar1-Ar2-Ar2-4) | 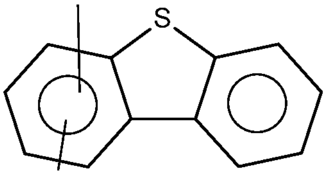 | ($Ar^2$-1-A-1) |
| (Ar1-Ar2-Ar2-5) | 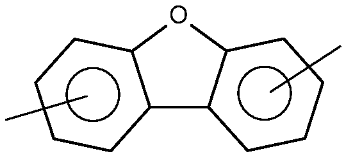 | ($Ar^2$-1-A-2) |
| (Ar1-Ar2-Ar2-6) | 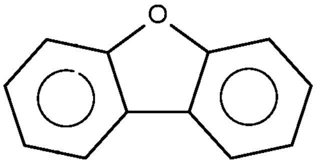 | ($Ar^2$-1-A-2) |
| (Ar1-Ar2-Ar2-7) | 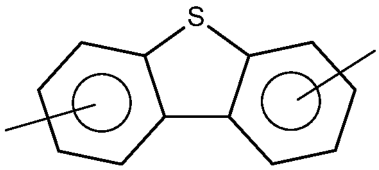 | ($Ar^2$-1-A-2) |
| (Ar1-Ar2-Ar2-8) | 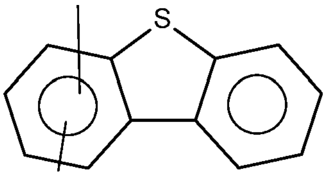 | ($Ar^2$-1-A-2) |
| (Ar1-Ar2-Ar2-9) | 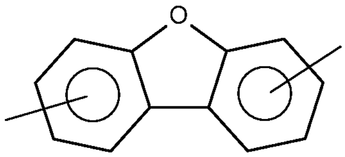 | ($Ar^2$-1-A-3) |
| (Ar1-Ar2-Ar2-10) | 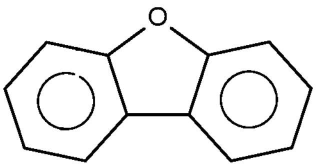 | ($Ar^2$-1-A-3) |
| (Ar1-Ar2-Ar2-11) | 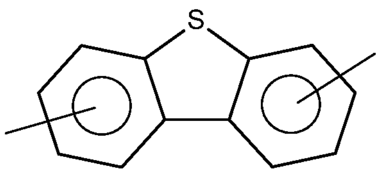 | ($Ar^2$-1-A-3) |
| (Ar1-Ar2-Ar2-12) | 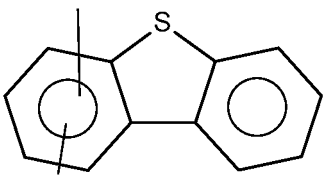 | ($Ar^2$-1-A-3) |

(fortgesetzt)

| Formel | -Ar²- | -Ar² |
|---|---|---|
| (Ar1-Ar2-Ar2-13) | 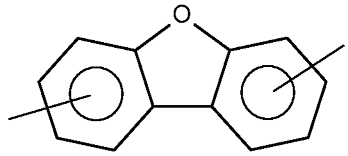 | ($Ar^2$-1-A-4) |
| (Ar1-Ar2-Ar2-14) | 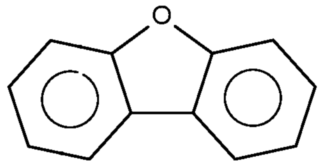 | ($Ar^2$-1-A-4) |
| (Ar1-Ar2-Ar2-15) | 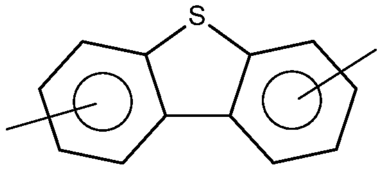 | ($Ar^2$-1-A-4) |
| (Ar1-Ar2-Ar2-16) | 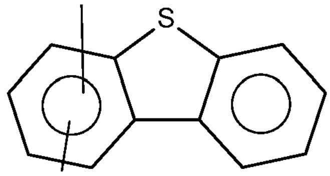 | ($Ar^2$-1-A-4) |
| (Ar1-Ar2-Ar2-17) | 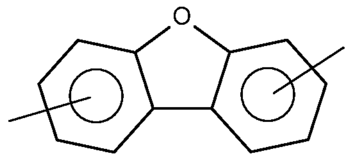 | ($Ar^2$-2-A-1) |
| (Ar1-Ar2-Ar2-18) | 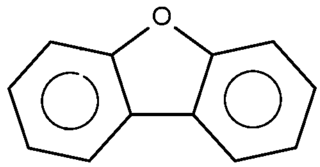 | ($Ar^2$-2-A-1) |
| (Ar1-Ar2-Ar2-19) | 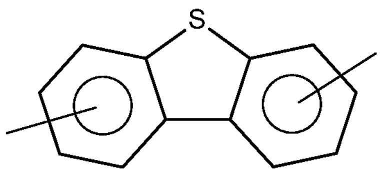 | ($Ar^2$-2-A-1) |
| (Ar1-Ar2-Ar2-20) | 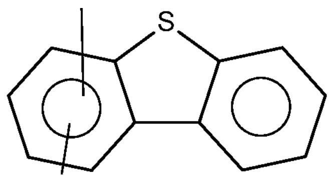 | ($Ar^2$-2-A-1) |
| (Ar1-Ar2-Ar2-21) | 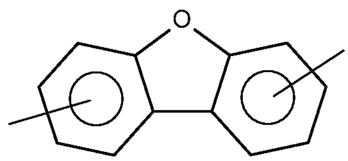 | ($Ar^2$-2-A-2) |

(fortgesetzt)

| **Formel** | **-Ar²-** | **-Ar²** |
|---|---|---|
| (Ar1-Ar2-Ar2-22) | 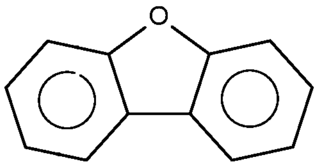 | (Ar$^2$-2-A-2) |
| (Ar1-Ar2-Ar2-23) | 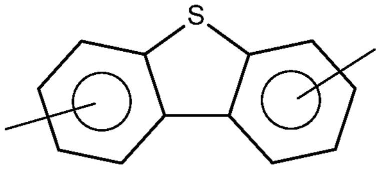 | (Ar$^2$-2-A-2) |
| (Ar1-Ar2-Ar2-24) | 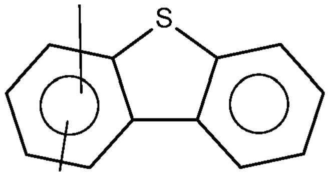 | (Ar$^2$-2-A-2) |
| (Ar1-Ar2-Ar2-25) | 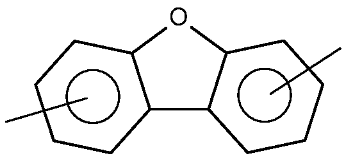 | (Ar$^2$-2-A-3) |
| (Ar1-Ar2-Ar2-26) | 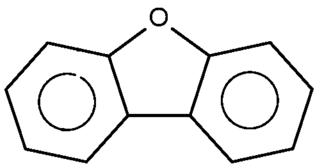 | (Ar$^2$-2-A-3) |
| (Ar1-Ar2-Ar2-27) | 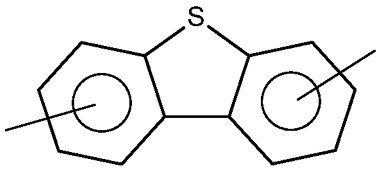 | (Ar$^2$-2-A-3) |
| (Ar1-Ar2-Ar2-28) | 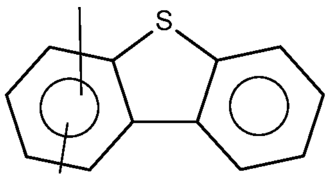 | (Ar$^2$-2-A-3) |
| (Ar1-Ar2-Ar2-29) | 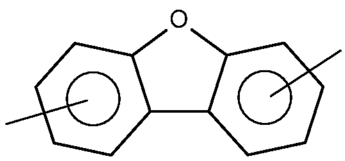 | (Ar$^2$-2-A-4) |
| (Ar1-Ar2-Ar2-30) | 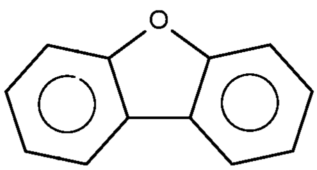 | (Ar$^2$-2-A-4) |

(fortgesetzt)

| Formel | -Ar2- | -Ar2 |
|---|---|---|
| (Ar1-Ar2-Ar2-31) | 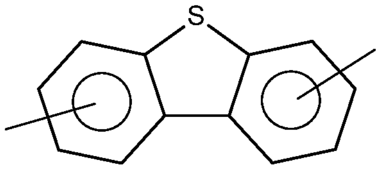 | ($Ar^2$-2-A-4) |
| (Ar1-Ar2-Ar2-32) | 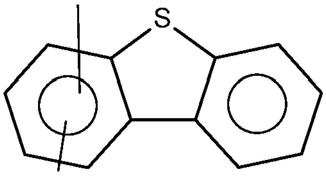 | ($Ar^2$-2-A-4) |

und wobei die Einheiten -$Ar^2$- an ihren freien Positionen jeweils mit einem Rest $R^3$ substituiert sein können.

Bevorzugte Einheiten -$[Ar^1]_c$-$[Ar^2]_d$-$[Ar^1]_e$- in Formel (II-A) sind gewählt aus Einheiten -$Ar^2$- gemäß den folgenden Formeln

| Formel | -$Ar^2$- |
|---|---|
| ($Ar^2$-1-5) | 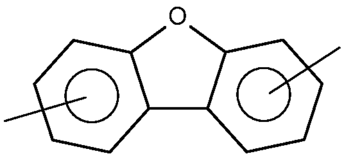 |
| ($Ar^2$-1-6) | 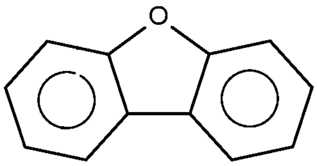 |
| ($Ar^2$-2-5) | 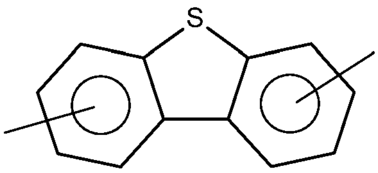 |
| ($Ar^2$-2-6) | 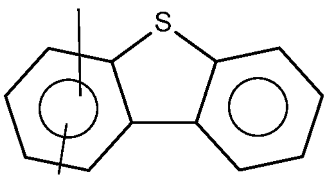 |

wobei die Einheiten -$Ar^2$- an ihren freien Positionen jeweils mit einem Rest $R^3$ substituiert sein können, oder aus Einheiten -$Ar^2$-$Ar^2$- gemäß den folgenden Formeln

| Formel | -Ar2- | -Ar2- |
|---|---|---|
| (Ar2-Ar2-1) | 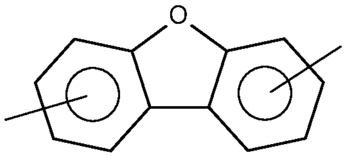 | 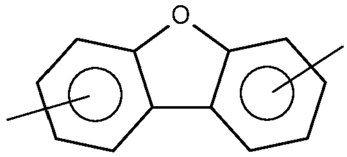 |
| (Ar2-Ar2-2) | 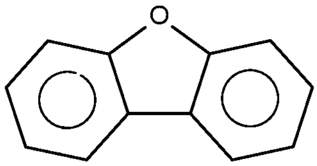 | 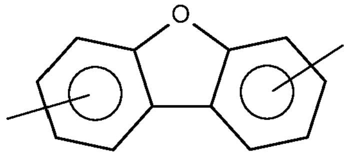 |
| (Ar2-Ar2-3) | 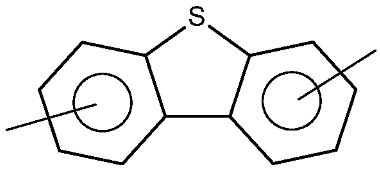 | 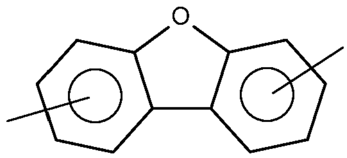 |
| (Ar2-Ar2-4) | 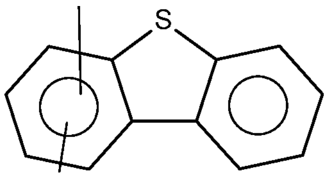 | 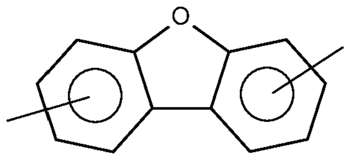 |
| (Ar2-Ar2-5) | 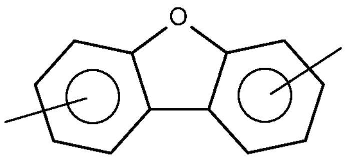 | 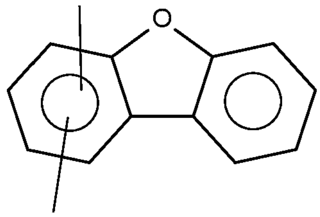 |
| (Ar2-Ar2-6) | 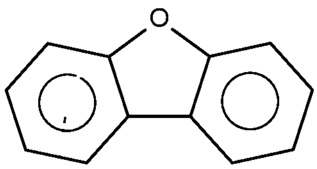 | 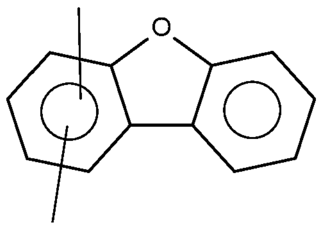 |
| (Ar2-Ar2-7) | 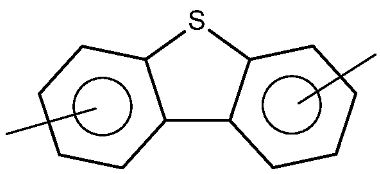 | 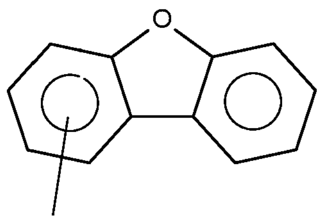 |
| (Ar2-Ar2-8) | 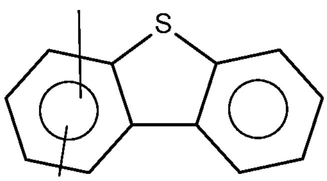 | 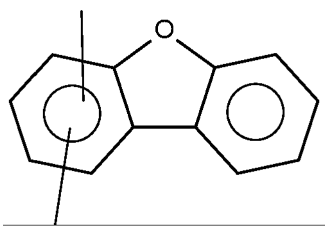 |

(fortgesetzt)

| Formel | -Ar²- | -Ar²- |
|---|---|---|
| (Ar2-Ar2-9) | 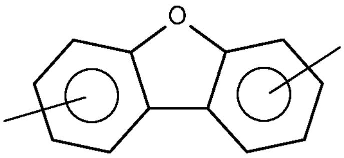 | 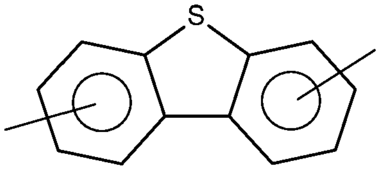 |
| (Ar2-Ar2-10) | 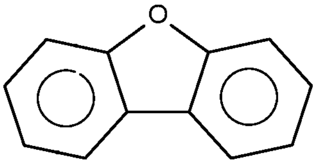 | 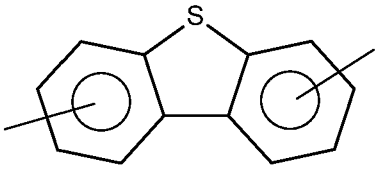 |
| (Ar2-Ar2-11) | 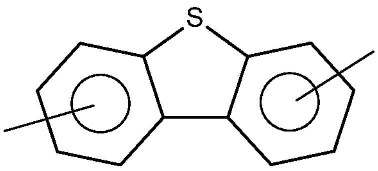 | 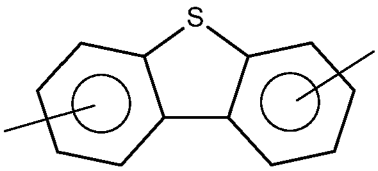 |
| (Ar2-Ar2-12) | 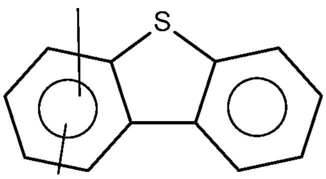 | 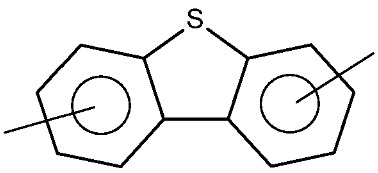 |
| (Ar2-Ar2-13) | 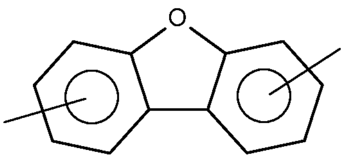 | 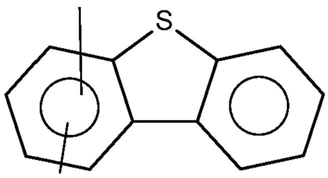 |
| (Ar2-Ar2-14) | 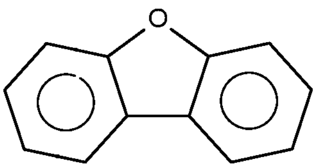 | 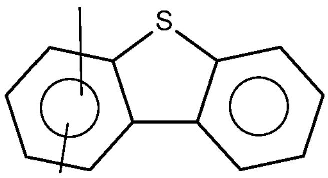 |
| (Ar2-Ar2-15) | 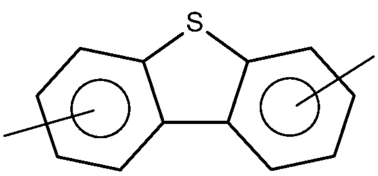 | 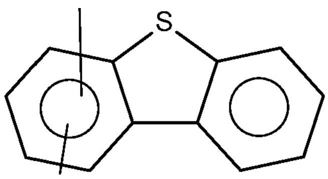 |
| (Ar2-Ar2-16) | 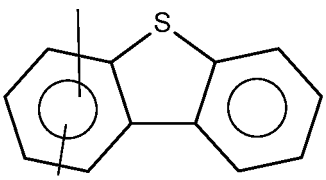 | 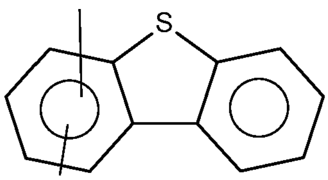 |

wobei die Einheiten -$Ar^2$- an ihren freien Positionen jeweils mit einem Rest $R^3$ substituiert sein können.

$R^1$ ist bevorzugt bei jedem Auftreten gleich oder verschieden gewählt aus H, D, F, CN, $Si(R^4)_3$, geradkettigen Alkyl- oder Alkoxygruppen mit 1 bis 20 C-Atomen, verzweigten oder cyclischen Alkyl- oder Alkoxygruppen mit 3 bis 20

C-Atomen, aromatischen Ringsystemen mit 6 bis 40 aromatischen Ringatomen, und heteroaromatischen Ringsystemen mit 5 bis 40 aromatischen Ringatomen; wobei die genannten Alkyl- und Alkoxygruppen, die genannten aromatischen Ringsysteme und die genannten heteroaromatischen Ringsysteme jeweils mit einem oder mehreren Resten $R^4$ substituiert sein können; und wobei in den genannten Alkyl- oder Alkoxygruppen eine oder mehrere $CH_2$-Gruppen durch -C≡C-, $-R^4C=CR^4-$, $Si(R^4)_2$, C=O, $C=NR^4$, -O-, -S-, -C(=O)O- oder $-C(=O)NR^4-$ ersetzt sein können. Besonders bevorzugt ist $R^1$ bei jedem Auftreten gleich oder verschieden gewählt aus H, D, F, CN, aromatischen Ringsystemen mit 6 bis 40 aromatischen Ringatomen, die jeweils mit einem oder mehreren Resten $R^4$ substituiert sein können, insbesondere Phenyl, Biphenyl, Fluorenyl, Spirobifluorenyl und Naphthyl, die jeweils mit einem oder mehreren Resten $R^4$ substituiert sein können, und heteroaromatischen Ringsystemen mit 5 bis 40 aromatischen Ringatomen, die jeweils mit einem oder mehreren Resten $R^4$ substituiert sein können, insbesondere Dibenzofuranyl, Dibenzothiophenyl, Pyridyl, Pyrimidyl, Benzochinolin und Triazinyl, die jeweils mit einem oder mehreren Resten $R^4$ substituiert sein können. Ganz besonders bevorzugt ist $R^1$ gleich H.

$R^2$ ist bevorzugt bei jedem Auftreten gleich oder verschieden gewählt aus H, D, F, CN, $Si(R^4)_3$, geradkettigen Alkyl- oder Alkoxygruppen mit 1 bis 20 C-Atomen, verzweigten oder cyclischen Alkyl- oder Alkoxygruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkyl- und Alkoxygruppen, die genannten aromatischen Ringsysteme und die genannten heteroaromatischen Ringsysteme jeweils mit einem oder mehreren Resten $R^4$ substituiert sein können; und wobei in den genannten Alkyl- oder Alkoxygruppen eine oder mehrere $CH_2$-Gruppen durch -C≡C-, $-R^4C=CR^4-$, $Si(R^4)_2$, C=O, $C=NR^4$, -O-, -S-, -C(=O)O- oder $-C(=O)NR^4-$ ersetzt sein können. Besonders bevorzugt ist $R^2$ bei jedem Auftreten gleich oder verschieden gewählt aus H, D, F, CN, aromatischen Ringsystemen mit 6 bis 40 aromatischen Ringatomen, die jeweils mit einem oder mehreren Resten $R^4$ substituiert sein können, insbesondere Phenyl, Biphenyl, Fluorenyl, Spirobifluorenyl und Naphthyl, die jeweils mit einem oder mehreren Resten $R^4$ substituiert sein können, und heteroaromatischen Ringsystemen mit 5 bis 40 aromatischen Ringatomen, die jeweils mit einem oder mehreren Resten $R^4$ substituiert sein können, insbesondere Dibenzofuranyl, Dibenzothiophenyl, Pyridyl, Pyrimidyl, Benzochinolinyl und Triazinyl, die jeweils mit einem oder mehreren Resten $R^4$ substituiert sein können. Ganz besonders bevorzugt ist $R^2$ gleich H.

$R^3$ ist bevorzugt bei jedem Auftreten gleich oder verschieden gewählt aus H, D, F, CN, $Si(R^4)_3$, geradkettigen Alkyl- oder Alkoxygruppen mit 1 bis 20 C-Atomen, verzweigten oder cyclischen Alkyl- oder Alkoxygruppen mit 3 bis 20 C-Atomen, aromatischen Ringsystemen mit 6 bis 40 aromatischen Ringatomen, und elektronenarmen Heteroarylgruppen mit 6 bis 40 aromatischen Ringatomen; wobei die genannten Alkyl- und Alkoxygruppen, die genannten aromatischen Ringsysteme und die genannten elektronenarmen Heteroarylgruppen jeweils mit einem oder mehreren Resten $R^4$ substituiert sein können; und wobei in den genannten Alkyl- oder Alkoxygruppen eine oder mehrere $CH_2$-Gruppen durch -C≡C-, $-R^4C=CR^4-$, $Si(R^4)_2$, C=O, $C=NR^4$, -O-, -S-, -C(=O)O- oder $-C(=O)NR^4-$ersetzt sein können. Besonders bevorzugt ist $R^3$ bei jedem Auftreten gleich oder verschieden gewählt aus H, D, F, CN, aromatischen Ringsystemen mit 6 bis 40 aromatischen Ringatomen, die jeweils mit einem oder mehreren Resten $R^4$ substituiert sein können, insbesondere Phenyl, Biphenyl, Fluorenyl, Spirobifluorenyl und Naphthyl, die jeweils mit einem oder mehreren Resten $R^4$ substituiert sein können, und elektronenarmen Heteroarylgruppen mit 6 bis 40 aromatischen Ringatomen, die jeweils mit einem oder mehreren Resten $R^4$ substituiert sein können, insbesondere Pyridyl, Pyrimidyl, Benzochinolinyl und Triazinyl, die jeweils mit einem oder mehreren Resten $R^4$ substituiert sein können.

$R^4$ ist bevorzugt bei jedem Auftreten gleich oder verschieden gewählt aus H, D, F, CN, $Si(R^5)_3$, geradkettigen Alkyl- oder Alkoxygruppen mit 1 bis 20 C-Atomen, verzweigten oder cyclischen Alkyl- oder Alkoxygruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkyl- und Alkoxygruppen, die genannten aromatischen Ringsysteme und die genannten heteroaromatischen Ringsysteme jeweils mit einem oder mehreren Resten $R^5$ substituiert sein können; und wobei in den genannten Alkyl- oder Alkoxygruppen eine oder mehrere $CH_2$-Gruppen durch -C≡C-, $-R^5C=CR^5-$, $Si(R^5)_2$, C=O, $C=NR^5$, -O-, -S-, -C(=O)O- oder $-C(=O)NR^5-$ ersetzt sein können. Besonders bevorzugt ist $R^4$ gleich H.

Indices a, b, c, d und e sind bevorzugt bei jedem Auftreten gleich oder verschieden gewählt aus 1 und 2.

Gemäß einer bevorzugten Ausführungsform ist in Formel (I) a=1 und b =1. Gemäß einer alternativen bevorzugten Ausführungsform ist in Formel (I) a=1 und b=2, wobei die beiden Gruppen $Ar^2$ gleich oder verschieden gewählt sind.

Gemäß einer bevorzugten Ausführungsform ist in Formel (II) c=1, e=1 und d =1. Gemäß einer alternativen bevorzugten Ausführungsform ist in Formel (II) c=1, e=1 und d=2, wobei die beiden Gruppen $Ar^2$ gleich oder verschieden gewählt sind.

Erfindungsgemäße Ausführungsformen entsprechen den folgenden Formeln (I-A), (I-B), (I-C) und (I-D):

| | |
|---|---|
| 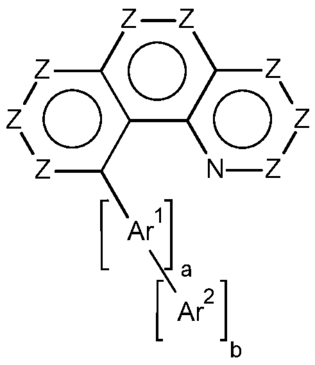 | 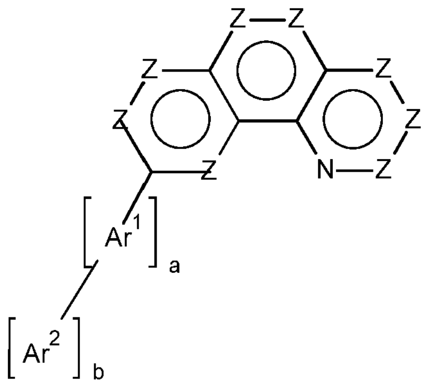 |
| Formel (I-A) | Formel (I-B) |
| 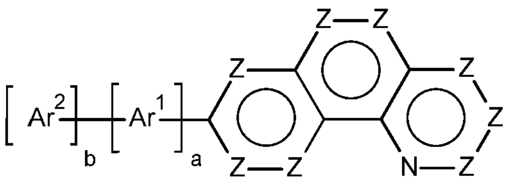 | 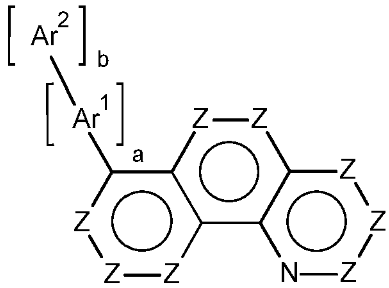 |
| Formel (I-C) | Formel (I-D) |

wobei die auftretenden Gruppen definiert sind wie oben. Erfindungsgemäß ist Z gleich $CR^1$ in den oben genannten Formeln. Bevorzugt unter den Formeln ist die Formel (I-A).

Eine erfindungsgemäße Ausführungsform ist die folgende Formel (II-A):

| |
|---|
| 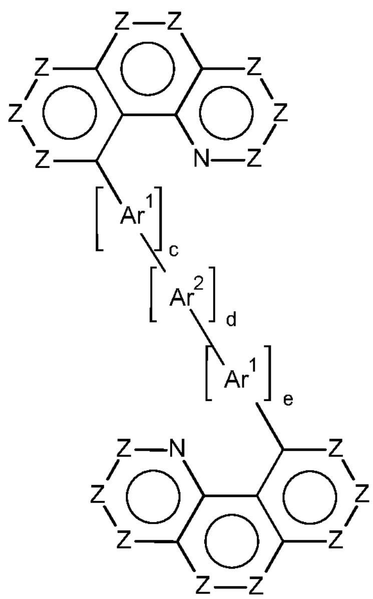 |
| Formel (II-A) |

wobei die auftretenden Gruppen definiert sind wie oben. Erfindungsgemäß ist Z in der Formel gleich $CR^1$.

Bevorzugte Ausführungsformen der Formel (I-A) entsprechen den folgenden Formeln

| | |
|---|---|
| 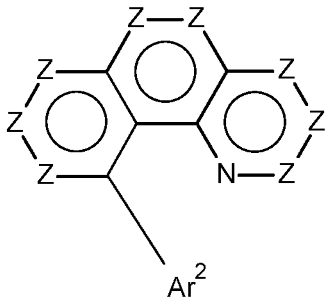 | 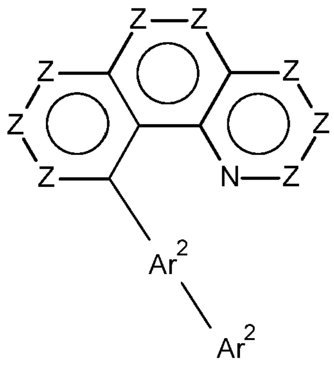 |
| Formel (I-A-1) | Formel (I-A-2) |
| 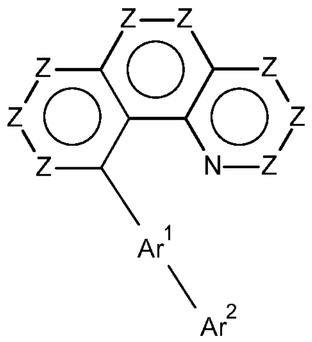 | |
| Formel (I-A-3) | |

wobei die auftretenden Gruppen definiert sind wie oben, mit der Ausnahme, dass $Ar^1$ keine Einfachbindung ist. Erfindungsgemäß ist Z in den Formeln gleich $CR^1$. Insbesondere können die Gruppen $Ar^2$ in Formel (I-A-2) jeweils gleich oder verschieden sein.

Bevorzugte Ausführungsformen der Formel (II-A) entsprechen den folgenden Formeln

| | |
|---|---|
| 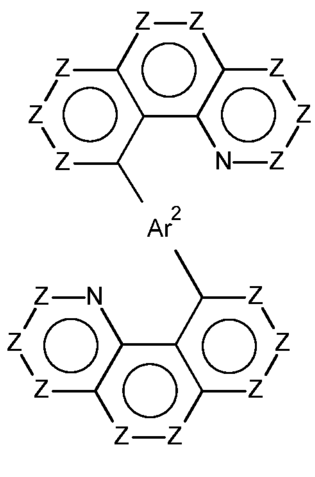 | 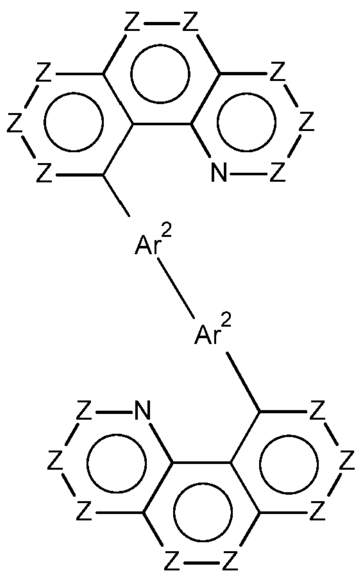 |
| Formel (II-A-1) | Formel (II-A-2) |

wobei die auftretenden Gruppen definiert sind wie oben. Erfindungsgemäß ist Z in den Formeln gleich $CR^1$. Insbesondere können die Gruppen $Ar^2$ in Formel (II-A-2) jeweils gleich oder verschieden sein.

Bevorzugte Ausführungsformen der Verbindungen sind im Folgenden aufgeführt:

| | | |
|---|---|---|
| 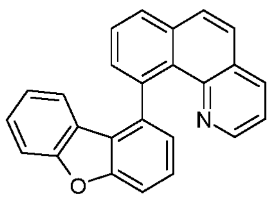 | 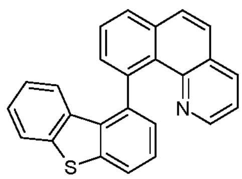 | 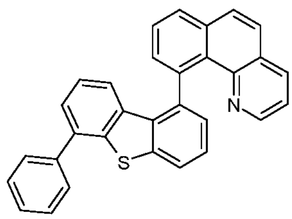 |
| 1 | 2 | 3 |
| 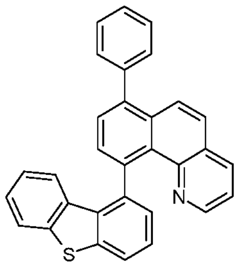 | 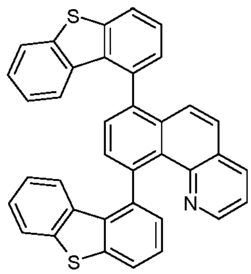 | 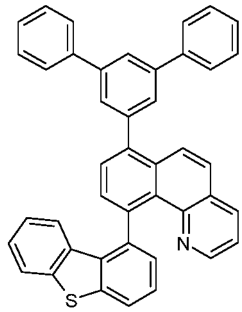 |
| 4 | 5 | 6 |
| 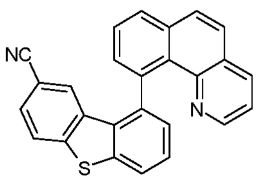 | 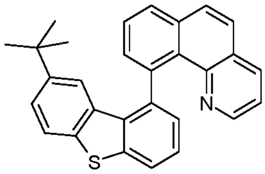 | 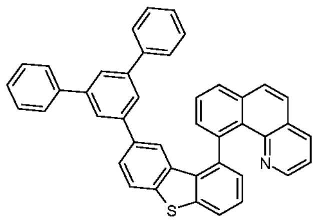 |
| 7 | 8 | 9 |
| 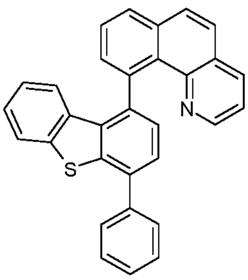 | 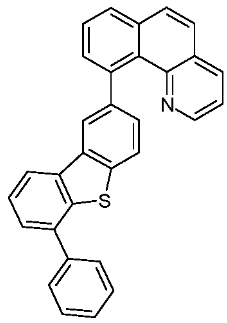 | 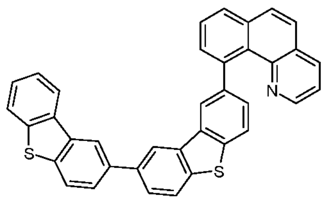 |
| 10 | 11 | 12 |
| 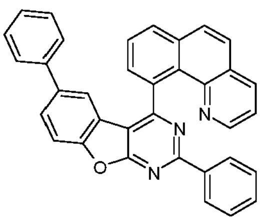 | 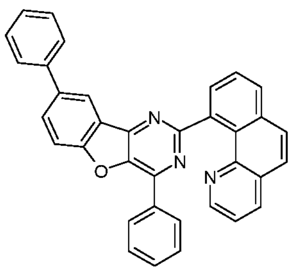 | 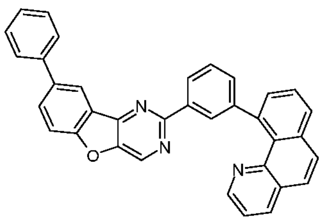 |
| 13 | 14 | 15 |

(fortgesetzt)

| | | |
|---|---|---|
| 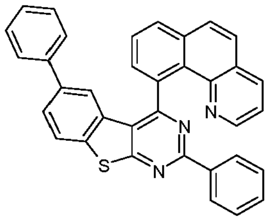 | 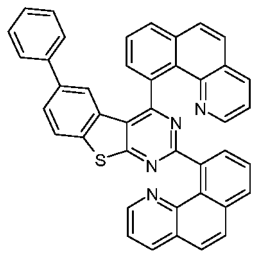 | 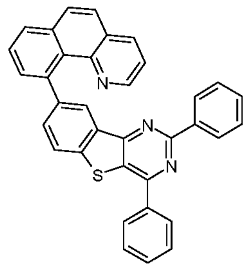 |
| 16 | 17 | 18 |
| 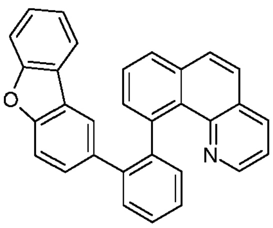 | 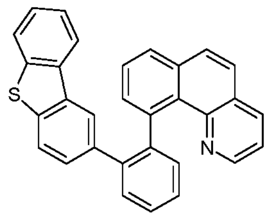 | 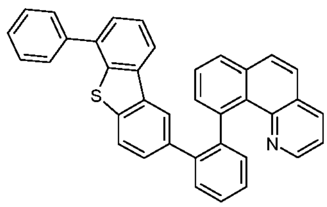 |
| 19 | 20 | 21 |
| 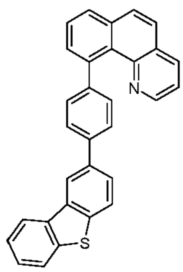 | 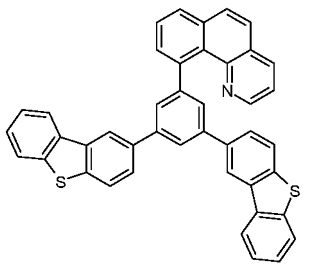 | 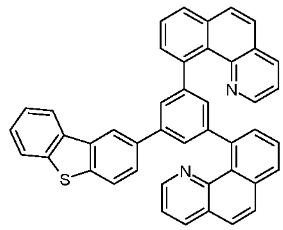 |
| 31 | 32 | 33 |
| 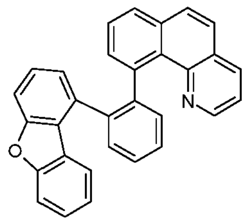 | 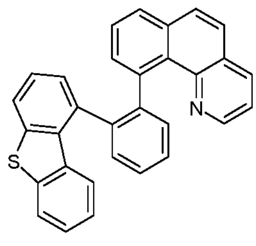 | 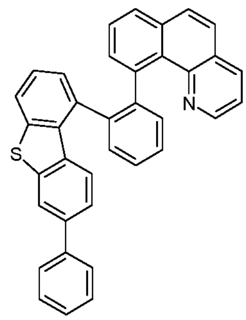 |
| 34 | 35 | 36 |
| 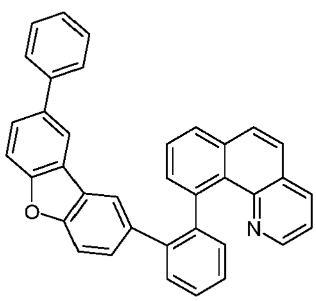 | 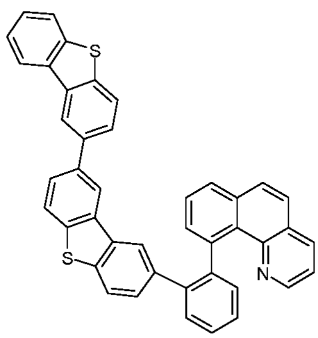 | 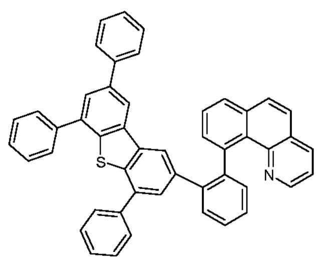 |
| 37 | 38 | 39 |

(fortgesetzt)

| | | |
|---|---|---|
| 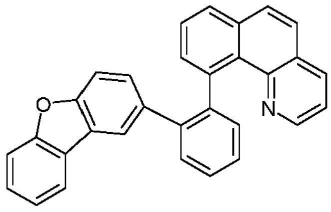 | 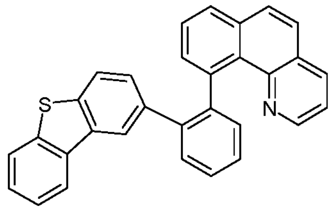 | 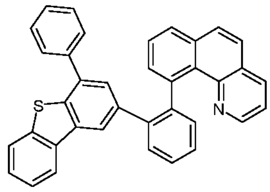 |
| 40 | 41 | 42 |
| 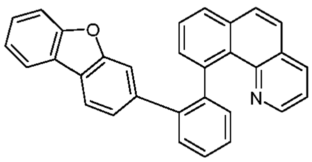 | 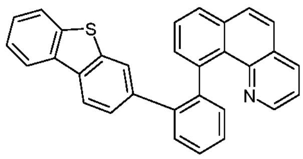 | 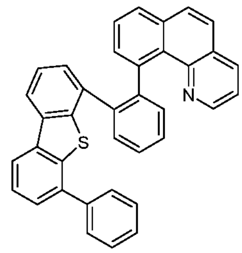 |
| 43 | 44 | 45 |
| 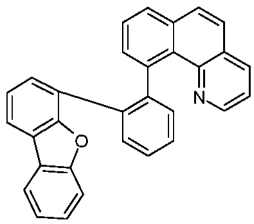 | 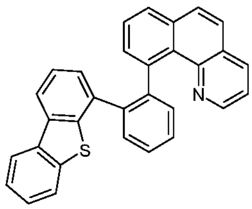 | 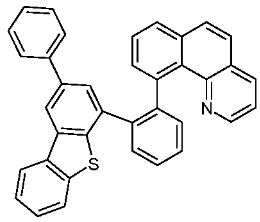 |
| 46 | 47 | 48 |
| 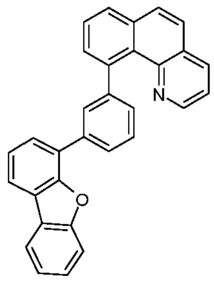 | 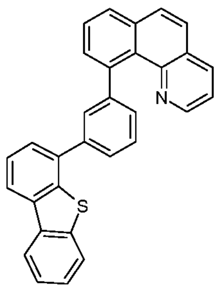 | 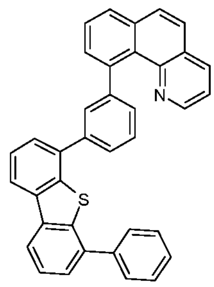 |
| 49 | 50 | 51 |
| 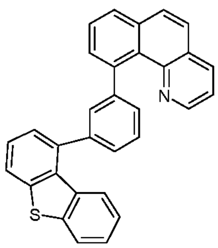 | 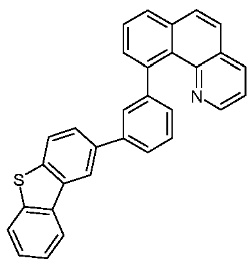 | 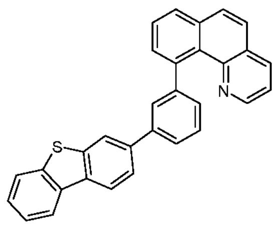 |
| 52 | 53 | 54 |

(fortgesetzt)

| | | |
|---|---|---|
| 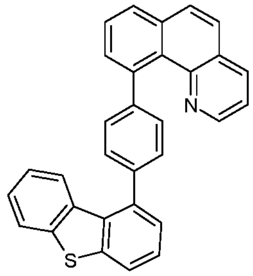 | 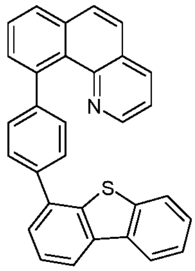 | 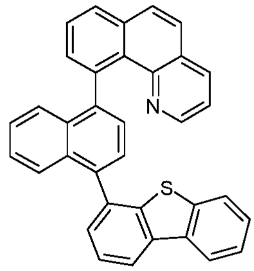 |
| 55 | 56 | 57 |
| 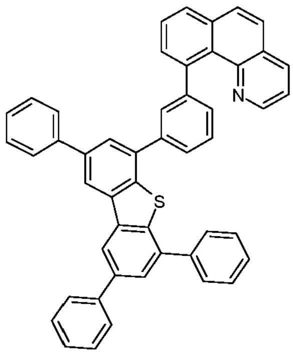 | 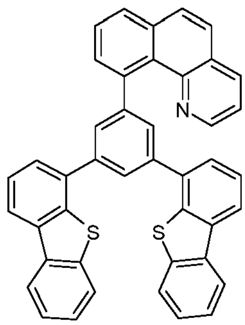 | 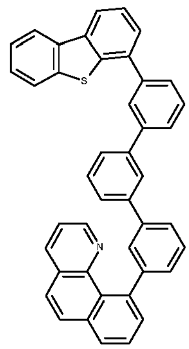 |
| 58 | 59 | 60 |
| 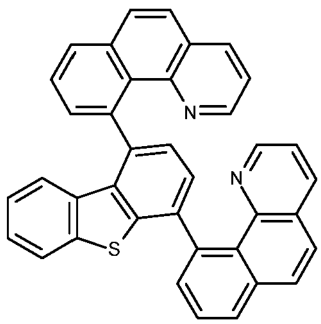 | 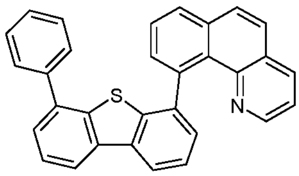 | 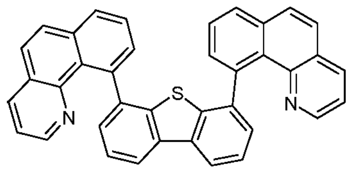 |
| 61 | 62 | 63 |
| 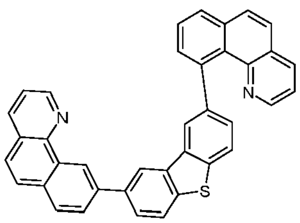 | 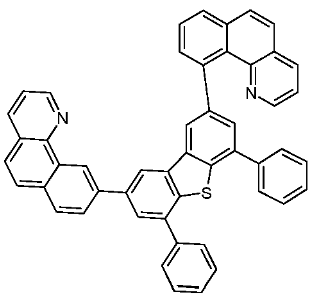 | 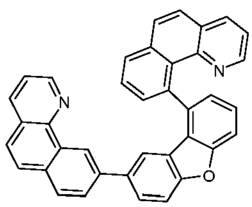 |
| 64 | 65 | 66 |

(fortgesetzt)

| | | |
|---|---|---|
| 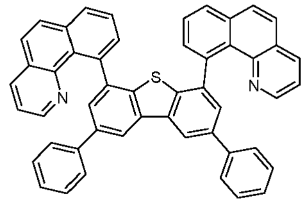 | 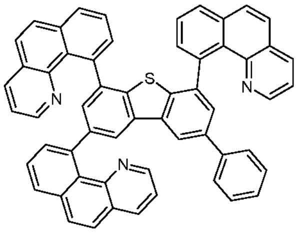 | 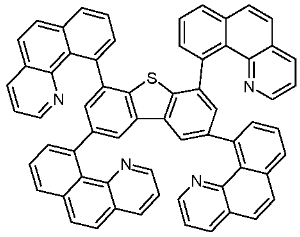 |
| 67 | 68 | 69 |
| 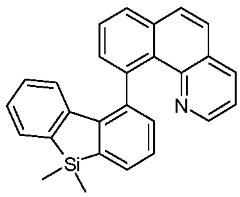 | 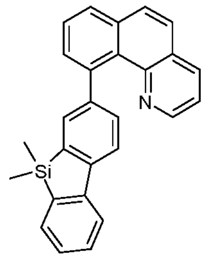 | 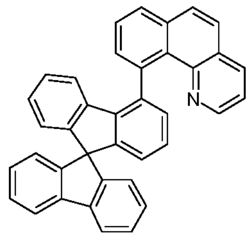 |
| 70 | 71 | 72 |
| 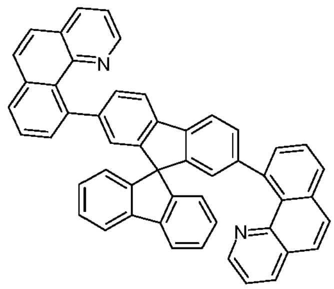 | 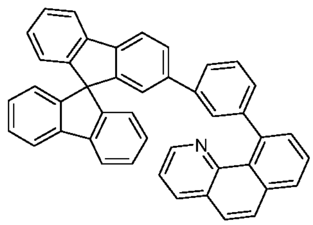 | 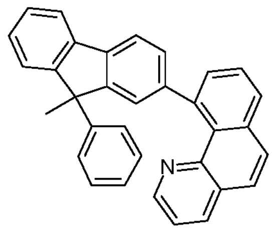 |
| 73 | 74 | 75 |
| 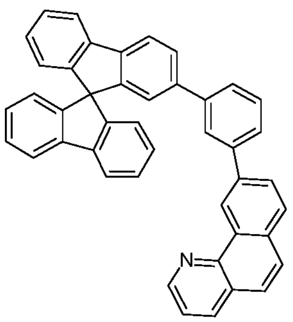 | 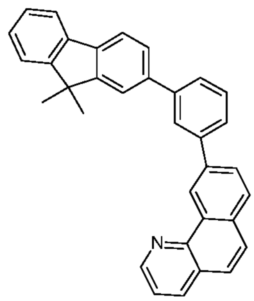 | 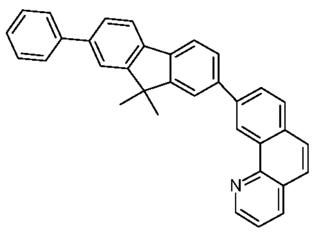 |
| 76 | 77 | 78 |
| 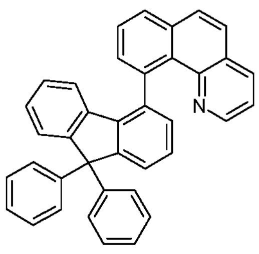 | 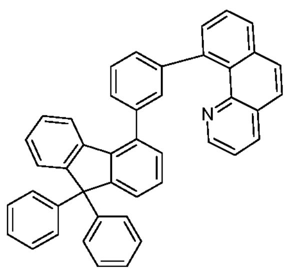 | 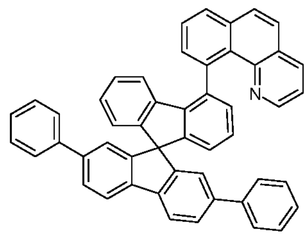 |
| 79 | 80 | 81 |

(fortgesetzt)

| | | |
|---|---|---|
| 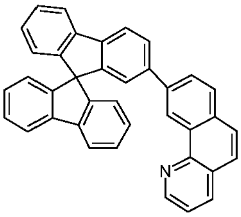 | 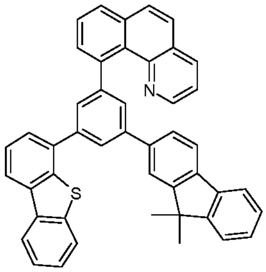 | 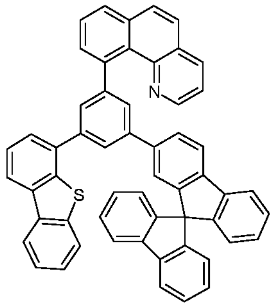 |
| 82 | 83 | 84 |
| 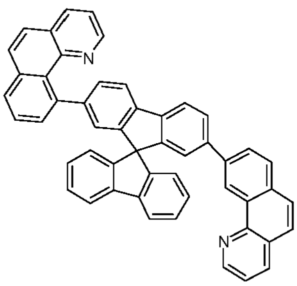 | 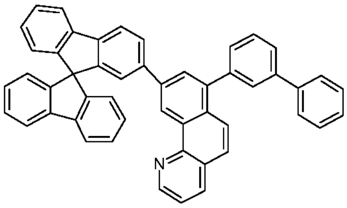 | 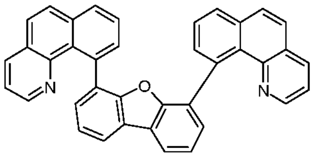 |
| 85 | 86 | 87 |
| 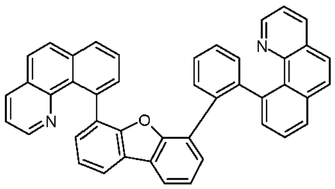 | 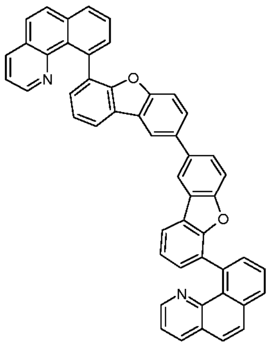 | 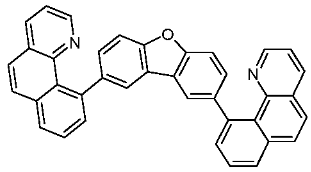 |
| 88 | 89 | 90 |
| 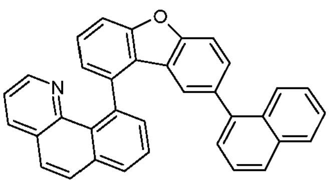 | 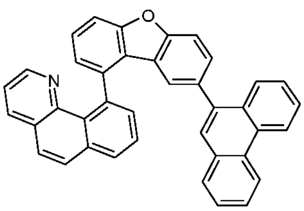 | 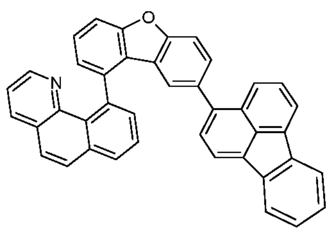 |
| 91 | 92 | 93 |
| 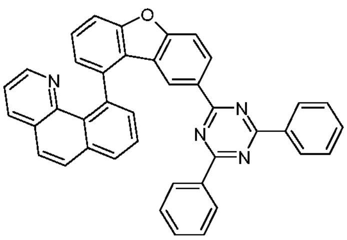 | 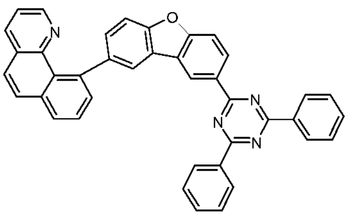 | 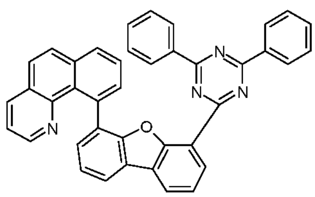 |
| 94 | 95 | 96 |

(fortgesetzt)

| | | |
|---|---|---|
| 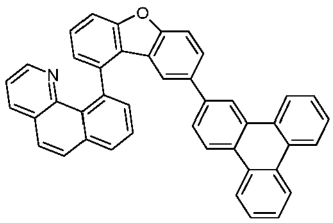 | 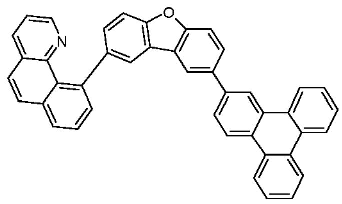 | 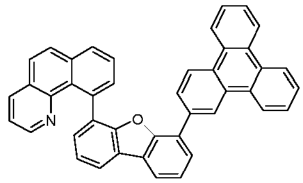 |
| 97 | 98 | 99 |
| 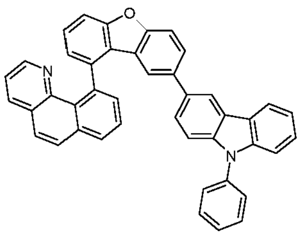 | 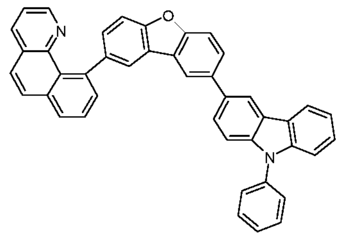 | 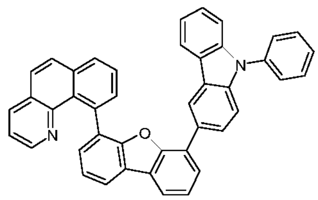 |
| 100 | 101 | 102 |
| 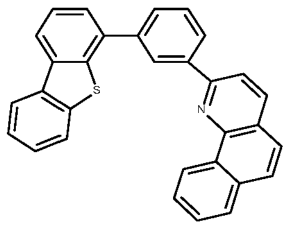 | 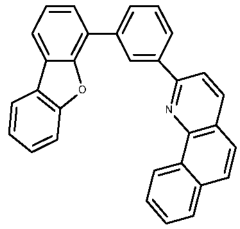 | 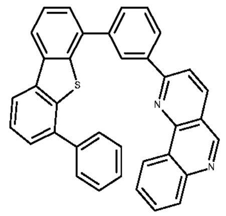 |
| 103 | 104 | 105 |
| 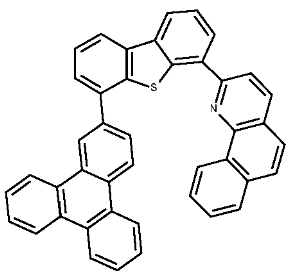 | 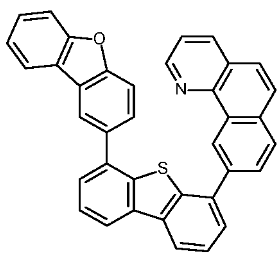 | 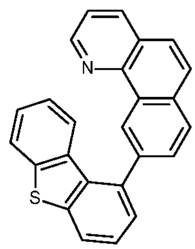 |
| 106 | 107 | 108 |
| 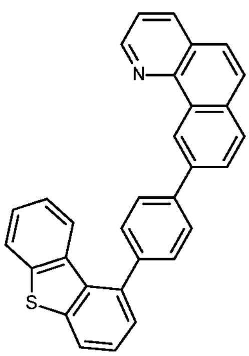 | 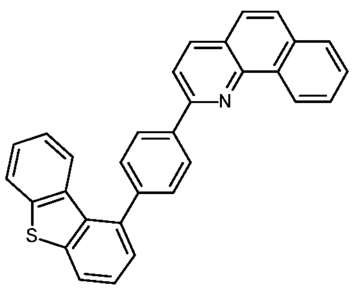 | 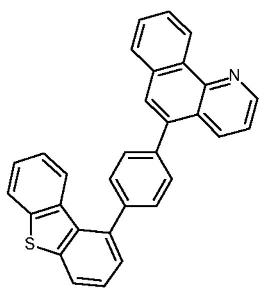 |
| 109 | 110 | 111 |

(fortgesetzt)

| | | |
|---|---|---|
| 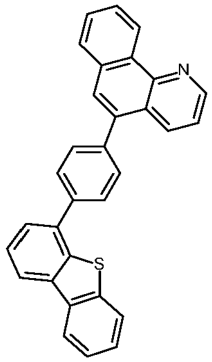 | 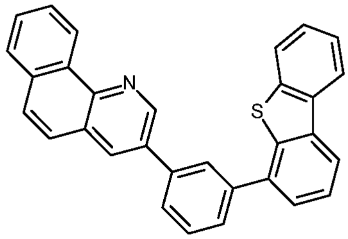 | 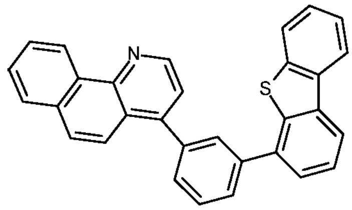 |
| 112 | 113 | 114 |
| 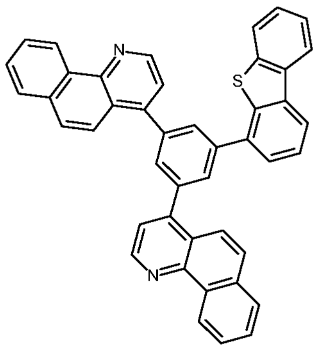 | 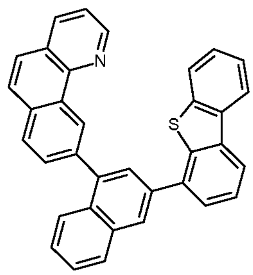 | 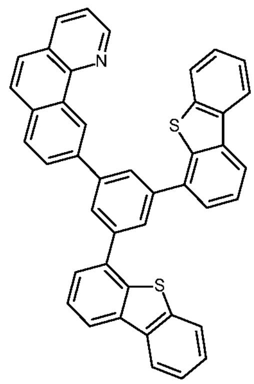 |
| 115 | 116 | 117 |
| 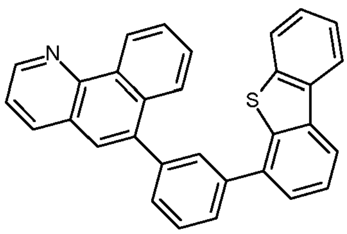 | 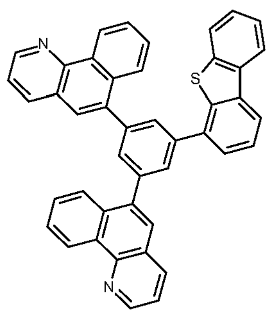 | 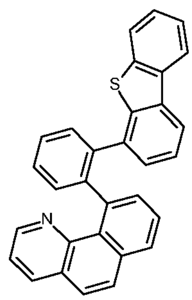 |
| 118 | 119 | 120 |
| 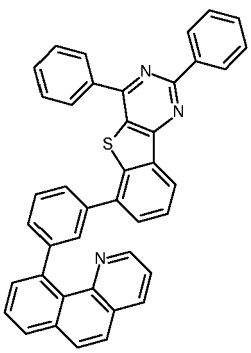 | 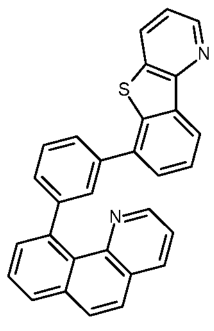 | 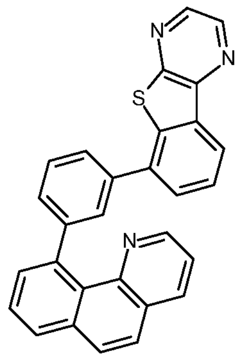 |
| 121 | 122 | 123 |

(fortgesetzt)

| | | |
|---|---|---|
| 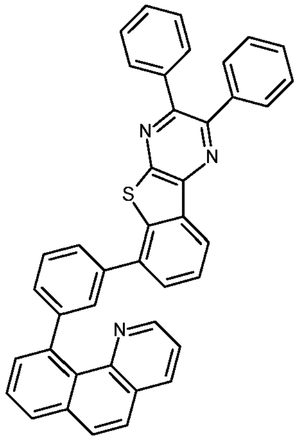 | 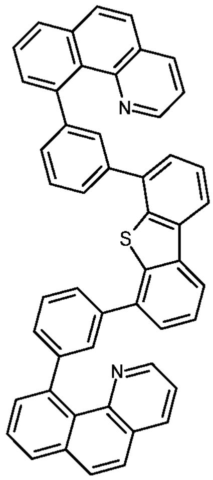 | 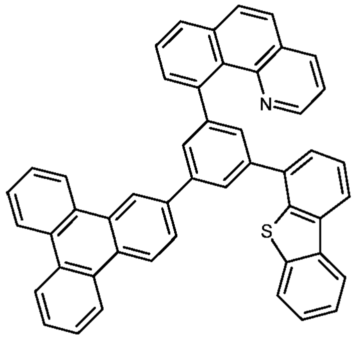 |
| 124 | 125 | 126 |
| 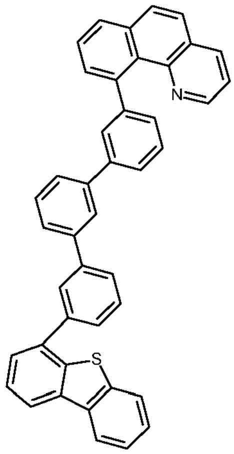 | 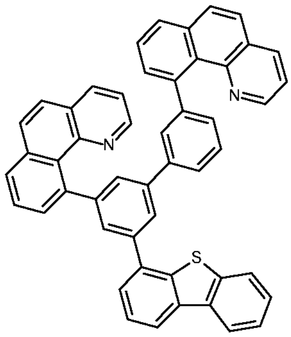 | 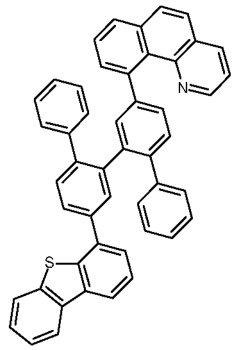 |
| 127 | 128 | 129 |
| 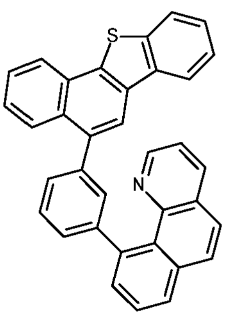 | 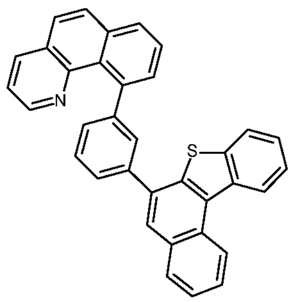 | 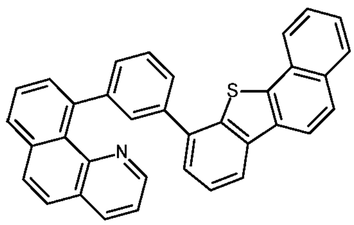 |
| 130 | 131 | 132 |

(fortgesetzt)

| | | |
|---|---|---|
| | 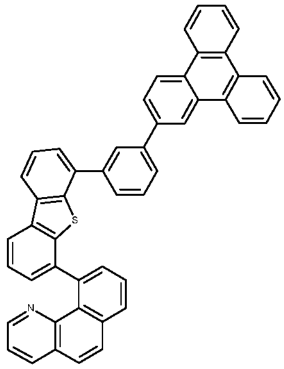 | 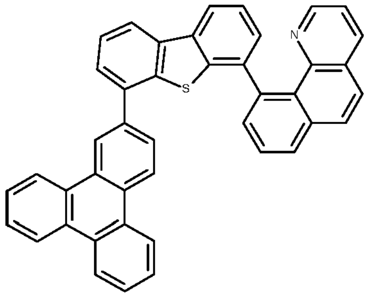 |
| | 137 | 138 |
| | | 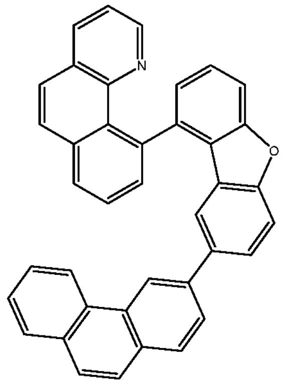 |
| | | 144 |
| | 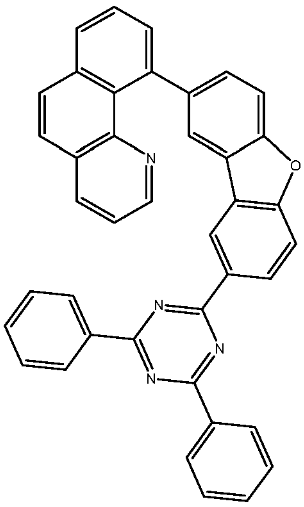 | 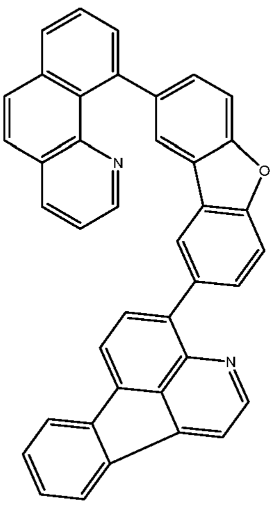 |
| | 152 | 153 |

(fortgesetzt)

| | | |
|---|---|---|
| 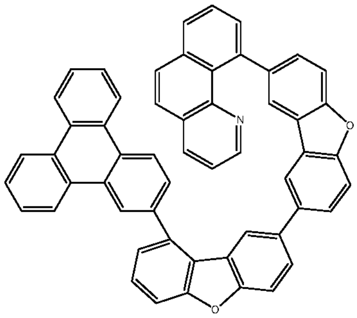 | 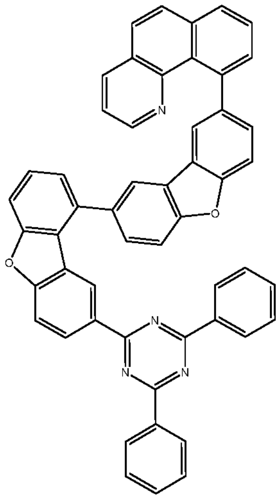 | 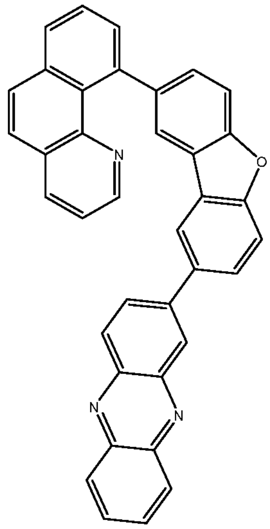 |
| 154 | 155 | 156 |
| 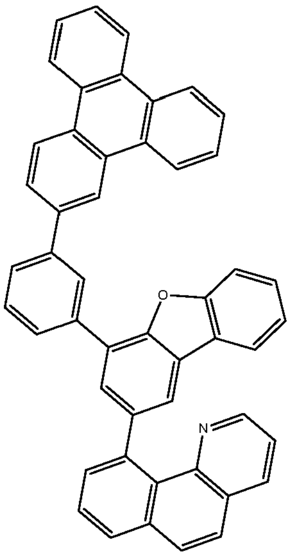 | 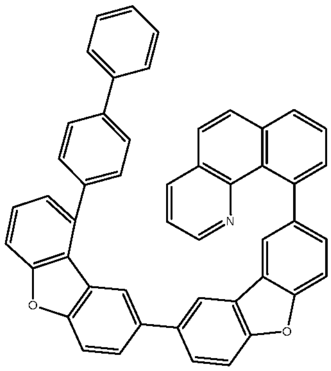 | |
| 157 | 158 | |

(fortgesetzt)

| | | |
|---|---|---|
| 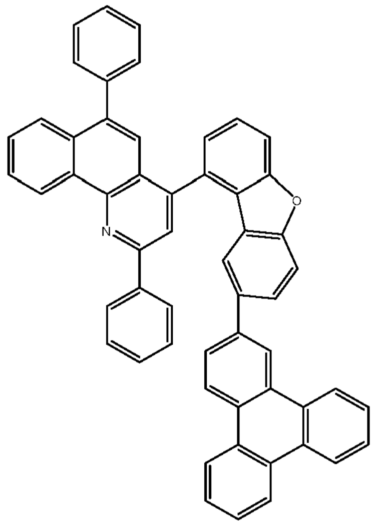 | | 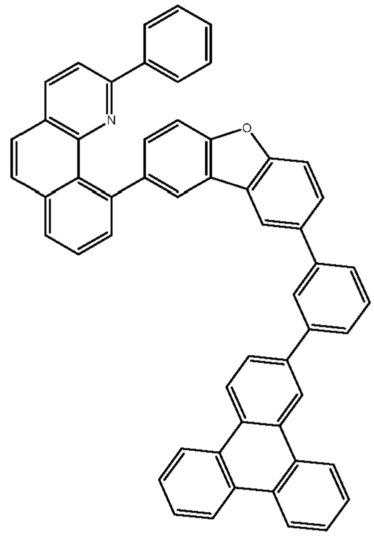 |
| 162 | | 165 |

Die Verbindung gemäß den oben genannten Formeln ist bevorzugt eine Verbindung mit einem hohen Energieabstand zwischen HOMO und LUMO, bevorzugt einem Energieabstand von 2,5 eV oder mehr, besonders bevorzugt 3 eV oder mehr, ganz besonders bevorzugt 3,5 eV oder mehr. Derartige Materialien werden als wide-bandgap-Materialien, insbesondere wide-bandgap-Matrixmaterialien bezeichnet.

Die HOMO- und LUMO- Energien werden dabei über quantenchemische Rechnungen bestimmt. Hierzu wird vorliegend das Programmpaket "Gaussian09, Revision D.01" (Gaussian Inc.) verwendet. Zur Berechnung organischer Substanzen ohne Metalle (mit Methode "org." bezeichnet) wird zuerst eine Geometrieoptimierung mit der semi-empirischen Methode AM1 (Gaussian-Eingabezeile "# AM1 opt") mit der Ladung (Charge) 0 und der Multiplizität (Multiplicity) 1 durchgeführt. Im Anschluss erfolgt auf Grundlage der optimierten Geometrie eine Energierechnung (single point) für den elektronischen Grundzustand und das Triplett-Niveau. Hierbei wird die TDDFT-Methode (time dependent density functional theory) B3PW91 mit dem Basissatz 6-31 G(d) (Gaussian-Eingabezeile "# B3PW91/6-31G(d) td=(50-50,nstates=4)") verwendet (Ladung 0, Multiplizität 1). Für metallorganische Verbindungen (mit Methode "M-org." bezeichnet) wird die Geometrie mit der Methode Hartree-Fock und dem Basissatz LanL2MB (Gaussian-Eingabezeile "# HF/LanL2MB opt") optimiert (Ladung 0, Multiplizität 1). Die Energierechnung erfolgt, wie oben beschrieben, analog zu der der organischen Substanzen, mit dem Unterschied, dass für das Metallatom der Basissatz "LanL2DZ" und für die Liganden der Basissatz "6-31G(d)" verwendet wird (Gaussian-Eingabezeile "# B3PW91/gen pseudo=lanl2 td=(50-50,nstates=4)"). Aus der Energierechnung erhält man das HOMO als das letzte mit zwei Elektronen besetzte Orbital (Alpha occ. eigenvalues) und LUMO als das erste unbesetzte Orbital (Alpha virt. eigenvalues) in Hartree-Einheiten, wobei HEh und LEh für die HOMO Energie in Hartree-Einheiten beziehungsweise für die LUMO-Energie in Hartree-Einheiten steht. Daraus wird der anhand von Cyclovoltammetriemessungen kalibrierte HOMO- und LUMO-Wert in Elektronenvolt wie folgt bestimmt:

$$\text{HOMO(eV)} = (\text{HEh}*27.212)*0.8308-1.118$$

$$\text{LUMO(eV)} = (\text{LEh}*27.212)*1.0658 -0.5049$$

Diese Werte sind im Sinne dieser Anmeldung als HOMO bzw. als LUMO der Materialien anzusehen. Der Betrag der Differenz beider Werte ist im Sinne dieser Anmeldung als Bandlücke (bandgap) anzusehen.

Die Verbindungen gemäß den oben genannten Formeln können mit bekannten Verfahren der organischen Synthesechemie hergestellt werden, insbesondere mittels Suzuki-Kupplungsreaktionen. Bevorzugte Verfahren zur Herstellung der anmeldungsgemäßen Verbindungen sind im Folgenden gezeigt.

Gemäß dem in Schema 1 gezeigten Verfahren wird ausgehend von einem Boronsäure-substituierten Benzochinolin-Derivat in einer Suzuki-Kupplung eine Einheit $Ar^1$-$Ar^2$ eingeführt, als halogeniertes Reagenz. Alternativ kann

auch das Benzochinolin-Derivat Halogen-substituiert sein, und die Einheit $Ar^1$-$Ar^2$ wird als Boronsäure-substituiertes Reagenz eingeführt.

Schema 1

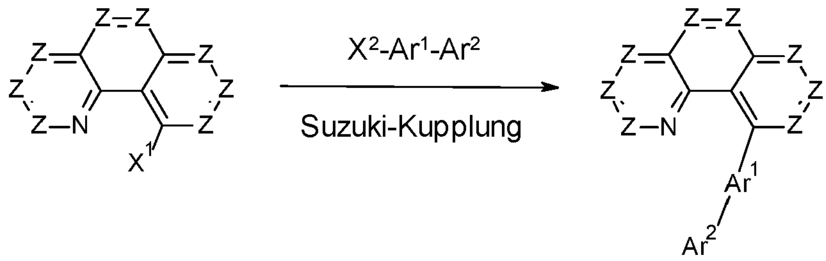


$X^1$, $X^2$ = Halogen oder Boronsäurederivat

Gemäß dem in Schema 2 gezeigten Verfahren wird zunächst ausgehend von einem Boronsäure-substituierten Benzochinolin-Derivat eine Spacer-Gruppe $Ar^1$ in einer Suzuki-Reaktion eingeführt. Letztere trägt eine weitere reaktive Gruppe, die in der ersten Suzuki-Reaktion nicht abreagiert. Dann wird in einer zweiten Suzuki-Reaktion eine Gruppe $Ar^2$ eingeführt.

Schema 2

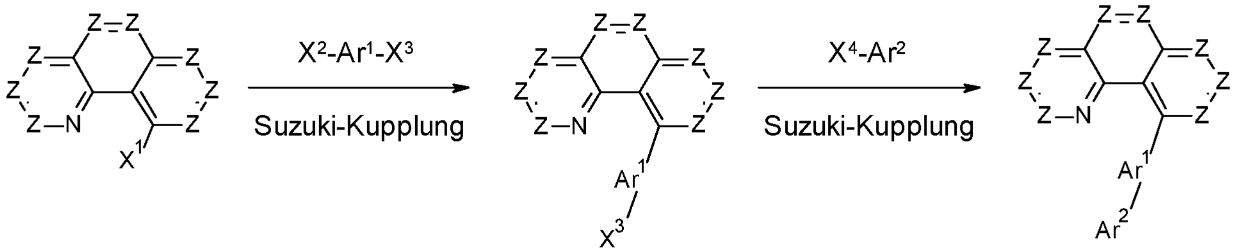


$X^1$, $X^2$, $X^3$, $X^4$ = Halogen oder Boronsäurederivat

Mit entsprechenden Verfahren können auch Verbindungen der Formel (II-A) hergestellt werden.

Gemäß einer weiteren Variation des erfindungsgemäßen Verfahrens können anstelle von Verbindungen

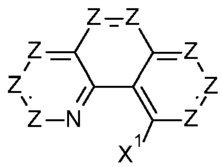

alternative Verbindungen verwendet werden, die die Gruppe $X^1$ an einer beliebigen anderen Position am Grundkörper tragen, beispielsweise Verbindungen der folgenden Formel

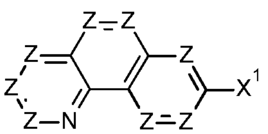

.

Weiterer Gegenstand der vorliegenden Anmeldung ist damit ein Verfahren zur Herstellung einer anmeldungsgemäßen Verbindung, dadurch gekennzeichnet, dass eine Verbindung der Formel (Z)

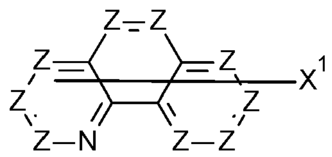

Formel (Z),

wobei $X^1$ gewählt ist aus $B(OR^4)_2$, Cl, Br und I, in einer Suzuki-Reaktion umgesetzt wird.

Die oben beschriebenen Verbindungen, insbesondere Verbindungen, welche mit reaktiven Abgangsgruppen, wie Brom, Iod, Chlor, Boronsäure oder Boronsäureester, substituiert sind, können als Monomere zur Erzeugung entsprechender Oligomere, Dendrimere oder Polymere Verwendung finden. Geeignete reaktive Abgangsgruppen sind beispielsweise Brom, Iod, Chlor, Boronsäuren, Boronsäureester, Amine, Alkenyl- oder Alkinylgruppen mit endständiger C-C-Doppelbindung bzw. C-C-Dreifachbindung, Oxirane, Oxetane, Gruppen, die eine Cycloaddition, beispielsweise eine 1,3-dipolare Cycloaddition, eingehen, wie beispielsweise Diene oder Azide, Carbonsäurederivate, Alkohole und Silane.

Weiterer Gegenstand der Erfindung sind daher Oligomere, Polymere oder Dendrimere, enthaltend eine oder mehrere Verbindungen gemäß den oben genannten Formeln, wobei die Bindung(en) zum Polymer, Oligomer oder Dendrimer an beliebigen, in den Formeln mit $R^1$, $R^2$ oder $R^3$ substituierten Positionen lokalisiert sein können. Je nach Verknüpfung der Verbindung ist die Verbindung Bestandteil einer Seitenkette des Oligomers oder Polymers oder Bestandteil der Hauptkette. Unter einem Oligomer im Sinne dieser Erfindung wird eine Verbindung verstanden, welche aus mindestens drei Monomereinheiten aufgebaut ist. Unter einem Polymer im Sinne der Erfindung wird eine Verbindung verstanden, die aus mindestens zehn Monomereinheiten aufgebaut ist. Die erfindungsgemäßen Polymere, Oligomere oder Dendrimere können konjugiert, teilkonjugiert oder nichtkonjugiert sein. Die erfindungsgemäßen Oligomere oder Polymere können linear, verzweigt oder dendritisch sein. In den linear verknüpften Strukturen können die Einheiten den oben genannten Formeln direkt miteinander verknüpft sein oder sie können über eine bivalente Gruppe, beispielsweise über eine substituierte oder unsubstituierte Alkylengruppe, über ein Heteroatom oder über eine bivalente aromatische oder heteroaromatische Gruppe miteinander verknüpft sein. In verzweigten und dendritischen Strukturen können beispielsweise drei oder mehrere Einheiten gemäß den oben genannten Formeln über eine trivalente oder höhervalente Gruppe, beispielsweise über eine trivalente oder höhervalente aromatische oder heteroaromatische Gruppe, zu einem verzweigten bzw. dendritischen Oligomer oder Polymer verknüpft sein.

Für die Wiederholeinheiten gemäß den oben genannten Formeln in Oligomeren, Dendrimeren und Polymeren gelten dieselben Bevorzugungen wie oben für Verbindungen gemäß den oben genannten Formeln beschrieben.

Zur Herstellung der Oligomere oder Polymere werden die erfindungsgemäßen Monomere homopolymerisiert oder mit weiteren Monomeren copolymerisiert. Geeignete und bevorzugte Comonomere sind gewählt aus Fluorenen, Spirobifluorenen, Paraphenylenen, Carbazolen, Thiophenen, Dihydrophenanthrenen, cis- und trans-Indenofluorenen, Ketonen, Phenanthrenen, oder mehreren dieser Einheiten. Die Polymere, Oligomere und Dendrimere enthalten üblicherweise noch weitere Einheiten, beispielsweise emittierende (fluoreszierende oder phosphoreszierende) Einheiten, wie z. B. Vinyltriarylamine oder phosphoreszierende Metallkomplexe, und/oder Ladungstransporteinheiten, insbesondere solche basierend auf Triarylaminen.

Die erfindungsgemäßen Polymere und Oligomere werden in der Regel durch Polymerisation von einer oder mehreren Monomersorten hergestellt, von denen mindestens ein Monomer im Polymer zu Wiederholungseinheiten gemäß den oben genannten Formeln führt. Geeignete Polymerisationsreaktionen sind dem Fachmann bekannt und in der Literatur beschrieben. Besonders geeignete und bevorzugte Polymerisationsreaktionen, die zu C-C- bzw. C-N-Verknüpfungen führen, sind die Suzuki-Polymerisation, die Yamamoto-Polymerisation; die Stille-Polymerisation; und die Hartwig-Buchwald-Polymerisation.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, $\alpha$-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropylenglycoldimethylether,

Tetraethylenglycoldimethylether, 2-Isopropylnaphthalin, Pentylbenzol, Hexylbenzol, Heptylbenzol, Octylbenzol, 1,1-Bis(3,4-Dimethylphenyl)ethan oder Mischungen dieser Lösemittel.

Gegenstand der Erfindung ist daher weiterhin eine Formulierung, insbesondere eine Lösung, Dispersion oder Emulsion, enthaltend mindestens eine Verbindung gemäß den oben genannten Formeln sowie mindestens ein Lösungsmittel, bevorzugt ein organisches Lösungsmittel. Wie solche Lösungen hergestellt werden können, ist dem Fachmann bekannt und beispielsweise in WO 2002/072714, WO 2003/019694 und der darin zitierten Literatur beschrieben.

Gemäß einer bevorzugten Ausführungsform der Erfindung enthält die Formulierung außer der anmeldungsgemäßen Verbindung noch mindestens ein weiteres Matrixmaterial und mindestens einen phosphoreszierenden Emitter. Dabei sind das mindestens eine weitere Matrixmaterial und der mindestens eine phosphoreszierende Emitter jeweils gewählt aus den unten als bevorzugt angegebenen Ausführungsformen. Nach Aufbringen und Verdampfen des Lösungsmittels aus der Formulierung verbleibt die Mischung der Materialien als phosphoreszierende emittierende Schicht mit einer gemischten Matrix (mixed matrix).

Die anmeldungsgemäßen Verbindungen eignen sich für den Einsatz in elektronischen Vorrichtungen, insbesondere in organischen Elektrolumineszenzvorrichtungen (OLEDs). Abhängig von der Substitution werden die Verbindungen in unterschiedlichen Funktionen und Schichten eingesetzt.

Weiterer Gegenstand der Erfindung ist daher die Verwendung der anmeldungsgemäßen Verbindungen in elektronischen Vorrichtungen. Dabei sind die elektronischen Vorrichtungen bevorzugt ausgewählt aus der Gruppe bestehend aus organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs), organischen Laserdioden (O-Laser) und besonders bevorzugt organischen Elektrolumineszenzvorrichtungen (OLEDs).

Weiterer Gegenstand der Erfindung ist, wie bereits oben ausgeführt, eine elektronische Vorrichtung, enthaltend mindestens eine Verbindung wie oben definiert. Dabei ist die elektronische Vorrichtung bevorzugt ausgewählt aus den oben genannten Vorrichtungen.

Besonders bevorzugt ist sie eine organische Elektrolumineszenzvorrichtung (OLED), enthaltend Anode, Kathode und mindestens eine emittierende Schicht, dadurch gekennzeichnet, dass mindestens eine organische Schicht, die bevorzugt gewählt ist aus emittierenden Schichten, Elektronentransportschichten und Lochblockierschichten, und die besonders bevorzugt gewählt ist aus emittierenden Schichten, ganz besonders phosphoreszierenden emittierenden Schichten, mindestens eine Verbindung wie oben definiert enthält.

Außer Kathode, Anode und mindestens einer emittierenden Schicht kann die organische Elektrolumineszenzvorrichtung noch weitere Schichten enthalten. Diese sind beispielsweise gewählt aus jeweils einer oder mehreren Lochinjektionsschichten, Lochtransportschichten, Lochblockierschichten, Elektronentransportschichten, Elektroneninjektionsschichten, Elektronenblockierschichten, Excitonenblockierschichten, Zwischenschichten (Interlayers), Ladungserzeugungsschichten (Charge-Generation Layers) und/oder organischen oder anorganischen p/n-Übergängen.

Die Abfolge der Schichten der organischen Elektrolumineszenzvorrichtung ist bevorzugt die folgende: Anode-Lochinjektionsschicht-Lochtransportschicht-wahlweise weitere Lochtransportschicht(en)-wahlweise Elektronenblockierschichtemittierende Schicht-wahlweise Lochblockierschicht-Elektronentransportschicht-wahlweise weitere Elektronentransportschicht(en)-Elektroneninjektionsschicht-Kathode. Es können zusätzlich weitere Schichten in der OLED vorhanden sein.

Es ist bevorzugt, wenn mindestens eine lochtransportierende Schicht der Vorrichtung p-dotiert ist, also mindestens einen p-Dotanden enthält. P-Dotanden sind bevorzugt gewählt aus Elektronenakzeptor-Verbindungen. Besonders bevorzugt als p-Dotanden sind Chinodimethanverbindungen, Azaindenofluorendione, Azaphenalene, Azatriphenylene, $I_2$, Metallhalogenide, bevorzugt Übergangsmetallhalogenide, Metalloxide, bevorzugt Metalloxide enthaltend mindestens ein Übergangsmetall oder ein Metall der 3. Hauptgruppe, und Übergangsmetallkomplexe, bevorzugt Komplexe von Cu, Co, Ni, Pd und Pt mit Liganden enthaltend mindestens ein Sauerstoffatom als Bindungsstelle. Bevorzugt sind weiterhin Übergangsmetalloxide als Dotanden, bevorzugt Oxide von Rhenium, Molybdän und Wolfram, besonders bevorzugt $Re_2O_7$, MoOs, $WO_3$ und ReOs. Weiterhin bevorzugt sind Bismuth-Komplexe, insbesondere Bi(III)-Komplexe, insbesondere Bismuth-Komplexe mit Benzoesäure-Derivaten als Komplexliganden.

Die erfindungsgemäße organische Elektrolumineszenzvorrichtung kann mehrere emittierende Schichten enthalten. Besonders bevorzugt weisen diese Emissionsschichten insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues, grünes, gelbes, orangefarbenes oder rotes Licht emittieren. Insbesondere bevorzugt sind Dreischichtsysteme, also Systeme mit drei emittierenden Schichten, wobei jeweils eine der drei Schichten blaue, jeweils eine der drei Schichten grüne und jeweils eine der drei Schichten orangefarbene oder rote Emission zeigt. Die erfindungsgemäßen Verbindungen sind dabei bevorzugt in der emittierenden Schicht vorhanden. Für die Erzeugung von weißem Licht kann anstelle mehrerer farbig emittierender Emitterverbindungen auch eine einzeln verwendete Emitterverbindung geeignet sein, welche in

einem breiten Wellenlängenbereich emittiert.

Es ist erfindungsgemäß bevorzugt, wenn die Verbindungen in einer elektronischen Vorrichtung eingesetzt werden, die eine oder mehrere phosphoreszierende emittierende Verbindungen in einer emittierenden Schicht enthält. Dabei sind die Verbindungen bevorzugt in der emittierenden Schicht in Kombination mit der phosphoreszierenden emittierenden Verbindung enthalten, besonders bevorzugt in Mischung mit mindestens einem weiteren Matrixmaterial. Dieses ist bevorzugt gewählt aus lochleitenden Matrixmaterialien, elektronenleitenden Matrixmaterialien und Matrixmaterialien, welche sowohl lochleitende Eigenschaften als auch elektronenleitende Eigenschaften aufweisen (bipolare Matrixmaterialien).

Vom Begriff phosphoreszierende emittierende Verbindungen sollen bevorzugt solche Verbindungen als umfasst gelten, bei denen die Lichtemission durch einen spin-verbotenen Übergang erfolgt, beispielsweise einen Übergang aus einem angeregten Triplettzustand oder einem Zustand mit einer höheren Spinquantenzahl, beispielsweise einem Quintett-Zustand.

In einer bevorzugten Ausführungsform der vorliegenden Erfindung wird die Verbindung gemäß den oben genannten Formeln in einer emittierenden Schicht als Matrixmaterial in Kombination mit einer oder mehreren phosphoreszierenden emittierenden Verbindungen eingesetzt. Die phosphoreszierende emittierende Verbindung ist dabei bevorzugt ein rot oder grün phosphoreszierender Emitter, besonders bevorzugt ein grün phosphoreszierender Emitter.

Der Gesamtanteil aller Matrixmaterialien in der phosphoreszierenden emittierenden Schicht beträgt in diesem Fall zwischen 50.0 und 99.9 Vol.-%, bevorzugt zwischen 80.0 und 99.5 Vol.-% und besonders bevorzugt zwischen 85.0 und 97.0 Vol.-%.

Entsprechend beträgt der Anteil der phosphoreszierenden emittierenden Verbindung zwischen 0.1 und 50.0 Vol.-%, bevorzugt zwischen 0.5 und 20.0 Vol.-% und besonders bevorzugt für zwischen 3.0 und 15.0 Vol.-%.

Die emittierende Schicht der organischen Elektrolumineszenzvorrichtung umfasst bevorzugt zwei oder mehr Matrixmaterialien (Mixed-Matrix-Systeme). Die Mixed-Matrix-Systeme umfassen bevorzugt zwei oder drei verschiedene Matrixmaterialien, besonders bevorzugt zwei verschiedene Matrixmaterialien.

Gemäß einer bevorzugten Ausführungform erfüllt dabei eines der beiden Matrixmaterialien die Funktion eines lochtransportierenden Materials, und das andere der beiden Matrixmaterialien erfüllt die Funktion eines elektronentransportierenden Materials.

Gemäß einer weiteren bevorzugten Ausführungsform der Erfindung stellt eines der beiden Materialien ein wide-bandgap-Material dar, und es sind ein oder zwei weitere Matrixmaterialien in der emittierenden Schicht vorhanden, durch die eine elektronentransportierende Funktion und/oder eine lochtransportierende Funktion der mixed Matrix erfüllt werden. Dies kann gemäß einer bevorzugten Ausführungsform dadurch geschehen, dass neben dem wide-bandgap-Material ein weiteres Matrixmaterial in der emittierenden Schicht vorhanden ist, welches elektronentransportierende Eigenschaften aufweist, und ein nochmals weiteres Matrixmaterial in der emittierenden Schicht vorhanden ist, welches lochtransportierende Eigenschaften aufweist. Alternativ und besonders bevorzugt kann dies dadurch geschehen, dass neben dem wide-bandgap-Material ein einziges weiteres Matrixmaterial in der emittierenden Schicht vorhanden ist, welches sowohl elektronentransportierende als auch lochtransportierende Eigenschaften aufweist. Derartige Matrixmaterialien werden auch als bipolare Matrixmaterialien bezeichnet.

Das bipolare Matrixmaterial zur Verwendung in Kombination mit der anmeldungsgemäßen Verbindung in einer phosphoreszierenden emittierenden Schicht ist bevorzugt gewählt aus Verbindungen enthaltend mindestens eine Triazingruppe. Besonders bevorzugt sind die in der noch nicht offengelegten Anmeldung EP17201480.5 offenbarten TriazinVerbindungen der Formel (1). Die diesbezügliche Offenbarung dieser Anmeldung wird hiermit in die vorliegende Anmeldung miteinbezogen.

Ganz besonders bevorzugt sind Verbindungen der unten gezeigten Formeln

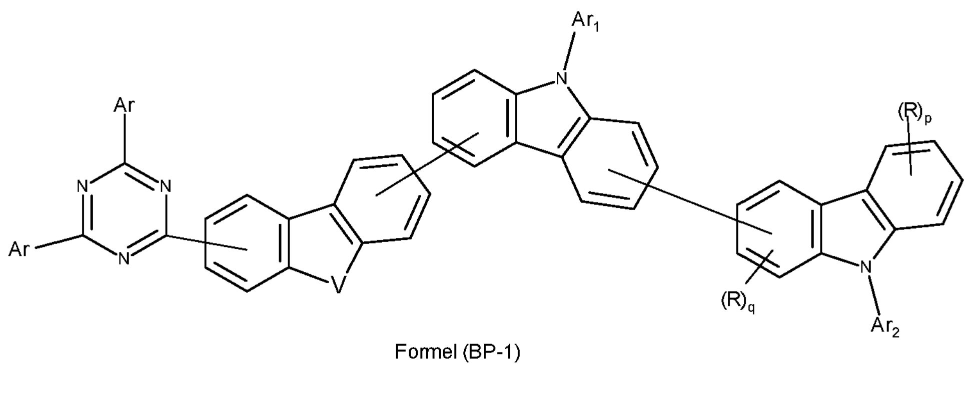

Formel (BP-1)

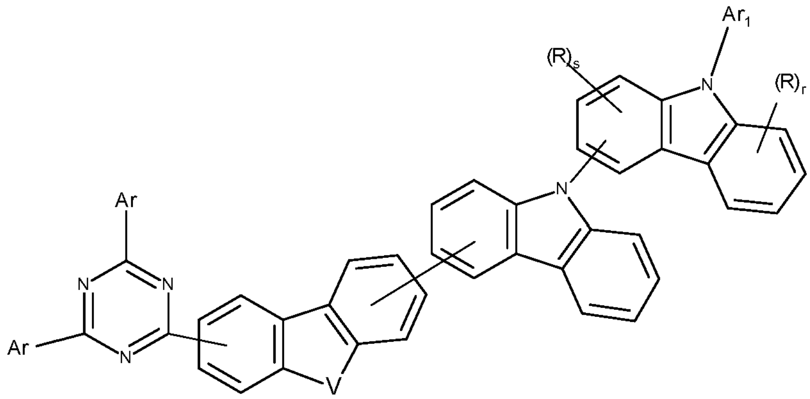

Formel (BP-2))

wobei für die auftretenden Variablen gilt:

V ist O oder S;

Ar, $Ar_1$, $Ar_2$ sind jeweils unabhängig voneinander bei jedem Auftreten eine Aryl- oder Heteroarylgruppe mit 5 bis 40 Ringatomen, die mit einem oder mehreren Resten $R^3$ substituiert sein können oder ein aromatisches oder heteroaromatisches Ringsystem mit 6 bis 40 Ringatomen, das mit einem oder mehreren Resten $R^3$ substituiert sein kann;

p, q sind jeweils unabhängig voneinander 0, 1, 2, 3 oder 4;

s, r sind jeweils unabhängig voneinander 0, 1, 2, 3 oder 4;

R ist bei jedem Auftreten gleich oder verschieden ausgewählt aus der Gruppe bestehend aus D, F, Cl, Br, I, CN, $NO_2$, $N(Ar)_2$, $N(R_2)_2$, C(=O)Ar, $C(=O)R_2$, $P(=O)(Ar)_2$, $P(Ar)_2$, $B(Ar)_2$, $Si(Ar)_3$, $Si(R_2)_3$, einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen oder einer Alkenylgruppe mit 2 bis 20 C-Atomen, die jeweils mit einem oder mehreren Resten $R^2$ substituiert sein kann, wobei eine oder mehrere nicht-benachbarte $CH_2$-Gruppen durch $R_2C=CR_2$, $Si(R_2)_2$, C=O, C=S, $C=NR_2$, $P(=O)(R_2)$, SO, $SO_2$, $NR_2$, O, S oder $CONR_2$ ersetzt sein können und wobei ein oder mehrere H-Atome durch D, F, Cl, Br, I, CN oder $NO_2$ ersetzt sein können, einem aromatischen oder heteroaromatischen Ringsystem mit 5 bis 40 Ringatomen, das jeweils mit einem oder mehreren Resten $R^2$ substituiert sein kann, einer Aryloxy- oder Heteroaryloxygruppe mit 5 bis 40 Ringatomen, die mit einem oder mehreren Resten $R^2$ substituiert sein kann, oder einer Aralkyl- oder Heteroaralkylgruppe mit 5 bis 40 Ringatomen, die mit einem oder mehreren Resten $R^2$ substituiert sein kann; dabei kann maximal ein Substituent R zusammen mit $Ar_1$ ein monocyclisches oder polycyclisches, aliphatisches, aromatisches oder heteroaromatisches Ringsystem bilden, das mit einem oder mehreren Resten $R^2$ substituiert sein kann;

$R^2$ ist bei jedem Auftreten gleich oder verschieden ausgewählt aus der Gruppe bestehend aus H, D, F, Cl, Br, I, CN, $NO_2$, $N(Ar)_2$, $NH_2$, $N(R_3)_2$, C(=O)Ar, C(=O)H, $C(=O)R_3$, $P(=O)(Ar)_2$, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, die jeweils mit einem oder mehreren Resten $R^3$ substituiert sein kann, wobei eine oder mehrere nicht-benachbarte $CH_2$-Gruppen durch HC=CH, $R_3C=CR_3$, C=C, $Si(R_3)_2$, $Ge(R_3)_2$, $Sn(R_3)_2$, C=O, C=S, C=Se, $C=NR_3$, $P(=O)(R_3)$, SO, $SO_2$, NH, $NR_3$, O, S, CONH oder $CONR_3$ ersetzt sein können und wobei ein oder mehrere H-Atome durch D, F, Cl, Br, I, CN oder $NO_2$ ersetzt sein können, einem aromatischen oder heteroaromatischen Ringsystem mit 5 bis 60 Ringatomen, das jeweils mit einem oder mehreren Resten $R^3$ substituiert sein kann, einer Aryloxy- oder Heteroaryloxygruppe mit 5 bis 60 Ringatomen, die mit einem oder mehreren Resten $R^3$ substituiert sein kann, oder einer Kombination dieser Systeme, wobei optional zwei oder mehr benachbarte Substituenten $R^2$ ein monocyclisches oder polycyclisches, aliphatisches, aromatisches oder heteroaromatisches Ringsystem bilden können, das mit einem oder mehreren Resten $R^3$ substituiert sein kann;

$R^3$ ist bei jedem Auftreten gleich oder verschieden ausgewählt aus der Gruppe bestehend aus H, D, F, CN, einem aliphatischen Kohlenwasserstoffrest mit 1 bis 20 C-Atomen oder einem aromatischen oder heteroaromatischen Ringsystem mit 5 bis 30 Ringatomen, in dem ein oder mehrere H-Atome durch D, F, Cl, Br, I oder CN ersetzt sein können und das durch ein oder mehrere Alkylgruppen mit jeweils 1 bis 4 Kohlenstoffatomen substituiert sein kann; dabei können zwei oder mehr benachbarte Substituenten $R^3$ miteinander ein mono- oder polycyclisches, aliphatisches Ringsystem bilden.

Gemäß einer nochmals alternativen Ausführungsform kann neben dem wide-bandgap-Matrixmaterial lediglich ein einziges weiteres Matrixmaterial in der emittierenden Schicht vorhanden sein, welches entweder überwiegend lochtransportierende Eigenschaften oder überwiegend elektronentransportierende Eigenschaften aufweist.

Im bevorzugten Fall, dass in der emittierenden Schicht zwei verschiedene Matrixmaterialien vorliegen, können diese in einem Volumenverhältnis von 1:50 bis 1:1, bevorzugt 1:20 bis 1:1, besonders bevorzugt 1:10 bis 1:1 und ganz besonders bevorzugt 1:4 bis 1:1 vorliegen. Bevorzugt liegt dabei die Verbindung gemäß den oben genannten Formeln im gleichen Anteil wie die weitere Matrixverbindung vor, oder sie liegt in einem höheren Anteil als die weitere Matrixverbindung vor. Bevorzugt beträgt das Verhältnis von Verbindung gemäß oben genannten Formeln (M1) und weiterer Matrixverbindung (M2), gleich M1:M2 zwischen 4:1 und 1:1.

Der absolute Anteil der Verbindung gemäß den oben genannten Formeln in der Mischung der emittierenden Schicht, bei Verwendung als Matrixmaterial in einer phosphoreszierenden emittierenden Schicht, beträgt bevorzugt 10 Vol.-% bis 85 Vol.-%, mehr bevorzugt 20 Vol.-% bis 85 Vol.-%, noch mehr bevorzugt 30 Vol.-% bis 80 Vol.-%, ganz besonders bevorzugt 20 Vol.-% bis 60 Vol.-% und am meisten bevorzugt 30 Vol.-% bis 50 Vol.-%. Der absolute Anteil der zweiten Matrixverbindung ist in diesem Fall bevorzugt 15 Vol.-% bis 90 Vol.-%, mehr bevorzugt 15 Vol.-% bis 80 Vol.-%, noch mehr bevorzugt 20 Vol.-% bis 70 Vol.-%, ganz besonders bevorzugt 40 Vol.-% bis 80 Vol.-%, und am meisten bevorzugt 50 Vol.-% bis 70 Vol.-%.

Zur Herstellung von phosphoreszierenden emittierenden Schichten des mixed-Matrix-Typs kann gemäß einer bevorzugten Ausführungsform der Erfindung eine Lösung enthaltend den phosphoreszierenden Emitter und die zwei oder mehr Matrixmaterialien hergestellt werden. Diese kann mittels Spincoating, Druckverfahren oder anderen Verfahren aufgebracht werden. Nach Verdampfen des Lösungsmittels verbleibt in diesem Fall die phosphoreszierende emittierende Schicht des mixed-Matrix-Typs.

Gemäß einer alternativen, stärker bevorzugten Ausführungsform der Erfindung wird die phosphoreszierende emittierende Schicht des mixed-Matrix-Typs durch Gasphasenabscheidung hergestellt. Hierzu bestehen zwei Möglichkeiten, wie die Schicht aufgebracht werden kann. Zum einen kann jedes der mindestens zwei verschiedenen Matrixmaterialien jeweils in einer Materialquelle vorgelegt werden, woraufhin aus den zwei oder mehr verschiedenen Materialquellen gleichzeitig verdampft wird ("co-evaporation"). Zum anderen können die mindestens zwei Matrixmaterialien vorgemischt und das erhaltene Gemisch in einer einzigen Materialquelle vorgelegt werden, aus der es schließlich verdampft wird. Letzteres Verfahren wird als Premix-Verfahren bezeichnet.

Gegenstand der vorliegenden Anmeldung ist daher auch eine Mischung enthaltend eine Verbindung gemäß den oben angegebenen Formeln sowie mindestens eine weitere Verbindung, die gewählt ist aus Matrixverbindungen, und bevorzugt gewählt ist aus den oben angegebenen bipolaren Matrixverbindungen, insbesondere aus Verbindungen der Formeln (BP-1) und (BP-2). Hierfür gelten die in dieser Anmeldung angegebenen bevorzugten Ausführungsformen bezüglich Anteilen der Matrixverbindungen und ihrer chemischen Struktur ebenfalls als bevorzugt.

In einer alternativen bevorzugten Ausführungsform der Erfindung wird die Verbindung als elektronentransportierendes Material eingesetzt. Dies gilt insbesondere, wenn die Verbindung mindestens eine Gruppe gewählt aus elektronenarmen Heteroarylgruppen, bevorzugt Azingruppen, inbesondere Triazingruppen, Pyrimidingruppen und Pyridingruppen, und Benzimidazolgruppen enthält.

Wenn die Verbindung als elektronentransportierendes Material eingesetzt wird, dann wird sie bevorzugt in einer Lochblockierschicht, einer Elektronentransportschicht oder in einer Elektroneninjektionsschicht eingesetzt. In einer bevorzugten Ausführungsform ist die genannte Schicht dabei n-dotiert. Die Verbindung kann in der betreffenden Schicht aber auch als Reinmaterial vorliegen.

Unter einem n-Dotanden wird vorliegend eine organische oder anorganische Verbindung verstanden, die in der Lage ist, Elektronen abzugeben (Elektronendonator), d.h. eine Verbindung, die als Reduktionsmittel wirkt. Die zur n-Dotierung eingesetzten Verbindungen können als Precursor eingesetzt werden, wobei diese Precursor-Verbindungen durch Aktivierung n-Dotanden freisetzen. Bevorzugt sind n-Dotanden ausgewählt aus elektronenreichen Metallkomplexen; P=N-Verbindungen; N-Heterocyclen, besonders bevorzugt Naphthylencarbodiimiden, Pyridinen, Acridinen und Phenazinen; Fluorenen und Radikal-Verbindungen.

Im Folgenden werden bevorzugte Ausführungsformen für die unterschiedlichen Funktionsmaterialien der elektronischen Vorrichtung aufgeführt.

Bevorzugte fluoreszierende emittierende Verbindungen sind ausgewählt aus der Klasse der Arylamine. Unter einem Arylamin bzw. einem aromatischen Amin im Sinne dieser Erfindung wird eine Verbindung verstanden, die drei

substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind. Weitere bevorzugte emittierende Verbindungen sind Indenofluorenamine bzw. - diamine, Benzoindenofluorenamine bzw. -diamine, und Dibenzoindenofluorenamine bzw. -diamine, sowie Indenofluorenderivate mit kondensierten Arylgruppen. Ebenfalls bevorzugt sind Pyren-Arylamine.

Ebenfalls bevorzugt sind Benzoindenofluoren-Amine, Benzofluoren-Amine, erweiterte Benzoindenofluorene, Phenoxazine, und Fluoren-Derivate, die mit Furan-Einheiten oder mit Thiophen-Einheiten verbunden sind.

Bevorzugte Matrixmaterialien für fluoreszierende Emitter sind ausgewählt aus den Klassen der Oligoarylene (z. B. 2,2‘,7,7‘-Tetraphenylspirobifluoren), insbesondere der Oligoarylene enthaltend kondensierte aromatische Gruppen, der Oligoarylenvinylene, der polypodalen Metallkomplexe, der lochleitenden Verbindungen, der elektronenleitenden Verbindungen, insbesondere Ketone, Phosphinoxide, und Sulfoxide; der Atropisomere, der Boronsäurederivate oder der Benzanthracene. Besonders bevorzugte Matrixmaterialien sind ausgewählt aus den Klassen der Oligoarylene, enthaltend Naphthalin, Anthracen, Benzanthracen und/oder Pyren oder Atropisomere dieser Verbindungen, der Oligoarylenvinylene, der Ketone, der Phosphinoxide und der Sulfoxide. Ganz besonders bevorzugte Matrixmaterialien sind ausgewählt aus den Klassen der Oligoarylene, enthaltend Anthracen, Benzanthracen, Benzphenanthren und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind.

Als phosphoreszierende emittierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als phosphoreszierende emittierende Verbindungen Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten. Dabei werden im Sinne der vorliegenden Erfindung alle lumineszierenden Iridium-, Platin- oder Kupferkomplexe als phosphoreszierende emittierende Verbindungen angesehen.

Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind. Explizite Beispiele für besonders geeignete Komplexe sind in der folgenden Tabelle aufgeführt.

| | | |
|---|---|---|
| 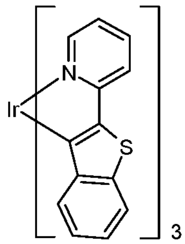 | 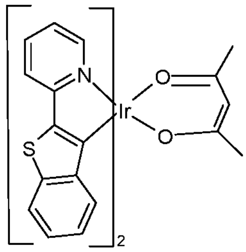 | 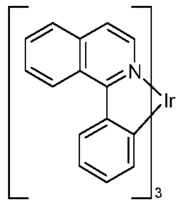 |
| 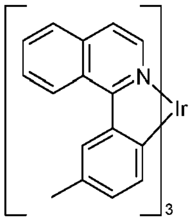 | 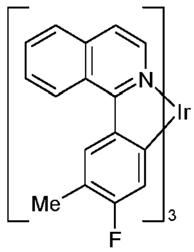 | 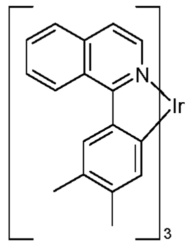 |
| 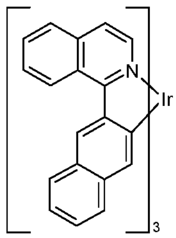 | 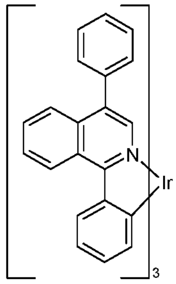 | 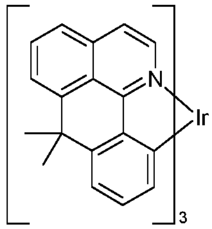 |
| 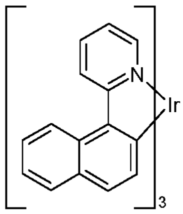 | 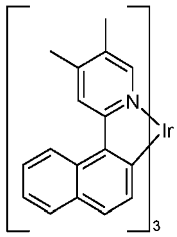 | 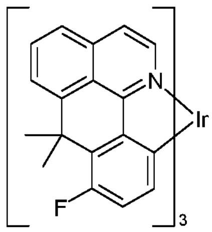 |
| 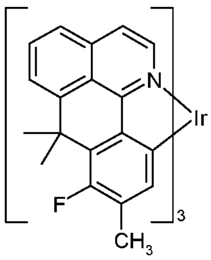 | 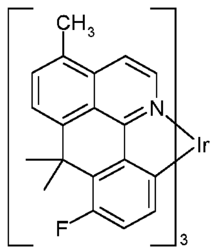 | 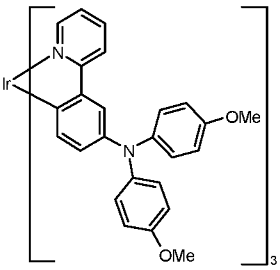 |

| | | |
|---|---|---|
| 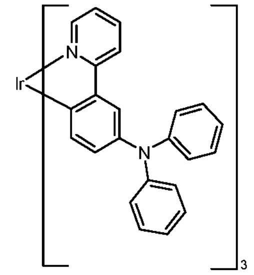 | 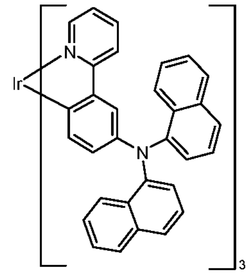 | 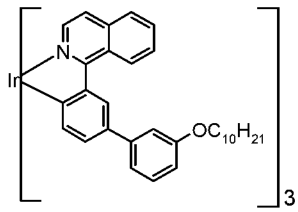 |
| 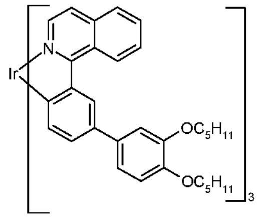 | 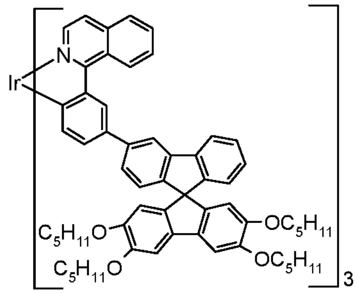 | 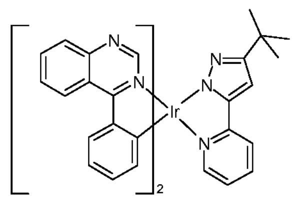 |
| 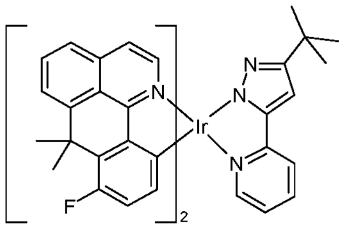 | 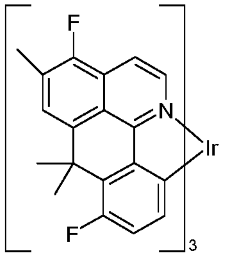 | 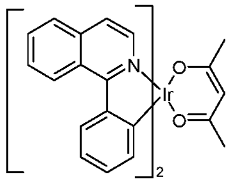 |
| 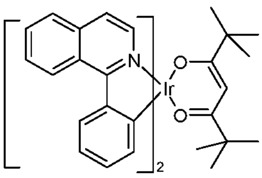 | 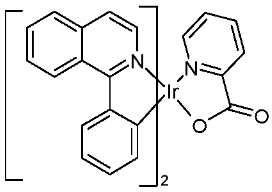 | 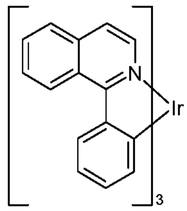 |
| 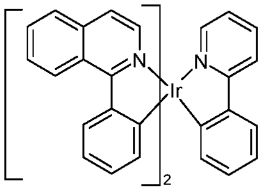 | 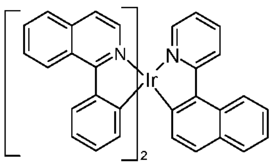 | 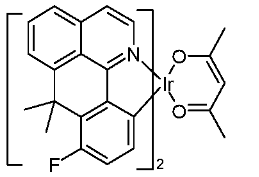 |
| 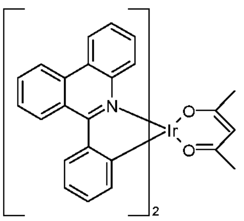 | 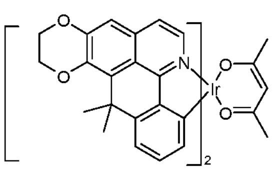 | 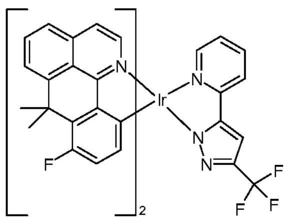 |

| | | |
|---|---|---|
| 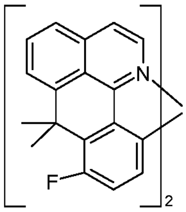 | 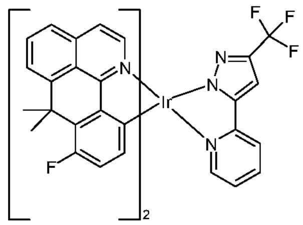 | 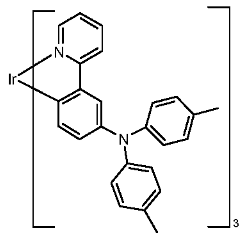 |
| 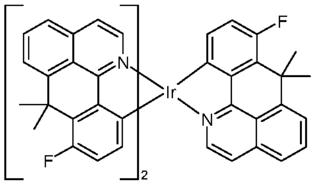 | 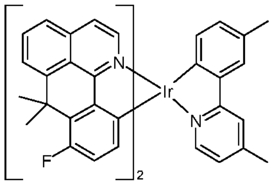 | 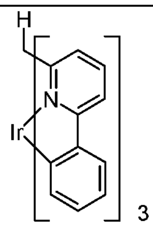 |
| 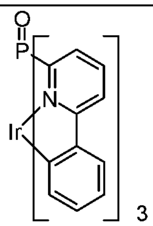 | 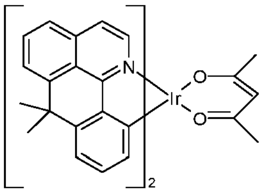 | 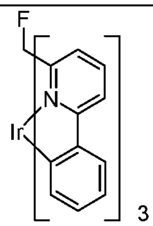 |
| 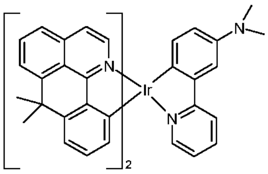 | 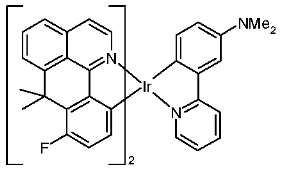 | 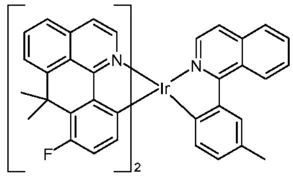 |
| 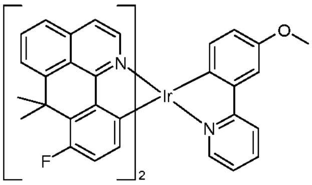 | 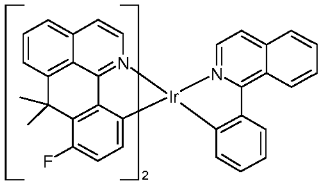 | 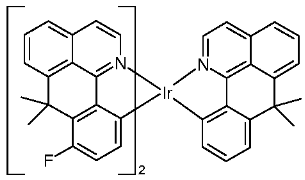 |
| 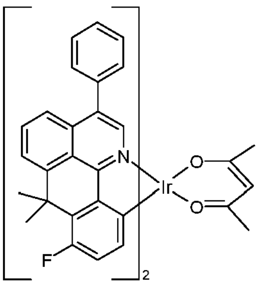 | 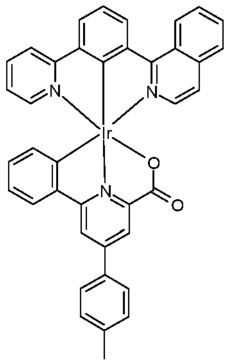 | 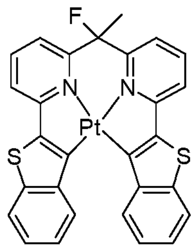 |

| | | |
|---|---|---|
| 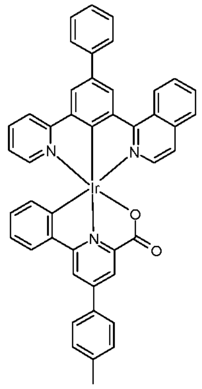 | 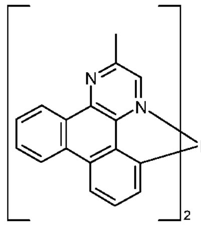 | 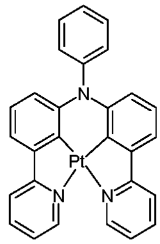 |
| 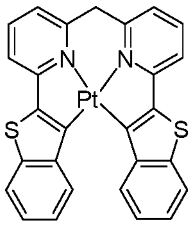 | 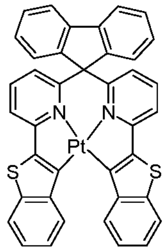 | 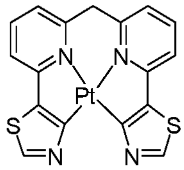 |
| 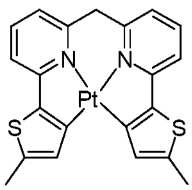 | 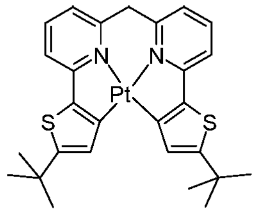 | 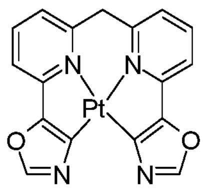 |
| 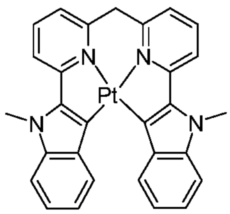 | 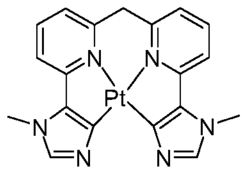 | 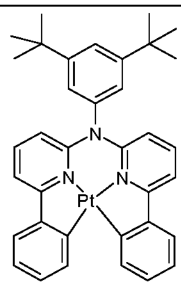 |
| 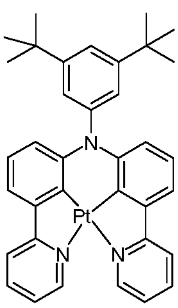 | 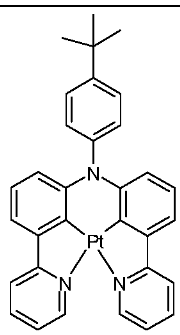 | 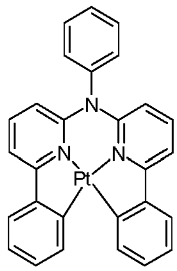 |

| | | |
|---|---|---|
| 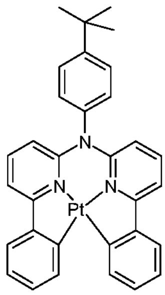 | 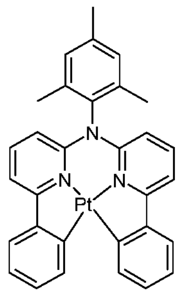 | 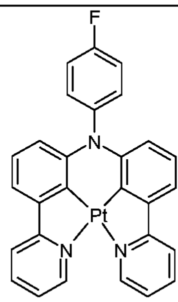 |
| 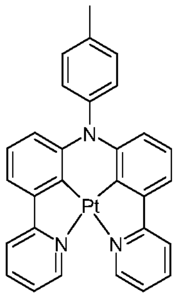 | 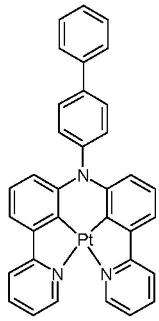 | 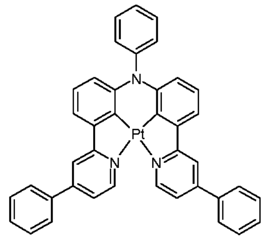 |
| 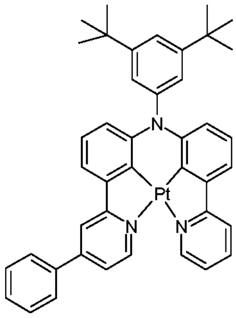 | 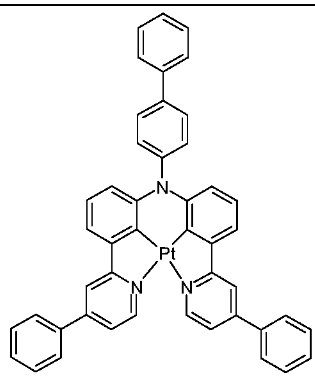 | 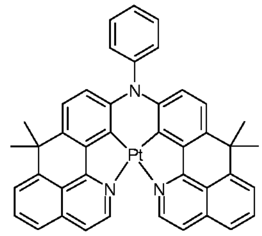 |
| 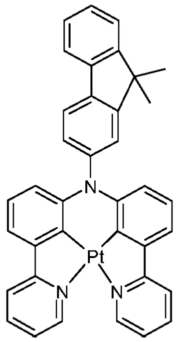 | 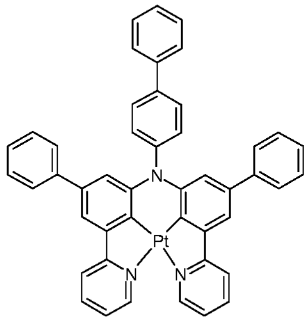 | 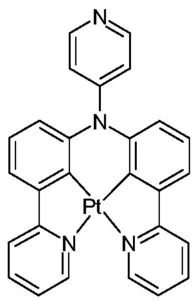 |
| 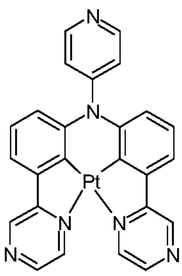 | 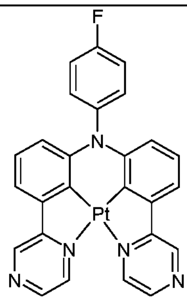 | 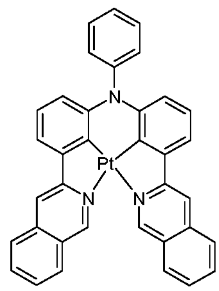 |

| | | |
|---|---|---|
| 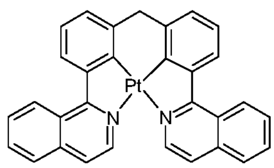 | 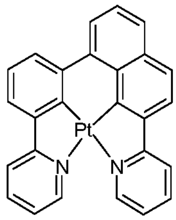 | 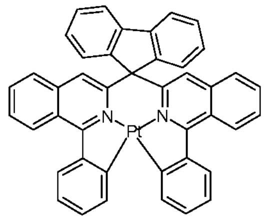 |
| 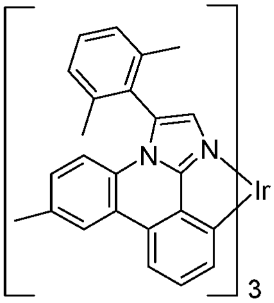 | 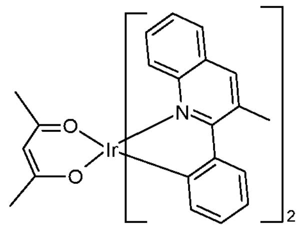 | 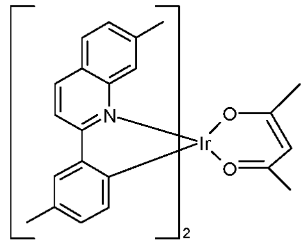 |
| 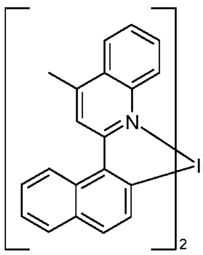 | 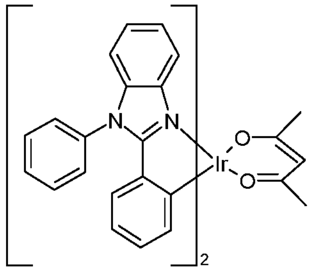 | 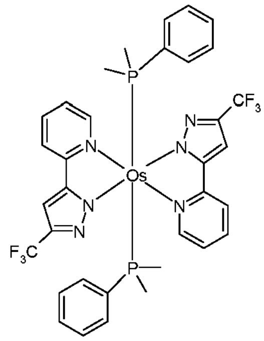 |
| 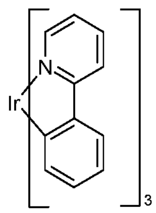 | 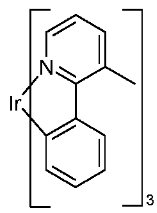 | 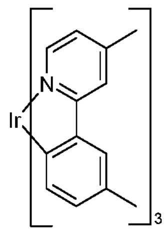 |
| 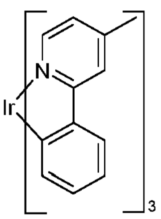 | 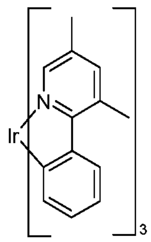 | 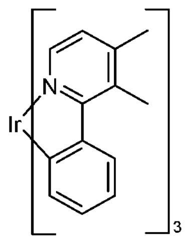 |
| 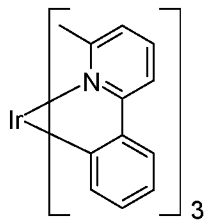 | 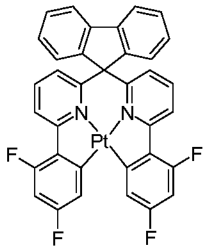 | 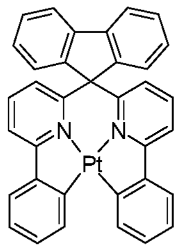 |

| | | |
|---|---|---|
| 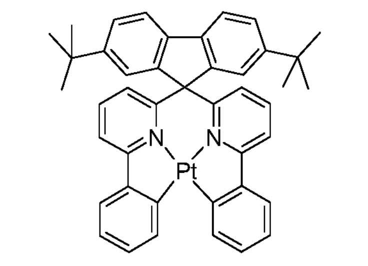 | 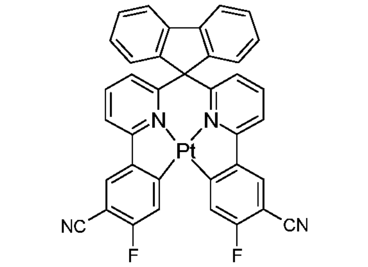 | 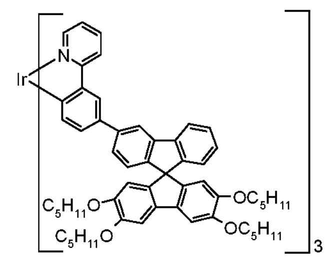 |
| 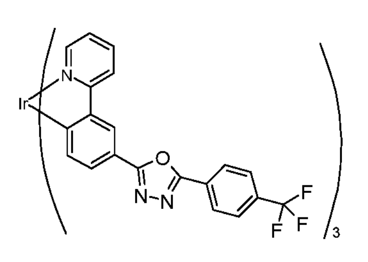 | 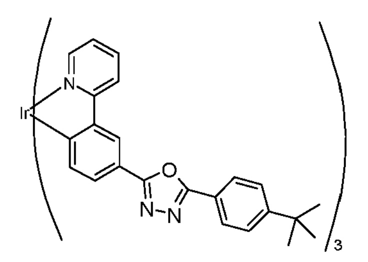 | 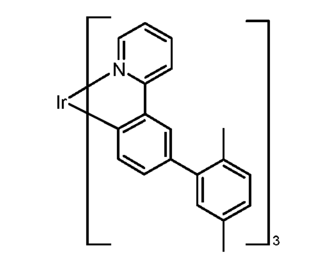 |
| 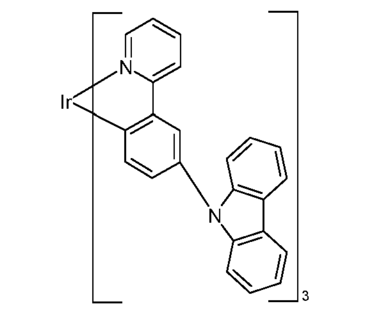 | 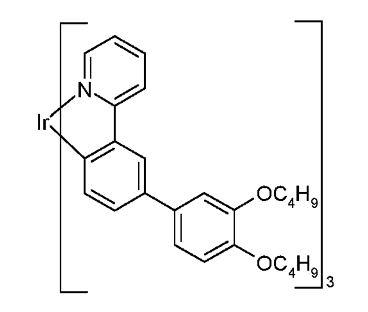 | 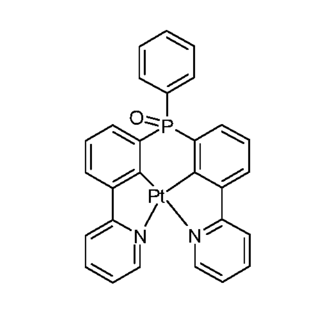 |
| 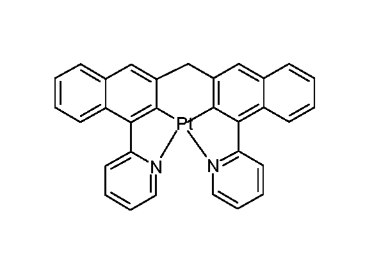 | 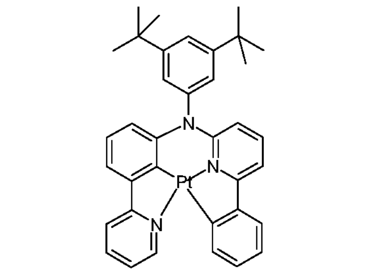 | 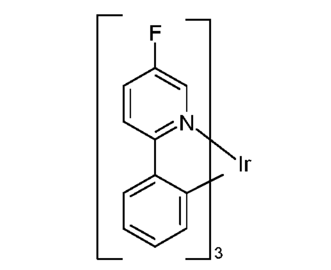 |
| 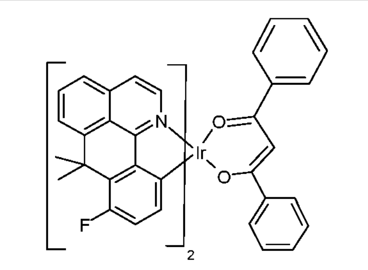 | 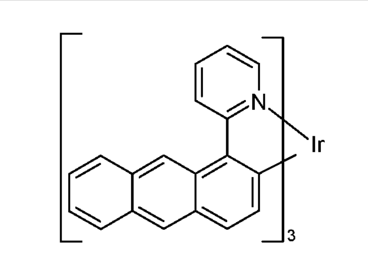 | 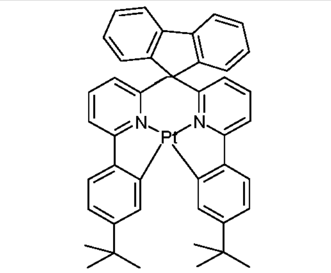 |
| 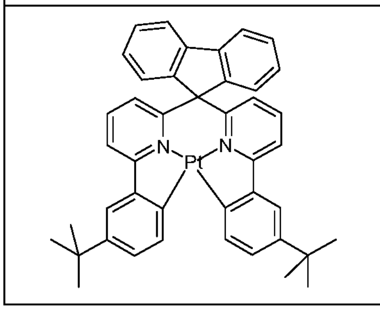 | 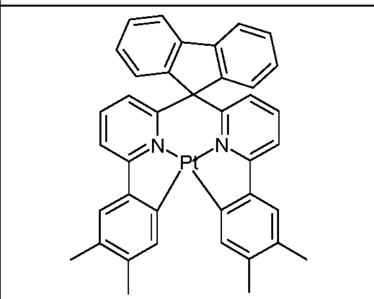 | 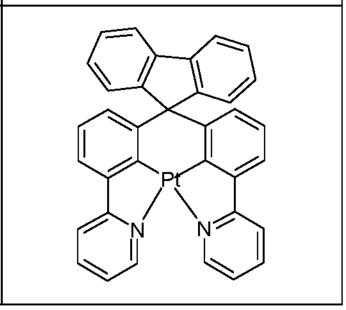 |

| | | |
|---|---|---|
| 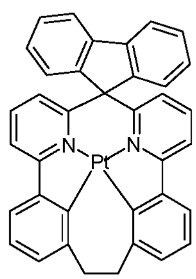 | 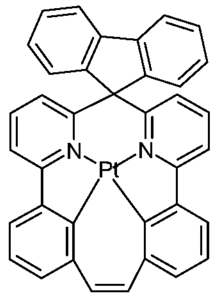 | 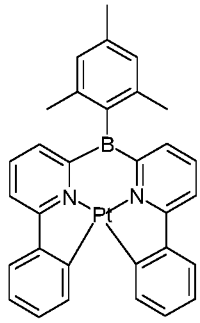 |
| 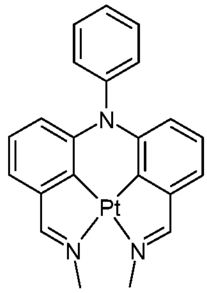 | 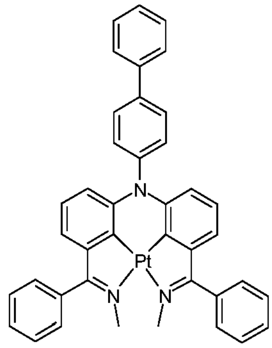 | 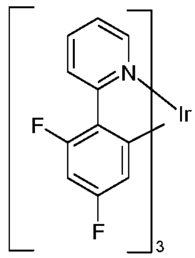 |
| 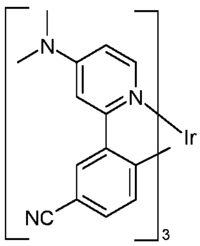 | 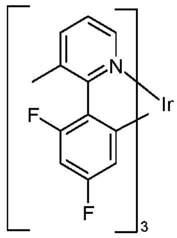 | 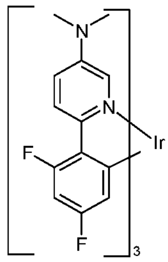 |
| 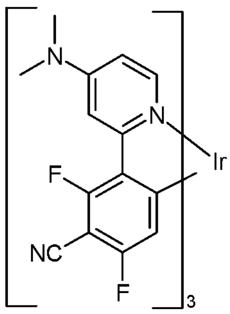 | 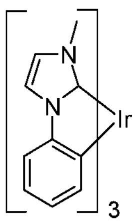 | 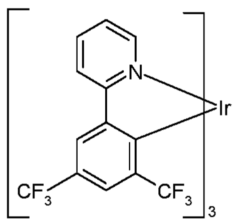 |
| 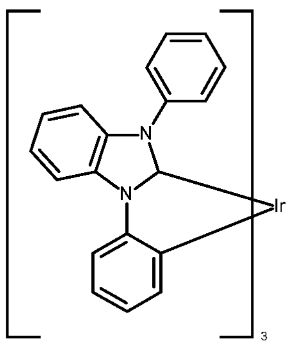 | 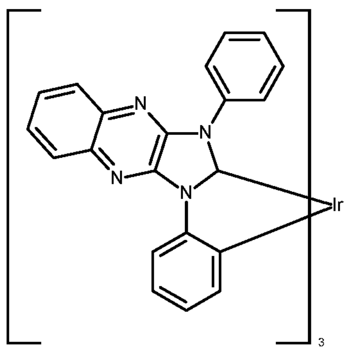 | 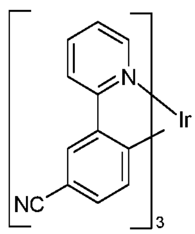 |

| | | |
|---|---|---|
| 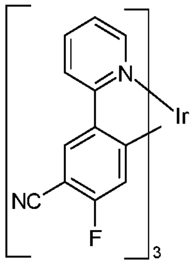 | 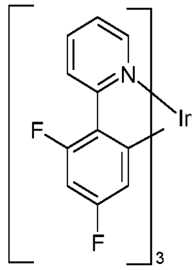 | 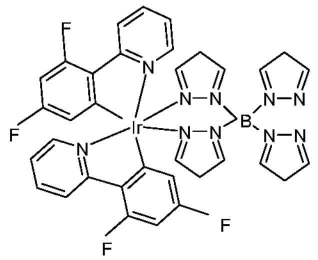 |
| 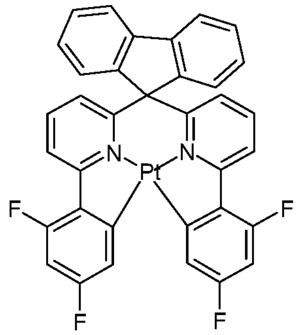 | 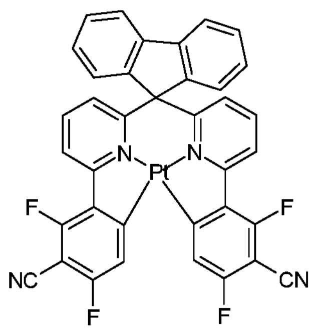 | 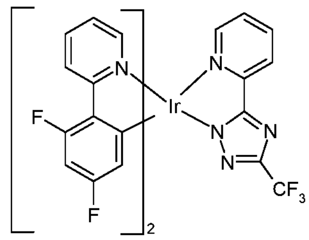 |
| 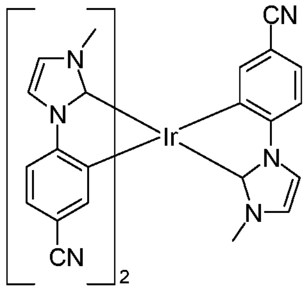 | 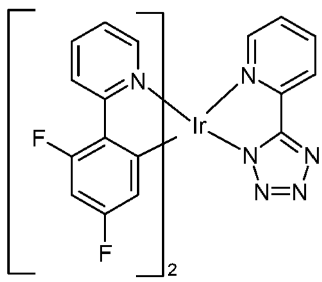 | 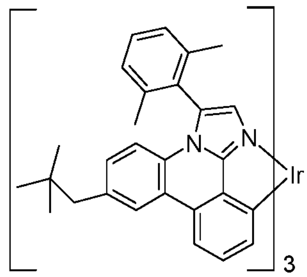 |
| 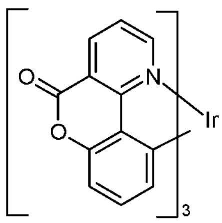 | 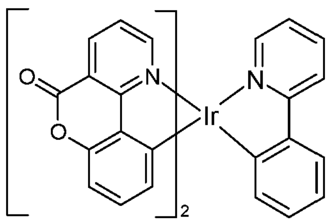 | 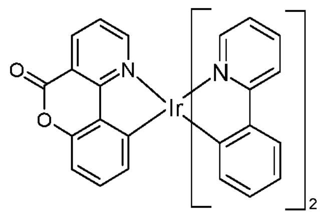 |
| 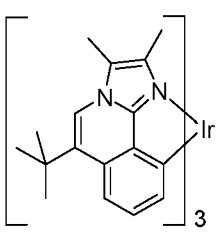 | 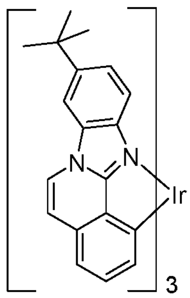 | 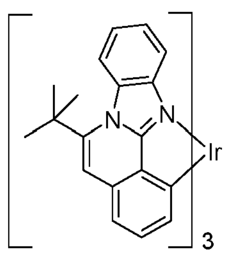 |

| | | |
|---|---|---|
| 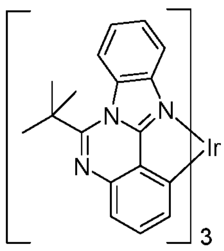 | 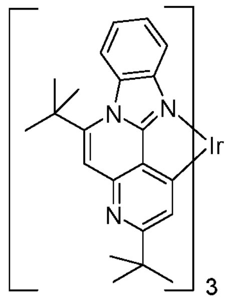 | 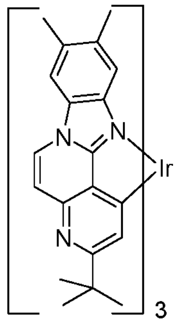 |
| 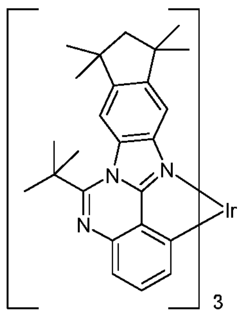 | 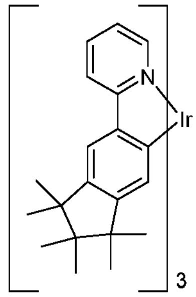 | 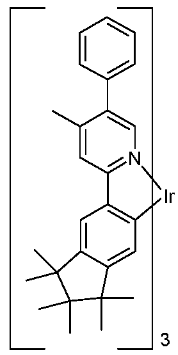 |
| 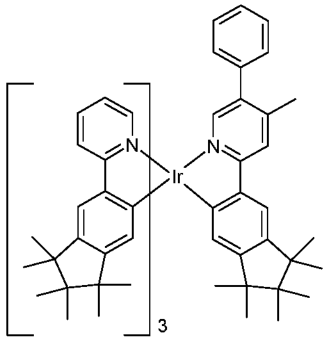 | 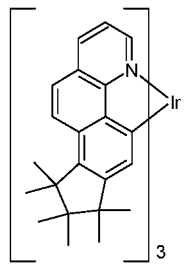 | 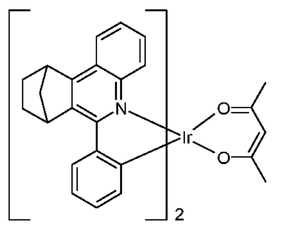 |
| 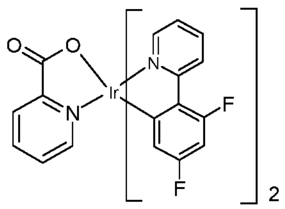 | 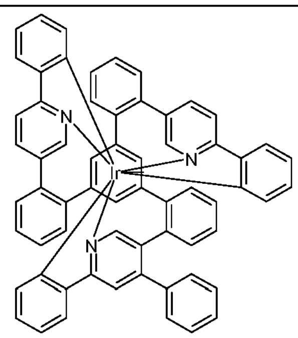 | 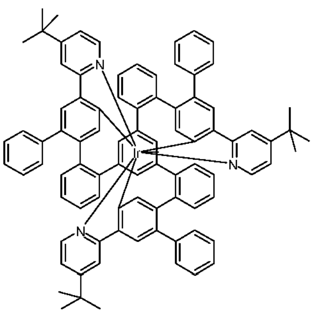 |

| | | |
|---|---|---|
| 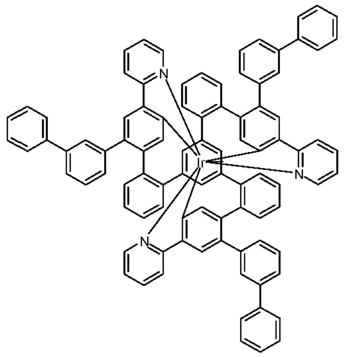 | 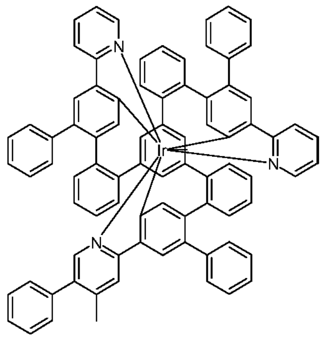 | 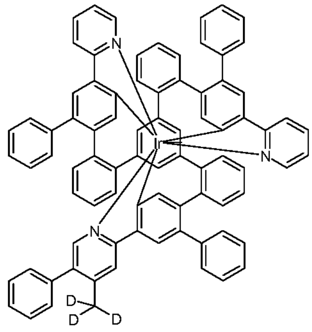 |
| 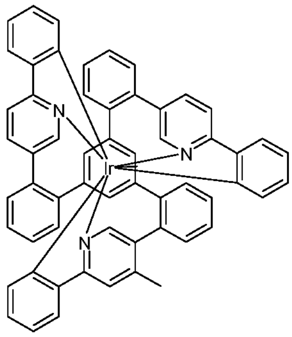 | | |

Bevorzugte Matrixmaterialien für phosphoreszierende Emitter sind neben den anmeldungsgemäßen Verbindungen aromatische Ketone, aromatische Phosphinoxide oder aromatische Sulfoxide oder Sulfone, Triarylamine, Carbazolderivate, z. B. CBP (N,N-Biscarbazolylbiphenyl) oder Carbazol-derivate, Indolocarbazolderivate, Indenocarbazolderivate, Azacarbazolderivate, bipolare Matrixmaterialien, Silane, Azaborole oder Boronester, Triazinderivate, Zinkkomplexe, Diazasilol- bzw. Tetraazasilol-Derivate, Diazaphosphol-Derivate, überbrückte Carbazol-Derivate, Triphenylenderivate, oder Lactame.

Bevorzugte Matrixmaterialien zur Verwendung in Mischung mit den anmeldungsgemäßen Verbindungen in phosphoreszierenden emittierenden Schichten sind gewählt aus den folgenden Verbindungen:

| | | |
|---|---|---|
| 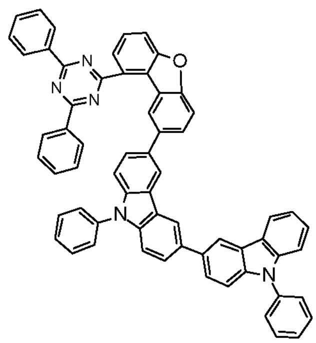 | 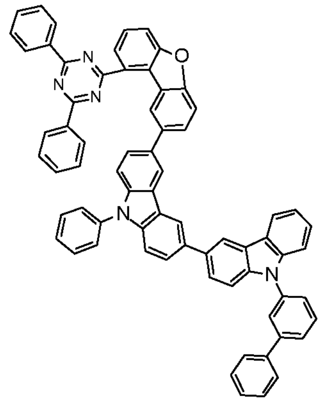 | 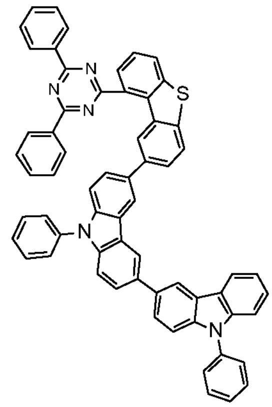 |
| 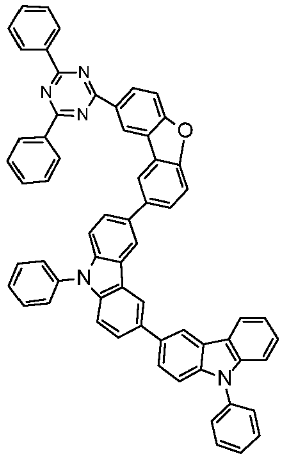 | 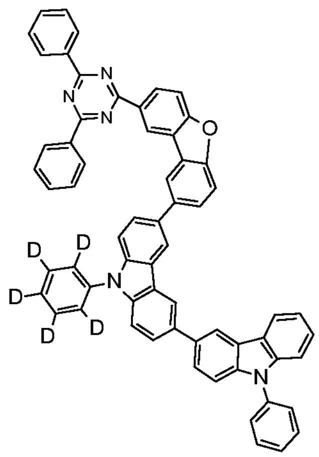 | 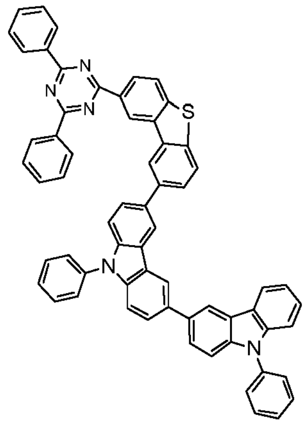 |
| 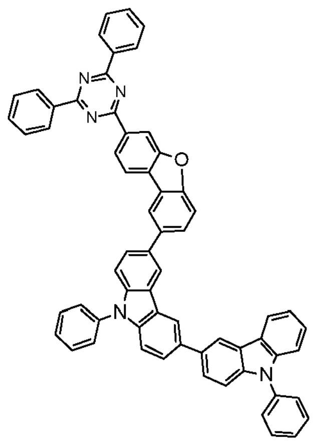 | 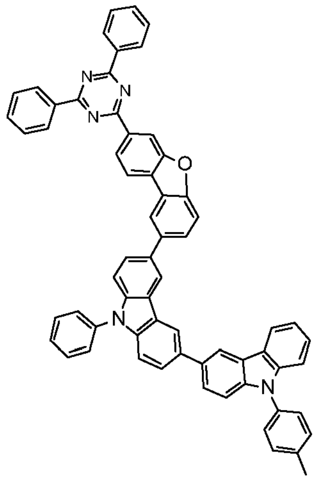 | 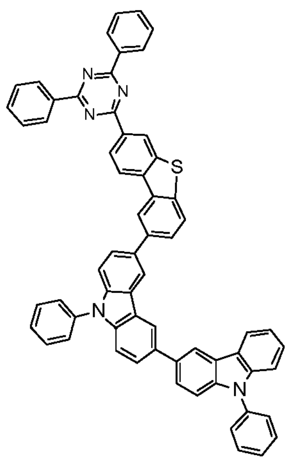 |

| | | |
|---|---|---|
| 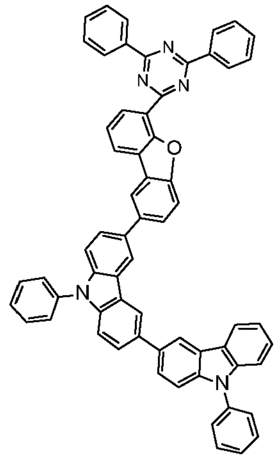 | 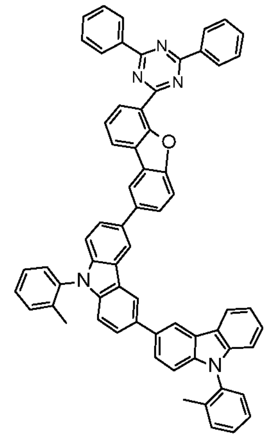 | 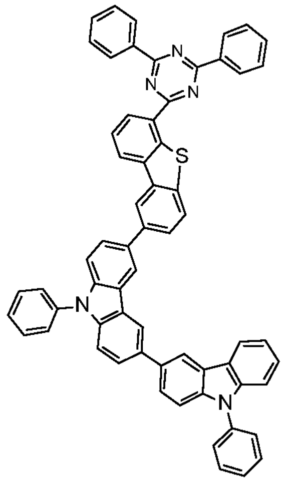 |
| 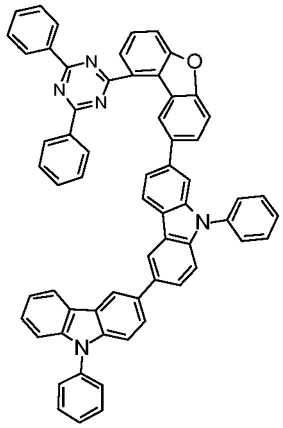 | 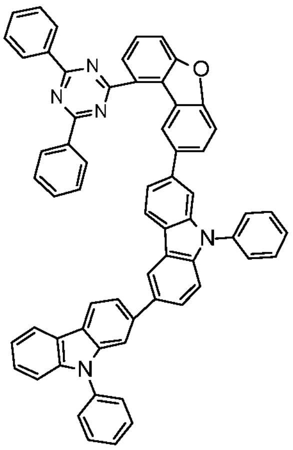 | 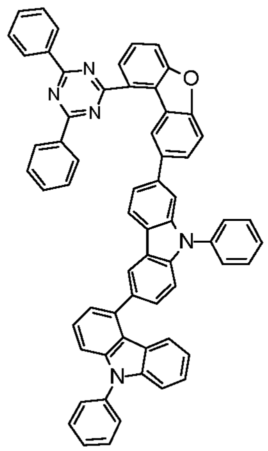 |
| 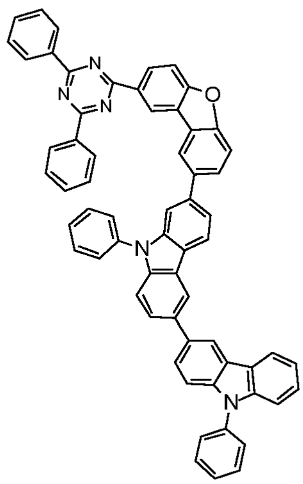 | 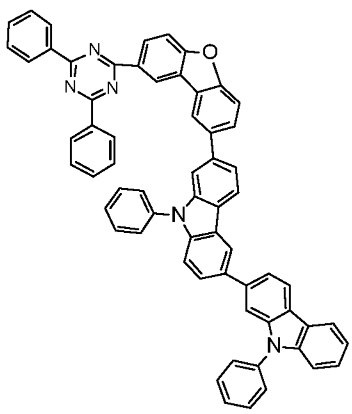 | 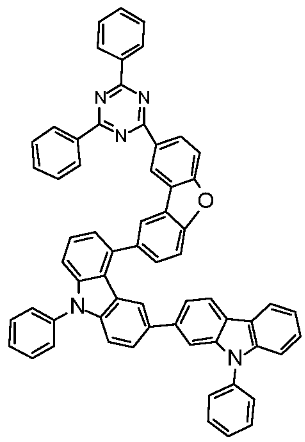 |

| | | |
|---|---|---|
| 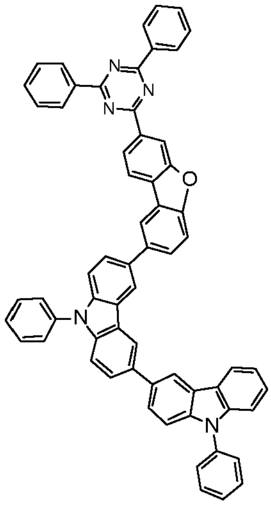 | 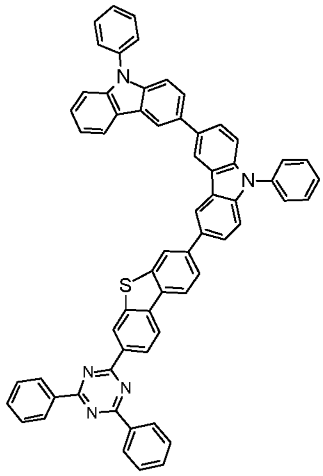 | 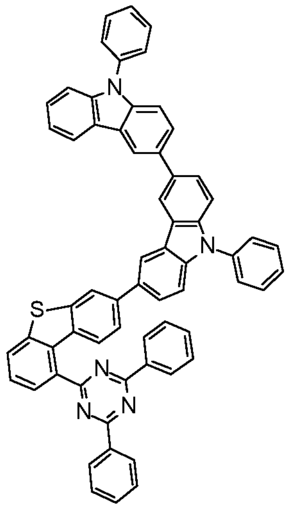 |
| 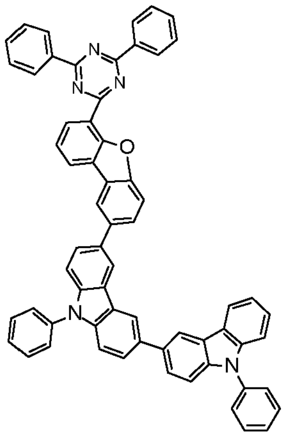 | 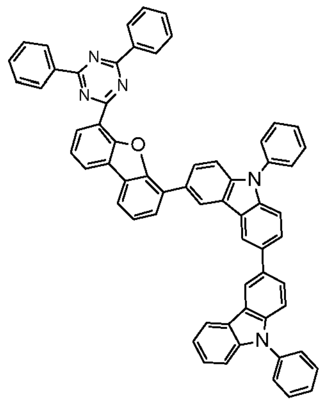 | 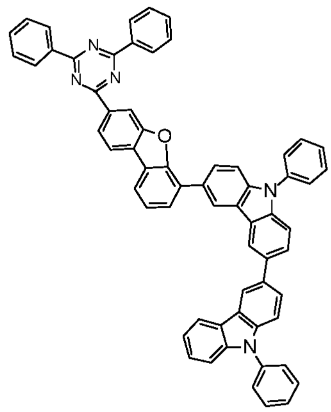 |
| 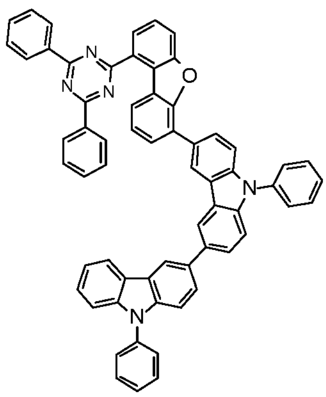 | 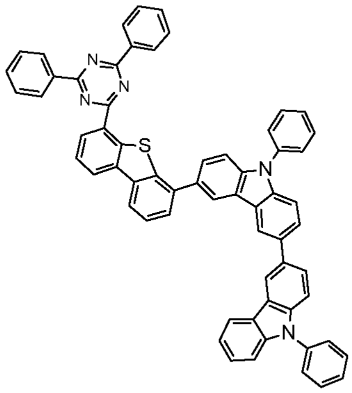 | 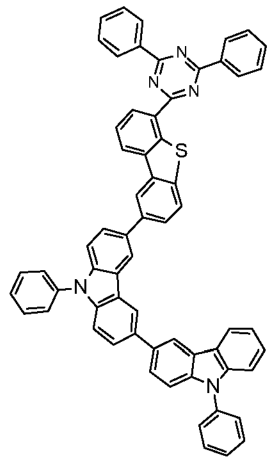 |

| | | |
|---|---|---|
| 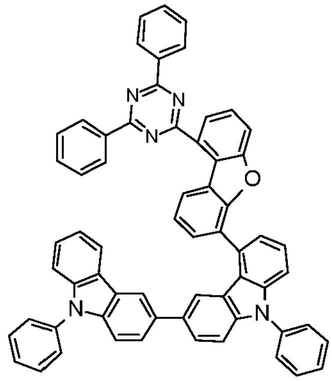 | 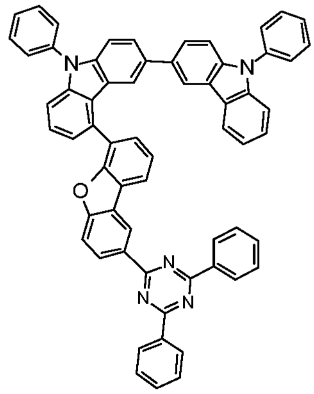 | 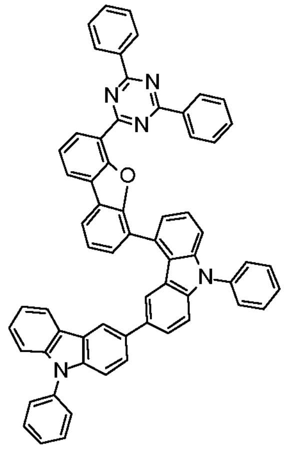 |
| 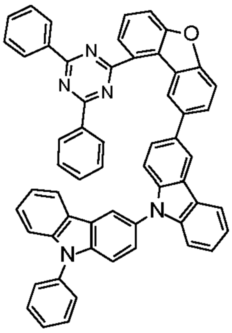 | 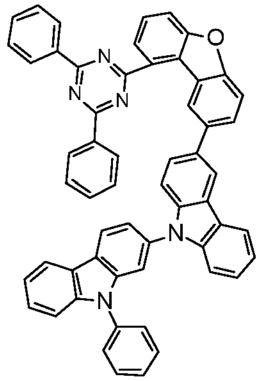 | 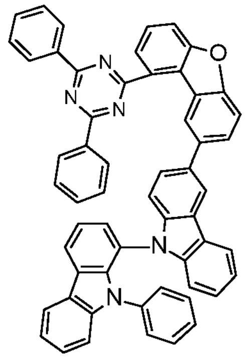 |
| 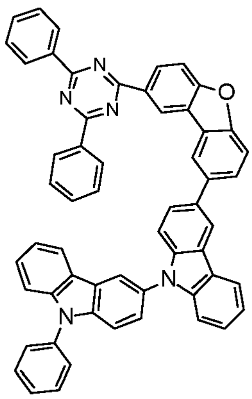 | 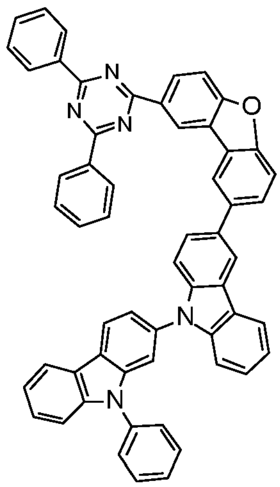 | 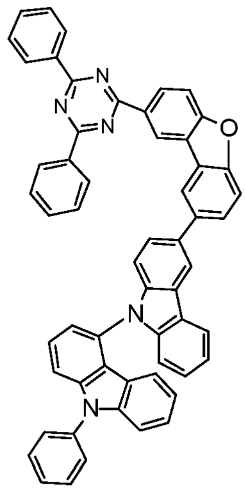 |

| | | |
|---|---|---|
| 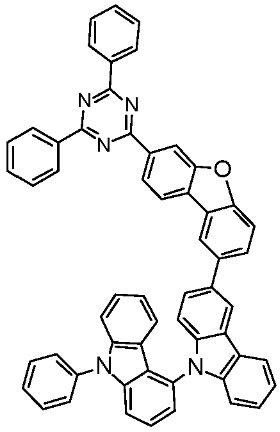 | 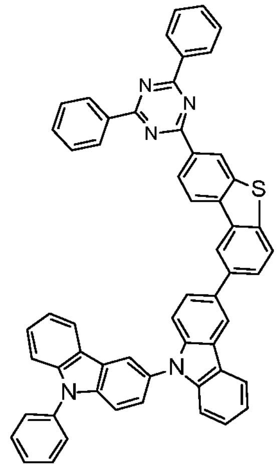 | 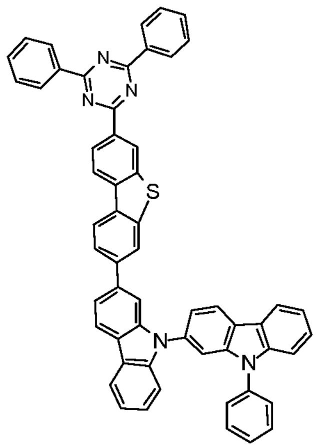 |
| 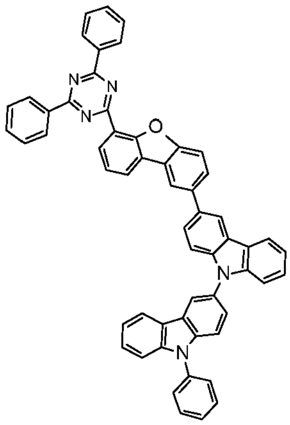 | 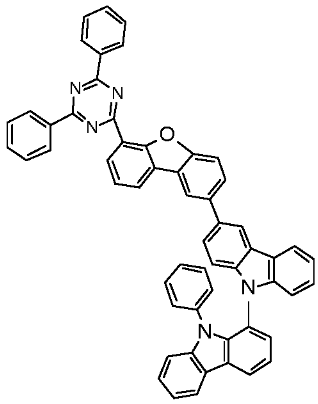 | 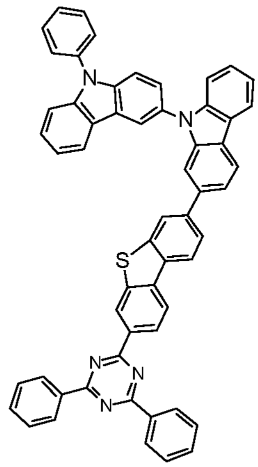 |
| 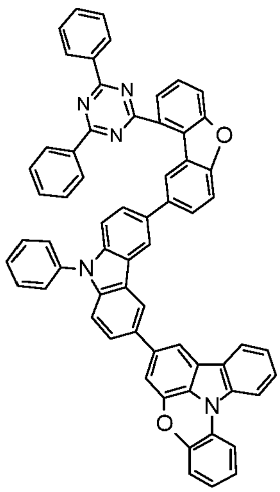 | 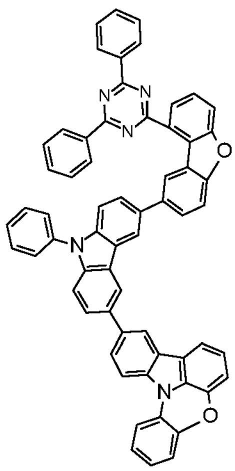 | 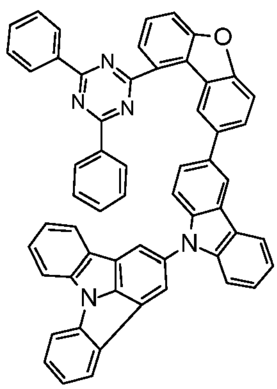 |

| | | |
|---|---|---|
| 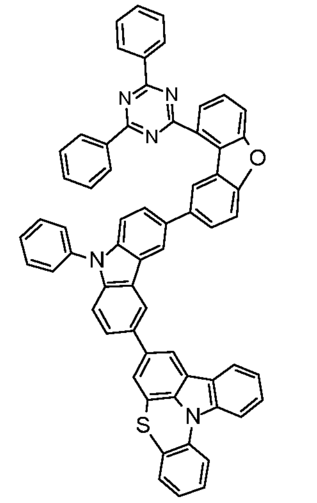 | 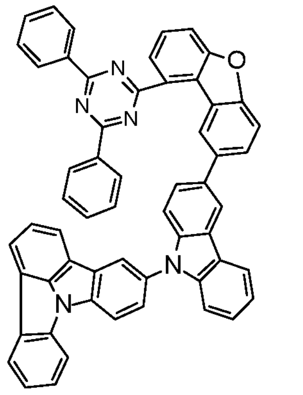 | 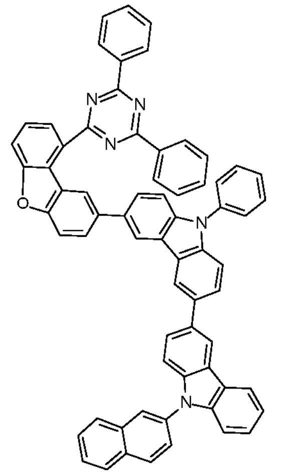 |

Geeignete Ladungstransportmaterialien, wie sie in der Lochinjektions- bzw. Lochtransportschicht bzw. Elektronenblockierschicht oder in der Elektronentransportschicht der erfindungsgemäßen elektronischen Vorrichtung verwendet werden können, sind beispielsweise die in Y. Shirota et al., Chem. Rev. 2007, 107(4), 953-1010 offenbarten Verbindungen oder andere Materialien, wie sie gemäß dem Stand der Technik in diesen Schichten eingesetzt werden.

Als Materialien für die elektronentransportierenden Schichten der Vorrichtung eignen sich insbesondere Aluminiumkomplexe, beispielsweise $Alq_3$, Zirkoniumkomplexe, beispielsweise $Zrq_4$, Lithiumkomplexe, beispielsweise Liq, Benzimidazolderivate, Triazinderivate, Pyrimidinderivate, Pyridinderivate, Pyrazinderivate, Chinoxalinderivate, Chinolinderivate, Oxadiazolderivate, aromatische Ketone, Lactame, Borane, Diazaphospholderivate und Phosphinoxidderivate.

Besonders bevorzugte Verbindungen zur Verwendung in elektronentransportierenden Schichten sind in der folgenden Tabelle gezeigt:

| | | |
|---|---|---|
| 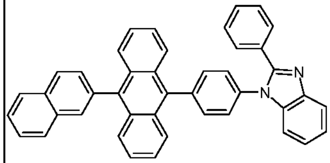 | 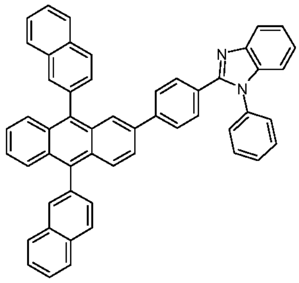 | 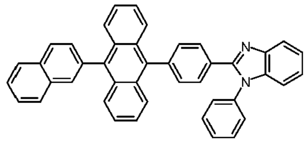 |
| 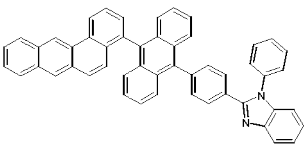 | 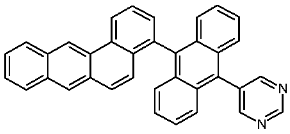 | 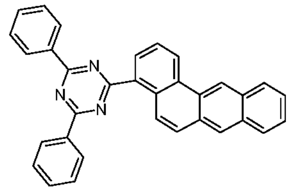 |
| 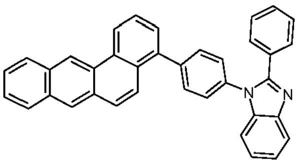 | 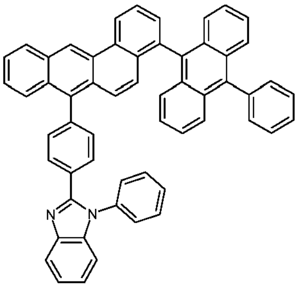 | 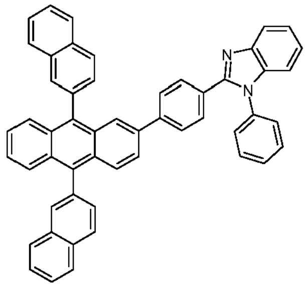 |
| 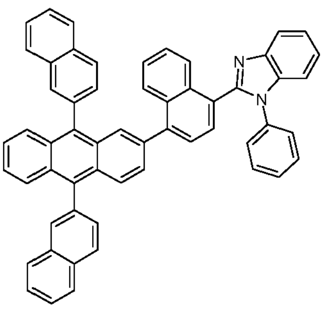 | 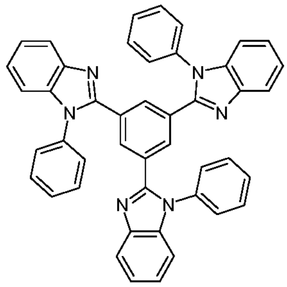 | 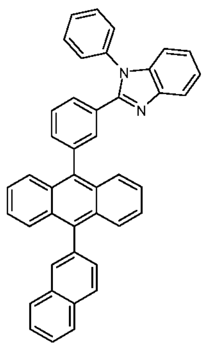 |
| 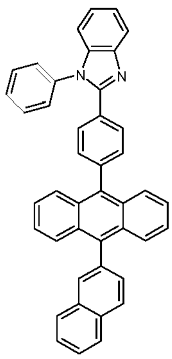 | 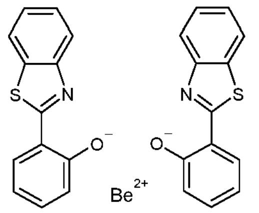 | 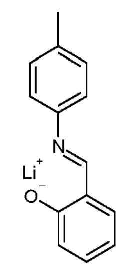 |

| | | |
|---|---|---|
| 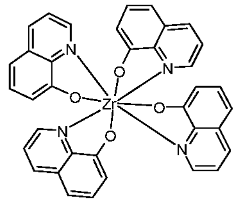 | 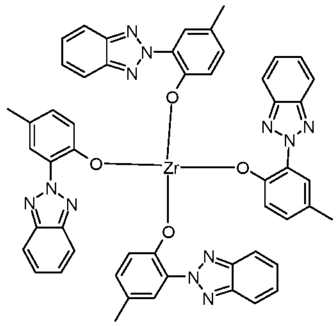 | 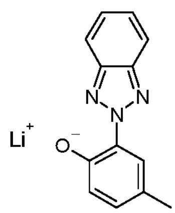 |
| 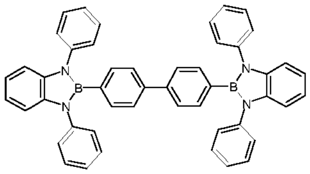 | 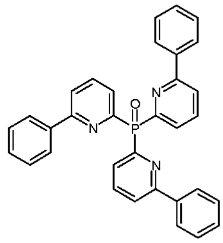 | 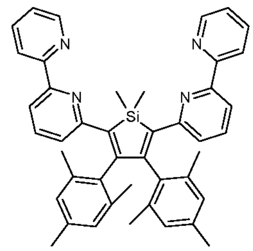 |
| 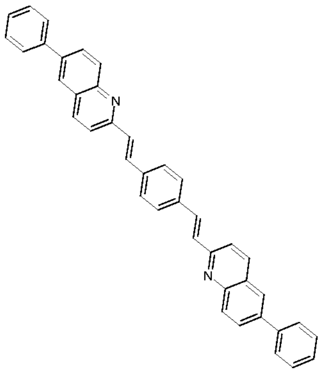 | 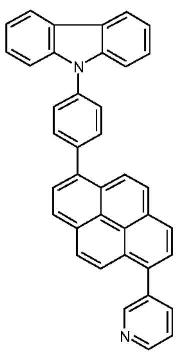 | 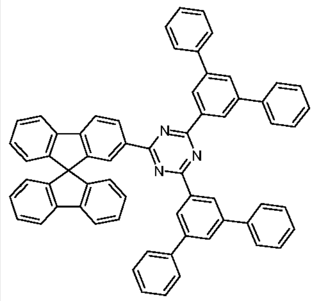 |
| 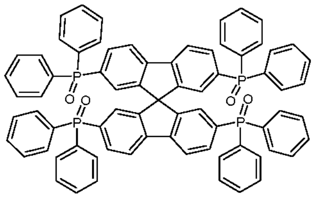 | 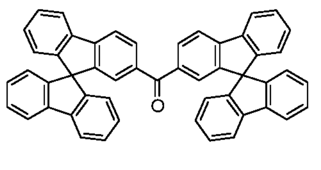 | 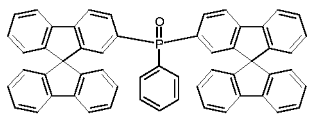 |
| 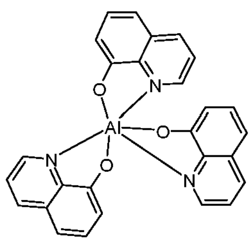 | | |

Als Materialien für lochtransportierende Schichten von OLEDs können bevorzugt Indenofluorenamin-Derivate, Aminderivate, Hexaazatriphenylenderivate, Aminderivate mit kondensierten Aromaten, Monobenzoindenofluorenamine, Dibenzoindenofluorenamine, Spirobifluoren-Amine, Fluoren-Amine, Spiro-Dibenzopyran-Amine, Dihydroacridin-Derivate, Spirodibenzofurane und Spirodibenzothiophene, Phenanthren-Diarylamine, Spiro-Tribenzotropolone, Spirobifluorene mit meta-Phenyldiamingruppen, Spiro-Bisacridine, Xanthen-Diarylamine, und 9,10-Dihydroanthracen-Spiroverbindungen mit Diarylaminogruppen eingesetzt werden. Insbesondere eignen sich hierfür die folgenden Verbindungen:

| | | |
|---|---|---|
| 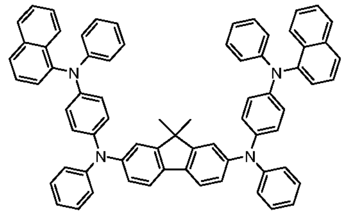 | 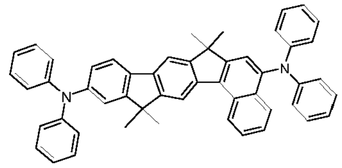 | 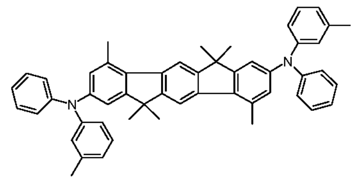 |
| 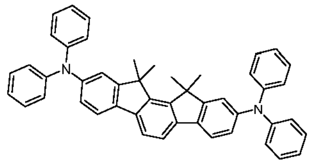 | 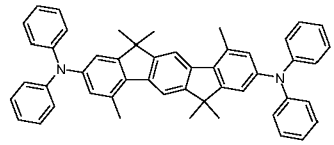 | 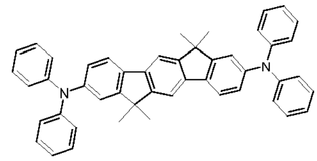 |
| 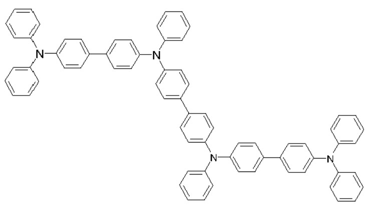 | 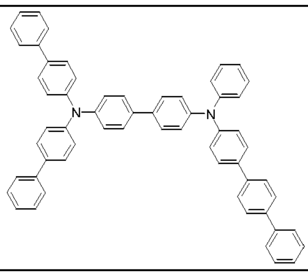 | 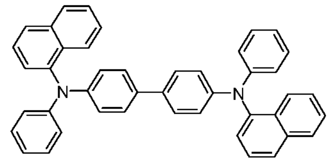 |
| 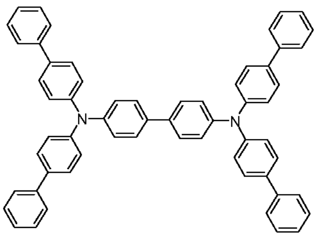 | 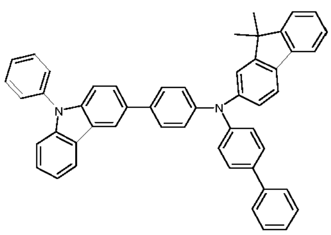 | 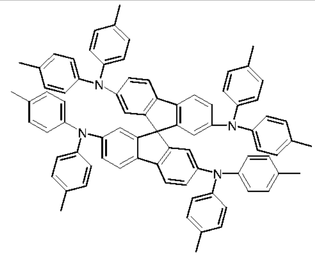 |
| 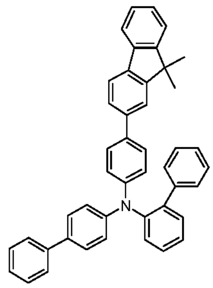 | 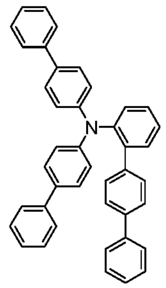 | 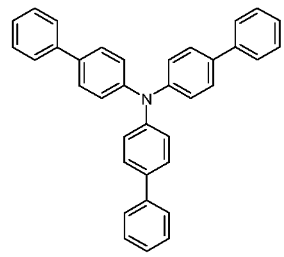 |

| | | |
|---|---|---|
| 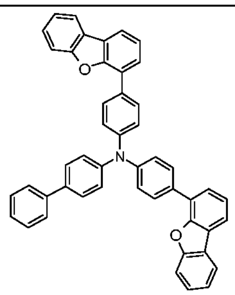 | 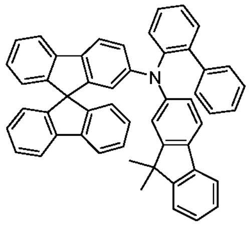 | 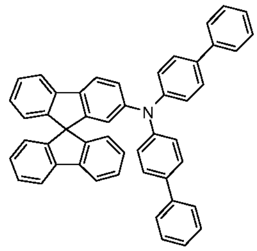 |
| 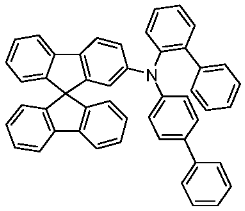 | 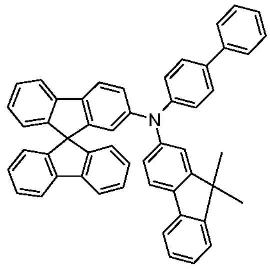 | 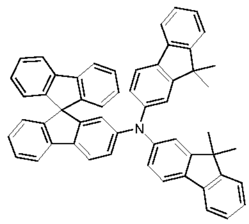 |
| 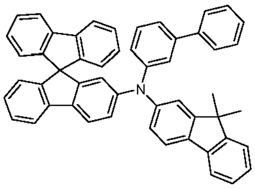 | 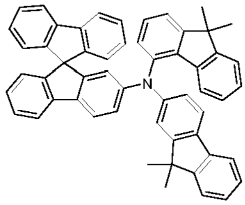 | 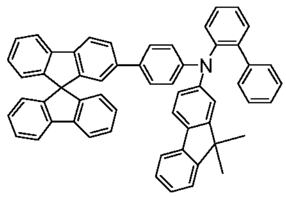 |
| 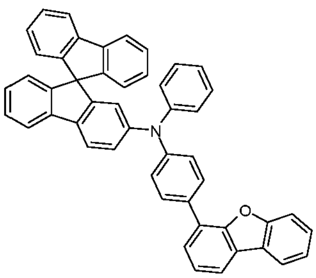 | 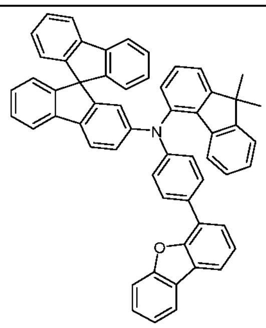 | 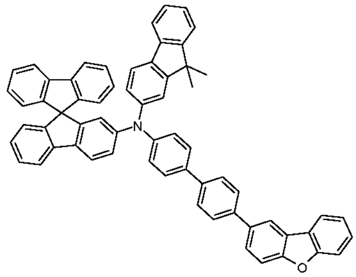 |
| 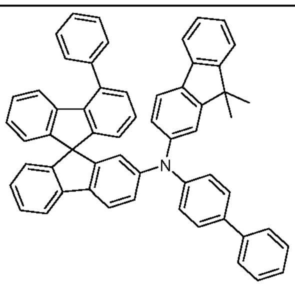 | 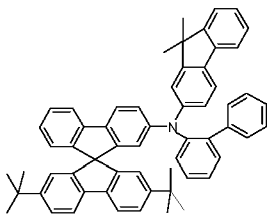 | 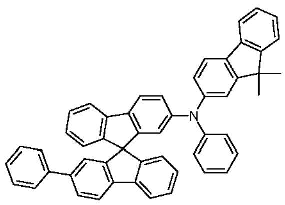 |

| | | |
|---|---|---|
| 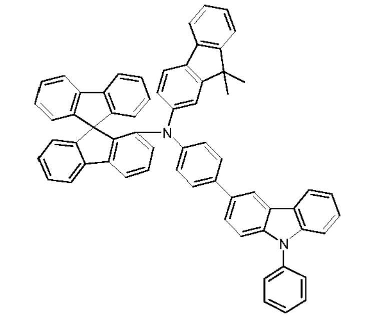 | 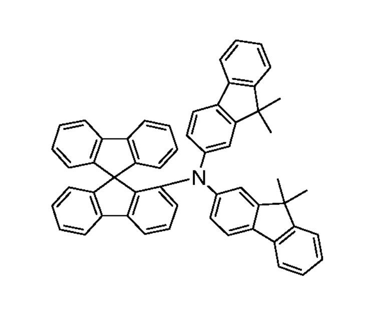 | 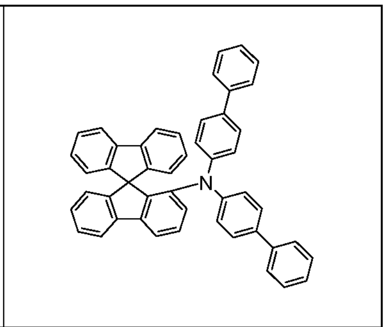 |
| 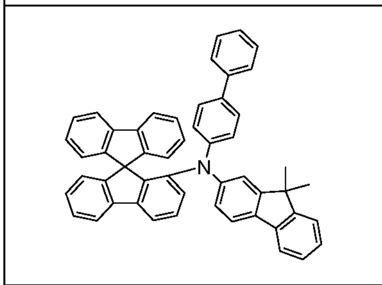 | 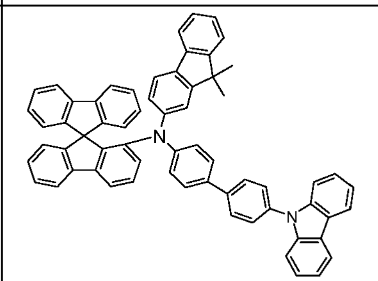 | 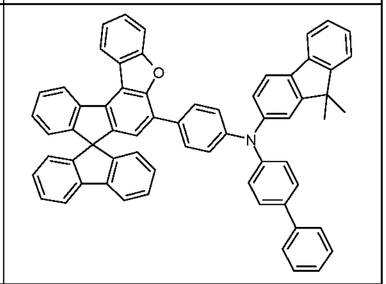 |
| 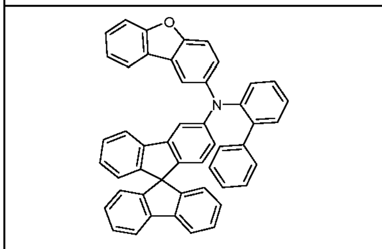 | 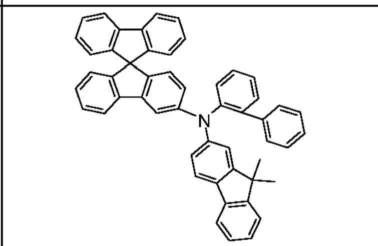 | 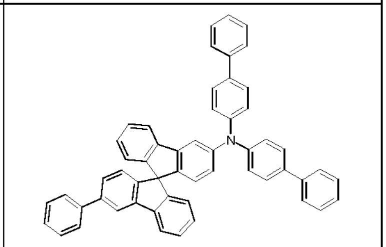 |
| 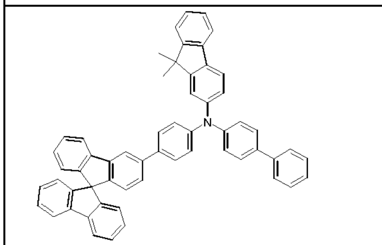 | 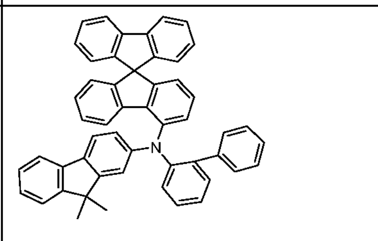 | 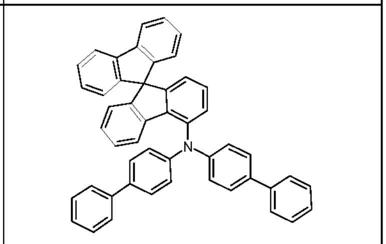 |
| 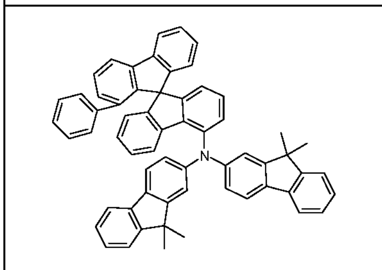 | 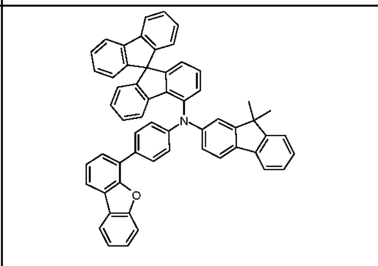 | 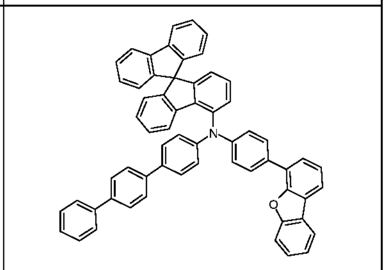 |
| 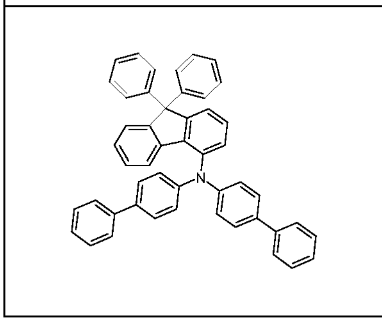 | 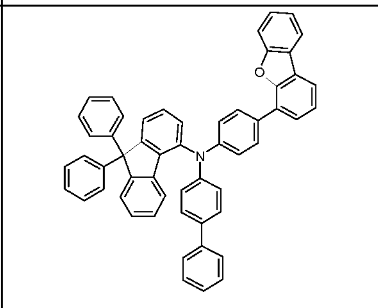 | 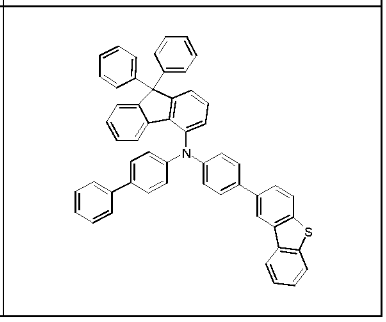 |

| | | |
|---|---|---|
| 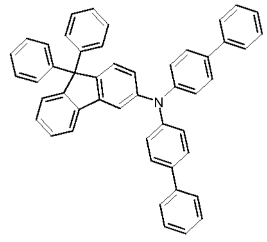 | 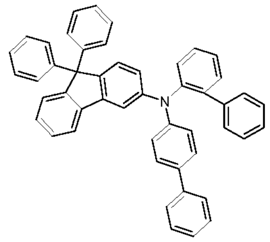 | 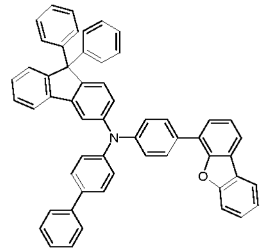 |
| 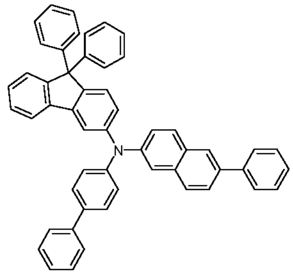 | 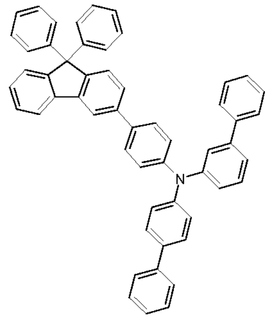 | 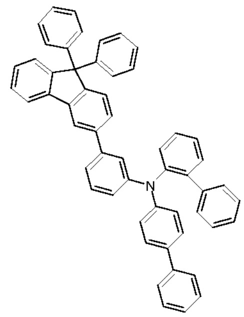 |
| 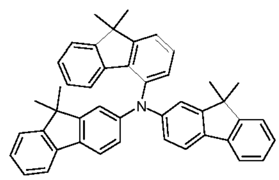 | 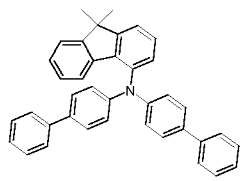 | 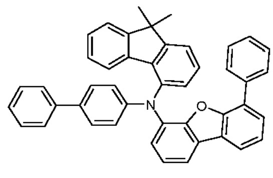 |
| 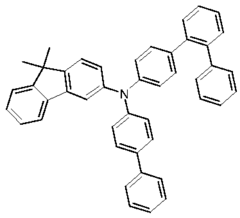 | 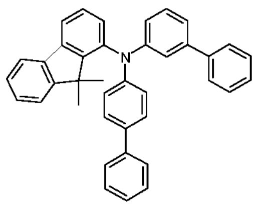 | 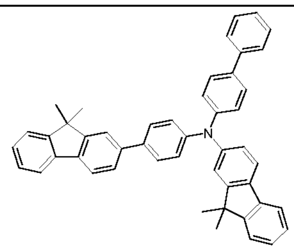 |
| 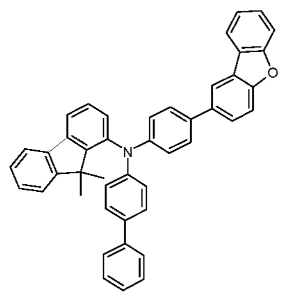 | 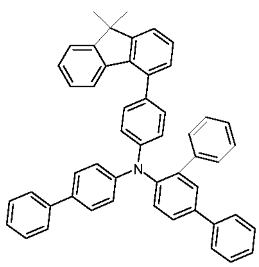 | 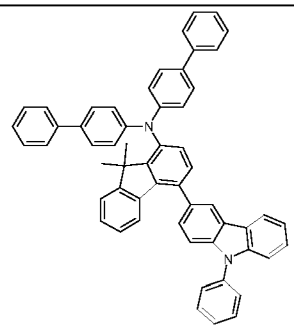 |

| | | |
|---|---|---|
| 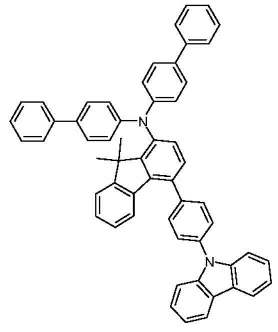 | 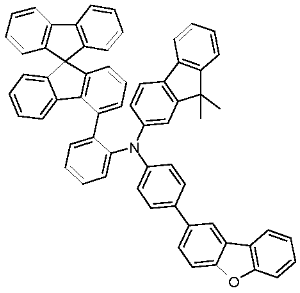 | 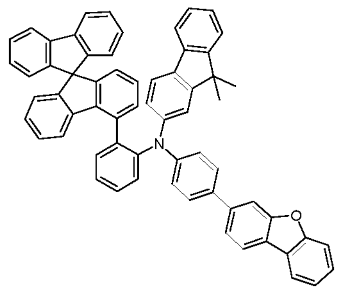 |
| 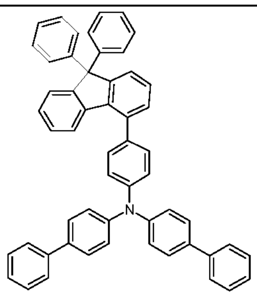 | 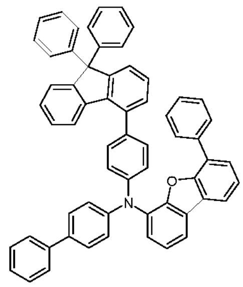 | |

Als Kathode der elektronischen Vorrichtung sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder Al, wobei dann in der Regel Kombinationen der Metalle, wie beispielsweise Ca/Ag, Mg/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, $Li_2O$, $BaF_2$, MgO, NaF, CsF, $Cs_2CO_3$, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/$NiO_x$, Al/$PtO_x$) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-ZinnOxid (ITO) oder Indium-Zink Oxid (IZO). Bevorzugt sind weiterhin leitfähige, dotierte organische Materialien, insbesondere leitfähige dotierte Polymere. Weiterhin kann die Anode auch aus mehreren Schichten bestehen, beispielsweise aus einer inneren Schicht aus ITO und einer äußeren Schicht aus einem Metalloxid, bevorzugt Wolframoxid, Molybdänoxid oder Vanadiumoxid.

Die Vorrichtung wird entsprechend (je nach Anwendung) strukturiert, kontaktiert und schließlich versiegelt, um schädigende Effekte von Wasser und Luft auszuschließen.

In einer bevorzugten Ausführungsform ist die elektronische Vorrichtung dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner $10^{-5}$ mbar, bevorzugt kleiner $10^{-6}$ mbar aufgedampft. Dabei ist es jedoch auch möglich, dass der Anfangsdruck noch geringer ist, beispielsweise kleiner $10^{-7}$ mbar.

Bevorzugt ist ebenfalls eine elektronische Vorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen $10^{-5}$ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine elektronische Vorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen nötig. Hohe Löslichkeit lässt sich durch geeignete Substitution der Verbindungen erreichen.

Weiterhin bevorzugt ist es, dass zur Herstellung einer erfindungsgemäßen elektronischen Vorrichtung eine oder mehrere Schichten aus Lösung und eine oder mehrere Schichten durch ein Sublimationsverfahren aufgetragen werden.

Elektronische Vorrichtungen enthaltend eine oder mehrere Verbindungen wie oben definiert werden bevorzugt in Displays, als Lichtquellen in Beleuchtungsanwendungen sowie als Lichtquellen in medizinischen und/oder kosmetischen Anwendungen (z.B. Lichttherapie) eingesetzt.

## Beispiele

### A) Synthesebeispiele

### Schritt a: Synthese von Zwischenstufen

### Beispiel a: 10-(4-Bromo-phenyl)-benzo[h]chinolin

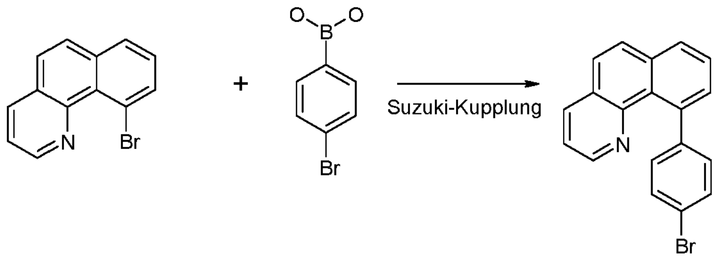


15,47 g (75 mmol) 4-Brom-benzolboronsäure, 19,4 g 10-Bromo-benzo[h]chinolin (75 mmol) und 110 mL einer 2M wäßrigen $NaHCO_3$ Lösung (163 mmol) werden in 500 mL Dimethoxyethan suspendiert. Zu dieser Suspension werden 3,0 g (3,45 mmol) Tetrakis-(triphenyl)phosphin-palladium(0) gegeben und die Reaktionsmischung wird 22 h unter Rückfluss erhitzt. Nach Erkalten wird die organische Phase abgetrennt, über Kieselgel filtriert, viermal mit 400 mL Wasser gewaschen und anschließend zur Trockene eingeengt. Nach Filtration des Rohproduktes über Kieselgel mit Heptan/Toluol (10:1) erhält man 39 g (71 %) 10-(4-Bromo-phenyl)-benzo[h]chinolin.

Analog dazu werden folgende Verbindungen hergestellt:

| | Edukt 1 | Edukt 2 | Produkt | Ausbeute |
|---|---|---|---|---|
| 1a | 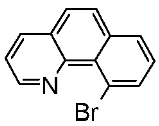 [152583-10-3] | 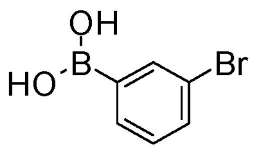 | 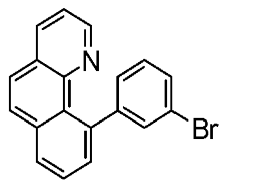 | 58% |
| 2a | 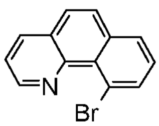 [152583-10-3] | 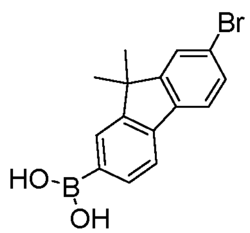 | 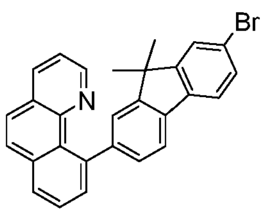 | 49% |

(fortgesetzt)

| | Edukt 1 | Edukt 2 | Produkt | Ausbeute |
|---|---|---|---|---|
| 3a | 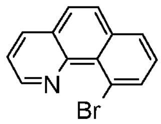 [152583-10-3] | 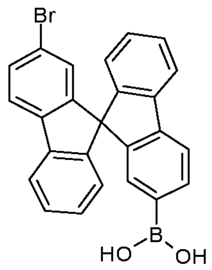 | 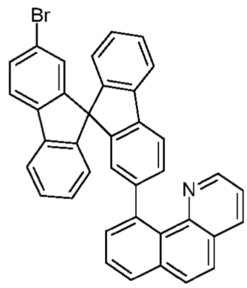 | 51% |
| 4a | 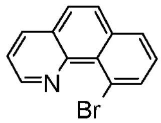 [152583-10-3] | 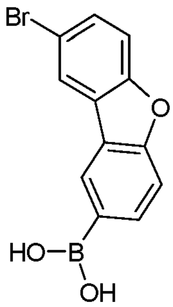 | 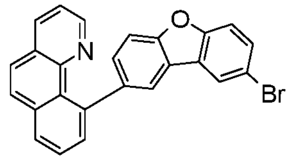 | 56% |
| 5a | 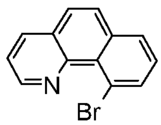 | 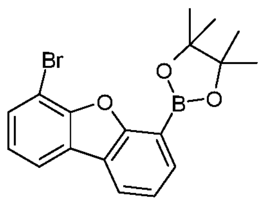 [1801142-52-8 ] | 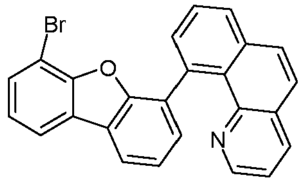 | 43% |
| 6a | 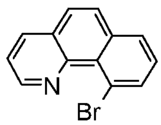 [152583-10-3] | 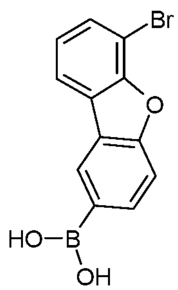 | 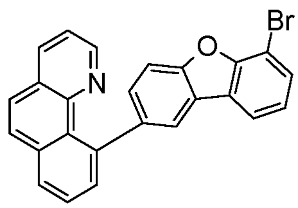 | 54% |
| 7a | 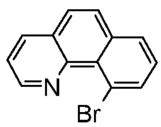 [152583-10-3] | 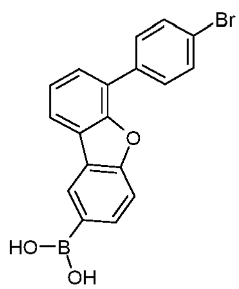 | 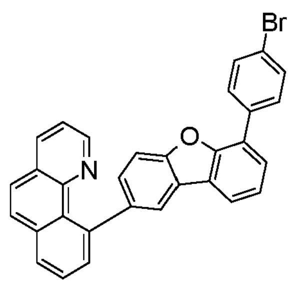 | 49% |

(fortgesetzt)

| | Edukt 1 | Edukt 2 | Produkt | Ausbeute |
|---|---|---|---|---|
| 8a | 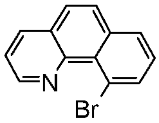 [152583-10-3] | 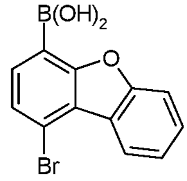 [2086712-52-7 ] | 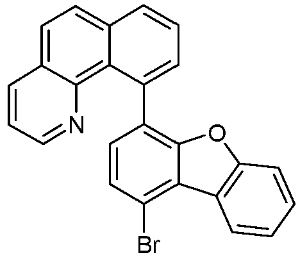 | 52% |
| 9a | 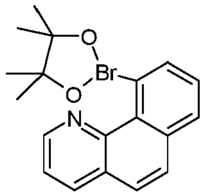 [1349171-34-1 ] | 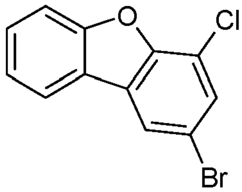 [1960445-63-9] | 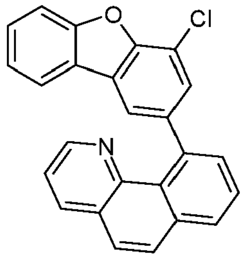 | 66% |
| 10a | 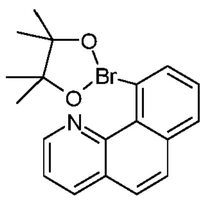 [1349171-34-1 ] | 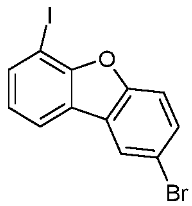 [1643716-58-8 ] | 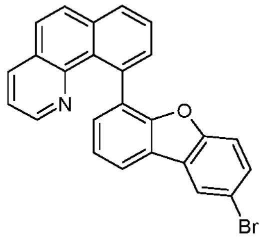 | 71% |
| 11a | 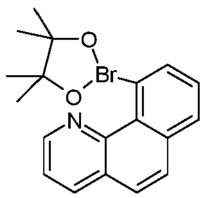 [1349171-34-1 ] | 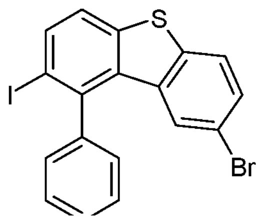 [1883821-34-8 ] | 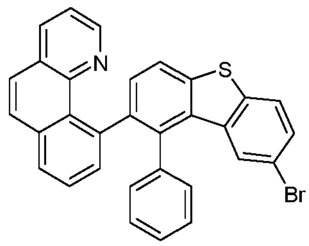 | 68% |
| 12a | 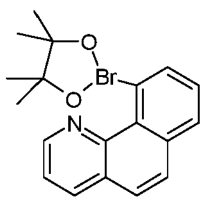 [1349171-34-1 ] | 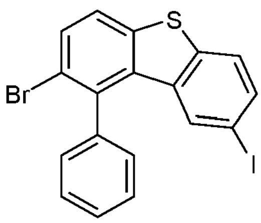 [1883821-17-7 ] | 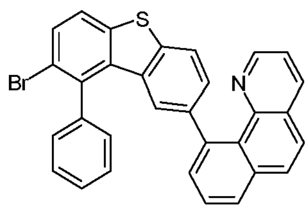 | 75% |
| 13a | 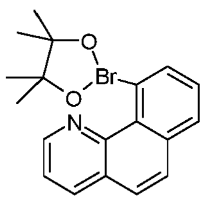 [1349171-34-1 ] | 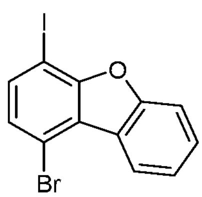 [1923736-35-9 ] | 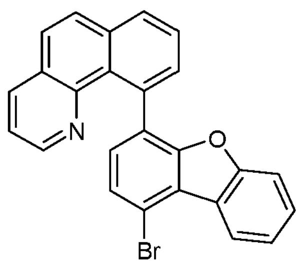 | 70% |

**Schritt b: Synthese von erfindungsgemäßen Verbindungen**

**Beispiel b: 10-(4-Dibenzothiophen-4-yl-phenyl)-benzo[h]chinolin**

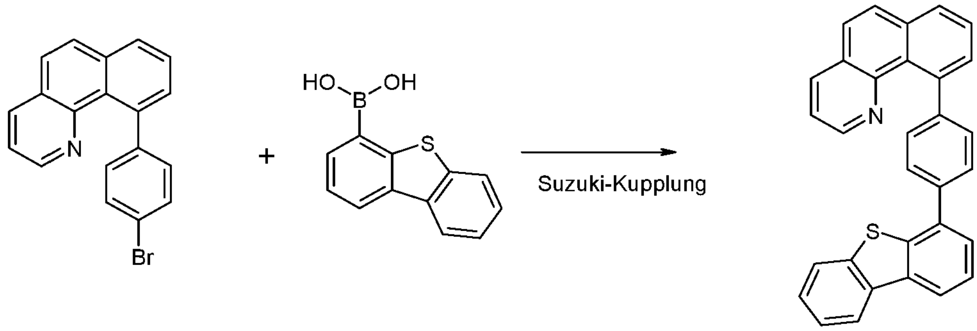


Unter analogen Bedingungen wie im Beispiel 1 werden folgende Verbindungen hergestellt:

| | Edukt 1 | Edukt 2 | Produkt | Ausbeute |
|---|---|---|---|---|
| 1b | 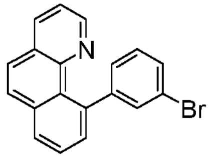 | 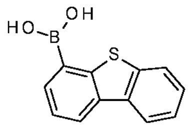 [108847-20-7 ] | 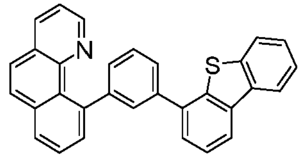 | 67% |
| 2b | 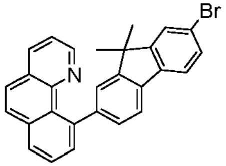 | 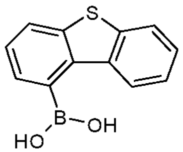 [1245943-60-5 ] | 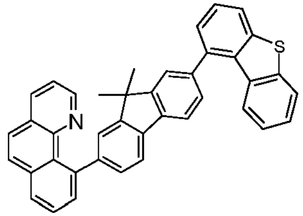 | 68% |
| 3b | 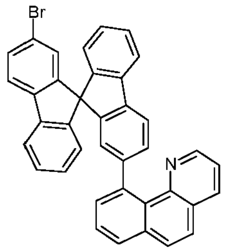 | 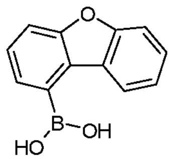 [162607-19-4 ] | 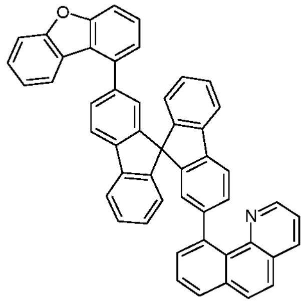 | 60% |
| 4b | 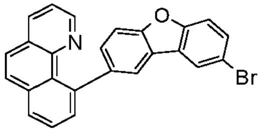 | 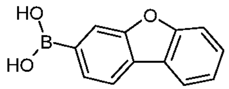 [056113-50-8 ] | 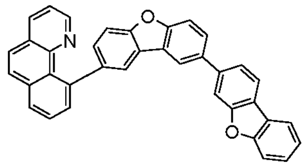 | 72% |
| 5b | 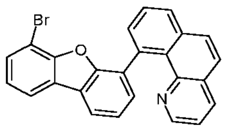 | 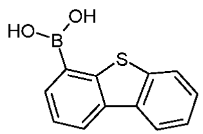 [108847-20-7 ] | 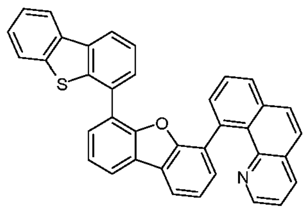 | 65% |

(fortgesetzt)

| | Edukt 1 | Edukt 2 | Produkt | Ausbeute |
|---|---|---|---|---|
| 6b | 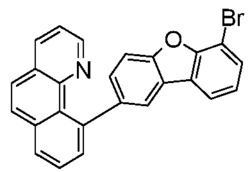 | 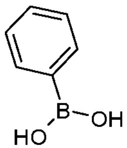 | 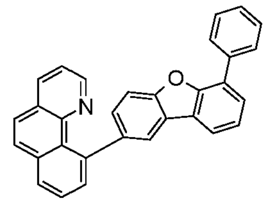 | 74% |
| 7b | 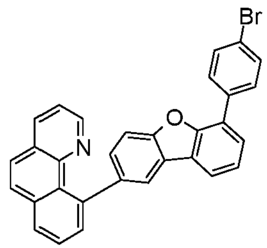 | 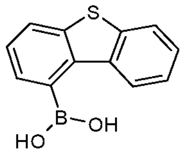 [1245943-60-5 ] | 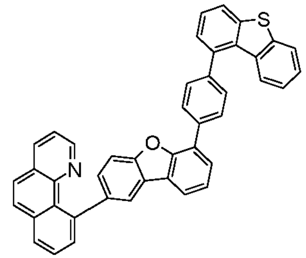 | 73% |
| 8b | 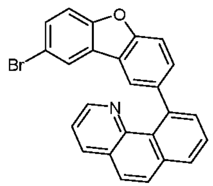 [1694627-38-7 ] | 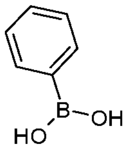 | 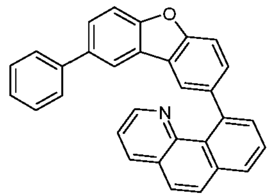 | 80% |
| 9b | 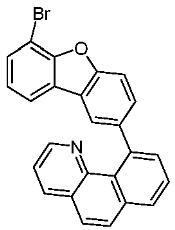 [1694627-42-3 ] | 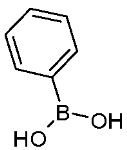 | 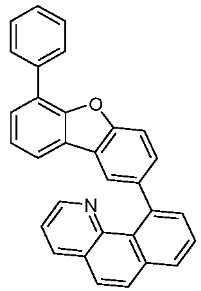 | 82% |

(fortgesetzt)

| | Edukt 1 | Edukt 2 | Produkt | Ausbeute |
|---|---|---|---|---|
| 10b | 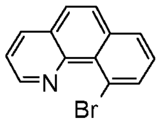 [152583-10-3] | 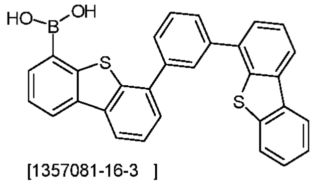 [1357081-16-3 ] | 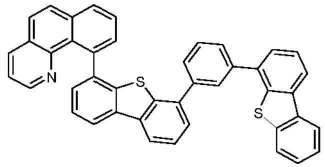 | 73% |
| Ref. 11b | 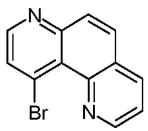 [1195222-30-0 ] | 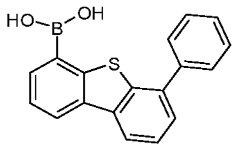 [1115640-18-0 ] | 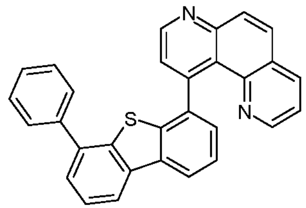 | 70% |
| Ref. 12b | 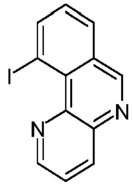 [347371-36-2 ] | 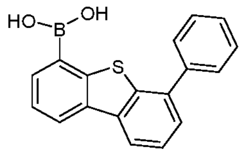 [1115640-18-0 ] | 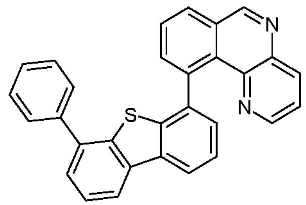 | 72% |
| Ref. 13b | 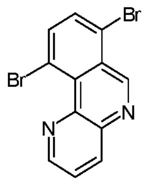 [66693-81-0 ] | 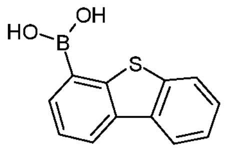 [108847-20-7 ] | 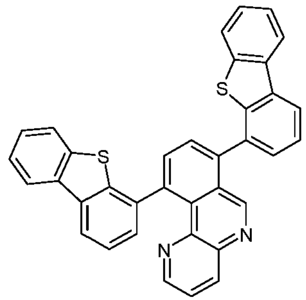 | 69% |

(fortgesetzt)

| | Edukt 1 | Edukt 2 | Produkt | Ausbeute |
|---|---|---|---|---|
| Ref. 14b | 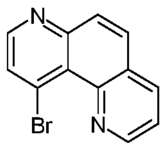 [1195222-30-0 ] | 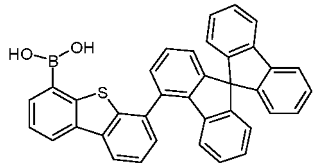 [2035077-91-7 ] | 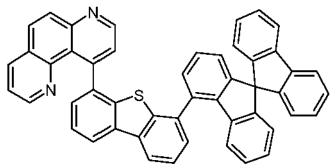 | 66% |
| 15b | 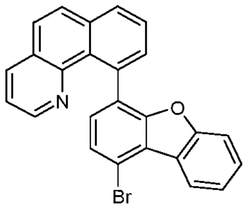 | 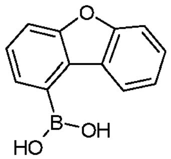 [162607-19-4 ] | 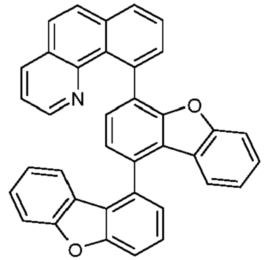 | 75% |
| 16b | 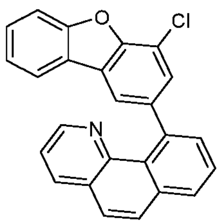 | 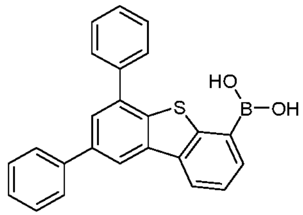 [1858289-84-5 ] | 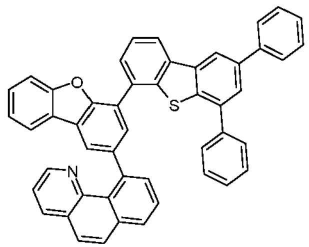 | 65% |

(fortgesetzt)

| | Edukt 1 | Edukt 2 | Produkt | Ausbeute |
|---|---|---|---|---|
| 17b | 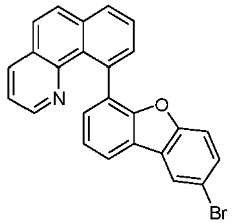 | 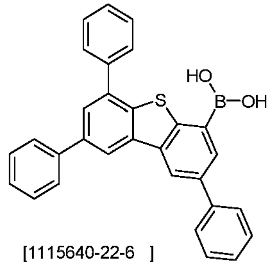 [1115640-22-6 ] | 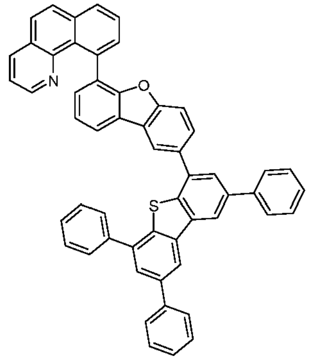 | 63% |
| 18b | 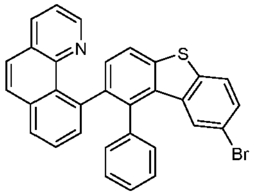 | 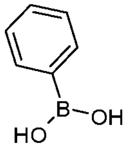 | 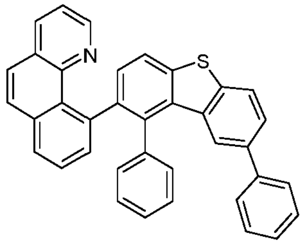 | 81% |
| 19b | 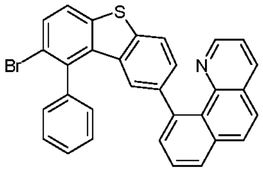 | 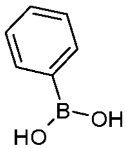 | 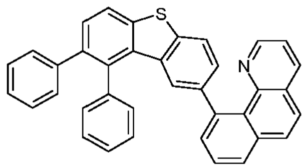 | 84% |

(fortgesetzt)

| | Edukt 1 | Edukt 2 | Produkt | Ausbeute |
|---|---|---|---|---|
| 20b | 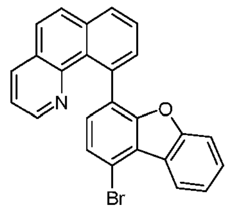 | 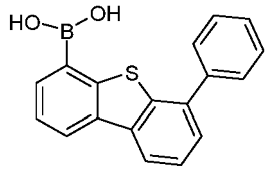 [1115640-18-0 ] | 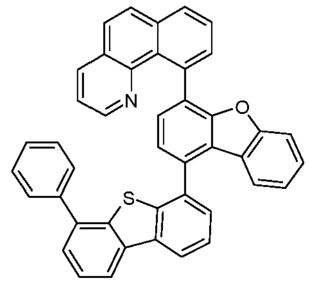 | 80% |
| Ref. 21b | 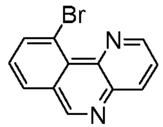 [66693-82-1] | 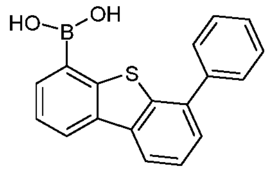 [1115640-18-0 ] | 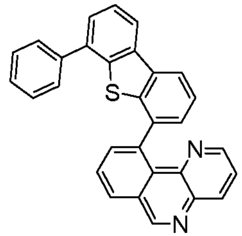 | 78% |
| Ref. 22b | 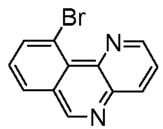 [66693-82-1] | 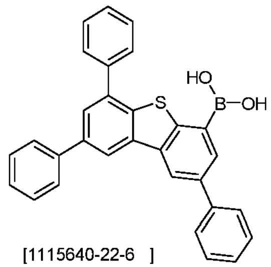 [1115640-22-6 ] | 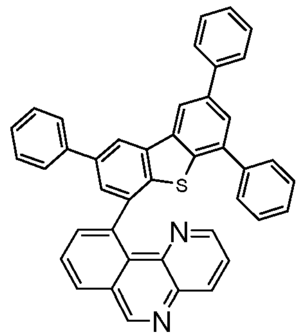 | 71% |

(fortgesetzt)

| | Edukt 1 | Edukt 2 | Produkt | Ausbeute |
|---|---|---|---|---|
| 23b | 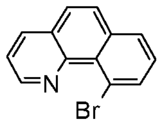 [152583-10-3] | 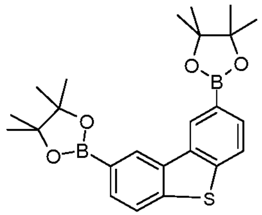 [552855-12-6] | 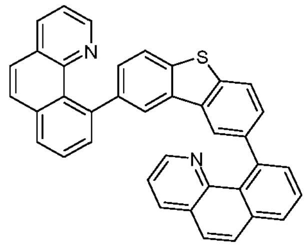 | 82% |
| 24b | 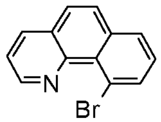 [152583-10-3] | 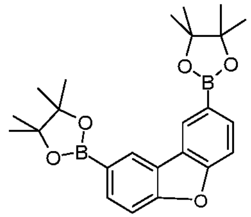 [1197989-83-5] | 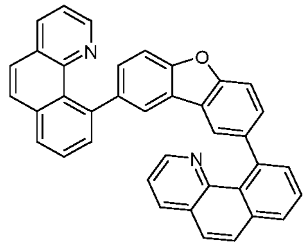 | 80% |

**Schritt c: Synthese von erfindungsgemäßen Verbindungen**

**Beispiel c: 10-(4-Biphenyl-4-yl-dibenzofuran-1-yl)-benzo[h]chinolin**

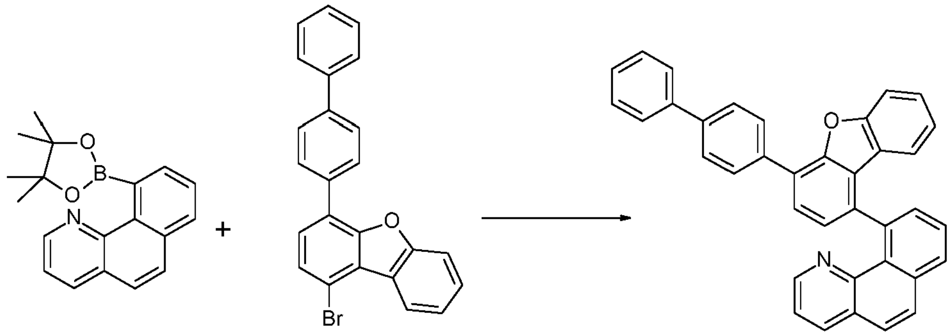

Unter analogen Bedingungen wie in Beispiel c werden ausgehend von 10-(4,4,5,5-Tetramethyl-[1,3,2]dioxaborolan-2-yl)-benzo[h]chinolin folgende Verbindungen hergestellt:

| | Edukt 1 | Edukt 2 | Produkt | Ausbeute |
|---|---|---|---|---|
| 1c | 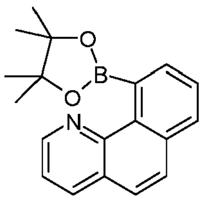 [1349171-34-1 ] | 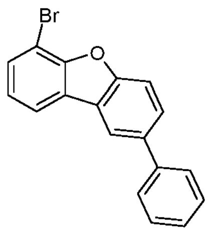 [1642127-10-3 ] | 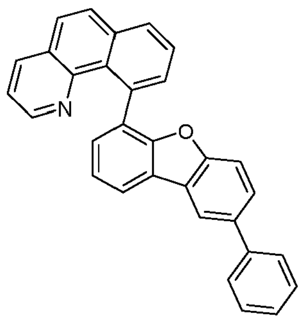 | 86% |
| 2c | 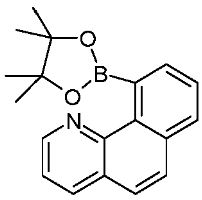 [1349171-34-1 ] | 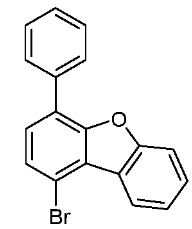 [1821235-69-1 ] | 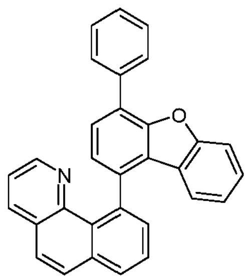 | 81% |
| 3c | 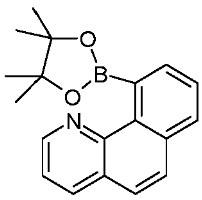 [1349171-34-1 ] | 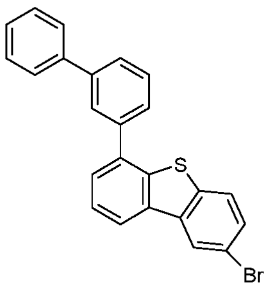 [2055856-63-6 ] | 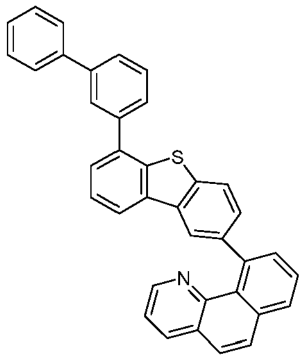 | 80% |

(fortgesetzt)

| | Edukt 1 | Edukt 2 | Produkt | Ausbeute |
|---|---|---|---|---|
| 4c | 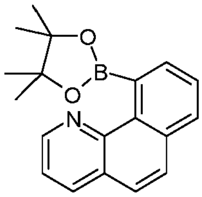 [1349171-34-1 ] | 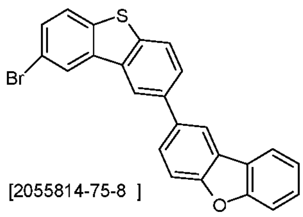 [2055814-75-8 ] | 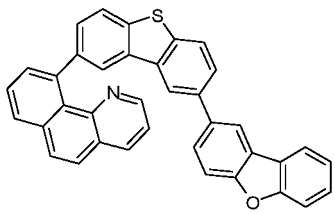 | 79% |
| 5c | 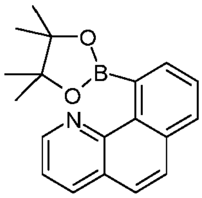 [1349171-34-1 ] | 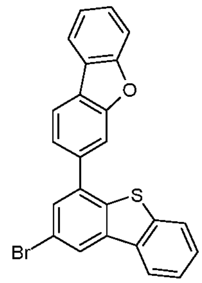 [2055814-72-5 ] | 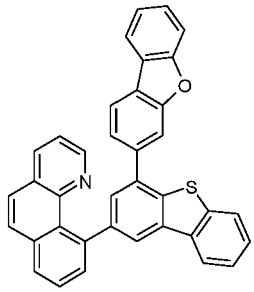 | 77% |
| 6c | 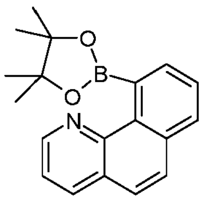 [1349171-34-1 ] | 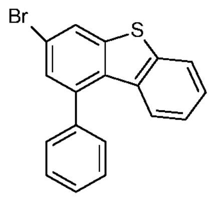 [1947345-85-8 ] | 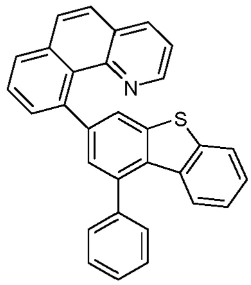 | 82% |
| 7c | 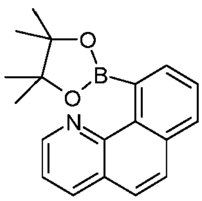 [1349171-34-1 ] | 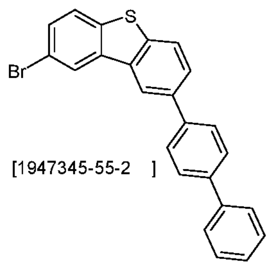 [1947345-55-2 ] | 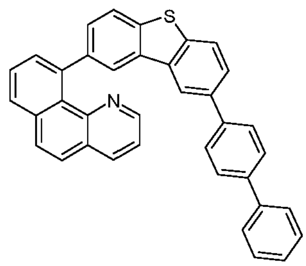 | 81% |
| 8c | 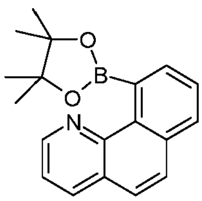 [1349171-34-1 ] | 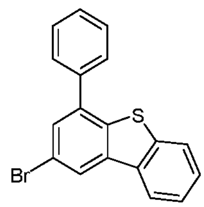 [1946844-48-9 ] | 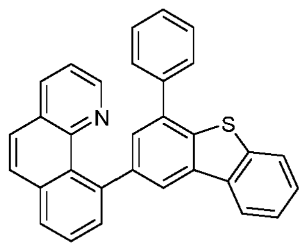 | 60% |

(fortgesetzt)

| | Edukt 1 | Edukt 2 | Produkt | Ausbeute |
|---|---|---|---|---|
| 9c | 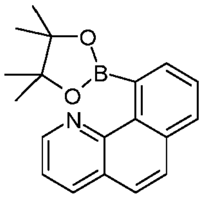 [1349171-34-1 ] | 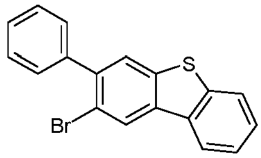 [1935723-63-9] | 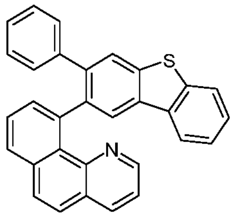 | 62% |
| 10c | 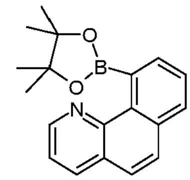 [1349171-34-1 ] | 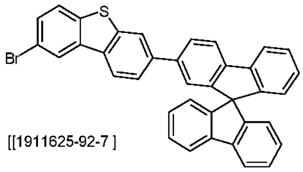 [[1911625-92-7 ] | 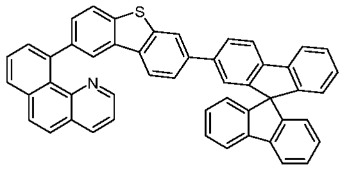 | 66% |
| 11c | 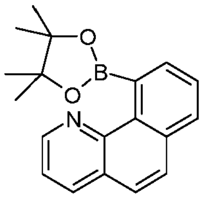 [1349171-34-1 ] | 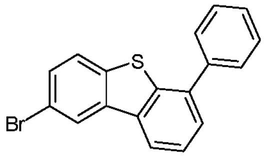 [1899869-83-0 ] | 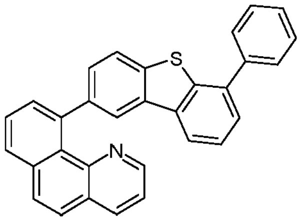 | 78% |
| 12c | 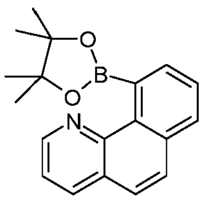 [1349171-34-1 ] | 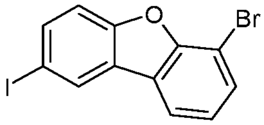 [916435-45-5 ] | 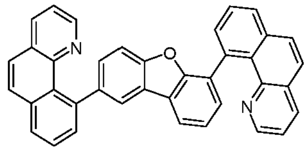 | 65% |
| 13c | 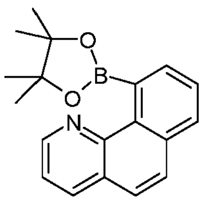 [1349171-34-1 ] | 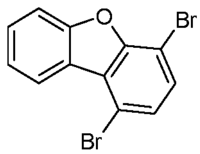 [617707-25-2 ] | 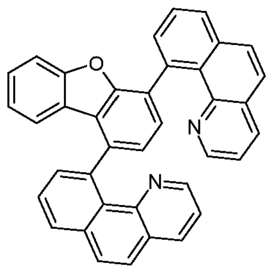 | 69% |
| 14c | 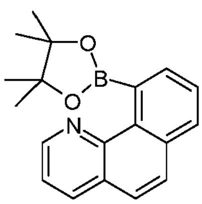 [1349171-34-1 ] | 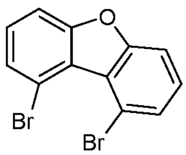 [617707-29-6 ] | 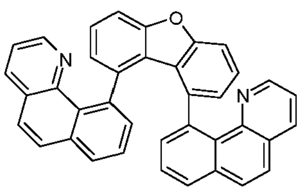 | 54% |

(fortgesetzt)

| | Edukt 1 | Edukt 2 | Produkt | Ausbeute |
|---|---|---|---|---|
| 15c | 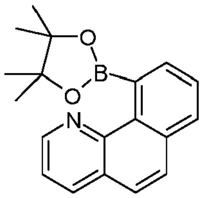 [1349171-34-1 ] | 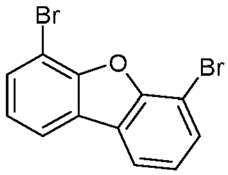 [201138-91-2 ] | 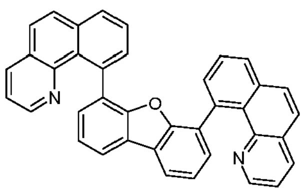 | 87% |
| 16c | 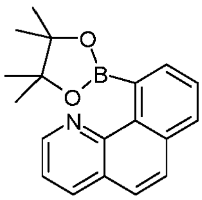 [1349171-34-1 ] | 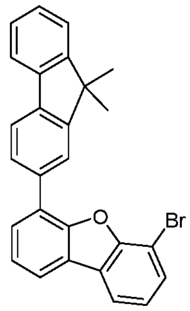 [1010069-01-8 ] | 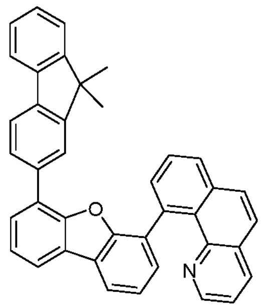 | 82% |
| 17c | 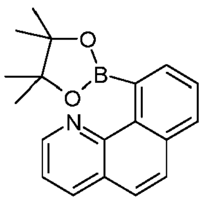 [1349171-34-1 ] | 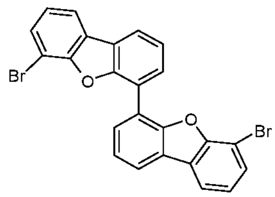 [86532-15-2 ] | 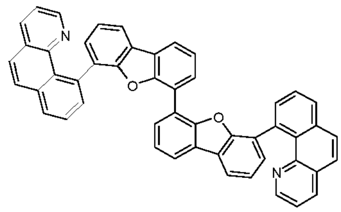 | 75% |
| 18c | 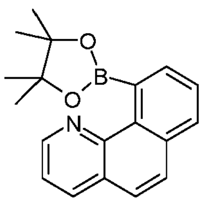 [1349171-34-1 ] | 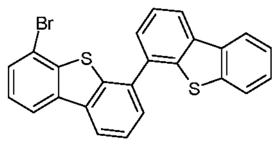 [1888414-21-8 ] | 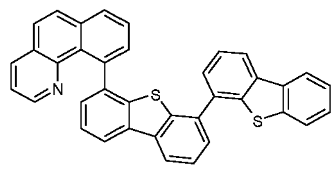 | 80% |
| 19c | 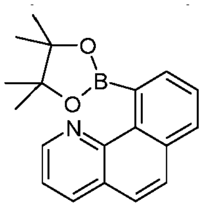 [1349171-34-1 ] | 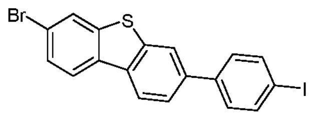 [1642127-31-8 ] | 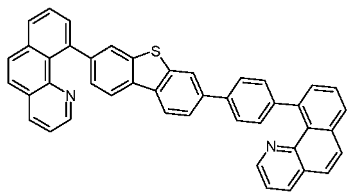 | 78% |
| 20c | 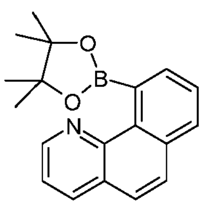 [1349171-34-1 ] | 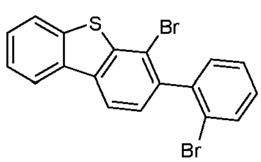 [1637248-08-8 ] | 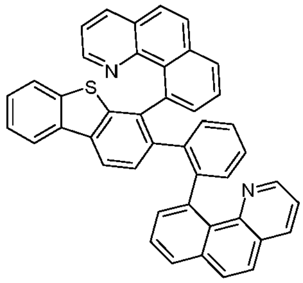 | 45% |

(fortgesetzt)

| | Edukt 1 | Edukt 2 | Produkt | Ausbeute |
|---|---|---|---|---|
| 21c | 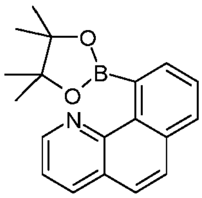 [1349171-34-1 ] | 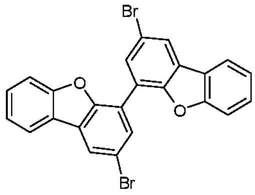 [1430422-03-9 ] | 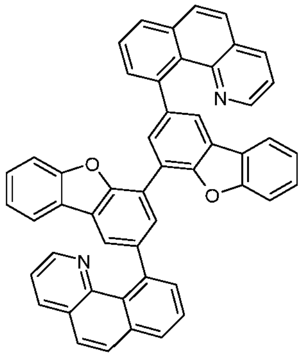 | 62% |
| 22c | 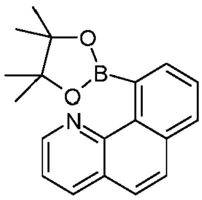 [1349171-34-1 ] | 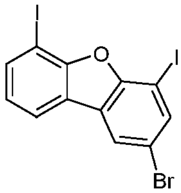 [1194601-88-1 ] | 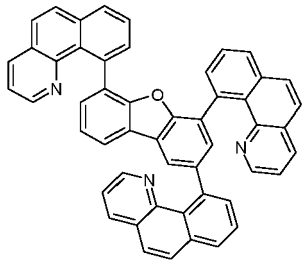 | 46% |
| 23c | 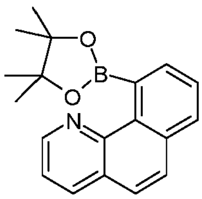 [1349171-34-1 ] | 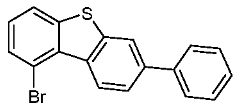 [1883821-17-7 ] | 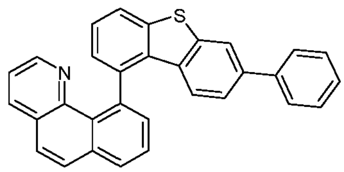 | 82% |
| 24c | 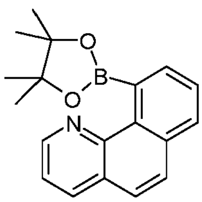 [1349171-34-1 ] | 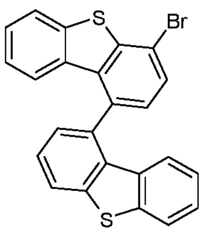 [1622179-84-3 ] | 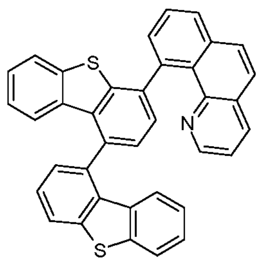 | 76% |
| 25c | 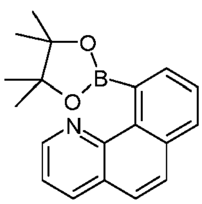 [1349171-34-1 ] | 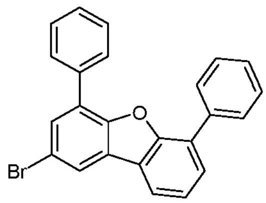 [1010069-07-4 ] | 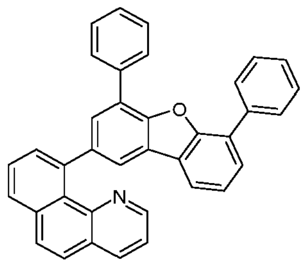 | 73% |

(fortgesetzt)

| | Edukt 1 | Edukt 2 | Produkt | Ausbeute |
|---|---|---|---|---|
| 26c | 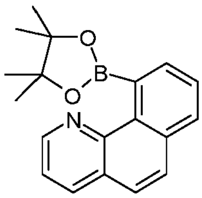 [1349171-34-1 ] | 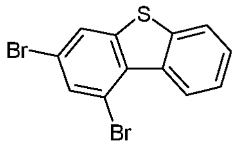 [1265215-55-1] | 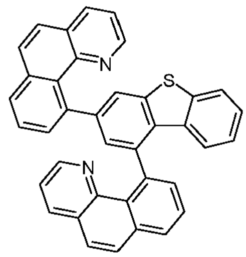 | 60% |
| 27c | 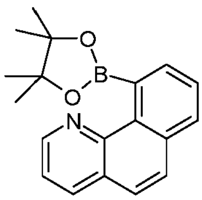 [1349171-34-1 ] | 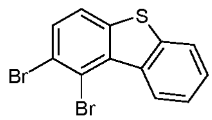 [1225467-28-6] | 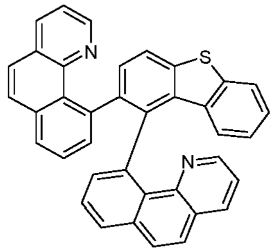 | 64% |
| 28c | 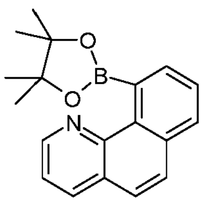 [1349171-34-1 ] | 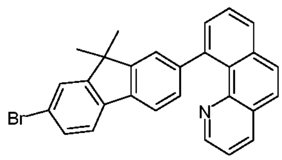 [1694627-35-4] | 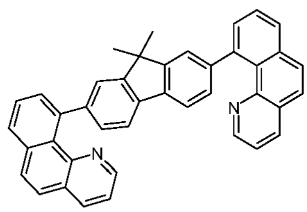 | 83% |
| 29c | 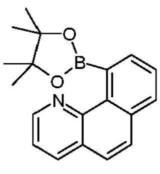 [1349171-34-1 ] | 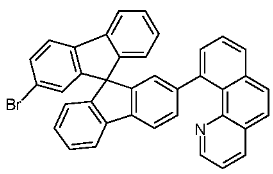 [1694627-37-6] | 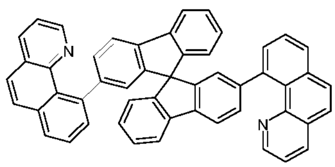 | 80% |
| 30c | 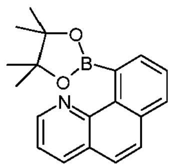 [1349171-34-1 ] | 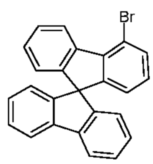 [1161009-88-6 ] | 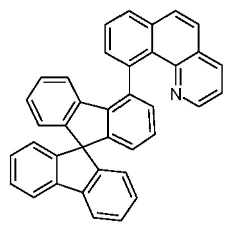 | 86% |
| 31c | 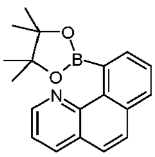 [1349171-34-1 ] | 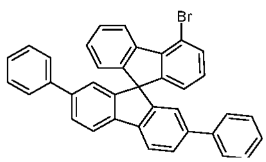 [1380085-41-5 ] | 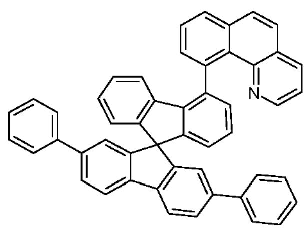 | 87% |

(fortgesetzt)

| | Edukt 1 | Edukt 2 | Produkt | Ausbeute |
|---|---|---|---|---|
| 32c | 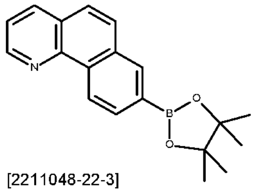 [2211048-22-3] | 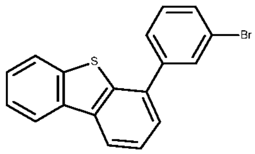 [1084334-28-0 ] | 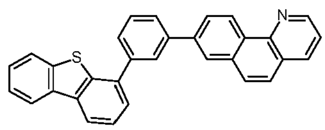 | 80% |
| 33c | 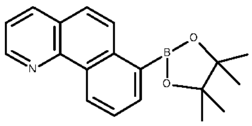 [1850288-92-4 ] | 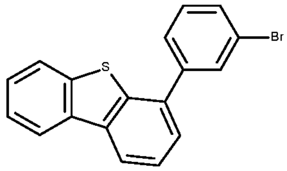 [1084334-28-0 ] | 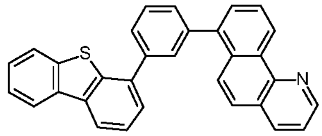 | 79% |
| 34c | 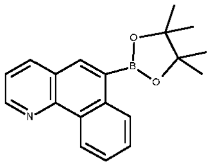 [1667729-79-4 ] | 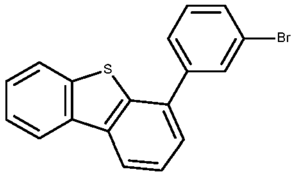 [1084334-28-0 ] | 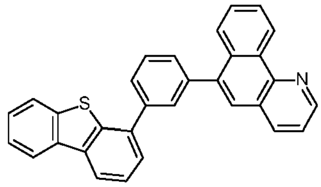 | 83% |
| 35c | 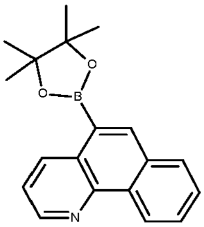 [1219975-87-7] | 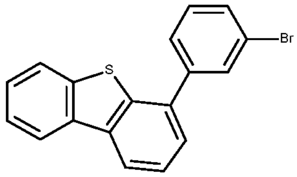 [1084334-28-0 ] | 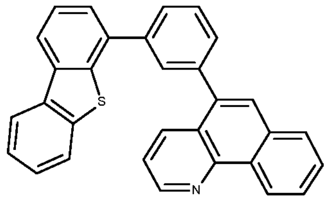 | 67% |
| 36c | 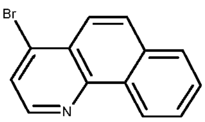 [1594678-66-6 ] | 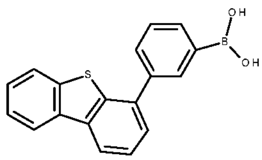 [1307859-67-1] | 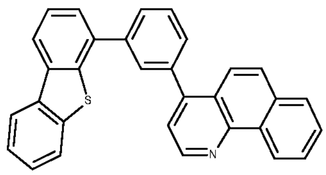 | 75% |
| 37c | 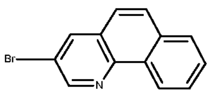 [1821675-85-7 ] | 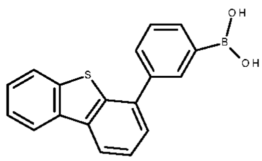 [1307859-67-1] | 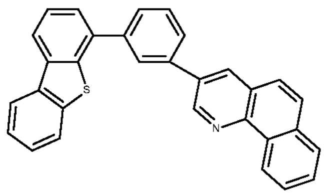 | 79% |

(fortgesetzt)

| | Edukt 1 | Edukt 2 | Produkt | Ausbeute |
|---|---|---|---|---|
| 38c | 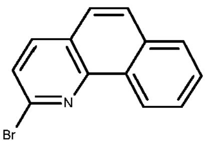 [1097204-18-6] | 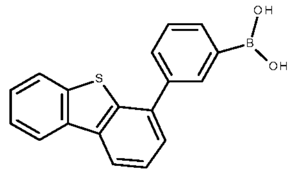 [1307859-67-1] | 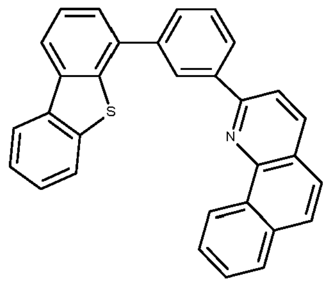 | 80% |
| 39c | 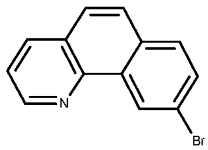 [53271-36-6 ] | 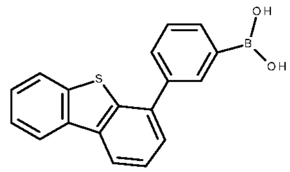 [1307859-67-1 ] | 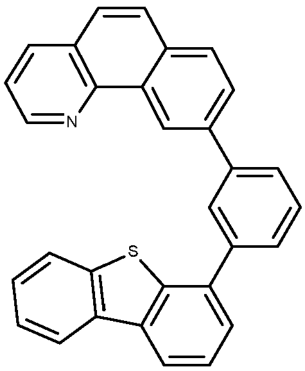 | 83% |
| 40c | 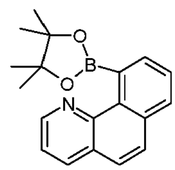 [1349171-34-1 ] | 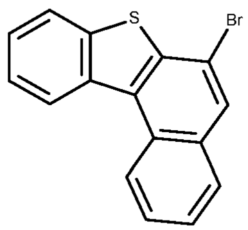 [1434286-63-19] | 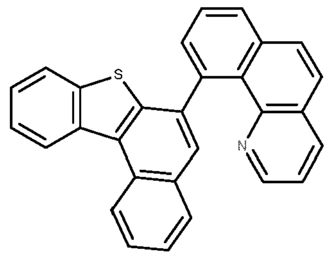 | 82% |

**B) Herstellung und Charakterisierung von OLEDs**

Glasplättchen, die mit strukturiertem ITO (Indium Zinn Oxid) der Dicke 50 nm beschichtet sind, werden vor der Beschichtung mit einem Sauerstoffplasma, gefolgt von einem Argonplasma, behandelt. Diese mit Plasma behandelten Glasplättchen bilden die Substrate, auf welche die OLEDs aufgebracht werden.

Die OLEDs haben den folgenden Schichtaufbau: Substrat / Lochinjektionsschicht (HIL) / Lochtransportschicht (HTL) / Elektronenblockierschicht (EBL) / Emissionsschicht (EML) / Lochblockierschicht (HBL) / Elektronentransportschicht (ETL) / Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs benötigten Materialien sind in Tabelle 2 gezeigt. Die Daten der OLEDs sind in Tabelle 3 aufgelistet.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Bei gemischten Schichten bedeutet eine Angabe wie IC1:1b:TEG1 (40%:40%:10%), dass das Material IC1 in einem Volumenanteil von 40%, 1b in einem Volumenanteil von 40% und TEG1 in einem Volumenanteil von 10% in der Schicht vorliegt.

Die OLEDs werden standardmäßig charakterisiert. Hierfür werden die Elektrolumineszenzspektren, die Betriebsspannung, die Stromeffizienz (SE, gemessen in cd/A) und die externe Quanteneffizienz (EQE, gemessen in %) in Abhängigkeit der Leuchtdichte, berechnet aus Strom-Spannungs-Leuchtdichte-Kennlinien unter Annahme einer lambertschen Abstrahlcharakteristik, bestimmt. Die Elektrolumineszenzspektren werden bei einer Leuchtdichte von 1000 $cd/m^2$ bestimmt und daraus die CIE 1931 x und y Farbkoordinaten berechnet. Die Angabe U1000 in Tabelle 3 bezeichnet die Spannung, die für eine Leuchtdichte von 1000 $cd/m^2$ benötigt wird. SE1000 und EQE1000 bezeichnen die Stromeffizienz bzw. die externe Quanteneffizienz, die bei 1000cd/m$^2$ erreicht werden.

Die erfindungsgemäßen Materialien können in grün phosphoreszierenden Emissionsschichten von OLEDs eingesetzt werden, wie die folgenden Beispiele zeigen:

Tabelle 1: Aufbau der OLEDs

| Bsp | HIL Dicke | HTL Dicke | EBL Dicke | EML Dicke | HBL Dicke | ETL Dicke | EIL Dicke |
|---|---|---|---|---|---|---|---|
| E1 | HATCN 5nm | SpMA1 230nm | SpMA2 20nm | IC1:1b:TEG1 (58%:30%:12%) 40nm | ST2 5nm | ST2:LiQ (50%:50%) 30nm | LiQ 1nm |
| E2 | HATCN 5nm | SpMA1 230nm | SpMA2 20nm | IC1:7b:TEG1 (58%:30%:12%) 40nm | ST2 5nm | ST2:LiQ (50%:50%) 30nm | LiQ 1nm |
| Ref. E3 | HATCN 5nm | SpMA1 230nm | SpMA2 20nm | IC1:Ref.11b:TEG1 (58%:30%:12%) 40nm | ST2 5nm | ST2:LiQ (50%:50%) 30nm | LiQ 1nm |
| E4 | HATCN 5nm | SpMA1 230nm | SpMA2 20nm | IC1:30c:TEG1 (58%:30%:12%) 40nm | ST2 5nm | ST2:LiQ (50%:50%) 30nm | LiQ 1nm |
| E5 | HATCN 5nm | SpMA1 230nm | SpMA2 20nm | IC1:32c:TEG1 (58%:30%:12%) 40nm | ST2 5nm | ST2:LiQ (50%:50%) 30nm | LiQ 1nm |
| E6 | HATCN 5nm | SpMA1 230nm | SpMA2 20nm | IC1:34c:TEG1 (58%:30%:12%) 40nm | ST2 5nm | ST2:LiQ (50%:50%) 30nm | LiQ 1nm |

| Tabelle 2: Verwendete Materialien | |
|---|---|
| 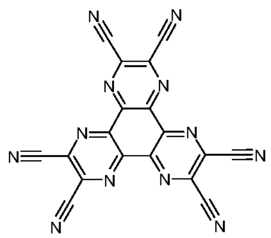 | 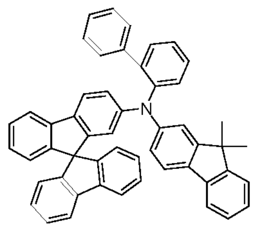 |
| HATCN | SpMA1 |
| 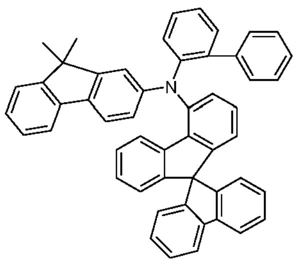 | 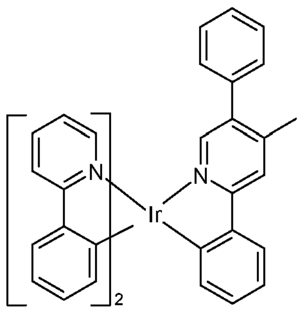 |
| SpMA3 | TEG1 |
| 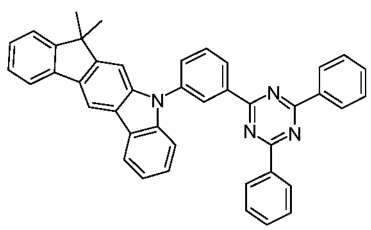 | 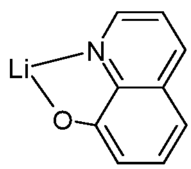 |
| IC1 | LiQ |

(fortgesetzt)

| Tabelle 2: Verwendete Materialien | |
|---|---|
| 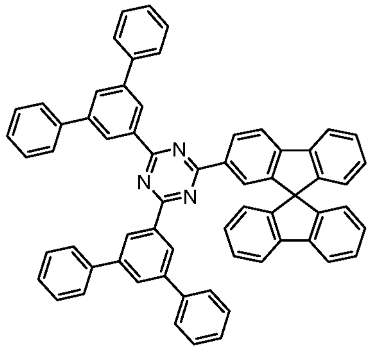 | 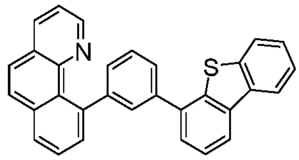 |
| ST2 | 1b |
| 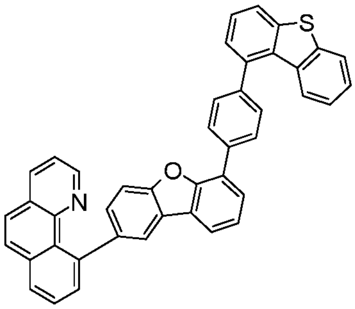 | 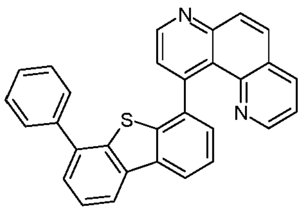 |
| 7b | Ref. 11b |
| 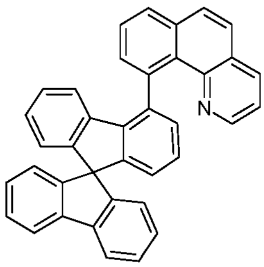 | 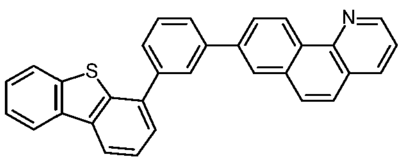 |
| 30c | 32c |
| 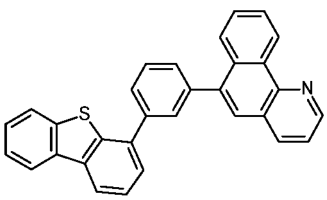 | |
| 34c | |

Tabelle 3: Daten der OLEDs

| Bsp. | U1000 (V) | SE1000 (cd/A) | EQE 1000 (%) | CIE x/y bei 1000 cd/m$^2$ |
|---|---|---|---|---|
| E1 | 3.4 | 62 | 17 | 0.32/0.62 |
| E2 | 37 | 62 | 17 | 0.32/0.62 |
| Ref. E3 | 37 | 56 | 16 | 0.32/0.62 |

(fortgesetzt)

| Bsp. | U1000 (V) | SE1000 (cd/A) | EQE 1000 (%) | CIE x/y bei 1000 cd/$m^2$ |
|---|---|---|---|---|
| E4 | 36 | 57 | 16 | 0.32/0.62 |
| E5 | 36 | 59 | 17 | 0.32/0.62 |
| E6 | 37 | 55 | 16 | 0.32/0.62 |

Die erhaltenen Daten zeigen, dass die anmeldungsgemäßen Verbindungen in OLEDs verwendet werden können. Insbesondere zeigen die oben aufgeführten OLED-Verwendungsbeispiele eine hohe Effizienz und eine niedrige Betriebsspannung.

Auch mit den Verbindungen 2b - 6b, 8b - 10b, 12b - 24b, 1c - 29c, 31c, 33c und 35c - 40c, deren Synthese oben in Teil A gezeigt wird, können OLEDs mit guter Effizienz und niedriger Betriebsspannung erhalten werden.

**Patentansprüche**

1. Verbindung gemäß Formel (I-A), (I-B), (I-C), (I-D) oder (II-A)

| | |
|---|---|
| 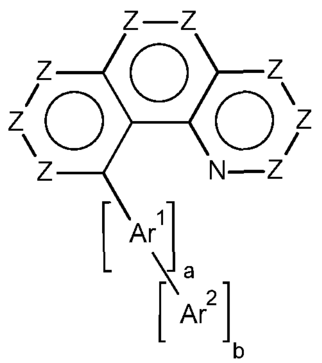 | 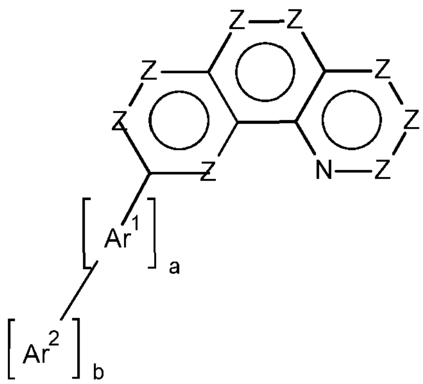 |
| Formel (I-A) | Formel (I-B) |
| 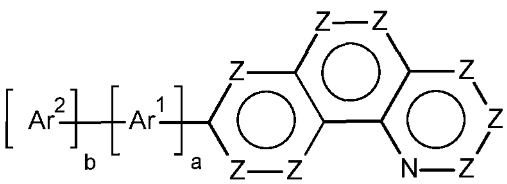 | 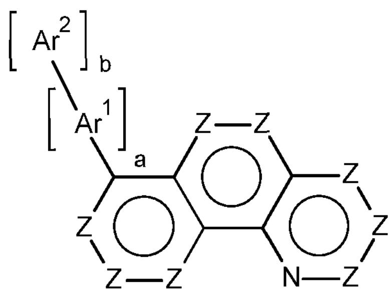 |
| Formel (I-C) | Formel (I-D) |

(fortgesetzt)

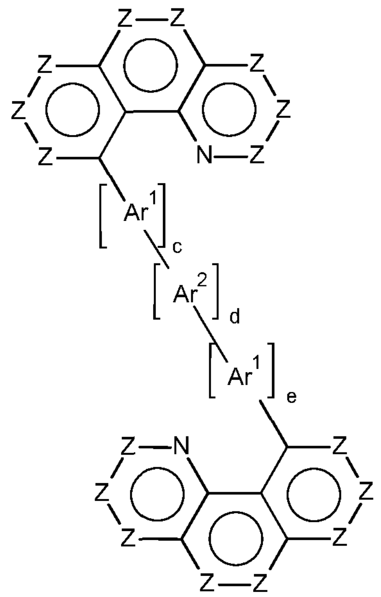

Formel (II-A)

wobei für die auftretenden Variablen gilt:

Z ist, wenn keine Einheit $Ar^1$ daran bindet, bei jedem Auftreten gleich oder verschieden gewählt aus $CR^1$; und Z ist, wenn eine Einheit $Ar^1$ daran bindet, gleich C;
$Ar^1$ ist bei jedem Auftreten gleich oder verschieden gewählt aus Einfachbindung, aromatischem Ringsystem mit 6 bis 40 aromatischen Ringatomen, das mit einem oder mehreren Resten $R^2$ substituiert sein kann, Dibenzothiophen, das mit einem oder mehreren Resten $R^2$ substituiert sein kann, und Dibenzofuran, das mit einem oder mehreren Resten $R^1$ substituiert sein kann;
$Ar^2$ ist bei jedem Auftreten gleich oder verschieden eine Gruppe gemäß Formel ($Ar^2$)

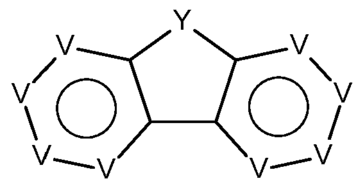

Formel ($Ar^2$),

Y ist bei jedem Auftreten gleich oder verschieden gewählt aus O, S, $C(R^3)_2$, $Si(R^3)_2$,

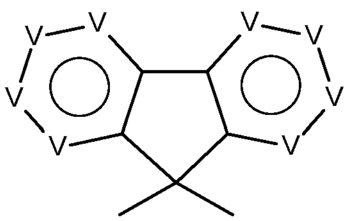

und

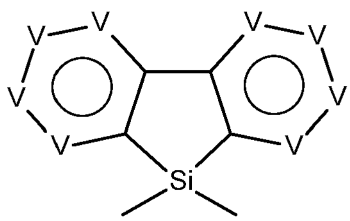

,

wobei in den Formeln

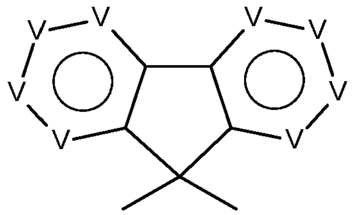

und

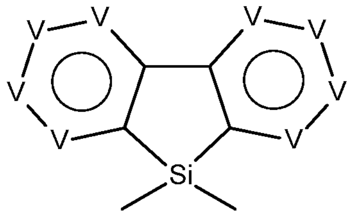

die freien Bindungen die Bindungen ausgehend von der Gruppe Y zum Rest der Gruppe der Formel ($Ar^2$) sind;
V ist bei jedem Auftreten gleich oder verschieden $CR^3$ oder N, sofern sich an der betreffenden Position nicht die Bindung an den Rest der Formel befindet, und V ist gleich C, sofern sich an der betreffenden Position die Bindung an den Rest der Formel befindet;
wobei eine oder mehrere Paare V-V jeweils durch eine Einheit gewählt aus den folgenden Einheiten

| | | |
|---|---|---|
| 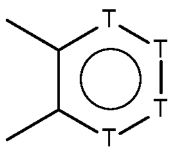 | 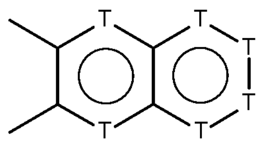 | 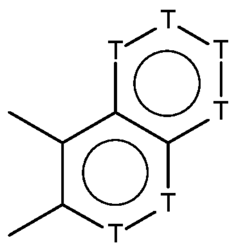 |
| (V-V-1 ) | (V-V-2) | (V-V-3) |

ersetzt sein können, wobei die freien Bindungen die Bindungen an den Rest der Formel kennzeichnen, und wobei T bei jedem Auftreten gleich oder verschieden $CR^3$ oder N ist, sofern sich an der betreffenden Position nicht die Bindung an den Rest der Formel befindet, und wobei T gleich C ist, sofern sich an der betreffenden Position die Bindung an den Rest der Formel befindet;
$R^1$, $R^2$ sind bei jedem Auftreten gleich oder verschieden gewählt aus H, D, F, $C(=O)R^4$, CN, $Si(R^4)_3$, $P(=O)(R^4)_2$, $OR^4$, $S(=O)R^4$, $S(=O)_2R^4$, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste $R^2$ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten $R^4$ substituiert sein können; und wobei eine oder mehrere $CH_2$-Gruppen in den genannten Alkyl-, Alkoxy-, Alkenyl- und Alkinylgruppen durch $-R^4C=CR^4-$, -C=C-, $Si(R^4)_2$, C=O, $C=NR^4$, -C(=O)O-, $-C(=O)NR^4-$, $P(=O)(R^4)$, -O-, -S-, SO oder $SO_2$ ersetzt sein können;
$R^3$ ist bei jedem Auftreten gleich oder verschieden gewählt aus H, D, F, $C(=O)R^4$, CN, $Si(R^4)_3$, $P(=O)(R^4)_2$, $OR^4$, $S(=O)R^4$, $S(=O)_2R^4$, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, heteroaromatischen N-freien Ringsystemen mit 5 bis 40 aromatischen Ringatomen; und elektronenarmen Heteroarylgruppen mit 6 bis 40 aromatischen Ringatomen, wobei zwei oder mehr Reste $R^3$ miteinander verknüpft sein können und einen Ring bilden können; wobei die genannten Alkyl-, Alkoxy-, Alkenyl- und Alkinylgruppen und die genannten aromatischen Ringsysteme und heteroaromatischen N-freien Ringsysteme und elektronenarmen Heteroarylgruppen jeweils mit einem oder mehreren Resten $R^4$ substituiert sein können; und wobei eine oder mehrere $CH_2$-Gruppen in den genannten Alkyl-, Alkoxy-, Alkenyl- und Alkinylgruppen durch $-R^4C=CR^4-$, -C=C-, $Si(R^4)_2$, C=O, $C=NR^4$, -C(=O)O-, $-C(=O)NR^4-$, $P(=O)(R^4)$, -O-, -S-, SO oder $SO_2$ ersetzt sein können;
$R^4$ ist bei jedem Auftreten gleich oder verschieden gewählt aus H, D, F, $C(=O)R^5$, CN, $Si(R^5)_3$, $P(=O)(R^5)_2$, $OR^5$, $S(=O)R^5$, $S(=O)_2R^5$, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste $R^4$ miteinander verknüpft sein können und einen Ring bilden können; wobei die genannten Alkyl-, Alkoxy-, Alkenyl- und Alkinylgruppen und

die genannten aromatischen Ringsysteme und heteroaromatischen Ringsysteme jeweils mit einem oder mehreren Resten $R^5$ substituiert sein können; und wobei eine oder mehrere $CH_2$-Gruppen in den genannten Alkyl-, Alkoxy-, Alkenyl- und Alkinylgruppen durch $-R^5C=CR^5-$, -C=C-, $Si(R^5)_2$, C=O, $C=NR^5$, -C(=O)O-, $-C(=O)NR^5-$, $P(=O)(R^5)$, -O-, -S-, SO oder $SO_2$ ersetzt sein können;
$R^5$ ist bei jedem Auftreten gleich oder verschieden gewählt aus H, D, F, CN, Alkyl- oder Alkoxygruppen mit 1 bis 20 C-Atomen, Alkenyl- oder Alkinylgruppen mit 2 bis 20 C-Atomen, aromatischen Ringsystemen mit 6 bis 40 aromatischen Ringatomen und heteroaromatischen Ringsystemen mit 5 bis 40 aromatischen Ringatomen; wobei die genannten Alkyl-, Alkoxy-, Alkenyl- und Alkinylgruppen, aromatischen Ringsysteme und heteroaromatischen Ringsysteme mit einem oder mehreren Resten gewählt aus F und CN substituiert sein können; und
a, b, c, d, e sind gleich oder verschieden gewählt aus 1, 2, 3 und 4.

**2.** Verbindung nach Anspruch 1, **dadurch gekennzeichnet, dass** $Ar^1$ bei jedem Auftreten gleich oder verschieden gewählt ist aus Einfachbindung und den Formeln:

| | | |
|---|---|---|
| 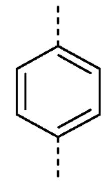 | 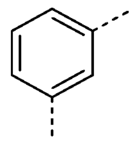 | 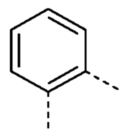 |
| $Ar^1$-1 | $Ar^1$-2 | $Ar^1$-3 |
| 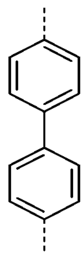 | 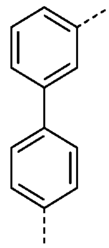 | 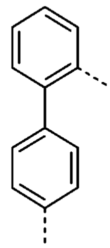 |
| $Ar^1$-4 | $Ar^1$-5 | $Ar^1$-6 |
| 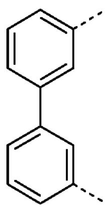 | 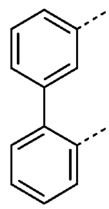 | 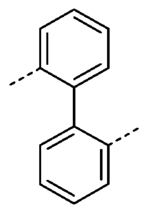 |
| $Ar^1$-7 | $Ar^1$-8 | $Ar^1$-9 |
| 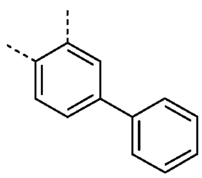 | 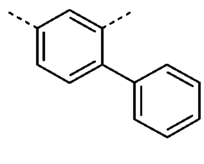 | 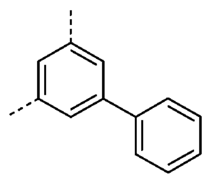 |
| $Ar^1$-10 | $Ar^1$-11 | $Ar^1$-12 |
| 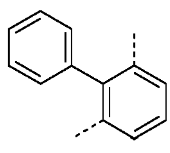 | 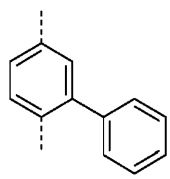 | 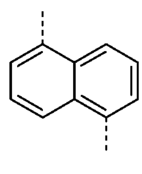 |
| $Ar^1$-13 | $Ar^1$-14 | $Ar^1$-15 |

(fortgesetzt)

| | | |
|---|---|---|
| 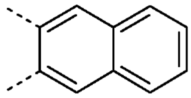 | 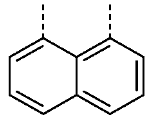 | 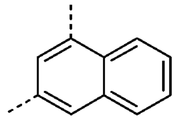 |
| $Ar^1$-16 | $Ar^1$-17 | $Ar^1$-18 |
| 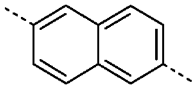 | 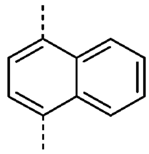 | 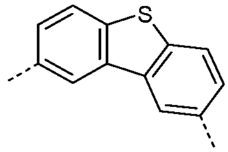 |
| $Ar^1$-19 | $Ar^1$-20 | $Ar^1$-21 |
| 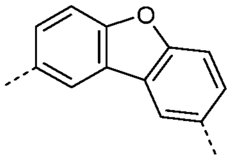 | 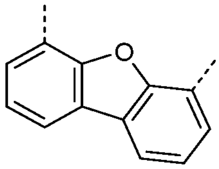 | 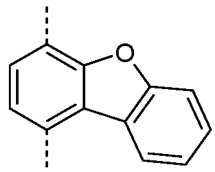 |
| $Ar^1$-22 | $Ar^1$-23 | $Ar^1$-24 |
| 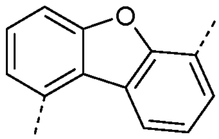 | 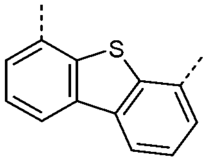 | 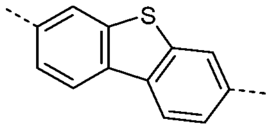 |
| $Ar^1$-25 | $Ar^1$-26 | $Ar^1$-27 |
| 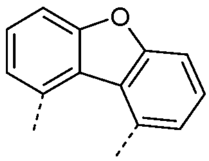 | 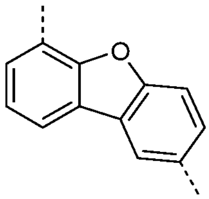 | 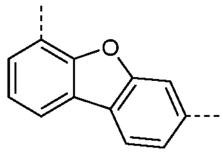 |
| $Ar^1$-28 | $Ar^1$-29 | $Ar^1$-30 |
| 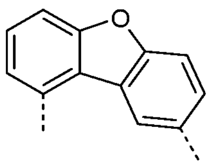 | 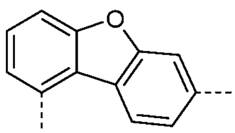 | 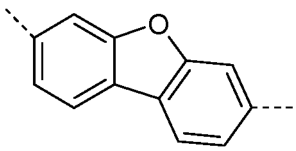 |
| $Ar^1$-31 | $Ar^1$-32 | $Ar^1$-33 |
| 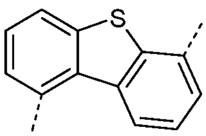 | 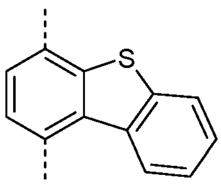 | 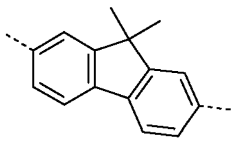 |
| $Ar^1$-34 | $Ar^1$-35 | $Ar^1$-36 |

(fortgesetzt)

| | | |
|---|---|---|
| 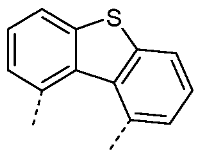 | 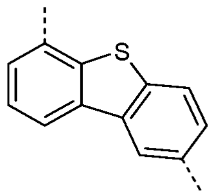 | 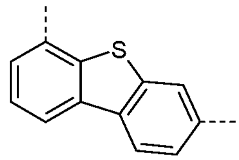 |
| $Ar^1$-37 | $Ar^1$-38 | $Ar^1$-39 |
| 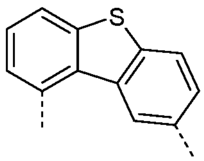 | 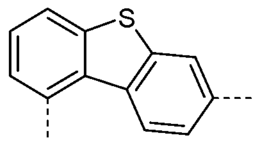 | 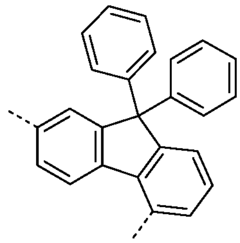 |
| $Ar^1$-40 | $Ar^1$-41 | $Ar^1$-42 |
| 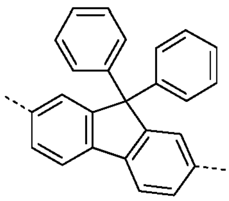 | 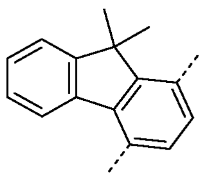 | 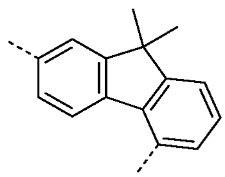 |
| $Ar^1$-43 | $Ar^1$-44 | $Ar^1$-45 |
| 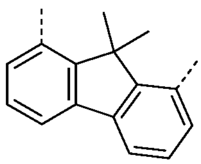 | 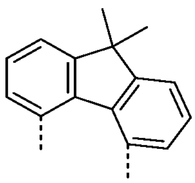 | 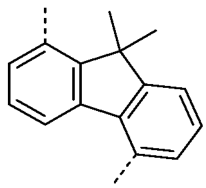 |
| $Ar^1$-46 | $Ar^1$-47 | $Ar^1$-48 |
| 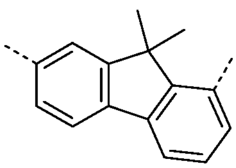 | 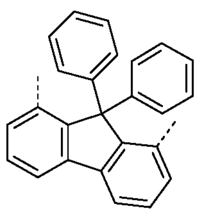 | 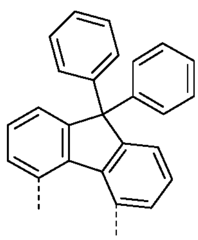 |
| $Ar^1$-49 | $Ar^1$-50 | $Ar^1$-51 |
| 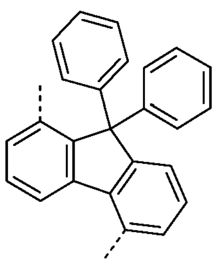 | 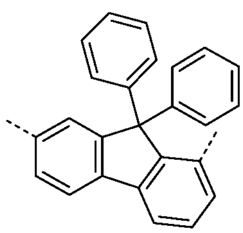 | 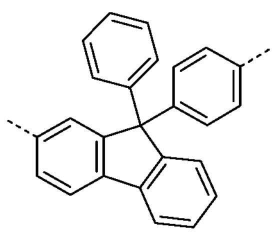 |
| $Ar^1$-52 | $Ar^1$-53 | $Ar^1$-54 |

(fortgesetzt)

| | | |
|---|---|---|
| 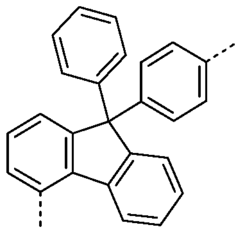 | 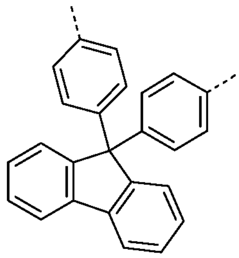 | 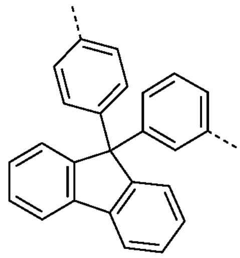 |
| $Ar^1$-55 | $Ar^1$-56 | $Ar^1$-57 |
| 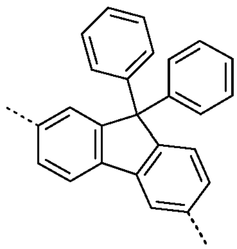 | 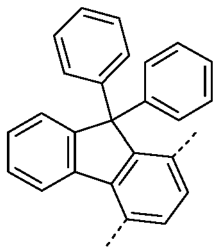 | 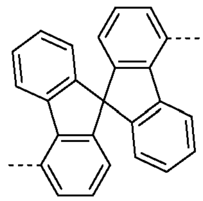 |
| $Ar^1$-58 | $Ar^1$-59 | $Ar^1$-60 |
| 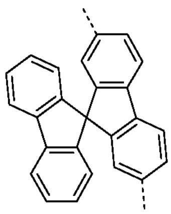 | 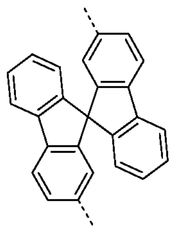 | 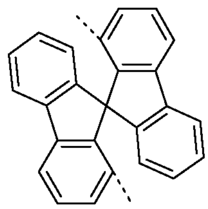 |
| $Ar^1$-61 | $Ar^1$-62 | $Ar^1$-63 |
| 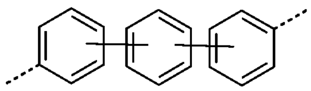 | | |
| $Ar^1$-64 | | |

wobei die Formeln jeweils mit einem oder mehreren Resten $R^2$ substituiert sein können.

**3.** Verbindung nach Anspruch 1 oder 2, **dadurch gekennzeichnet, dass** Y bei jedem Auftreten gleich oder verschieden gewählt ist aus O und S.

**4.** Verbindung nach einem oder mehreren der Ansprüche 1 bis 3, **dadurch gekennzeichnet, dass** $Ar^2$ einer Formel gewählt aus den folgenden Formeln entspricht:

| | |
|---|---|
| 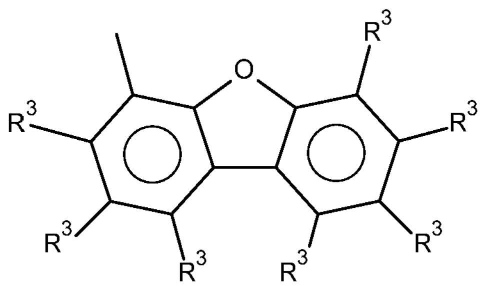 | 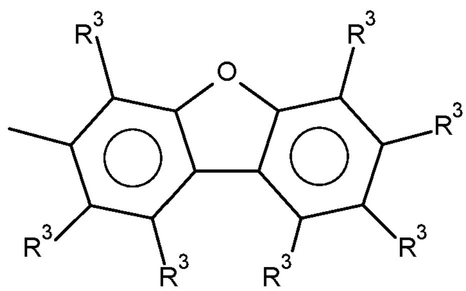 |
| Formel ($Ar^2$-1-A-1) | Formel ($Ar^2$-1-A-2) |

(fortgesetzt)

| | |
|---|---|
| 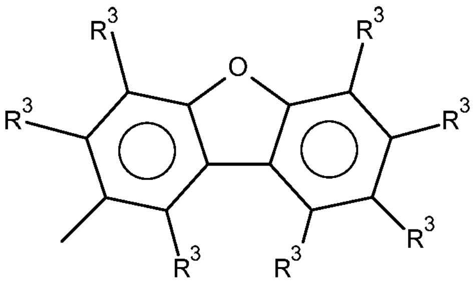 | 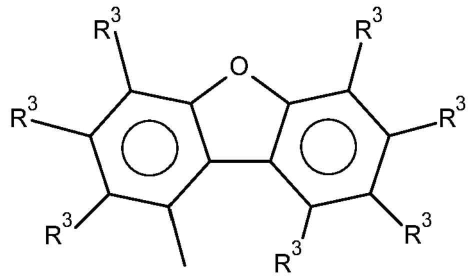 |
| Formel ($Ar^2$-1-A-3) | Formel ($Ar^2$-1-A-4) |
| 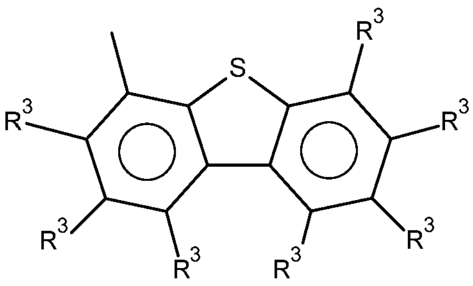 | 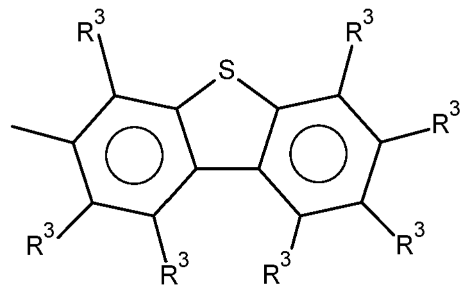 |
| Formel ($Ar^2$-2-A-1) | Formel ($Ar^2$-2-A-2) |
| 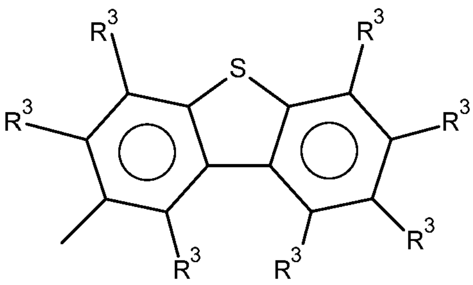 | 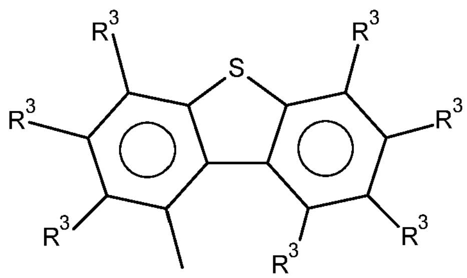 |
| Formel ($Ar^2$-2-A-3) | Formel ($Ar^2$-2-A-4) |

wobei die freie Bindung die Bindung an den Rest der Formel kennzeichnet.

5. Verbindung nach einem oder mehreren der Ansprüche 1 bis 4, **dadurch gekennzeichnet, dass** $R^1$ bei jedem Auftreten gleich oder verschieden gewählt ist aus H, D, F, CN, aromatischen Ringsystemen mit 6 bis 40 aromatischen Ringatomen, die jeweils mit einem oder mehreren Resten $R^4$ substituiert sein können, und heteroaromatischen Ringsystemen mit 5 bis 40 aromatischen Ringatomen, die jeweils mit einem oder mehreren Resten $R^4$ substituiert sein können.

6. Verbindung nach einem oder mehreren der Ansprüche 1 bis 5, **dadurch gekennzeichnet, dass** $R^2$ bei jedem Auftreten gleich oder verschieden gewählt ist aus H, D, F, CN, aromatischen Ringsystemen mit 6 bis 40 aromatischen Ringatomen, die jeweils mit einem oder mehreren Resten $R^4$ substituiert sein können, und heteroaromatischen Ringsystemen mit 5 bis 40 aromatischen Ringatomen, die jeweils mit einem oder mehreren Resten $R^4$ substituiert sein können.

7. Verbindung nach einem oder mehreren der Ansprüche 1 bis 6, **dadurch gekennzeichnet, dass** $R^3$ bei jedem Auftreten gleich oder verschieden gewählt ist aus H, D, F, CN, aromatischen Ringsystemen mit 6 bis 40 aromatischen Ringatomen, die jeweils mit einem oder mehreren Resten $R^4$ substituiert sein können, und elektronenarmen Heteroarylgruppen mit 6 bis 40 aromatischen Ringatomen, die jeweils mit einem oder mehreren Resten $R^4$ substituiert sein können.

8. Verbindung nach einem oder mehreren der Ansprüche 1 bis 7, **dadurch gekennzeichnet, dass** $R^4$ bei jedem

Auftreten gleich oder verschieden gewählt ist aus H, D, F, CN, $Si(R^5)_3$, geradkettigen Alkyl- oder Alkoxygruppen mit 1 bis 20 C-Atomen, verzweigten oder cyclischen Alkyl- oder Alkoxygruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkyl- und Alkoxygruppen, die genannten aromatischen Ringsysteme und die genannten heteroaromatischen Ringsysteme jeweils mit einem oder mehreren Resten $R^5$ substituiert sein können; und wobei in den genannten Alkyl- oder Alkoxygruppen eine oder mehrere $CH_2$-Gruppen durch -C≡C-, $-R^5C=CR^5-$, $Si(R^5)_2$, C=O, $C=NR^5$, -O-, -S-, -C(=O)O- oder $-C(=O)NR^5-$ ersetzt sein können.

9. Verbindung nach einem oder mehreren der Ansprüche 1 bis 8, **dadurch gekennzeichnet, dass** a=1 und b=1; oder **dadurch gekennzeichnet, dass** a=1 und b=2, wobei die beiden Gruppen $Ar^2$ gleich oder verschieden gewählt sind.

10. Verbindung nach einem oder mehreren der Ansprüche 1 bis 8, **dadurch gekennzeichnet, dass** c=1, e=1 und d=1; oder **dadurch gekennzeichnet, dass** c=1, e=1 und d=2, wobei die beiden Gruppen $Ar^2$ gleich oder verschieden gewählt sind.

11. Verbindung nach einem oder mehreren der Ansprüche 1 bis 10, **dadurch gekennzeichnet, dass** sie einer der Formeln (I-A-1), (I-A-2), (I-A-3), (II-A-1) oder (II-A-2) entspricht

| | |
|---|---|
| 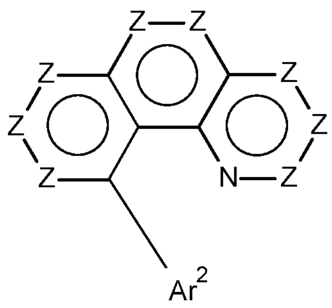 | 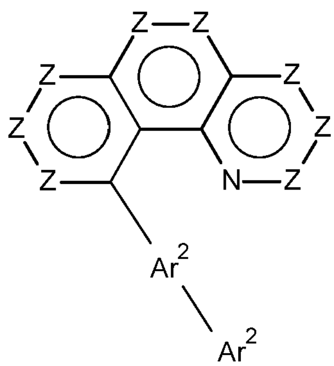 |
| Formel (I-A-1) | Formel (I-A-2) |
| 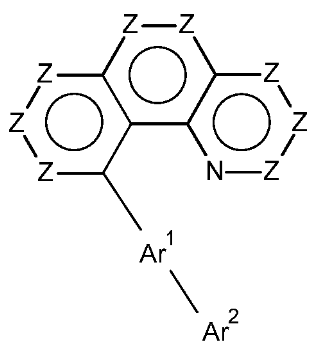 | |
| Formel (I-A-3) | |

(fortgesetzt)

| | |
|---|---|
| 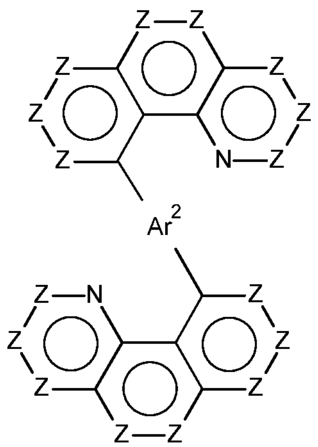 | 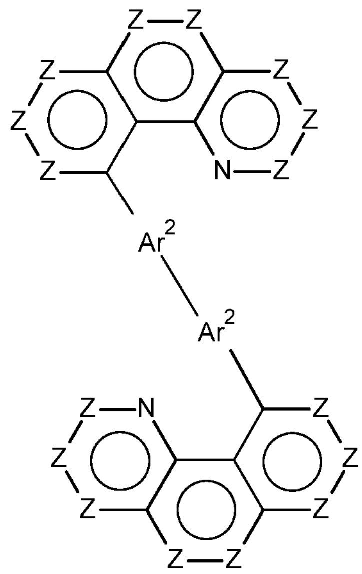 |
| Formel (II-A-1) | Formel (II-A-2) |

wobei die auftretenden Gruppen definiert sind wie in einem oder mehreren der Ansprüche 1 bis 10, mit der Ausnahme, dass $Ar^1$ keine Einfachbindung ist.

**12.** Verbindung nach einem oder mehreren der Ansprüche 1 bis 11, **dadurch gekennzeichnet, dass** sie einer der Formeln (I-A-1), (I-A-2) oder (I-A-3) entspricht

| | |
|---|---|
| 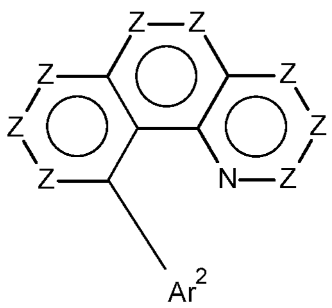 | 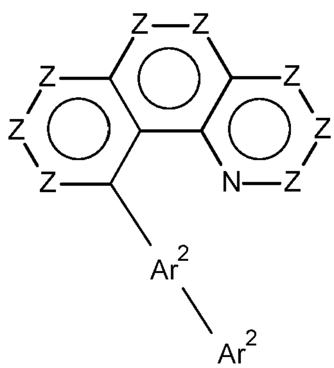 |
| Formel (I-A-1) | Formel (I-A-2) |
| 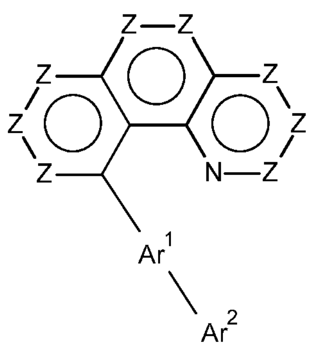 | |
| Formel (I-A-3) | |

wobei Z gleich $CR^3$ ist, $Ar^1$ definiert ist wie in Anspruch 2, $Ar^2$ definiert ist wie in Anspruch 4, $R^1$ definiert ist wie in Anspruch 5, $R^2$ definiert ist wie in Anspruch 6, $R^3$ definiert ist wie in Anspruch 7 und $R^4$ definiert ist wie in Anspruch 8.

**13.** Verfahren zur Herstellung einer Verbindung nach einem oder mehreren der Ansprüche 1 bis 12, **dadurch gekennzeichnet, dass** eine Verbindung der Formel (Z)

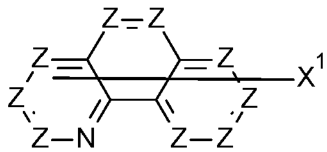

Formel (Z),

wobei $X^1$ gewählt ist aus $B(OR^4)_2$, Cl, Br und I, in einer Suzuki-Reaktion umgesetzt wird.

**14.** Oligomer, Polymer oder Dendrimer, enthaltend eine oder mehrere Verbindungen gemäß Formel (I) oder (II) nach einem oder mehreren der Ansprüche 1 bis 12, wobei die Bindung(en) zum Polymer, Oligomer oder Dendrimer an beliebigen, in Formel (I) oder (II) mit $R^1$, $R^2$, oder $R^3$ substituierten Positionen lokalisiert sein können.

**15.** Formulierung, enthaltend mindestens eine Verbindung nach einem oder mehreren der Ansprüche 1 bis 12 oder ein Polymer, Oligomer oder Dendrimer nach Anspruch 14, sowie mindestens ein Lösungsmittel.

**16.** Elektronische Vorrichtung, enthaltend mindestens eine Verbindung nach einem oder mehreren der Ansprüche 1 bis 12, oder ein Polymer, Oligomer oder Dendrimer nach Anspruch 14.

**17.** Elektronische Vorrichtung nach Anspruch 16, gewählt aus organischen Elektrolumineszenzvorrichtungen, **dadurch gekennzeichnet, dass** mindestens eine Verbindung nach einem oder mehreren der Ansprüche 1 bis 12 als Matrixmaterial in einer emittierenden Schicht zusammen mit mindestens einer phosphoreszierenden emittierenden Verbindung und mindestens einem weiteren Matrixmaterial vorliegt.

**18.** Elektronische Vorrichtung nach Anspruch 17, **dadurch gekennzeichnet, dass** das weitere Matrixmaterial gewählt ist aus bipolaren Matrixmaterialien.

**19.** Elektronische Vorrichtung nach Anspruch 17, **dadurch gekennzeichnet, dass** das weitere Matrixmaterial gewählt ist aus Verbindungen der Formeln (BP-1) oder (BP-2)

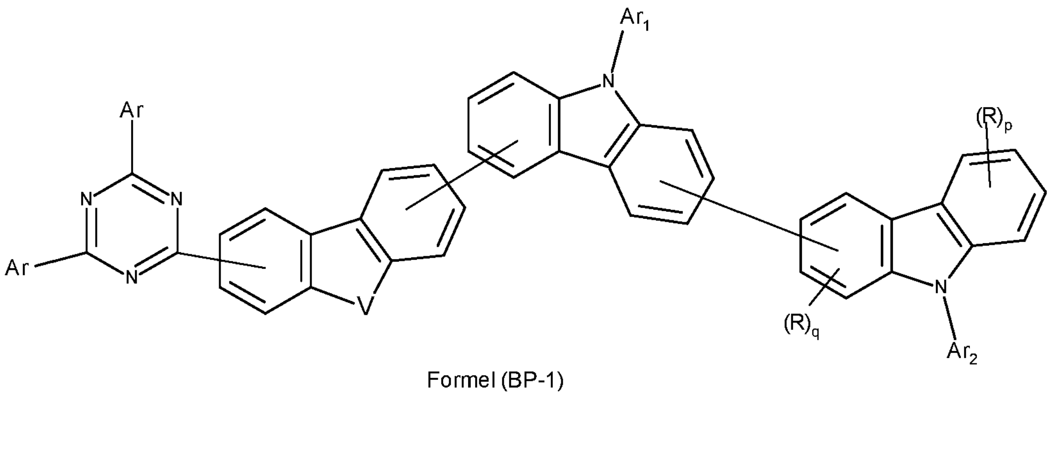

Formel (BP-1)

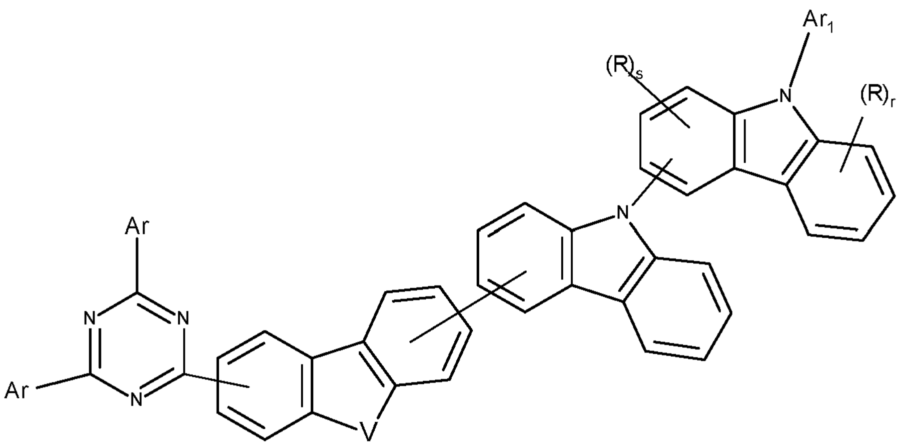

Formel (BP-2))

wobei für die auftretenden Variablen gilt:

V ist O oder S;

Ar, $Ar_1$, $Ar_2$ sind jeweils unabhängig voneinander bei jedem Auftreten eine Aryl- oder Heteroarylgruppe mit 5 bis 40 Ringatomen, die mit einem oder mehreren Resten $R_3$ substituiert sein können oder ein aromatisches oder heteroaromatisches Ringsystem mit 6 bis 40 Ringatomen, das mit einem oder mehreren Resten $R_3$ substituiert sein kann;

p, q sind jeweils unabhängig voneinander 0, 1, 2, 3 oder 4;

s, r sind jeweils unabhängig voneinander 0, 1, 2, 3 oder 4;

R ist bei jedem Auftreten gleich oder verschieden ausgewählt aus der Gruppe bestehend aus D, F, Cl, Br, I, CN, $NO_2$, $N(Ar)_2$, $N(R_2)_2$, C(=O)Ar, $C(=O)R_2$, $P(=O)(Ar)_2$, $P(Ar)_2$, $B(Ar)_2$, $Si(Ar)_3$, $Si(R_2)_3$, einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen oder einer Alkenylgruppe mit 2 bis 20 C-Atomen, die jeweils mit einem oder mehreren Resten $R^2$ substituiert sein kann, wobei eine oder mehrere nicht-benachbarte $CH_2$-Gruppen durch $R_2C=CR_2$, $Si(R_2)_2$, C=O, C=S, $C=NR_2$, $P(=O)(R_2)$, SO, $SO_2$, $NR_2$, O, S oder $CONR_2$ ersetzt sein können und wobei ein oder mehrere H-Atome durch D, F, Cl, Br, I, CN oder $NO_2$ ersetzt sein können, einem aromatischen oder heteroaromatischen Ringsystem mit 5 bis 40 Ringatomen, das jeweils mit einem oder mehreren Resten $R_2$ substituiert sein kann, einer Aryloxy- oder Heteroaryloxygruppe mit 5 bis 40 Ringatomen, die mit einem oder mehreren Resten $R_2$ substituiert sein kann, oder einer Aralkyl- oder Heteroaralkylgruppe mit 5 bis 40 Ringatomen, die mit einem oder mehreren Resten $R_2$ substituiert sein kann; dabei kann maximal ein Substituent R zusammen mit Ar1 ein monocyclisches oder polycyclisches, aliphatisches, aromatisches oder heteroaromatisches Ringsystem bilden, das mit einem oder mehreren Resten $R_2$ substituiert sein kann;

$R_2$ ist bei jedem Auftreten gleich oder verschieden ausgewählt aus der Gruppe bestehend aus H, D, F, Cl, Br, I, CN, $NO_2$, $N(Ar)_2$, $NH_2$, $N(R_3)_2$, C(=O)Ar, C(=O)H, $C(=O)R_3$, $P(=O)(Ar)_2$, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, die jeweils mit einem oder mehreren Resten $R_3$ substituiert sein kann, wobei eine oder mehrere nicht-benachbarte $CH_2$-Gruppen durch HC=CH, $R_3C=CR_3$, C=C, $Si(R^3)_2$, $Ge(R_3)_2$, $Sn(R_3)_2$, C=O, C=S, C=Se, $C=NR_3$, $P(=O)(R_3)$, SO, $SO_2$, NH, $NR_3$, O, S, CONH oder $CONR_3$ ersetzt sein können und wobei ein oder mehrere H-Atome durch D, F, Cl, Br, I, CN oder $NO_2$ ersetzt sein können, einem aromatischen oder heteroaromatischen Ringsystem mit 5 bis 60 Ringatomen, das jeweils mit einem oder mehreren Resten $R_3$ substituiert sein kann, einer Aryloxy- oder Heteroaryloxygruppe mit 5 bis 60 Ringatomen, die mit einem oder mehreren Resten $R_3$ substituiert sein kann, oder einer Kombination dieser Systeme, wobei optional zwei oder mehr benachbarte Substituenten $R_2$ ein monocyclisches oder polycyclisches, aliphatisches, aromatisches oder heteroaromatisches Ringsystem bilden können, das mit einem oder mehreren Resten $R_3$ substituiert sein kann;

$R_3$ ist bei jedem Auftreten gleich oder verschieden ausgewählt aus der Gruppe bestehend aus H, D, F, CN, einem aliphatischen Kohlenwasserstoffrest mit 1 bis 20 C-Atomen oder einem aromatischen oder heteroaromatischen Ringsystem mit 5 bis 30 Ringatomen, in dem ein oder mehrere H-Atome durch D, F, Cl, Br, I oder CN ersetzt sein können und das durch ein oder mehrere Alkylgruppen mit jeweils 1 bis 4 Kohlenstoffatomen substituiert sein kann; dabei können zwei oder mehr benachbarte Substituenten $R_3$ miteinander ein mono- oder polycyclisches, aliphatisches Ringsystem bilden.

**20.** Mischung, enthaltend mindestens eine Verbindung gemäß einem oder mehreren der Ansprüche 1 bis 12, und mindestens eine weitere Verbindung, die gewählt ist aus bipolaren Matrixmaterialien.

**21.** Verwendung einer Verbindung nach einem oder mehreren der Ansprüche 1 bis 12 in einer elektronischen Vorrichtung.

**Claims**

**1.** Compound of the formula (I-A), (I-B), (I-C), (I-D) or (II-A)

| | |
|---|---|
| 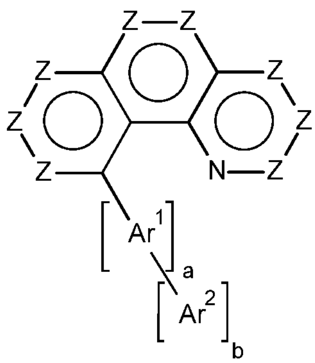 | 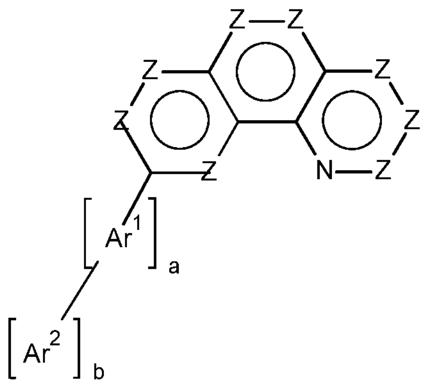 |
| formula (I-A) | formula (I-B) |
| 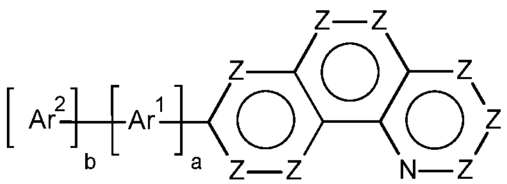 | 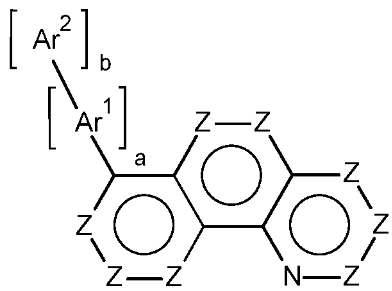 |
| formula (I-C) | formula (I-D) |
| 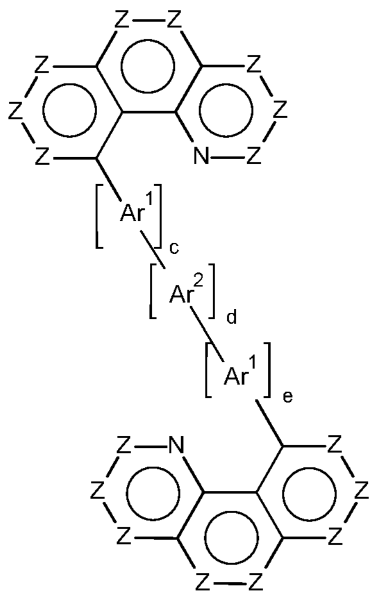 | |
| formula (II-A) | |

where the following applies to the variables occurring:

Z, if no unit $Ar^1$ is bonded thereto, is selected on each occurrence, identically or differently, from $CR^1$; and Z, if an unit $Ar^1$ is bonded thereto, is equal to C;
$Ar^1$ is selected on each occurrence, identically or differently, from a single bond, an aromatic ring system having 6 to 40 aromatic ring atoms, which may be substituted by one or more radicals $R^2$, dibenzothiophene, which may be substituted by one or more radicals $R^2$, and dibenzofuran, which may be substituted by one or more radicals $R^1$;
$Ar^2$ is on each occurrence, identically or differently, a group of the formula ($Ar^2$)

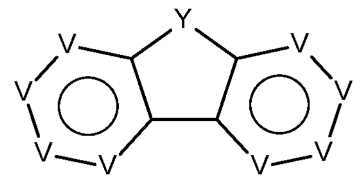

formula ($Ar^2$),

Y is selected on each occurrence, identically or differently, from O, S, $C(R^3)_2$, $Si(R^3)_2$,

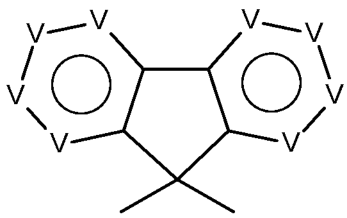 and 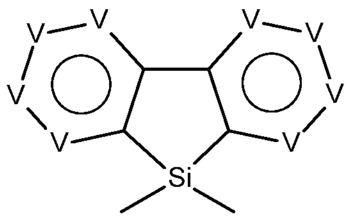 ,

where, in the formulae

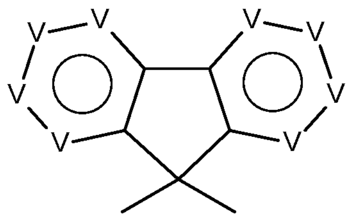 and 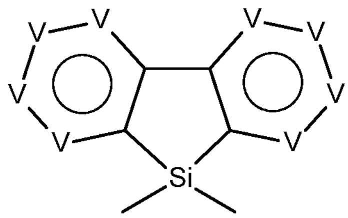 ,

the free bonds are the bonds emanating from the group Y to the remainder of the group of the formula ($Ar^2$);
V is on each occurrence, identically or differently, $CR^3$ or N if the bond to the remainder of the formula is not located at the position in question, and V is equal to C if the bond to the remainder of the formula is located at the position in question;
where one or more pairs V-V may in each case be replaced by a unit selected from the following units:

| | | |
|---|---|---|
| 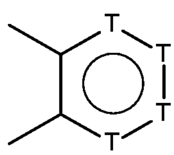 | 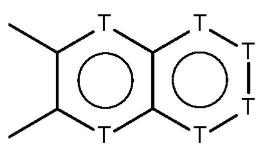 | 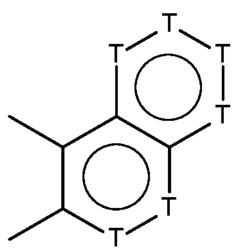 |
| (V-V-1) | (V-V-2) | (V-V-3) |

where the free bonds denote the bonds to the remainder of the formula, and where T is on each occurrence, identically or differently, $CR^3$ or N if the bond to the remainder of the formula is not located at the position in question, and where T is equal to C if the bond to the remainder of the formula is located at the position in question;
$R^1$, $R^2$ are selected on each occurrence, identically or differently, from H, D, F, $C(=O)R^4$, CN, $Si(R^4)_3$, $P(=O)(R^4)_2$, $OR^4$, $S(=O)R^4$, $S(=O)_2R^4$, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; where two or more radicals $R^2$ may be linked to one another and may form a ring; where the said alkyl, alkoxy, alkenyl and alkynyl groups and the said aromatic ring systems and heteroaromatic ring systems may in each case be substituted by one or more radicals $R^4$; and where one or more $CH_2$ groups in the said alkyl, alkoxy, alkenyl and alkynyl groups may be replaced by $-R^4C=CR^4-$, -C=C-, $Si(R^4)_2$, C=O, $C=NR^4$, -C(=O)O-, $-C(=O)NR^4-$, $P(=O)(R^4)$, -O-, -S-, SO or $SO_2$;
$R^3$ is selected on each occurrence, identically or differently, from H, D, F, $C(=O)R^4$, CN, $Si(R^4)_3$, $P(=O)(R^4)_2$, $OR^4$, $S(=O)R^4$, $S(=O)_2R^4$, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, heteroaromatic N-free ring systems having 5 to 40 aromatic ring atoms, and electron-deficient heteroaryl groups having 6 to 40 aromatic ring atoms; where two or more radicals $R^3$ may be linked to one another and may form a ring; where the said alkyl, alkoxy, alkenyl and alkynyl groups and the said aromatic ring systems and heteroaromatic N-free ring systems and electron-deficient heteroaryl groups may in each case be substituted by one or more radicals $R^4$; and where one or more $CH_2$ groups in the said alkyl, alkoxy, alkenyl and alkynyl groups may be replaced by $-R^4C=CR^4-$, -C≡C-, $Si(R^4)_2$, C=O, $C=NR^4$, -C(=O)O-, $-C(=O)NR^4-$, $P(=O)(R^4)$, -O-, -S-, SO or $SO_2$;
$R^4$ is selected on each occurrence, identically or differently, from H, D, F, $C(=O)R^5$, CN, $Si(R^5)_3$, $P(=O)(R^5)_2$, $OR^5$, $S(=O)R^5$, $S(=O)_2R^5$, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; where two or more radicals $R^4$ may be linked to one another and may form a ring; where the said alkyl, alkoxy, alkenyl and alkynyl groups and the said aromatic ring systems and heteroaromatic ring systems may in each case be substituted by one or more radicals $R^5$; and where one or more $CH_2$ groups in the said alkyl, alkoxy, alkenyl and alkynyl groups may be replaced by $-R^5C=CR^5-$, -C≡C-, $Si(R^5)_2$, C=O, $C=NR^5$, -C(=O)O-, $-C(=O)NR^5-$, $P(=O)(R^5)$, -O-, -S-, SO or $SO_2$;
$R^5$ is selected on each occurrence, identically or differently, from H, D, F, CN, alkyl or alkoxy groups having 1 to 20 C atoms, alkenyl or alkynyl groups having 2 to 20 C atoms, aromatic ring systems having 6 to 40 aromatic ring atoms and heteroaromatic ring systems having 5 to 40 aromatic ring atoms; where the said alkyl, alkoxy, alkenyl and alkynyl groups, aromatic ring systems and heteroaromatic ring systems may be substituted by one or more radicals selected from F and CN; and
a, b, c, d, e are selected, identically or differently, from 1, 2, 3 and 4.

2. Compound according to Claim 1, **characterised in that** $Ar^1$ is selected on each occurrence, identically or differently, from a single bond and the formulae:

| 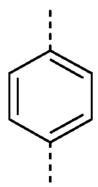 | 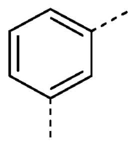 | 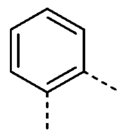 |
|---|---|---|
| $Ar^1$-1 | $Ar^1$-2 | $Ar^1$-3 |
| 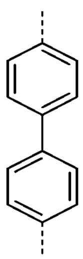 | 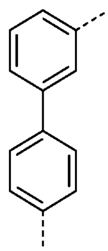 | 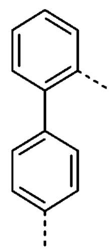 |

(continued)

| $Ar^1$-4 | $Ar^1$-5 | $Ar^1$-6 |
|---|---|---|
| 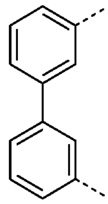 | 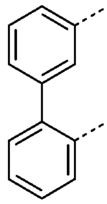 | 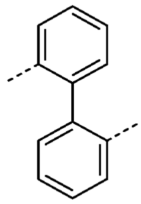 |
| $Ar^1$-7 | $Ar^1$-8 | $Ar^1$-9 |
| 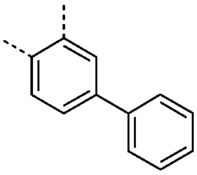 | 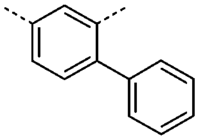 | 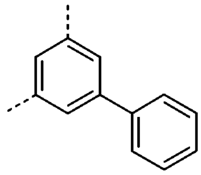 |
| $Ar^1$-10 | $Ar^1$-11 | $Ar^1$-12 |
| 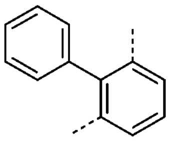 | 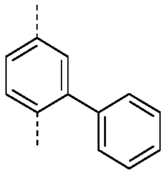 | 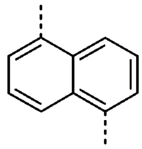 |
| $Ar^1$-13 | $Ar^1$-14 | $Ar^1$-15 |
| 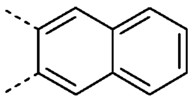 | 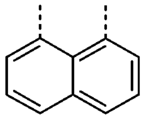 | 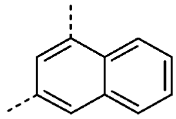 |
| $Ar^1$-16 | $Ar^1$-17 | $Ar^1$-18 |
| 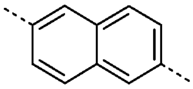 | 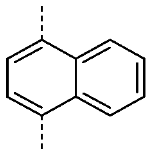 | 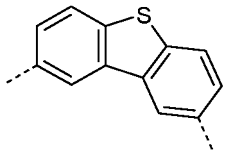 |
| $Ar^1$-19 | $Ar^1$-20 | $Ar^1$-21 |
| 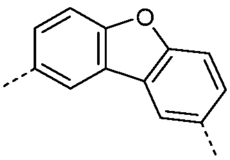 | 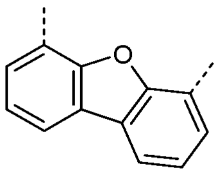 | 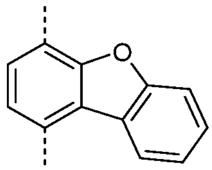 |
| $Ar^1$-22 | $Ar^1$-23 | $Ar^1$-24 |
| 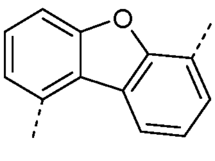 | 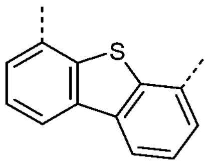 | 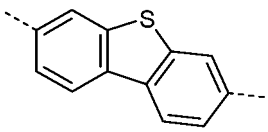 |
| $Ar^1$-25 | $Ar^1$-26 | $Ar^1$-27 |

(continued)

| | | |
|---|---|---|
| 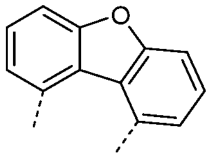 | 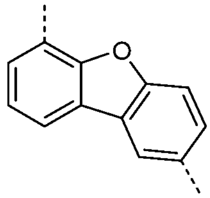 | 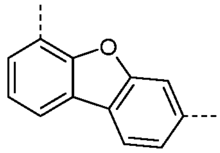 |
| $Ar^1$-28 | $Ar^1$-29 | $Ar^1$-30 |
| 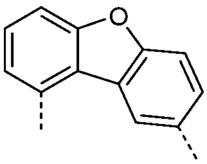 | 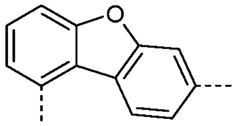 | 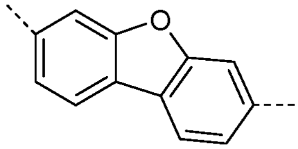 |
| $Ar^1$-31 | $Ar^1$-32 | $Ar^1$-33 |
| 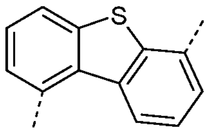 | 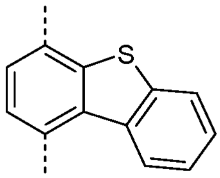 | 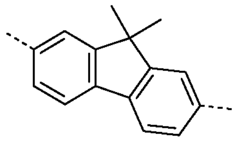 |
| $Ar^1$-34 | $Ar^1$-35 | $Ar^1$-36 |
| 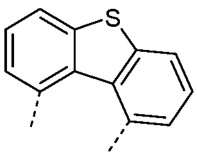 | 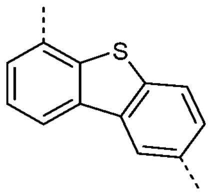 | 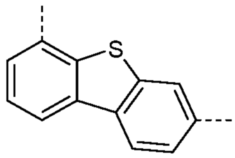 |
| $Ar^1$-37 | $Ar^1$-38 | $Ar^1$-39 |
| 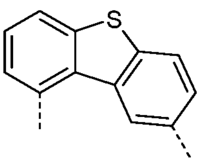 | 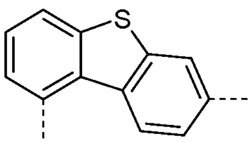 | 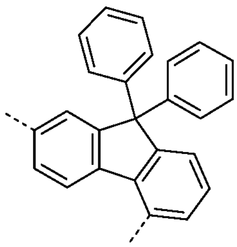 |
| $Ar^1$-40 | $Ar^1$-41 | $Ar^1$-42 |
| 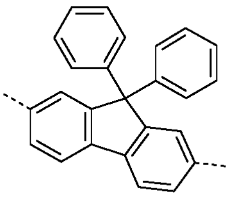 | 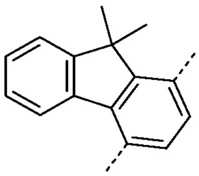 | 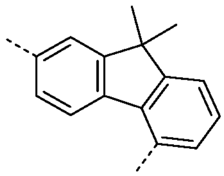 |
| $Ar^1$-43 | $Ar^1$-44 | $Ar^1$-45 |

(continued)

| | | |
|---|---|---|
| 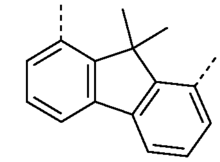 | 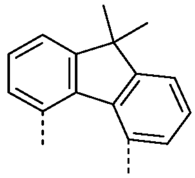 | 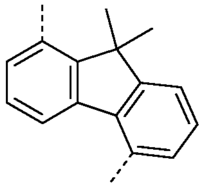 |
| Ar1-46 | Ar1-47 | Ar1-48 |
| 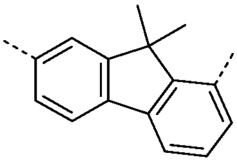 | 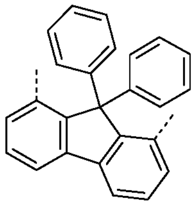 | 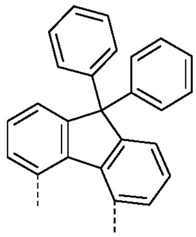 |
| Ar1-49 | Ar1-50 | Ar1-51 |
| 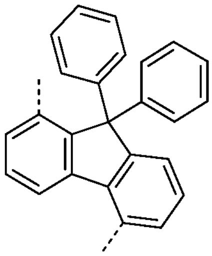 | 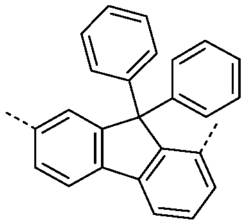 | 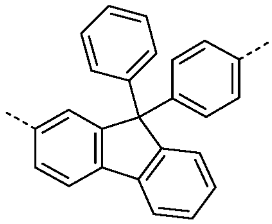 |
| Ar1-52 | Ar1-53 | Ar1-54 |
| 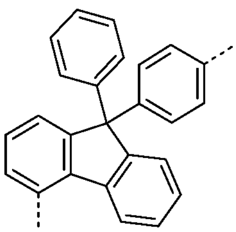 | 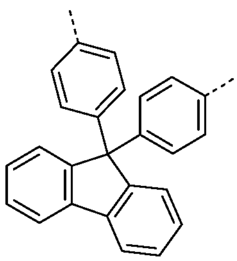 | 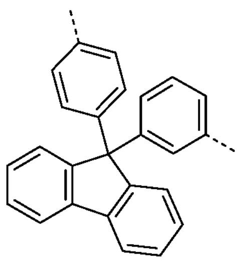 |
| Ar1-55 | Ar1-56 | Ar1-57 |
| 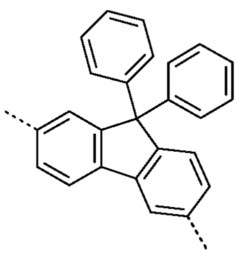 | 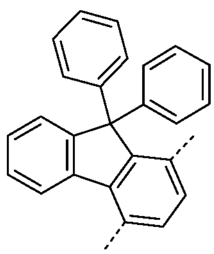 | 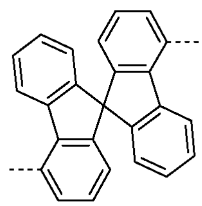 |
| Ar1-58 | Ar1-59 | Ar1-60 |

(continued)

| | | |
|---|---|---|
| 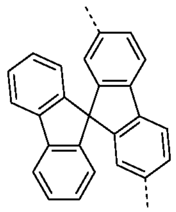 | 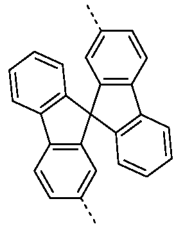 | 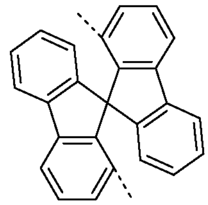 |
| $Ar^1$-61 | $Ar^1$-62 | $Ar^1$-63 |
| 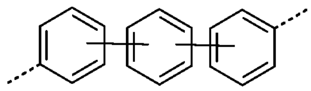 | | |
| $Ar^1$-64 | | |

where the formulae may in each case be substituted by one or more radicals $R^2$.

3. Compound according to Claim 1 or 2, **characterised in that** Y is selected on each occurrence, identically or differently, from O and S.

4. Compound according to one or more of Claims 1 to 3, **characterised in that** $Ar^2$ corresponds to a formula selected from the following formulae:

| | |
|---|---|
| 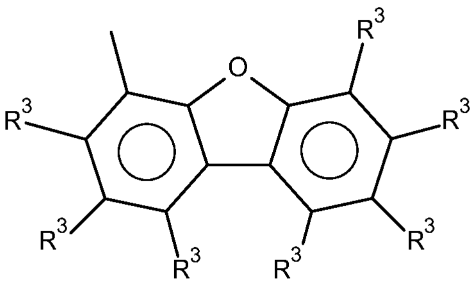 | 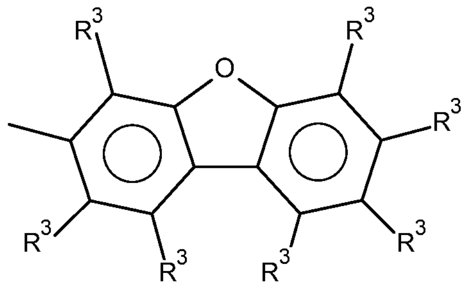 |
| formula ($Ar^2$-1-A-1) | formula ($Ar^2$-1-A-2) |
| 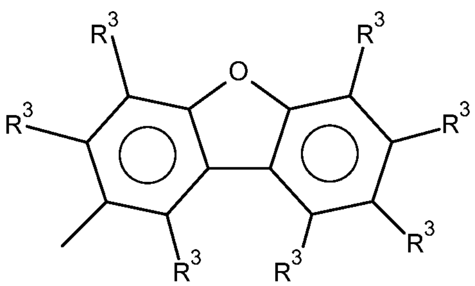 | 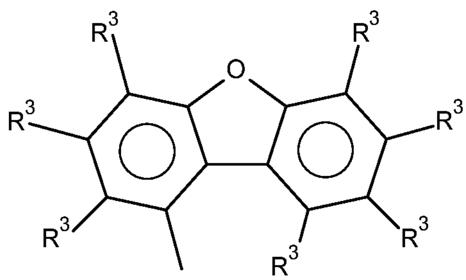 |
| formula ($Ar^2$-1-A-3) | formula ($Ar^2$-1-A-4) |

(continued)

| | |
|---|---|
| 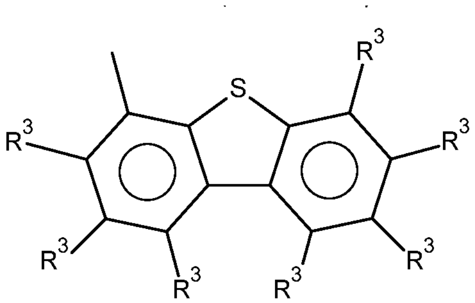 | 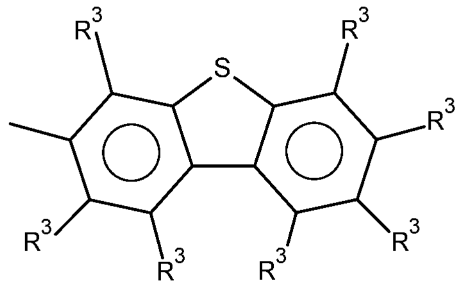 |
| formula ($Ar^2$-2-A-1) | formula ($Ar^2$-2-A-2) |
| 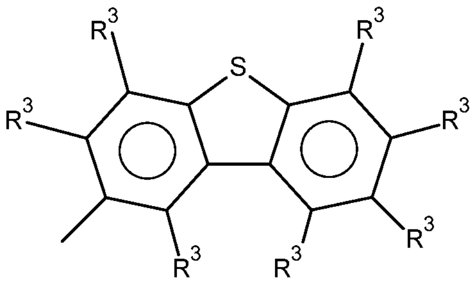 | 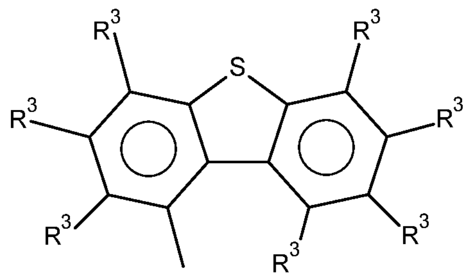 |
| formula ($Ar^2$-2-A-3) | formula ($Ar^2$-2-A-4) |

where the free bond denotes the bond to the remainder of the formula.

5. Compound according to one or more of Claims 1 to 4, **characterised in that** $R^1$ is selected on each occurrence, identically or differently, from H, D, F, CN, aromatic ring systems having 6 to 40 aromatic ring atoms, which may in each case be substituted by one or more radicals $R^4$, and heteroaromatic ring systems having 5 to 40 aromatic ring atoms, which may in each case be substituted by one or more radicals $R^4$.

6. Compound according to one or more of Claims 1 to 5, **characterised in that** $R^2$ is selected on each occurrence, identically or differently, from H, D, F, CN, aromatic ring systems having 6 to 40 aromatic ring atoms, which may in each case be substituted by one or more radicals $R^4$, and heteroaromatic ring systems having 5 to 40 aromatic ring atoms, which may in each case be substituted by one or more radicals $R^4$.

7. Compound according to one or more of Claims 1 to 6, **characterised in that** $R^3$ is selected on each occurrence, identically or differently, from H, D, F, CN, aromatic ring systems having 6 to 40 aromatic ring atoms, which may in each case be substituted by one or more radicals $R^4$, and electron-deficient heteroaryl groups having 6 to 40 aromatic ring atoms, which may in each case be substituted by one or more radicals $R^4$.

8. Compound according to one or more of Claims 1 to 7, **characterised in that** $R^4$ is selected on each occurrence, identically or differently, from H, D, F, CN, $Si(R^5)_3$, straight-chain alkyl or alkoxy groups having 1 to 20 C atoms, branched or cyclic alkyl or alkoxy groups having 3 to 20 C atoms, aromatic ring systems having 6 to 40 aromatic ring atoms, and heteroaromatic ring systems having 5 to 40 aromatic ring atoms; where the said alkyl and alkoxy groups, the said aromatic ring systems and the said heteroaromatic ring systems may in each case be substituted by one or more radicals $R^5$; and where one or more $CH_2$ groups in the said alkyl or alkoxy groups may be replaced by -C≡C-, $-R^5C=CR^5-$, $Si(R^5)_2$, C=O, $C=NR^5$, -O-, -S-, -C(=O)O- or $-C(=O)NR^5-$.

9. Compound according to one or more of Claims 1 to 8, **characterised in that** a = 1 and b = 1; or **characterised in that** a = 1 and b = 2, where the two groups $Ar^2$ are selected identically or differently.

10. Compound according to one or more of Claims 1 to 8, **characterised in that** c = 1, e = 1 and d = 1; or **characterised in that** c = 1, e = 1 and d = 2, where the two groups $Ar^2$ are selected identically or differently.

11. Compound according to one or more of Claims 1 to 10, **characterised in that** it corresponds to one of the formulae

(I-A-1), (I-A-2), (I-A-3), (II-A-1) or (II-A-2)

| | |
|---|---|
| 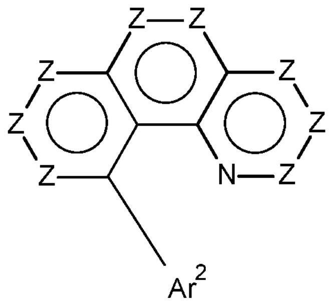 | 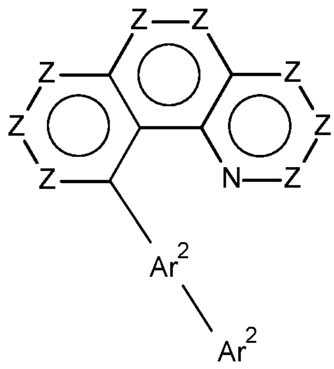 |
| formula (I-A-1) | formula (I-A-2) |
| 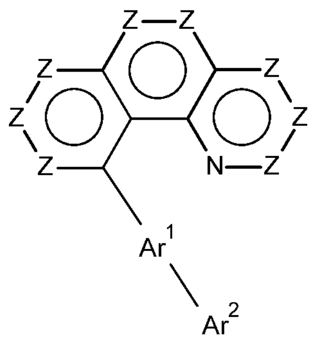 | |
| formula (I-A-3) | |
| 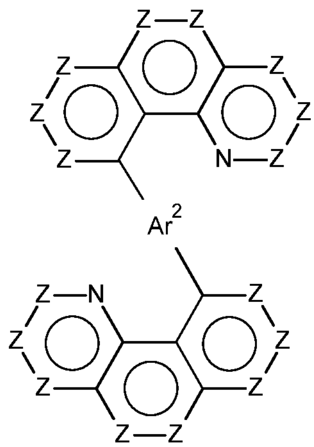 | 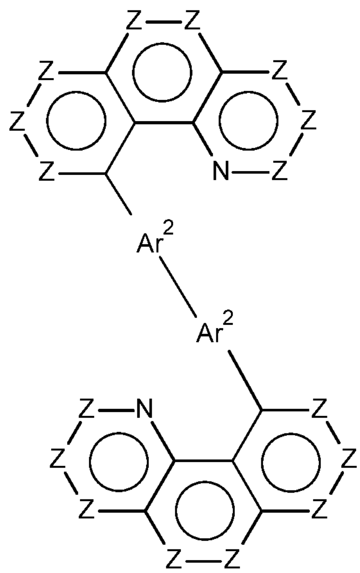 |
| formula (II-A-1) | formula (II-A-2) |

where the groups occurring are defined as in one or more of claims 1 to 10, with the exception that $Ar^1$ is not a single bond.

**12.** Compound according to one or more of Claims 1 to 11, **characterised in that** it corresponds to one of the formulae (I-A-1), (I-A-2) or (I-A-3)

| | |
|---|---|
| 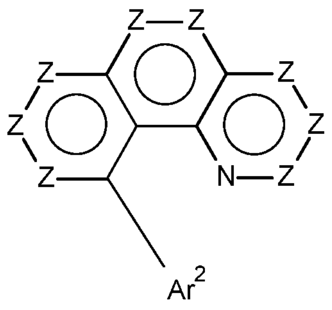 | 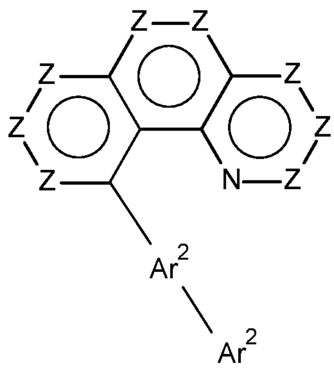 |
| formula (I-A-1) | formula (I-A-2) |
| 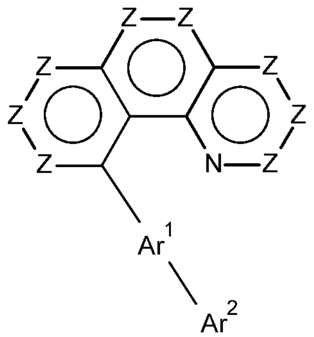 | |
| formula (I-A-3) | |

where Z is equal to $CR^3$, $Ar^1$ is defined as in Claim 2, $Ar^2$ is defined as in Claim 4, $R^1$ is defined as in Claim 5, $R^2$ is defined as in Claim 6, $R^3$ is defined as in Claim 7 and $R^4$ is defined as in Claim 8.

**13.** Process for the preparation of a compound according to one or more of Claims 1 to 12, **characterised in that** a compound of the formula (Z)

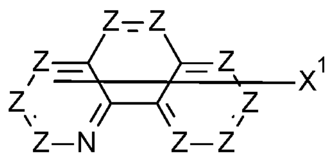

formula (Z)

where $X^1$ is selected from $B(OR^4)_2$, Cl, Br and I, is reacted in a Suzuki reaction.

**14.** Oligomer, polymer or dendrimer containing one or more compounds of the formula (I) or (II) according to one or more of Claims 1 to 12, where the bond(s) to the polymer, oligomer or dendrimer may be localised at any desired positions in formula (I) or (II) that are substituted by $R^1$, $R^2$ or $R^3$.

**15.** Formulation comprising at least one compound according to one or more of Claims 1 to 12 or a polymer, oligomer or dendrimer according to Claim 14, and at least one solvent.

**16.** Electronic device containing at least one compound according to one or more of Claims 1 to 12 or a polymer, oligomer or dendrimer according to Claim 14.

**17.** Electronic device according to Claim 16, selected from organic electroluminescent devices, **characterised in that**

at least one compound according to one or more of Claims 1 to 12 is present as matrix material in an emitting layer together with at least one phosphorescent emitting compound and at least one further matrix material.

**18.** Electronic device according to Claim 17, **characterised in that** the further matrix material is selected from bipolar matrix materials.

**19.** Electronic device according to Claim 17, **characterised in that** the further matrix material is selected from compounds of the formulae (BP-1) or (BP-2)

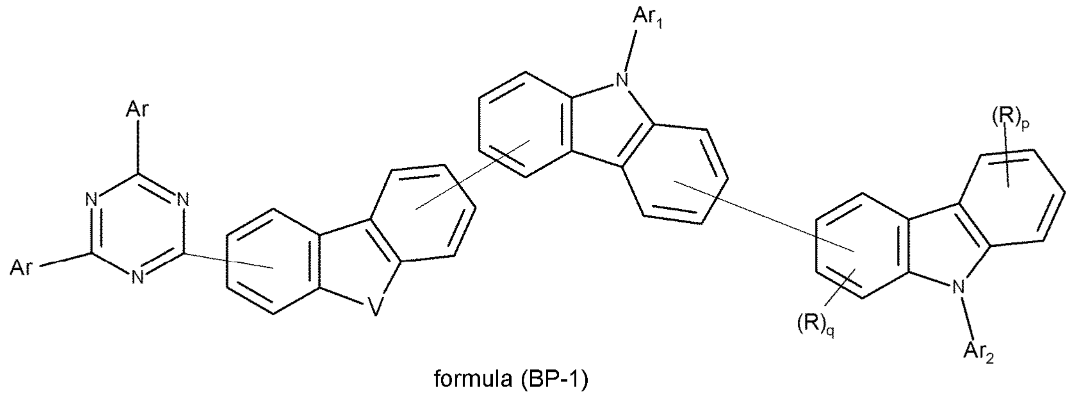

formula (BP-1)

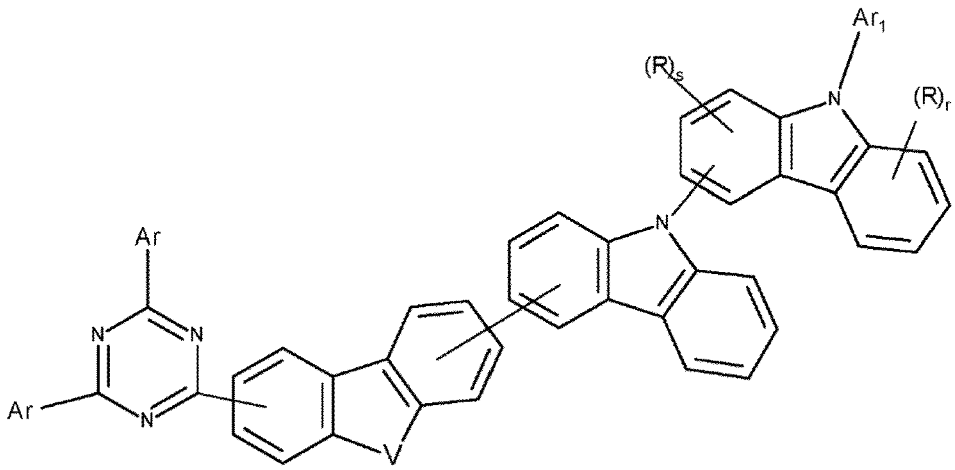

formula (BP-2)

where the following applies to the variables occurring:

V is O or S;
Ar, Ar1, $Ar_2$ are on each occurrence, in each case independently of one another, an aryl or heteroaryl group having 5 to 40 ring atoms, which may be substituted by one or more radicals $R_3$, or an aromatic or heteroaromatic ring system having 6 to 40 ring atoms, which may be substituted by one or more radicals $R_3$;
p, q are in each case, independently of one another, 0, 1, 2, 3 or 4;
s, r are in each case, independently of one another, 0, 1, 2, 3 or 4;
R is selected on each occurrence, identically or differently, from the group consisting of D, F, Cl, Br, I, CN, $NO_2$, $N(Ar)_2$, $N(R_2)_2$, C(=O)Ar, $C(=O)R_2$, $P(=O)(Ar)_2$, $P(Ar)_2$, $B(Ar)_2$, $Si(Ar)_3$, $Si(R_2)_3$, a straight-chain alkyl, alkoxy or thioalkyl group having 1 to 20 C atoms or a branched or cyclic alkyl, alkoxy or thioalkyl group having 3 to 20 C atoms or an alkenyl group having 2 to 20 C atoms, which may in each case be substituted by one or more radicals $R^2$, where one or more non-adjacent $CH_2$ groups may be replaced by $R_2C{=}CR_2$, $Si(R_2)_2$, C=O, C=S, $C{=}NR_2$, $P(=O)(R_2)$, SO, $SO_2$, $NR_2$, O, S or $CONR_2$ and where one or more H atoms may be replaced by D, F, Cl, Br, I, CN or $NO_2$, an aromatic or heteroaromatic ring system having 5 to 40 ring atoms, which may in each case be substituted by one or more radicals $R^2$, an aryloxy or heteroaryloxy group having 5 to 40 ring atoms, which may be substituted by one or more radicals $R^2$, or an aralkyl or heteroaralkyl group having 5 to 40 ring

atoms, which may be substituted by one or more radicals $R_2$; a maximum of one substituent R may together with $Ar_1$ form a monocyclic or polycyclic, aliphatic, aromatic or heteroaromatic ring system, which may be substituted by one or more radicals $R_2$;

$R_2$ is selected on each occurrence, identically or differently, from the group consisting of H, D, F, Cl, Br, I, CN, $NO_2$, $N(Ar)_2$, $NH_2$, $N(R_3)_2$, C(=O)Ar, C(=O)H, $C(=O)R_3$, $P(=O)(Ar)_2$, a straight-chain alkyl, alkoxy or thioalkyl group having 1 to 40 C atoms or a branched or cyclic alkyl, alkoxy or thioalkyl group having 3 to 40 C atoms or an alkenyl or alkynyl group having 2 to 40 C atoms, which may in each case be substituted by one or more radicals $R_3$, where one or more non-adjacent $CH_2$ groups may be replaced by HC=CH, $R_3C=CR_3$, C=C, $Si(R^3)_2$, $Ge(R_3)_2$, $Sn(R_3)_2$, C=O, C=S, C=Se, $C=NR_3$, $P(=O)(R_3)$, SO, $SO_2$, NH, $NR_3$, O, S, CONH or $CONR_3$ and where one or more H atoms may be replaced by D, F, Cl, Br, I, CN or $NO_2$, an aromatic or heteroaromatic ring system having 5 to 60 ring atoms, which may in each case be substituted by one or more radicals $R_3$, an aryloxy or heteroaryloxy group having 5 to 60 ring atoms, which may be substituted by one or more radicals $R_3$, or a combination of these systems, where two or more adjacent substituents $R_2$ may optionally form a monocyclic or polycyclic, aliphatic, aromatic or heteroaromatic ring system, which may be substituted by one or more radicals $R_3$;

$R_3$ is selected on each occurrence, identically or differently, from the group consisting of H, D, F, CN, an aliphatic hydrocarbon radical having 1 to 20 C atoms, or an aromatic or heteroaromatic ring system having 5 to 30 ring atoms, in which one or more H atoms may be replaced by D, F, Cl, Br, I or CN and which may be substituted by one or more alkyl groups having 1 to 4 carbon atoms in each case; two or more adjacent substituents $R_3$ may form a mono- or polycyclic, aliphatic ring system with one another.

**20.** Mixture comprising at least one compound according to one or more of Claims 1 to 12 and at least one further compound selected from bipolar matrix materials.

**21.** Use of a compound according to one or more of Claims 1 to 12 in an electronic device.

**Revendications**

**1.** Composé de formule (I-A), (I-B), (I-C), (I-D) ou (II-A)

| | |
|---|---|
| 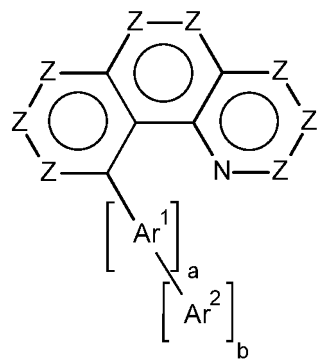 | 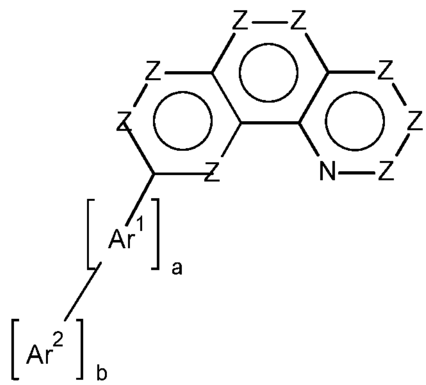 |
| formule (I-A) | formule (I-B) |
| 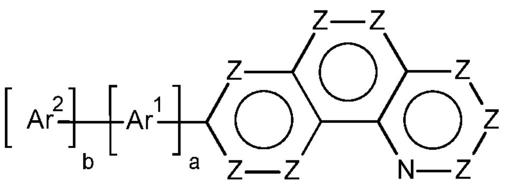 | 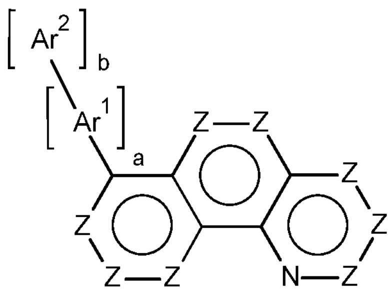 |
| formule (I-C) | formule (I-D) |

(suite)

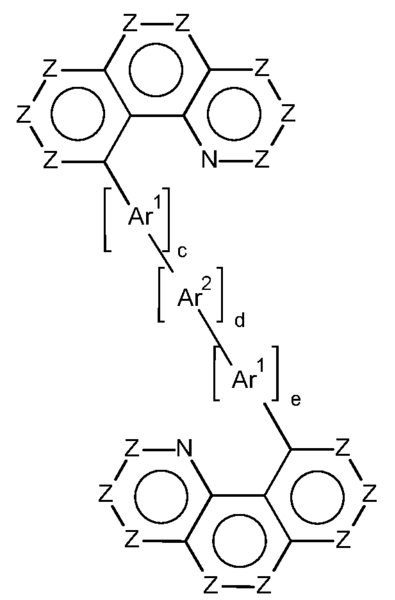

formule (II-A)

dans laquelle ce qui suit s'applique aux variables présentes :

Z, si aucun motif $Ar^1$ n'y est fixé, est choisi à chaque occurrence, de manière identique ou différente, parmi $CR^1$ ; et Z, si un motif $Ar^1$ y est fixé, est égal à C ;

$Ar^1$ est choisi à chaque occurrence, de manière identique ou différente, parmi une liaison simple, un noyau aromatique ayant de 6 à 40 atomes de cycle aromatique, qui peut être substitué par un ou plusieurs radicaux $R^2$, dibenzothiophène, qui peut être substitué par un ou plusieurs radicaux $R^2$, et dibenzofurane, qui peut être substitué par un ou plusieurs radicaux $R^1$ ;

$Ar^2$ est à chaque occurrence, de manière identique ou différente, un groupement de formule ($Ar^2$)

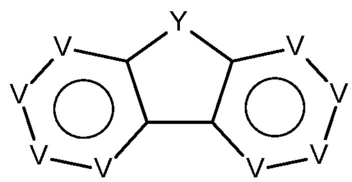

formule ($Ar^2$),

Y est choisi à chaque occurrence, de manière identique ou différente, parmi O, S, $C(R^3)_2$, $Si(R^3)_2$,

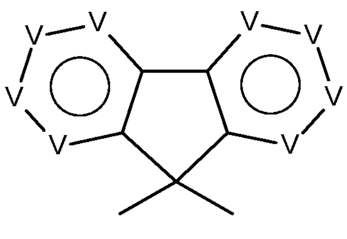

et

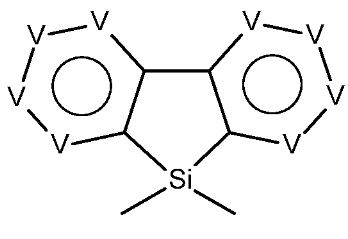

,

où, dans les formules

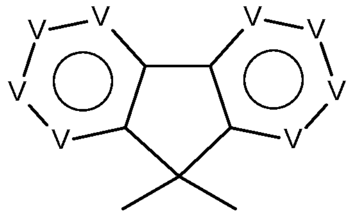

et

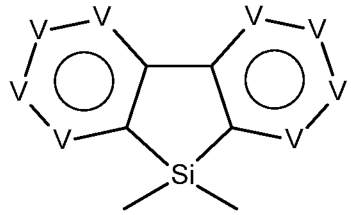

,

les liaisons libres sont les liaisons émanant du groupement Y au reste du groupement de formule ($Ar^2$) ;
V est à chaque occurrence, de manière identique ou différente, $CR^3$ ou N si la liaison au reste de la formule n'est pas située au niveau de la position en question, et V est égal à C si la liaison au reste de la formule est située au niveau de la position en question ;
où une ou plusieurs paires V-V peuvent être remplacées dans chaque cas par un motif choisi parmi les motifs suivants

| | | |
|---|---|---|
| 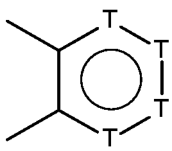 | 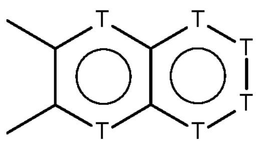 | 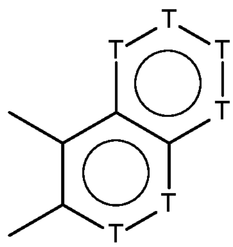 |
| (V-V-1) | (V-V-2) | (V-V-3) |

dans lesquels les liaisons libres désignent les liaisons au reste de la formule, et où T est à chaque occurrence, de manière identique ou différente, $CR^3$ ou N si la liaison au reste de la formule n'est pas située au niveau de la position en question, et où T est égal à C si la liaison au reste de la formule est située au niveau de la position en question ;
$R^1$, $R^2$ sont choisis à chaque occurrence, de manière identique ou différente, parmi H, D, F, $C(=O)R^4$, CN, $Si(R^4)_3$, $P(=O)(R^4)_2$, $OR^4$, $S(=O)R^4$, $S(=O)_2R^4$, des groupements alkyle ou alcoxy à chaîne linéaire ayant de 1 à 20 atomes de C, des groupements alkyle ou alcoxy ramifiés ou cycliques ayant de 3 à 20 atomes de C, des groupements alcényle ou alcynyle ayant de 2 à 20 atomes de C, des noyaux aromatiques ayant de 6 à 40 atomes de cycle aromatique, et des noyaux hétéroaromatiques ayant de 5 à 40 atomes de cycle aromatique ; où deux, ou plus, radicaux $R^2$ peuvent être liés les uns aux autres et peuvent former un cycle ; où lesdits groupements alkyle, alcoxy, alcényle et alcynyle et lesdits noyaux aromatiques et noyaux hétéroaromatiques peuvent dans chaque cas être substitués par un ou plusieurs radicaux $R^4$ ; et où un ou plusieurs groupements $CH_2$ dans lesdits groupements alkyle, alcoxy, alcényle et alcynyle peuvent être remplacés par $-R^4C=CR^4-$, $-C\equiv C-$, $Si(R^4)_2$, C=O, $C=NR^4$, -C(=O)O-, $-C(=O)NR^4-$, $P(=O)(R^4)$, -O-, -S-, SO ou $SO_2$ ;
$R^3$ est choisi à chaque occurrence, de manière identique ou différente, parmi H, D, F, $C(=O)R^4$, CN, $Si(R^4)_3$, $P(=O)(R^4)_2$, $OR^4$, $S(=O)R^4$, $S(=O)_2R^4$, des groupements alkyle ou alcoxy à chaîne linéaire ayant de 1 à 20 atomes de C, des groupements alkyle ou alcoxy ramifiés ou cycliques ayant de 3 à 20 atomes de C, des groupements alcényle ou alcynyle ayant de 2 à 20 atomes de C, des noyaux aromatiques ayant de 6 à 40 atomes de cycle aromatique, des noyaux hétéroaromatiques exempts de N ayant de 5 à 40 atomes de cycle aromatique, et des groupements hétéroaryles déficients en électrons ayant de 6 à 40 atomes de cycle aromatique ; où deux, ou plus, radicaux $R^3$ peuvent être liés les uns aux autres et peuvent former un cycle ; où lesdits groupements alkyle, alcoxy, alcényle et alcynyle et lesdits noyaux aromatiques et noyaux hétéroaromatiques exempts de N et groupements hétéroaryles déficients en électrons peuvent dans chaque cas être substitués par un ou plusieurs radicaux $R^4$ ; et où un ou plusieurs groupements $CH_2$ dans lesdits groupements alkyle, alcoxy, alcényle et alcynyle peuvent être remplacés par $-R^4C=CR^4-$, -C=C-, $Si(R^4)_2$, C=O, $C=NR^4$, -C(=O)O-, $-C(=O)NR^4-$, $P(=O)(R^4)$, -O-, -S-, SO ou $SO_2$ ;
$R^4$ est choisi à chaque occurrence, de manière identique ou différente, parmi H, D, F, $C(=O)R^5$, CN, $Si(R^5)_3$, $P(=O)(R^5)_2$, $OR^5$, $S(=O)R^5$, $S(=O)_2R^5$, des groupements alkyle ou alcoxy à chaîne linéaire ayant de 1 à 20 atomes de C, des groupements alkyle ou alcoxy ramifiés ou cycliques ayant de 3 à 20 atomes de C, des groupements alcényle ou alcynyle ayant de 2 à 20 atomes de C, des noyaux aromatiques ayant de 6 à 40

atomes de cycle aromatique, et des noyaux hétéroaromatiques ayant de 5 à 40 atomes de cycle aromatique ; où deux, ou plus, radicaux $R^4$ peuvent être liés les uns aux autres et peuvent former un cycle ; où lesdits groupements alkyle, alcoxy, alcényle et alcynyle et lesdits noyaux aromatiques et noyaux hétéroaromatiques peuvent dans chaque cas être substitués par un ou plusieurs radicaux $R^5$ ; et où un ou plusieurs groupements $CH_2$ dans lesdits groupements alkyle, alcoxy, alcényle et alcynyle peuvent être remplacés par $-R^5C=CR^5-$, -C=C-, $Si(R^5)_2$, C=O, $C=NR^5$, -C(=O)O-, $-C(=O)NR^5-$, $P(=O)(R^5)$, -O-, -S-, SO ou $SO_2$ ;

$R^5$ est choisi à chaque occurrence, de manière identique ou différente, parmi H, D, F, CN, des groupements alkyle ou alcoxy ayant de 1 à 20 atomes de C, des groupements alcényle ou alcynyle ayant de 2 à 20 atomes de C, des noyaux aromatiques ayant de 6 à 40 atomes de cycle aromatique et des noyaux hétéroaromatiques ayant de 5 à 40 atomes de cycle aromatique ; où lesdits groupements alkyle, alcoxy, alcényle et alcynyle, lesdits noyaux aromatiques et noyaux hétéroaromatiques peuvent être substitués par un ou plusieurs radicaux choisis parmi F et CN ; et

a, b, c, d, e sont choisis, de manière identique ou différente, parmi 1, 2, 3 et 4.

**2.** Composé selon la revendication 1, **caractérisé en ce que** $Ar^1$ est choisi à chaque occurrence, de manière identique ou différente, parmi une liaison simple et les formules :

| | | |
|---|---|---|
| 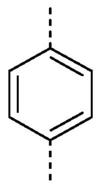 | 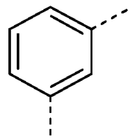 | 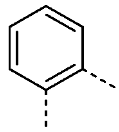 |
| $Ar^1$-1 | $Ar^1$-2 | $Ar^1$-3 |
| 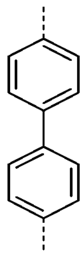 | 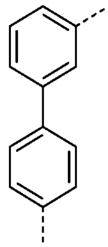 | 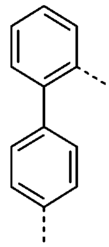 |
| $Ar^1$-4 | $Ar^1$-5 | $Ar^1$-6 |
| 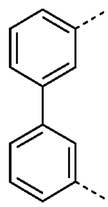 | 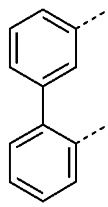 | 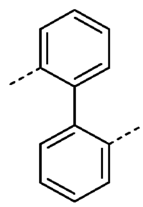 |
| $Ar^1$-7 | $Ar^1$-8 | Ar'-9 |
| 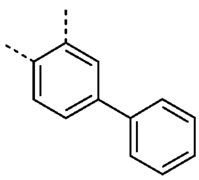 | 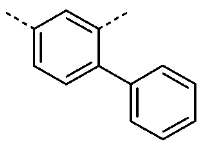 | 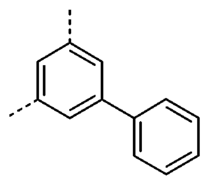 |
| $Ar^1$-10 | $Ar^1$-11 | $Ar^1$-12 |

(suite)

| | | |
|---|---|---|
| 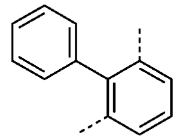 | 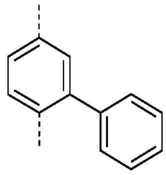 | 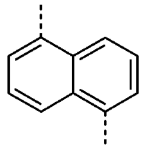 |
| Ar$^1$-13 | Ar$^1$-14 | Ar$^1$-15 |
| 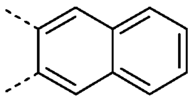 | 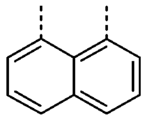 | 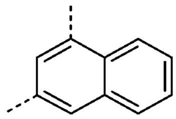 |
| Ar$^1$-16 | Ar$^1$-17 | Ar$^1$-18 |
| 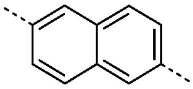 | 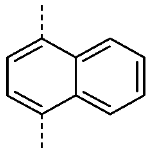 | 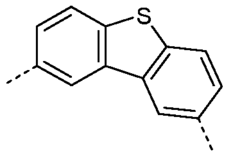 |
| Ar$^1$-19 | Ar$^1$-20 | Ar$^1$-21 |
| 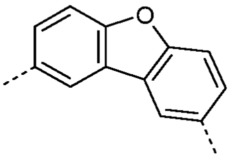 | 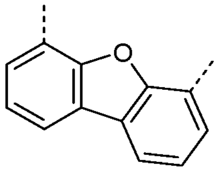 | 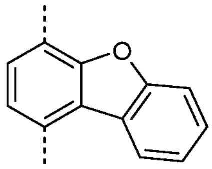 |
| Ar$^1$-22 | Ar$^1$-23 | Ar$^1$-24 |
| 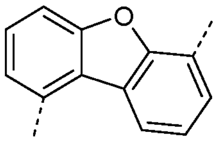 | 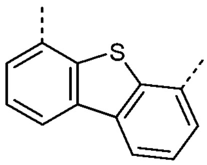 | 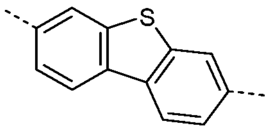 |
| Ar$^1$-25 | Ar$^1$-26 | Ar$^1$-27 |
| 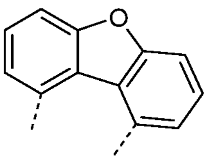 | 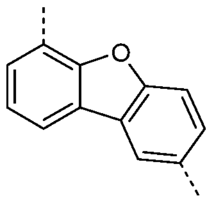 | 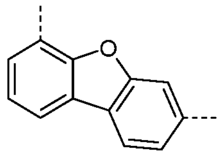 |
| Ar$^1$-28 | Ar$^1$-29 | Ar$^1$-30 |
| 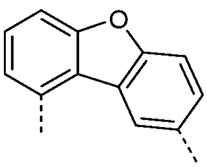 | 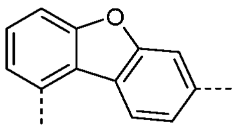 | 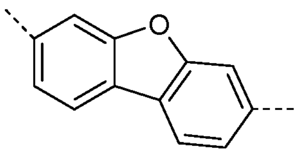 |
| Ar$^1$-31 | Ar$^1$-32 | Ar$^1$-33 |

(suite)

| | | |
|---|---|---|
| 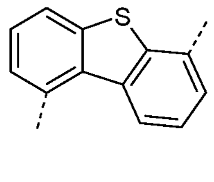 | 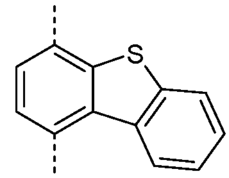 | 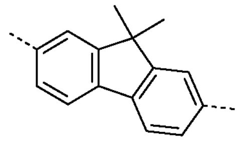 |
| $Ar^1$-34 | $Ar^1$-35 | $Ar^1$-36 |
| 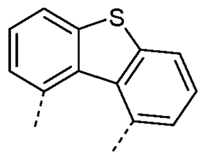 | 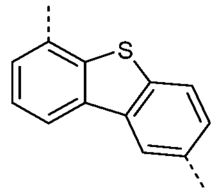 | 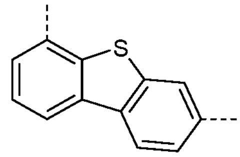 |
| $Ar^1$-37 | $Ar^1$-38 | $Ar^1$-39 |
| 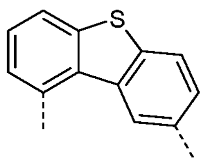 | 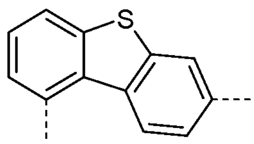 | 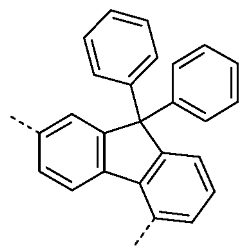 |
| $Ar^1$-40 | $Ar^1$-41 | $Ar^1$-42 |
| 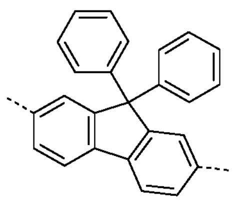 | 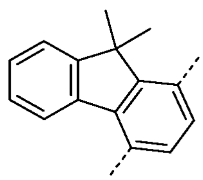 | 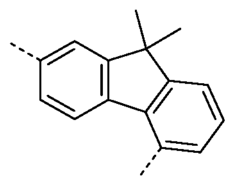 |
| $Ar^1$-43 | $Ar^1$-44 | $Ar^1$-45 |
| 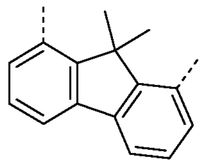 | 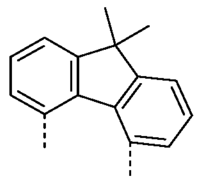 | 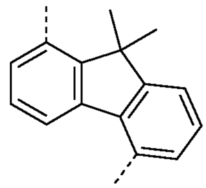 |
| $Ar^1$-46 | $Ar^1$-47 | $Ar^1$-48 |
| 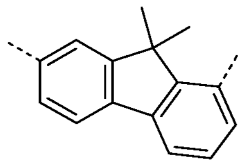 | 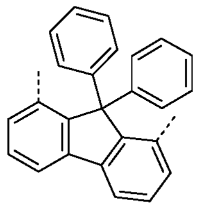 | 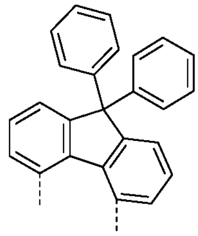 |
| $Ar^1$-49 | $Ar^1$-50 | $Ar^1$-51 |

(suite)

| | | |
|---|---|---|
| 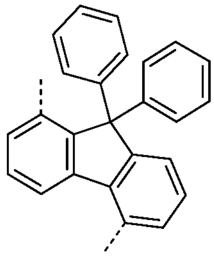 | 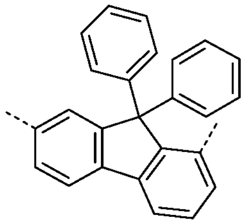 | 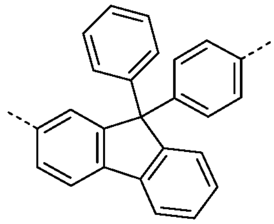 |
| $Ar^1$-52 | $Ar^1$-53 | $Ar^1$-54 |
| 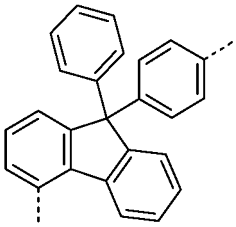 | 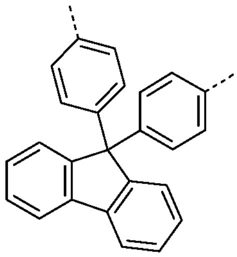 | 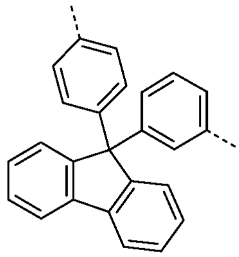 |
| $Ar^1$-55 | $Ar^1$-56 | $Ar^1$-57 |
| 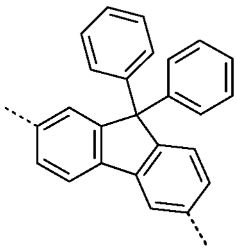 | 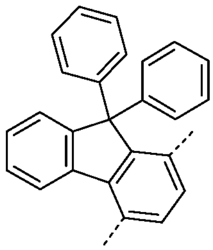 | 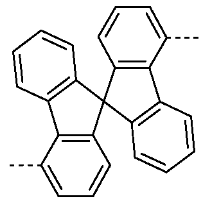 |
| $Ar^1$-58 | $Ar^1$-59 | $Ar^1$-60 |
| 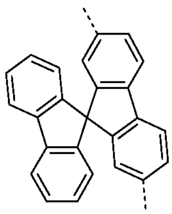 | 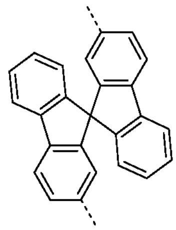 | 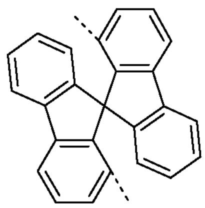 |
| $Ar^1$-61 | $Ar^1$-62 | $Ar^1$-63 |
| 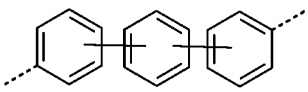 | | |
| $Ar^1$-64 | | |

les formules pouvant dans chaque cas être substituées par un ou plusieurs radicaux $R^2$.

3. Composé selon la revendication 1 ou 2, **caractérisé en ce que** Y est choisi à chaque occurrence, de manière identique ou différente, parmi O et S.

4. Composé selon l'une ou plusieurs parmi les revendications 1 à 3, **caractérisé en ce que** $Ar^2$ correspond à une formule choisie parmi les formules suivantes :

| | |
|---|---|
| 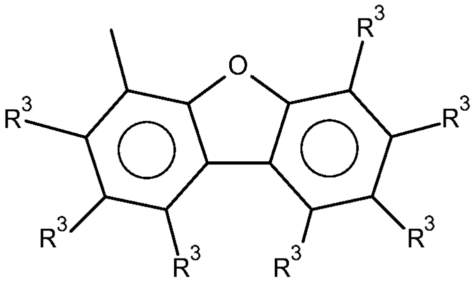 | 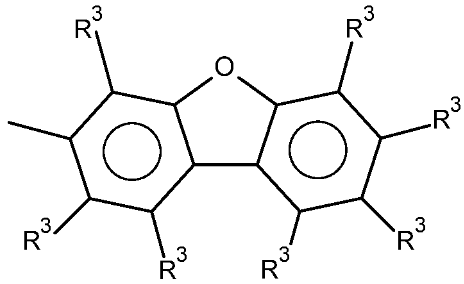 |
| formule ($Ar^2$-1-A-1) | formule ($Ar^2$-1-A-2) |
| 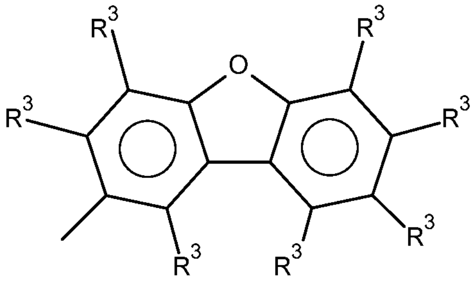 | 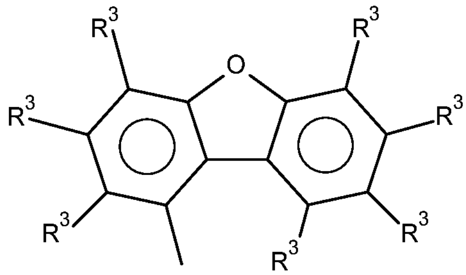 |
| formule ($Ar^2$-1-A-3) | formule ($Ar^2$-1-A-4) |
| 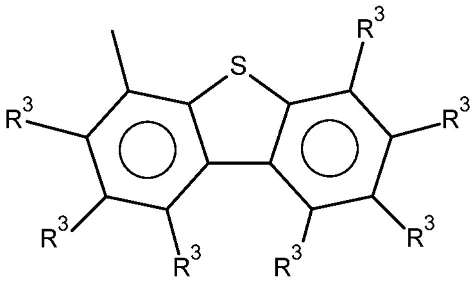 | 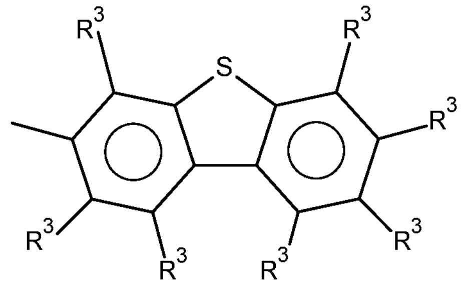 |
| formule ($Ar^2$-2-A-1) | formule ($Ar^2$-2-A-2) |
| 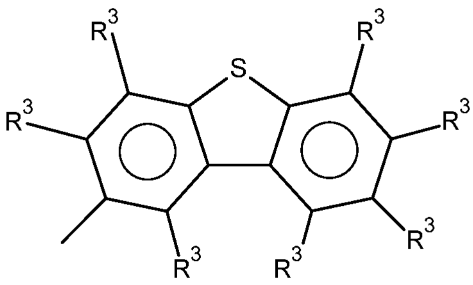 | 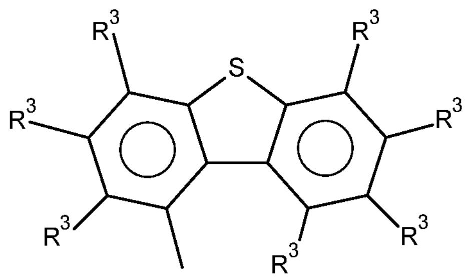 |
| formule ($Ar^2$-2-A-3) | formule ($Ar^2$-2-A-4) |

dans lesquelles la liaison libre désigne la liaison au reste de la formule.

**5.** Composé selon l'une ou plusieurs parmi les revendications 1 à 4, **caractérisé en ce que** $R^1$ est choisi à chaque occurrence, de manière identique ou différente, parmi H, D, F, CN, des noyaux aromatiques ayant de 6 à 40 atomes de cycle aromatique, qui peuvent dans chaque cas être substitués par un ou plusieurs radicaux $R^4$, et des noyaux hétéroaromatiques ayant de 5 à 40 atomes de cycle aromatique, qui peuvent dans chaque cas être substitués par un ou plusieurs radicaux $R^4$.

**6.** Composé selon l'une ou plusieurs parmi les revendications 1 à 5, **caractérisé en ce que** $R^2$ est choisi à chaque occurrence, de manière identique ou différente, parmi H, D, F, CN, des noyaux aromatiques ayant de 6 à 40 atomes

de cycle aromatique, qui peuvent dans chaque cas être substitués par un ou plusieurs radicaux $R^4$, et des noyaux hétéroaromatiques ayant de 5 à 40 atomes de cycle aromatique, qui peuvent dans chaque cas être substitués par un ou plusieurs radicaux $R^4$.

**7.** Composé selon l'une ou plusieurs parmi les revendications 1 à 6, **caractérisé en ce que** $R^3$ est choisi à chaque occurrence, de manière identique ou différente, parmi H, D, F, CN, des noyaux aromatiques ayant de 6 à 40 atomes de cycle aromatique, qui peuvent dans chaque cas être substitués par un ou plusieurs radicaux $R^4$, et des groupements hétéroaryles déficients en électrons ayant de 6 à 40 atomes de cycle aromatique, qui peuvent dans chaque cas être substitués par un ou plusieurs radicaux $R^4$.

**8.** Composé selon l'une ou plusieurs parmi les revendications 1 à 7, **caractérisé en ce que** $R^4$ est choisi à chaque occurrence, de manière identique ou différente, parmi H, D, F, CN, $Si(R^5)_3$, des groupements alkyle ou alcoxy à chaîne linéaire ayant de 1 à 20 atomes de C, des groupements alkyle ou alcoxy ramifiés ou cycliques ayant de 3 à 20 atomes de C, des noyaux aromatiques ayant de 6 à 40 atomes de cycle aromatique, et des noyaux hétéroaromatiques ayant de 5 à 40 atomes de cycle aromatique ; où lesdits groupements alkyle et alcoxy, lesdits noyaux aromatiques et lesdits noyaux hétéroaromatiques peuvent dans chaque cas être substitués par un ou plusieurs radicaux $R^5$ ; et où un ou plusieurs groupements $CH_2$ dans lesdits groupements alkyle ou alcoxy peuvent être remplacés par -C=C-, $-R^5C=CR^5-$, $Si(R^5)_2$, C=O, $C=NR^5$, -O-, -S-, -C(=O)O- ou $-C(=O)NR^5-$.

**9.** Composé selon l'une ou plusieurs parmi les revendications 1 à 8, **caractérisé en ce que** a = 1 et b = 1 ; ou **caractérisé en ce que** a = 1 et b = 2, où les deux groupements $Ar^2$ sont choisis de manière identique ou différente.

**10.** Composé selon l'une ou plusieurs parmi les revendications 1 à 8, **caractérisé en ce que** c = 1, e = 1 et d = 1 ; ou **caractérisé en ce que** c = 1, e = 1 et d = 2, où les deux groupements $Ar^2$ sont choisis de manière identique ou différente.

**11.** Composé selon l'une ou plusieurs parmi les revendications 1 à 10, **caractérisé en ce qu'**il correspond à l'une des formules (I-A-1), (I-A-2), (I-A-3), (II-A-1) ou (II-A-2)

| | |
|---|---|
| 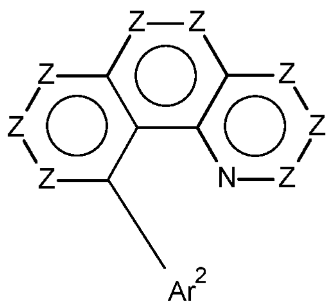 | 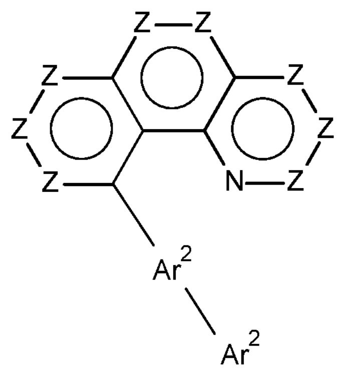 |
| formule (I-A-1) | formule (I-A-2) |
| 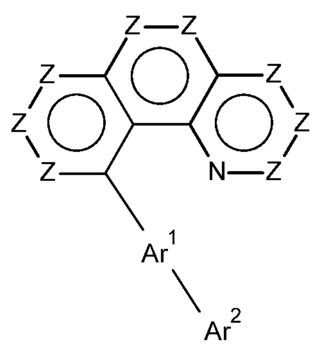 | |
| formule (I-A-3) | |

(suite)

| | |
|---|---|
| 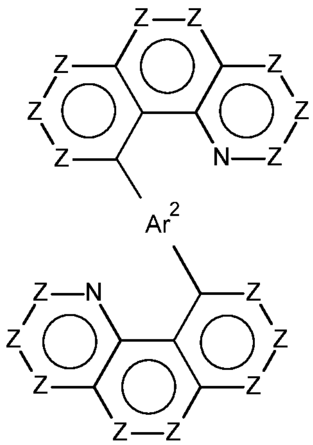 | 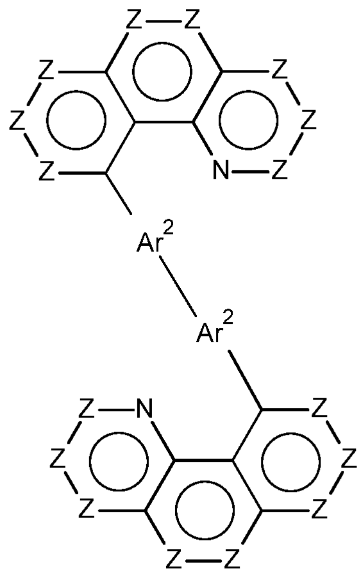 |
| formule (II-A-1) | formule (II-A-2) |

dans lesquelles les groupements présents sont tels que définis selon l'une ou plusieurs parmi les revendications 1 à 10, excepté que $Ar^1$ n'est pas une liaison simple.

**12.** Composé selon l'une ou plusieurs parmi les revendications 1 à 11, **caractérisé en ce qu'**il correspond à l'une des formules (I-A-1), (I-A-2) ou (I-A-3)

| | |
|---|---|
| 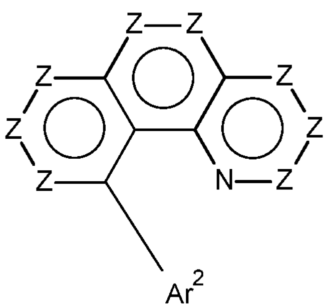 | 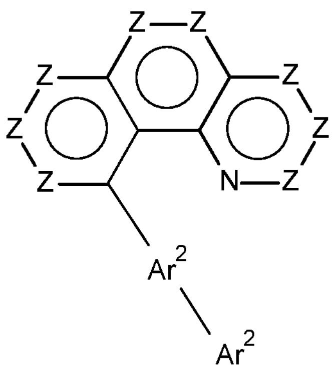 |
| formule (I-A-1) | formule (I-A-2) |
| 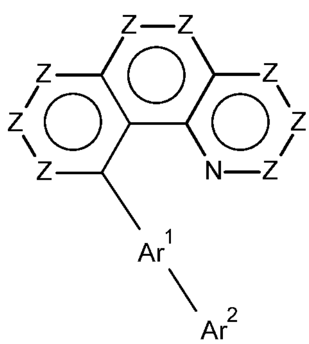 | |
| formule (I-A-3) | |

dans lesquelles Z est égal à $CR^3$, $Ar^1$ est tel que défini selon la revendication 2, $Ar^2$ est tel que défini selon la revendication 4, $R^1$ est tel que défini selon la revendication 5, $R^2$ est tel que défini selon la revendication 6, $R^3$ est tel que défini selon la revendication 7 et $R^4$ est tel que défini selon la revendication 8.

**13.** Procédé de préparation d'un composé selon l'une ou plusieurs parmi les revendications 1 à 12, **caractérisé en ce qu'**un composé de formule (Z)

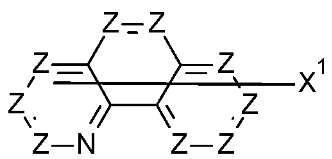

formule (Z)

dans laquelle $X^1$ est choisi parmi $B(OR^4)_2$, Cl, Br et I, est réagi dans une réaction de Suzuki.

**14.** Oligomère, polymère ou dendrimère contenant un ou plusieurs composés de formule (I) ou (II) selon l'une ou plusieurs parmi les revendications 1 à 12, où la ou les liaisons au polymère, à l'oligomère ou au dendrimère peuvent être situées au niveau de positions désirées quelconques dans la formule (I) ou (II) qui sont substituées par $R^1$, $R^2$ ou $R^3$.

**15.** Formulation comprenant au moins un composé selon l'une ou plusieurs parmi les revendications 1 à 12 ou un polymère, oligomère ou dendrimère selon la revendication 14, et au moins un solvant.

**16.** Dispositif électronique contenant au moins un composé selon l'une ou plusieurs parmi les revendications 1 à 12 ou un polymère, oligomère ou dendrimère selon la revendication 14.

**17.** Dispositif électronique selon la revendication 16, choisi parmi les dispositifs électroluminescents organiques, **caractérisé en ce qu'**au moins un composé selon l'une ou plusieurs parmi les revendications 1 à 12 est présent comme matériau de matrice dans une couche émettrice conjointement avec au moins un composé émetteur phosphorescent et au moins un matériau de matrice supplémentaire.

**18.** Dispositif électronique selon la revendication 17, **caractérisé en ce que** le matériau de matrice supplémentaire est choisi parmi les matériaux de matrice bipolaires.

**19.** Dispositif électronique selon la revendication 17, **caractérisé en ce que** le matériau de matrice supplémentaire est choisi parmi les composés de formules (BP-1) ou (BP-2)

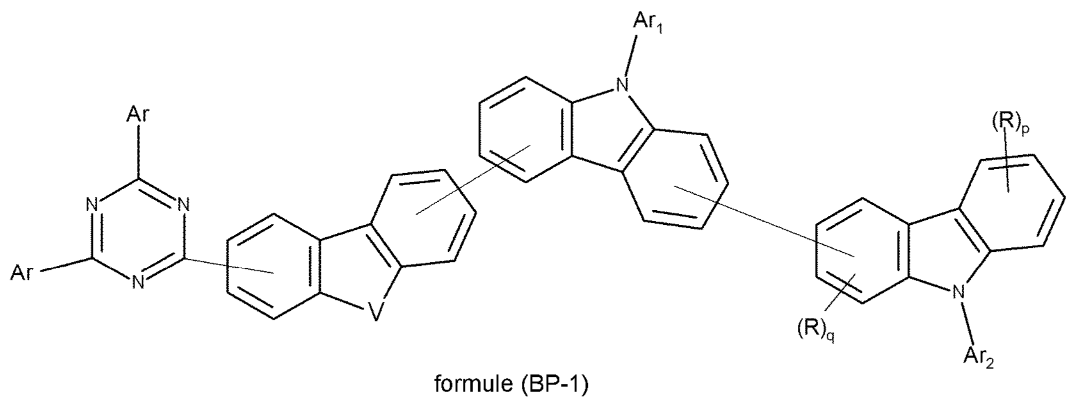

formule (BP-1)

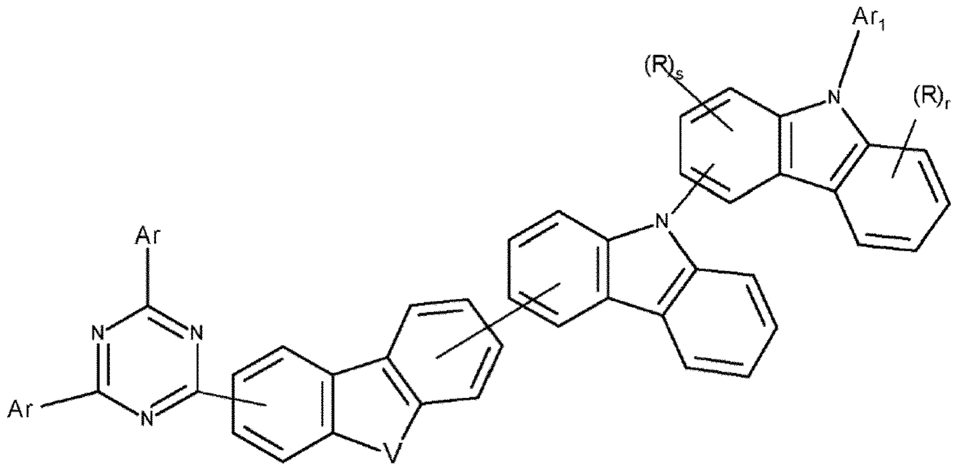

formule (BP-2)

dans lesquelles ce qui suit s'applique aux variables présentes :

V est O ou S ;

Ar, $Ar_1$, $Ar_2$ sont à chaque occurrence, dans chaque cas indépendamment les uns des autres, un groupement aryle ou hétéroaryle ayant de 5 à 40 atomes de cycle, qui peut être substitué par un ou plusieurs radicaux $R_3$, ou un noyau aromatique ou hétéroaromatique ayant de 6 à 40 atomes de cycle, qui peut être substitué par un ou plusieurs radicaux $R_3$ ;

p, q valent dans chaque cas, indépendamment l'un de l'autre, 0, 1, 2, 3 ou 4 ;

s, r valent dans chaque cas, indépendamment l'un de l'autre, 0, 1, 2, 3 ou 4 ;

R est choisi à chaque occurrence, de manière identique ou différente, dans le groupe constitué par D, F, Cl, Br, I, CN, $NO_2$, $N(Ar)_2$, $N(R_2)_2$, C(=O)Ar, C(=O)$R_2$, P(=O)$(Ar)_2$, $P(Ar)_2$, $B(Ar)_2$, $Si(Ar)_3$, $Si(R_2)_3$, un groupement alkyle, alcoxy ou thioalkyle à chaîne linéaire ayant de 1 à 20 atomes de C ou un groupement alkyle, alcoxy ou thioalkyle ramifié ou cyclique ayant de 3 à 20 atomes de C ou un groupement alcényle ayant de 2 à 20 atomes de C, qui peuvent dans chaque cas être substitués par un ou plusieurs radicaux $R^2$, où un ou plusieurs groupements $CH_2$ non adjacents peuvent être remplacés par $R_2C=CR_2$, $Si(R_2)_2$, C=O, C=S, $C=NR_2$, $P(=O)(R_2)$, SO, $SO_2$, $NR_2$, O, S ou $CONR_2$ et où un ou plusieurs atomes de H peuvent être remplacés par D, F, Cl, Br, I, CN ou $NO_2$, un noyau aromatique ou hétéroaromatique ayant de 5 à 40 atomes de cycle, qui peut dans chaque cas être substitué par un ou plusieurs radicaux $R^2$, un groupement aryloxy ou hétéroaryloxy ayant de 5 à 40 atomes de cycle, qui peut être substitué par un ou plusieurs radicaux $R^2$, ou un groupement aralkyle ou hétéroaralkyle ayant de 5 à 40 atomes de cycle, qui peut être substitué par un ou plusieurs radicaux $R_2$ ; un maximum d'un seul substituant R peut, conjointement avec $Ar_1$, former un noyau monocyclique ou polycyclique, aliphatique, aromatique ou hétéroaromatique, qui peut être substitué par un ou plusieurs radicaux $R^2$ ;

$R^2$ est choisi à chaque occurrence, de manière identique ou différente, dans le groupe constitué par H, D, F, Cl, Br, I, CN, $NO_2$, $N(Ar)_2$, $NH_2$, $N(R_3)_2$, C(=O)Ar, C(=O)H, C(=O)$R_3$, P(=O)$(Ar)_2$, un groupement alkyle, alcoxy ou thioalkyle à chaîne linéaire ayant de 1 à 40 atomes de C ou un groupement alkyle, alcoxy ou thioalkyle ramifié ou cyclique ayant de 3 à 40 atomes de C ou un groupement alcényle ou alcynyle ayant de 2 à 40 atomes de C, qui peuvent dans chaque cas être substitués par un ou plusieurs radicaux $R_3$, où un ou plusieurs groupements $CH_2$ non adjacents peuvent être remplacés par HC=CH, $R_3C=CR_3$, C=C, $Si(R_3)_2$, $Ge(R_3)_2$, $Sn(R_3)_2$, C=O, C=S, C=Se, $C=NR_3$, $P(=O)(R_3)$, SO, $SO_2$, NH, $NR_3$, O, S, CONH ou $CONR_3$ et où un ou plusieurs atomes de H peuvent être remplacés par D, F, Cl, Br, I, CN ou $NO_2$, un noyau aromatique ou hétéroaromatique ayant de 5 à 60 atomes de cycle, qui peut dans chaque cas être substitué par un ou plusieurs radicaux $R_3$, un groupement aryloxy ou hétéroaryloxy ayant de 5 à 60 atomes de cycle, qui peut être substitué par un ou plusieurs radicaux $R_3$, ou une combinaison de ces systèmes, où deux, ou plus, substituants $R_2$ adjacents peuvent éventuellement former un noyau monocyclique ou polycyclique, aliphatique, aromatique ou hétéroaromatique, qui peut être substitué par un ou plusieurs radicaux $R_3$ ;

$R_3$ est choisi à chaque occurrence, de manière identique ou différente, dans le groupe constitué par H, D, F, CN, un radical hydrocarboné aliphatique ayant de 1 à 20 atomes de C, ou un noyau aromatique ou hétéroaromatique ayant de 5 à 30 atomes de cycle, où un ou plusieurs atomes de H peuvent être remplacés par D, F, Cl, Br, I ou CN et qui peut être substitué par un ou plusieurs groupements alkyle ayant de 1 à 4 atomes de carbone dans chaque cas ; deux, ou plus, substituants $R^3$ adjacents peuvent former un noyau monoou polycyclique, aliphatique les uns avec les autres.

**20.** Mélange comprenant au moins un composé selon l'une ou plusieurs parmi les revendications 1 à 12 et au moins un composé supplémentaire choisi parmi les matériaux de matrice bipolaires.

**21.** Utilisation d'un composé selon l'une ou plusieurs parmi les revendications 1 à 12, dans un dispositif électronique.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 2014077191 A1
- WO 2006128800 A1
- US 2015060824 A1
- EP 2983227 A1
- US 2013009543 A1
- CN 106893581 A
- WO 2002072714 A
- WO 2003019694 A
- EP 17201480


### In der Beschreibung aufgeführte Nicht-Patentliteratur


- **Y. SHIROTA et al.** *Chem. Rev.,* 2007, vol. 107 (4), 953-1010
- **M. S. ARNOLD et al.** *Appl. Phys. Lett.,* 2008, vol. 92, 053301